# FASCICULE DE BREVET EUROPEEN

(11) **EP 3 237 403 B1**
(45) Date de publication et mention de la délivrance du brevet: **06.05.2020**
(21) Numéro de dépôt: 15826043.0
(22) Date de dépôt: 22.12.2015
(51) Int. Cl.: C07D 401/14, C07D 413/14, A61K 31/513, A61K 31/517, A61K 31/519, A61K 31/537, A61K 31/5513, C07D 401/06, C07D 403/06, C07D 407/14, C07D 409/14, C07D 471/04, C07D 498/20

(54) **NOUVEAUX COMPOSÉS HÉTÉROCYCLIQUES ET LEUR UTILISATION EN MÉDECINE AINSI QU'EN COSMÉTIQUE**
NEUARTIGE HETEROCYCLISCHE VERBINDUNGEN UND VERWENDUNG DAVON IN DER MEDIZIN UND IN DER KOSMETIK
NOVEL HETEROCYCLIC COMPOUNDS AND THE USE THEREOF IN MEDICINE AND IN COSMETICS

(30) Priorité: 23.12.2014 FR 1463212
(43) Date de publication de la demande: 01.11.2017
(73) Titulaire: Galderma Research & Development, 06410 Biot (FR)
(72) Inventeur: FOURNIER, Jean-François, 06160 Juan les Pins (FR); CLARY, Laurence, 06480 La Colle sur Loup (FR); THOREAU, Etienne, 06460 Saint Vallier de Thiey (FR)
(74) Mandataire: Nederlandsch Octrooibureau
(86) Numéro de dépôt international: PCT/FR2015/053709
(87) Numéro de publication internationale: WO 2016/102882

(56) Documents cités:
- WO-A1-2004/083187
- WO-A1-2008/130512
- WO-A1-2010/139717
- WO-A2-2006/029153
- WO-A2-2008/112159

## Description

L'invention concerne de nouveaux composés hétérocycliques de formule générale (I) :

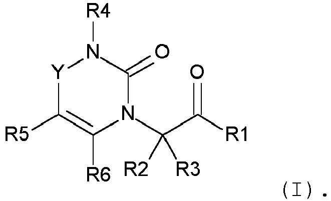

Elle concerne également leur utilisation comme médicament, notamment dans la prévention et/ou le traitement de maladies inflammatoires avec une composante neurogène ou comme cosmétique. Les composés de la présente invention agissent comme antagonistes du récepteur CGRP-R.

Le peptide relié au gène calcitonine (CGRP ou calcitonin gene-related peptide) est un neuropeptide de 37 acides aminés comportant un groupe amide en C-terminal et une structure cyclique formée avec un pont disulfure Cys-Cys en N-terminal (Poyner et al., « The mammalian calcitonin gene-related peptides, adrenomedullin, amylin, and calcitonin receptors », 2002) . CGRP est largement exprimé dans le système nerveux central et périphérique et est impliqué dans un certain nombre de fonctions biologiques (Edvinsson et al., « CGRP antagonism and migraine », 2010). Il y a de plus en plus de preuves que le CGRP joue un rôle important comme neuromodulateur (Ho et al., « CGRP and its receptors provide new insights into migraine pathophysiology », 2010), mais il est surtout connu comme un puissant vasodilatateur (Brain et al., « Calcitonin gene-related peptide is a potent vasodilatator », 1985).

CGRP signale principalement via un récepteur (CGRP-R) qui est un hétérodimère formé par l'association d'un récepteur couplé à une protéine G (RCPG) de classe B (récepteur de la calcitonine (CLR ou calcitonin-like receptor)) avec une protéine transmembranaire modifiant l'activité des récepteurs (RAMP1 ou receptor activity-modifying protein 1) (McLatchie et al., « RAMPs regulate the transport and ligand specificity of the calcitonin receptor-like receptor », 1998).

Le rôle de CGRP et l'activité du récepteur CGRP ont fait l'objet de nombreuses études.

On peut plus particulièrement citer à titre indicatif des études qui ont suggéré que la crise de migraine est associée à une libération de CGRP par les nocicepteurs méningés du ganglion trigéminé et que le CGRP puisse donc jouer un rôle actif dans la migraine (Edvinsson et al., « Functional role of perivascular peptides in the control of cerebral circulation », 1985 ; Goadsby et al., « Vasoactive peptide release in the extracerebral circulation of humans during migraine headache », 1990 ; Lassen et al., « CGRP may play a causative role in migraine », 2002).

Le nombre croissant de preuves reliant CGRP à la migraine a conduit à un intérêt dans le développement d'agents capables de bloquer les effets de ce neuropeptide. Des efforts ont notamment porté sur le développement de petites molécules antagonistes directs du récepteur CGRP (les gépants) et non vasoconstricteurs pour le traitement de la migraine.

Ces molécules sont décrites dans la littérature pour une utilisation par voie orale et présentent ainsi une stabilité métabolique au niveau hépatique (olcegepant, telcagepant et MK-3207) .

Toutefois, leur développement s'est heurté à des problèmes de toxicité hépatique.

Par ailleurs, outre son rôle dans la migraine, la libération de CGRP par le nerf trigéminal innervant la face pourrait être impliquée dans l'inflammation neurogène responsable notamment de l'érythème permanent dans la rosacée de type 1. Cette hypothèse est soutenue par des caractéristiques communes entre la rosacée et la migraine, qui comporte une composante neurogène avec des phénomènes vasoactifs, une activation du système nerveux trigéminé et la libération de peptides neuro-inflammatoires (CGRP, substance P...) . Le CGRP exercerait son action vasodilatatrice en agissant principalement sur les cellules musculaires lisses et endothéliales des vaisseaux sous-cutanées.

La rosacée est une dermatose inflammatoire commune chronique et progressive liée à une relaxation vasculaire. Elle affecte principalement la partie centrale du visage et se caractérise par un rougissement du visage ou des bouffées de chaleur, un érythème facial, des papules, des pustules, une télangiectasie et parfois des lésions oculaires appelées rosacée oculaire.

Par ailleurs, il s'associe à ces caractéristiques primaires une composante neurogène secondaire, c'est-à-dire une hyperréactivité cutanée de la peau du visage et du cou, caractérisée par l'apparition de rougeurs et de sensations subjectives du type démangeaisons ou prurit, de sensations de brûlures ou d'échauffement, de sensations de picotements, de fourmillements, d'inconforts, de tiraillements, etc.

La rosacée est ainsi classiquement traitée par voie orale ou par voie topique par des actifs tels que les antiséborrhéiques et les antiinfectieux, par exemple le peroxyde de benzoyle, l'acide rétinoïque, le métronidazole ou les cyclines.

Toutefois, ces traitements qui agissent sur l'infection et l'hyperséborrhée ne permettent pas de traiter et/ou prévenir efficacement l'ensemble des symptômes associés à la rosacée, en particulier de traiter la composante neurogène de cette affection, et notamment l'hyperréactivité de la peau et la rougeur.

Aussi, on connait plus particulièrement le brevet EP0734729 qui divulgue l'utilisation d'un antagoniste de CGRP pour traiter les rougeurs cutanées d'origine neurogène, et notamment la rosacée et/ou l'érythème pudique.

Toutefois, le seul antagoniste décrit est le CGRP 8-37, un peptide anti-CGRP. Il doit diminuer la vasodilatation induite par la capsaïcine et/ou provoquer une inhibition de la libération de CGRP par les fibres nerveuses sensitives et/ou provoquer une inhibition de la contraction du muscle lisse du canal déférent induite par le CGRP.

WO 2006/029153 décrit des composés inhibiteurs des récepteurs CGRP comprenant comme noyau initial la 2-oxo-hexahydropyrimidine, le 2-oxo-tétrahydroimidazole, ou le 2-oxo-dihydroimidazole.

WO 2004/083147 décrit des antagonistes des récepteurs CGRP de type anilide monocyclique de spirohydantoine.

WO 2010/139717 décrit des pipérazines substituées en tant quántagoniste des récepteurs CGRP.

WO 2008/130512 décrit des antagonistes des récepteurs CGRP hétérocycliques d'anilide bicyclique.

WO 2008/112159 décrit des antagonistes des récepteurs CGRP monocyclique dánilide spirolactame.

Considérant ce qui précède, il existe donc un besoin de disposer d'actifs efficaces dans le traitement de maladies inflammatoires avec une composante neurogène telle que la la rosacée, qui soient utilisables pendant de longues périodes, avec des effets secondaires aussi limités que possible.

Un problème que se propose de résoudre la présente invention est de proposer un traitement permettant de réduire ou abolir totalement cette inflammation, en utilisant un antagoniste non peptidique du récepteur au CGRP afin de cibler l'inflammation neurogène, avec pour résultat un effet persistant à l'arrêt du traitement, tout en limitant les effets secondaires pour le patient, en particulier les risques de toxicité hépatique.

Le Demandeur a ainsi identifié de nouveaux composés hétérocycliques antagonistes du récepteur CGRP, sélectifs du récepteur humain, qui présentent un très bon potentiel sur le plan de l'activité biologique. De manière inattendue, ces nouveaux composés présentent une instabilité métabolique au niveau hépatique, ce qui devrait limiter le risque d'hépatotoxicité rencontré avec de nombreux antagonistes CGRP connus de l'art antérieur comme par exemple l'olcegepant, le telcagepant et MK-3207.

L'objet de la présente invention est tel que défini par les revendications 1-10. Les mises en œuvre qui ne sont pas couvertes par l'etendue des revendications sont fournies à titre illustratif.

L'invention a pour objet un composé hétérocyclique choisi parmi les composés de formule générale (I) : 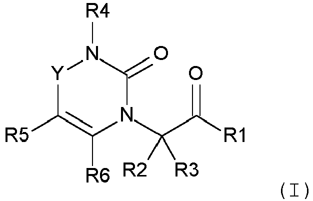 dans laquelle R1, R2, R3, R4, R5, R6 et Y sont tels que définis dans la description détaillée ci-dessous, ainsi que les sels pharmaceutiquement acceptables des composés de formule générale (I) et les énantiomères des composés de formule générale (I).

L'invention a également pour objet une composition pharmaceutique comprenant au moins un composé de formule générale (I) et un véhicule pharmaceutiquement acceptable, de préférence adapté pour une administration par voie topique.

Elle a encore pour objet l'utilisation cosmétique d'un composé de formule générale (I).

L'invention a aussi pour objet un composé de formule générale (I) pour son utilisation comme médicament.

Elle a en particulier pour objet un composé de formule générale (I) pour son utilisation dans la prévention et/ou le traitement de maladies inflammatoires avec une composante neurogène.

Un aspect de la divulgation porte sur l'utilisation d'un composé de formule générale (I) pour préparer un médicament destiné à la prévention et/ou au traitement de maladies inflammatoires avec une composante neurogène.

Un autre aspect de la divulgation porte sur_un procédé pour prévenir et/ou traiter les maladies inflammatoires avec une composante neurogène, comprenant l'administration d'au moins un composé de formule générale (I).

L'invention et les avantages qui en découlent seront mieux compris à la lecture de la description et des modes de réalisation non limitatifs qui suivent, rédigés au regard des figures annexées, dans lesquelles :

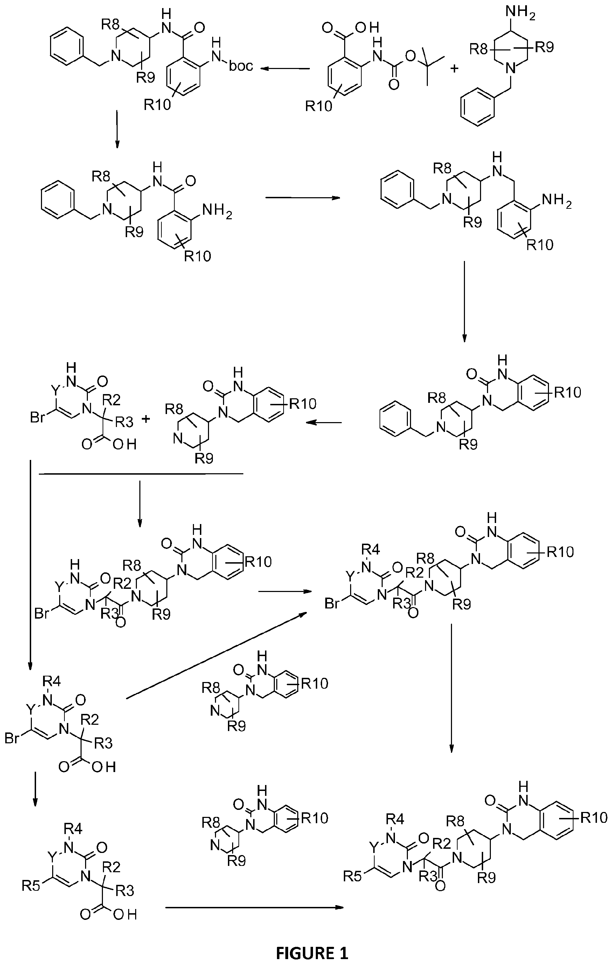

- la figure 1 représente une voie de synthèse générale dans des conditions classiques de composés selon le schéma réactionnel n°1 ;

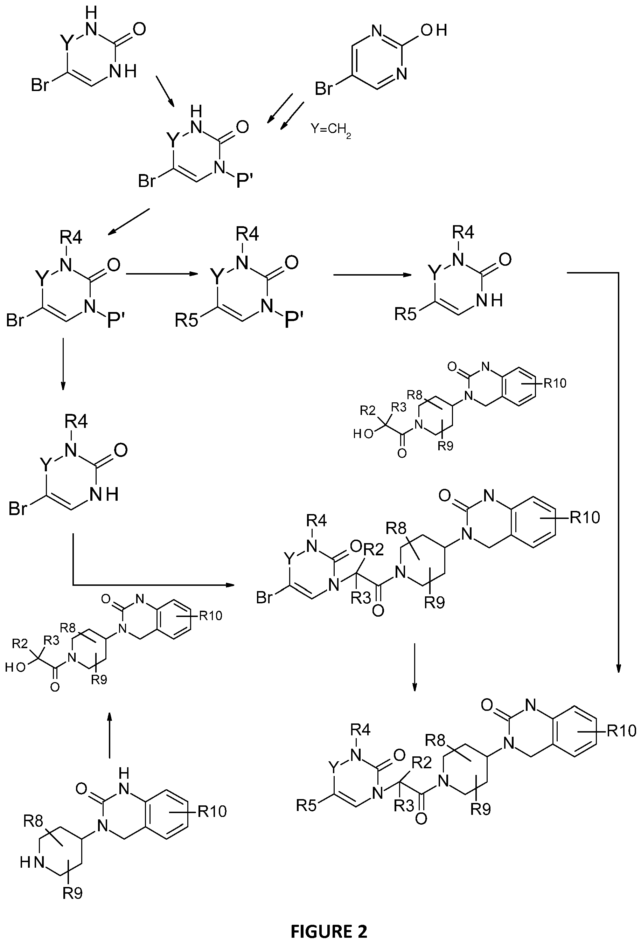

- la figure 2 représente une voie de synthèse générale dans des conditions classiques de composés selon le schéma réactionnel n°2, dans lequel P' désigne un groupement protecteur de type benzyle, 4-méthoxy-benzyle, benzhydrile, -COOtBu ou autre ;

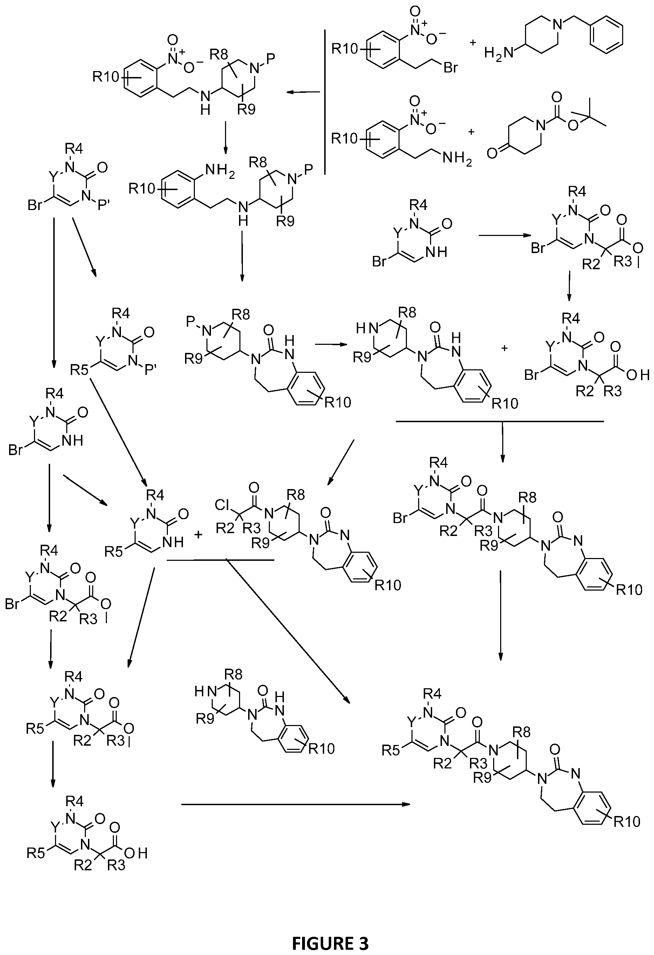

- la figure 3 représente une voie de synthèse générale dans des conditions classiques de composés selon le schéma réactionnel n°3, dans lequel P' désigne un groupement protecteur de type benzyle, 4-méthoxy-benzyle, benzhydrile, -COOtBu ou autre ; et

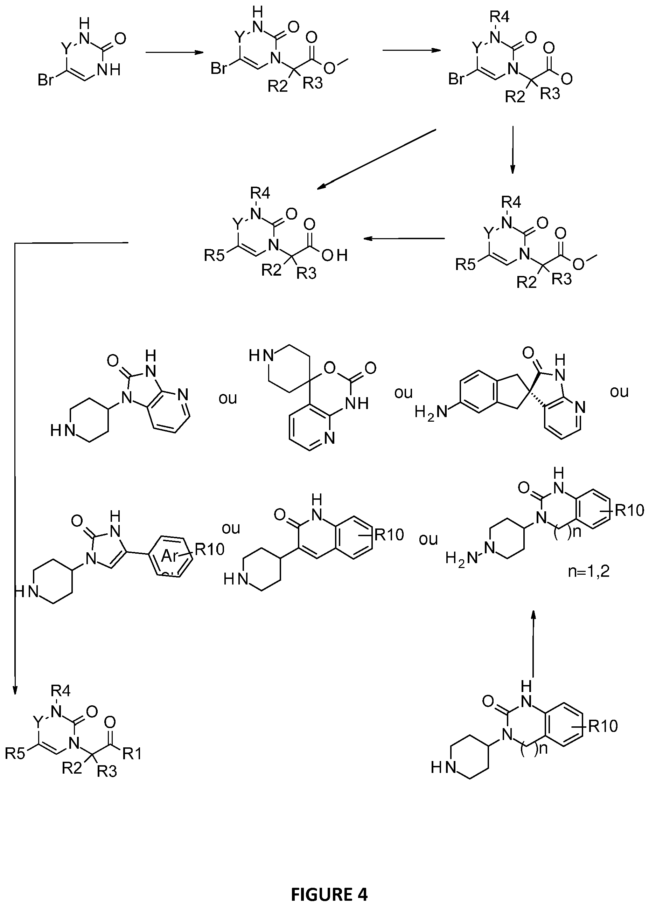

- la figure 4 représente une voie de synthèse générale dans des conditions classiques de composés selon le schéma réactionnel n°4.

Dans l'ensemble de la présente description, à moins qu'il ne soit spécifié autrement, il est entendu que, lorsque des intervalles de concentrations sont donnés, ils incluent les bornes supérieures et inférieures dudit intervalle.

Les composés selon l'invention sont de nouveaux composés hétérocycliques antagonistes du récepteur CGRP choisis parmi les composés de formule générale (I) : 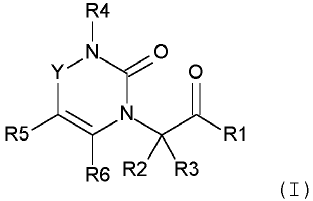 dans laquelle,
- Y est -C(O ;
- R1 est (1-1) : 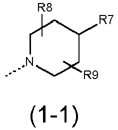
- R2, R3, R6 sont un atome d'hydrogène;
- R4 est groupement (4-8): 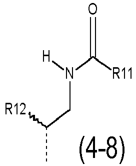
- R5 est (5-1) : 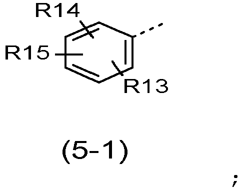
- R7 est (7-1) : 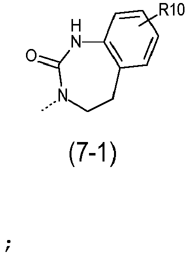
- R8, R9 sont identiques, et un atome d'hydrogène;
- R10 est -OR11 ;
- R11, R12 sont identiques ou différents, et choisis parmi -CH3 ou -CH2CH3;
- R13, R14, R15 sont identiques ou différents, et choisis parmi un atome d'hydrogène, -OR16, un halogène choisi parmi F et Cl;
- R16 est un alkyle C1-C3 ;
ainsi que leurs sels pharmaceutiquement acceptables, et leurs énantiomères.

« Sel pharmaceutiquement acceptable » désigne les sels d'un composé d'intérêt qui possèdent l'activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine. Une liste de sels pharmaceutiquement acceptables est notamment publiée dans la revue de Berge et al. (J. Pharm. Sci. 1977, 66(1), 1-19) .

Le terme énantiomère désigne les configurations R ou S des composés de formule générale (I) selon l'invention.

A moins qu'il n'en soit précisé autrement, les définitions données ci-après s'appliquent par défaut à l'ensemble des radicaux utilisés pour définir la structure des composés de formule générale (I).

Lorsqu'une caractéristique du substituant diffère de celle indiquée dans les définitions ci-dessous, cette différence est explicitement indiquée.

A titre d'exemples, un alkyle qui ne comprendrait que 3 à 7 atomes de carbone serait défini par alkyle C3-C7 ; un alcène qui ne comprendrait que 3 à 7 atomes de carbone et qui serait uniquement linéaire serait défini par alcène C3-C7 linéaire, toutes autres caractéristiques étant égales par ailleurs à celles définies par défaut pour chaque radical ci-dessous.

Un alkyle désigne, en particulier, une chaîne hydrocarbonée saturée, linéaire ou ramifiée, comprenant de 1 à 8 atomes de carbone, éventuellement substituée par un ou plusieurs atomes de fluor.

Un alcène désigne, en particulier, une chaîne hydrocarbonée insaturée, linéaire ou ramifiée, comprenant de 2 à 8 atomes de carbone et comprenant une ou deux doubles liaisons, éventuellement substituée par un ou plusieurs atomes de fluor.

Un alcyne désigne, en particulier, une chaîne hydrocarbonée insaturée, linéaire ou ramifiée, comprenant de 2 à 8 atomes de carbone et comprenant une ou deux triples liaisons, éventuellement substituée par un ou plusieurs atomes de fluor.

Un cycloalkyle désigne, en particulier, une chaine hydrocarbonée, saturée, cyclique comprenant de 3 à 7 atomes de carbones, éventuellement substituée par un ou plusieurs atomes de fluor.

Un cycloalcène désigne, en particulier, une chaine hydrocarbonée, insaturée, cyclique comprenant de 4 à 7 atomes de carbones et comprenant une ou deux doubles liaisons, éventuellement substituée par un ou plusieurs atomes de fluor.

Un aryle désigne, en particulier, un cycle hydrocarboné aromatique ou deux cycles fusionnés hydrocarbonés aromatiques. Les radicaux aryles préférés sont choisis parmi les radicaux phényle et naphtyle, éventuellement substitué par un ou plusieurs groupes d'atomes choisis parmi un alkyle, un alkoxyle, un aryle, un halogène, un hydroxyle, un cyano, un trifluorométhyle, un sulfure, un pentafluorosulfure, un sulfoxyde, une sulfone, un acide carboxylique, un ester et un nitro.

Un aralkyle désigne, en particulier, un alkyle substitué par un aryle.

Un hétéroaryle désigne, en particulier, un radical hétérocyclique aromatique c'est-à-dire une chaîne hydrocarbonée cyclique ou polycyclique, aromatique, comprenant un ou plusieurs hétéroatomes choisis parmi O, S et N, éventuellement substituée par un ou plusieurs groupes d'atomes choisis par exemple parmi un alkyle, un alkoxy, un aryle, un aryle substitué, un halogène, un hydroxy, un cyano, un trifluorométhyle, un sulfure, un pentafluorosulfure, un sulfoxyde, une sulfone, un acide carboxylique, un ester et un nitro.

Un hétéroaralkyle désigne, en particulier, un alkyle substitué par un hétéroaryle.

Un hétérocycle désigne, en particulier, une chaine hydrocarbonée cyclique ou bicylique, saturée ou insaturée, comprenant un ou plusieurs hétéroatomes choisis parmi O, S et N, éventuellement substitué par un ou plusieurs groupes choisis parmi un alkyle, un alkoxyle, un halogène, un hydroxyle, un cyano, un trifluorométhyle, un sulfure, un pentafluorosulfure, un sulfoxyde, une sulfone, un acide carboxylique, un ester et un nitro.

Un halogène désigne un atome de fluor, chlore ou brome.

Un alkoxyle désigne un atome d'oxygène substitué par un radical alkyle.

Un ether désigne, en particulier, une chaîne hydrocarbonée saturée, linéaire ou ramifiée, comprenant de 1 à 8 atomes de carbone, et dans laquelle 1 à 3 atomes de carbone sont remplacés respectivement par 1 à 3 atomes d'oxygène, deux atomes d'oxygène étant toujours séparés par au moins un atome de carbone, ladite chaine étant éventuellement substituée par un ou plusieurs atomes de fluor

Les composés sont préférentiellement choisis parmi les composés de formule générale (I) dans laquelle :
- Y est -C(O) ;
- R1 est choisi parmi les groupements suivants : 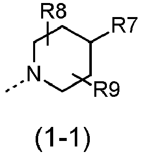
- R2, R3, R6 sont un atome d'hydrogène;
- R4 est 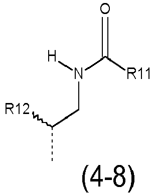
- R5 est le groupement suivant : 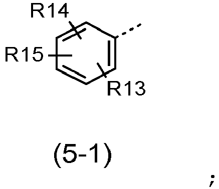
- R7 est le groupement suivant : 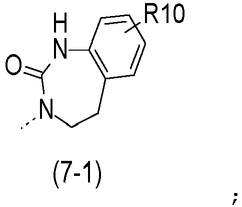
- R8, R9 sont un atome d'hydrogène ;
- R10 est -OR11 ;
- R11, R12 sont identiques ou différents, et choisis parmi -CH3 ou -CH2CH3;
- R13, R14, R15 sont identiques ou différents, et choisis parmi un atome d'hydrogène, -OR16, un halogène choisi parmi F et Cl; et
- R16 est un alkyle C1-C3.

Décrit sont_les composés suivants :
Composé 1 : 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 2 : 5-(2-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 3 : 5-(2,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 4 : 5-(3-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 5 : 3-méthyl-1-{2-oxo-2-[4-(2-oxo-1,4-dihydro-2H-quinazolin-3-yl)-pipéridin-1-yl]-éthyl}-5-(1H-pyrazol-3-yl)-1H-pyrimidine-2,4-dione ;
Composé 6 : 5-(3-méthoxy-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 7 : 5-(3-hydroxy-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 8 : 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 9 : 5-cyclohex-1-ényl-3-méthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 10 : 3-méthyl-1-{2-oxo-2-[4-(2-oxo-1,4-dihydro-2H-quinazolin-3-yl)-pipéridin-1-yl]-éthyl}-5-phényl-1H-pyrimidine-2,4-dione ;
Composé 11 : 5-bromo-1-{2-oxo-2-[4-(2-oxo-1,4-dihydro-2H-quinazolin-3-yl)-pipéridin-1-yl]-éthyl}-3-(2,2,2-trifluoro-éthyl)-1H-pyrimidine-2,4-dione ;
Composé 12 : 5-(2,3-difluoro-phényl)-3-éthyl-1-{2-oxo-2-[4-(2-oxo-1,4-dihydro-2H-quinazolin-3-yl)-pipéridin-1-yl]-éthyl}-1H-pyrimidine-2,4-dione ;
Composé 13 : 5-(2-chloro-3-fluoro-phényl)-3-isopropyl-1-{2-oxo-2-[4-(2-oxo-1,4-dihydro-2H-quinazolin-3-yl)-pipéridin-1-yl]-éthyl}-1H-pyrimidine-2,4-dione ;
Composé 14 : 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione ;
Composé 15 : 5-(2,3-difluoro-phényl)-3-méthyl-1-[1-méthyl-2-oxo-2-[4-(2-oxo-1,4-dihydroquinazolin-3-yl)-1-pipéridyl]éthyl]pyrimidine-2,4-dione ;
Composé 16 : 5-(2,3-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 17 : 5-(2,3-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 18 : 5-(2-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 19 : 3-méthyl-1-{2-oxo-2-[4-(2-oxo-1,2,4,5-tétrahydro-benzo[d][1,3]diazépin-3-yl)-pipéridin-1-yl]-éthyl}-5-(2-trifluorométhoxy-phényl)-1H-pyrimidine-2,4-dione ;
Composé 20 : 3-méthyl-1-{2-oxo-2-[4-(2-oxo-1,2,4,5-tétrahydro-benzo[d][1,3]diazépin-3-yl)-pipéridin-1-yl]-éthyl}-5-thiophen-3-yl-1H-pyrimidine-2,4-dione ;
Composé 21 : 5-(2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 22 : 5-(3-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 23 : 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 24 : 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 25 : 5-(2-méthoxy-3-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 26 : 5-(3-chloro-2-hydroxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 27 : 5-(2,3-diméthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 28 : 5-(3-fluoro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 29 : 5-(3-chloro-2-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 30 : 5-(2,3-diméthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 31 : 5-(2-chloro-3-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 32 : 5-bromo-3-méthyl-1-{2-oxo-2-[4-(2-oxo-1,2,4,5-tétrahydro-benzo [d][1,3]diazépin-3-yl)-pipéridin-1-yl]-éthyl}-1H-pyrimidine-2,4-dione ;
Composé 33 : 5-(3-fluoro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 34 : 5-(2,6-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 35 : 5-(3,5-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 36 : 4-(3-méthyl-2,4-dioxo-1-{2-oxo-2-[4-(2-oxo-1,2,4,5-tétrahydro-benzo [d][1,3]diazépin-3-yl)-pipéridin-1-yl]-éthyl}-1,2,3,4-tétrahydro-pyrimidin-5-yl)-benzonitrile ;
Composé 37 : 3-(3-méthyl-2,4-dioxo-1-{2-oxo-2-[4-(2-oxo-1,2,4,5-tétrahydro-benzo [d][1,3]diazépin-3-yl)-pipéridin-1-yl]-éthyl}-1,2,3,4-tétrahydro-pyrimidin-5-yl)-benzonitrile ;
Composé 38: 2-(3-méthyl-2,4-dioxo-1-{2-oxo-2-[4-(2-oxo-1,2,4,5-tétrahydro-benzo [d][1,3]diazépin-3-yl)-pipéridin-1-yl]-éthyl}-1,2,3,4-tétrahydro-pyrimidin-5-yl)-benzonitrile ;
Composé 39 : 3-méthyl-5-(3-méthyl-pyridin-4-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 40 : 3-méthyl-5-(2-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 41 : 3-méthyl-5-(4-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 42 : 3-méthyl-5-(6-méthyl-pyridin-2-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 43 : 3-méthyl-5-(6-méthyl-1-oxy-pyridin-2-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 44 : 3-méthyl-5-(1-méthyl-1H-pyrazol-4-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 45 : 5-(5-chloro-pyridin-3-yl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 46 : 5-benzyl-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione
Composé 47 : 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 48 : 5-(5-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 49 : 5-(4,5-diméthyl-pyridin-3-yl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 50 : 3-méthyl-1-{2-oxo-2-[4-(2-oxo-1,2,4,5-tétrahydro-benzo[d][1,3]diazépin-3-yl)-pipéridin-1-yl]-éthyl}-5-phénoxy-1H-pyrimidine-2,4-dione ;
Composé 51 : 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 52 : 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 53 : 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 54 : 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 55 : 5-(3-chloro-2-méthoxy-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 56 : 5-(2,3-diméthyl-phényl)-1-{2-oxo-2-[4-(2-oxo-1,2,4,5-tétrahydro-benzo[d] [1,3]diazépin-3-yl)-pipéridin-1-yl]-éthyl}-3-propyl-1H-pyrimidine-2,4-dione ;
Composé 57 : 5-(2-chloro-3-fluoro-phényl)-1-{2-oxo-2-[4-(2-oxo-1,2,4,5-tétrahydro-benzo[d][1,3]diazépin-3-yl)-pipéridin-1-yl]-éthyl}-3-propyl-1H-pyrimidine-2,4-dione ;
Composé 58 : 1-{2-oxo-2-[4-(2-oxo-1,2,4,5-tétrahydro-benzo[d][1,3]diazépin-3-yl)-pipéridin-1-yl]-éthyl}-3-propyl-5-pyridazin-3-yl-1H-pyrimidine-2,4-dione ;
Composé 59 : 5-(3-chloro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 60 : 5-(3-chloro-2-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 61 : 5-(3-fluoro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 62 : 5-(2,3-difluoro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 63 : 5-(2-chloro-3-fluoro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 64 : 3-isopropyl-5-(2-méthoxy-3-méthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 65 : 5-(3-chloro-4-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 66 : 5-(2-fluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 67 : 5-(3,4-dichloro-phényl)-3-isopropyl -1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 68 : 5-(2,3-diméthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 69 : 5-(2-chloro-3-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 70 : 5-(5-chloro-2-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 71 : 5-(2,3-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 72 : (5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-acetonitrile ;
Composé 73 : acide 3-(5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-propanoïque ;
Composé 74 : 3-(3-isopropyl-2,4-dioxo-1-{2-oxo-2-[4-(2-oxo-1,2,4,5-tétrahydro-benzo[d] [1,3]diazépin-3-yl)-pipéridin-1-yl]-éthyl}-1,2,3,4-tétrahydro-pyrimidin-5-yl)-benzoic acétate de méthyle ;
Composé 75 : acide 3-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïque ;
Composé 76 : 5-(2-chloro-3-méthylsulfanyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 77 : 5-(2-chloro-3-méthanesulfinyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 78 : 5-(2-chloro-3-méthanesulfonyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 79 : 2-(3-isopropyl-2,4-dioxo-1-{2-oxo-2-[4-(2-oxo-1,2,4,5-tétrahydro-benzo[d] [1,3]diazépin-3-yl)-pipéridin-1-yl]-éthyl}-1,2,3,4-tétrahydro-pyrimidin-5-yl)-benzoïc acétate de méthyle ;
Composé 80 : acide 2-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïque ;
Composé 81 : 5-(3,4-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 82 : 5-(3,5-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 83 : 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 84 : 5-(2-chloro-3-fluoro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 85 : 5-(2,3-diméthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 86 : 5-(3-fluoro-2-méthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 87 : [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamic acid tert-butyl ester ;
Composé 88 : 3-(2-amino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 89 : 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 90 : 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 91 : 3-((R)-2,3-dihydroxy-propyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 92 : 3-(3,4-dihydroxy-butyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 93 : 5-(2,3-diméthyl-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 94 : 5-(3,5-dichloro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 95 : 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 96 : 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 97 : 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 98 : 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 99 : 3-(2-diméthylamino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 100 : 5-(2,3-diméthyl-phényl)-3-(2-méthoxyméthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 101 : N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide ;
Composé 102 : 5-(2,3-diméthyl-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 103 : (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle ;
Composé 104 : acide (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétique ;
Composé 105 : 5-(2-chloro-3-fluoro-phényl)-3-oxétan-3-ylméthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 106 : 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 107 : (5-bromo-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle ;
Composé 108 : 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 109 : 5-(2,3-difluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 110 : 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 111 : 2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle ;
Composé 112 : 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 113 : 5-(2,3-difluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione ;
Composé 114 : 3-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle ;
Composé 115 : N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 116 : N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 117 : N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide ;
Composé 118 : N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide ;
Composé 119 : 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 120 : N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-méthanesulfonamide ;
Composé 121 : 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-[(S)-1-méthyl-2-(3-méthyl-oxétan-3-ylamino)-éthyl]-1H-pyrimidine-2,4-dione ;
Composé 122 : 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 123 : (S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-acétate de propyle ;
Composé 124 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 126 : 5-(2,3-difluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 127 : 5-(2,3-dichloro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 128 : 5-(2,3-difluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 129 : 5-(2,3-diméthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 130 : 5-(2-chloro-3-méthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 131 : 5-(2-chloro-3-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 132 : 5-(2,3-dichloro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 133 : 5-(2,3-diméthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 134 : 5-(3-bromo-2-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 135 : 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 136 : 5-(2,3-difluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 137 : 5-(2,3-dichloro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 138 : 5-(2,3-diméthyl-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 139 : 5-(2,3-diméthoxy-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 140 : 5-(2-chloro-3-méthoxy-phényl)-3-(2-methanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 141 : 3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-5-méthyl-1H-pyrimidine-2,4-dione ;
Composé 142 : 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo [d][1,3]diazépin-3-yl)-pipéridin-1-yl]-2-oxo-éthyl}-1H-pyrimidine-2,4-dione ;
Composé 143 : 5-(2-chloro-3-fluoro-phényl)-3-((R)-2-méthanesulfonyl-1-méthyl-ethyl)-1-{2-[4-(7-méthoxy-2-oxo-1,2,4,5-tétrahydro-benzo [d][1,3]diazépin-3-yl)-pipéridin-1-yl]-2-oxo-éthyl}-1H-pyrimidine-2,4-dione ;
Composé 144 : 5-(2,3-dichloro-phényl)-3-((S)-2-méthanesulfonyl-1-méthyl-éthyl)-1-{2-[4-(7-méthoxy-2-oxo-1,2,4,5-tétrahydro-benzo [d][1,3]diazépin-3-yl)-pipéridin-1-yl]-2-oxo-éthyl}-1H-pyrimidine-2,4-dione ;
Composé 145 : 5-(2,3-dichloro-phényl)-3-((R)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 146 : 5-(2-chloro-3-fluoro-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 147 : 5-(2,3-diméthoxy-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 148 : 5-(2-chloro-3-méthoxy-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 149 : 5-(2-fluoro-3-méthoxy-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 150 : 3-isopropyl-1-{2-[4-(7-méthoxy-2-oxo-1,2,4,5-tétrahydro-benzo [d][1,3]diazépin-3-yl)-pipéridin-1-yl]-2-oxo-éthyl}-5-(2-trifluorométhoxy-phényl)-1H-pyrimidine-2,4-dione ;
Composé 151 : 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(3-trifluorométhoxy-phényl)-1H-pyrimidine-2,4-dione ;
Composé 152 : 3-isopropyl-1-{2-[4-(7-méthoxy-2-oxo-1,2,4,5-tétrahydro-benzo [d][1,3]diazépin-3-yl)-pipéridin-1-yl]-2-oxo-éthyl}-5-(3-méthoxy-2-trifluorométhoxy-phényl)-1H-pyrimidine-2,4-dione ;
Composé 153 : 5-(2,3-difluoro-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 154 : 5-(2-chloro-3-éthoxy-phényl)-3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 155 : 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione ;
Composé 156 : 5-(2-chloro-3-fluoro-phényl)-3-(3-méthanesulfonyl-propyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 157 : 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione ;
Composé 158 : N-[(S)-2-(5-(2-chloro-3-fluoro-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-propionamide ;
Composé 159 : 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-((S)-2-methoxy-1-methyl-ethyl)-1H-pyrimidine-2,4-dione ;
Composé 160 : 5-(2-Chloro-3-fluoro-phenyl)-1-{2-[4-(7-fluoro-2-oxo-1,2,4,5-tetrahydro-benzo[d][1,3]diazepin-3-yl)-piperidin-1-yl]-2-oxo-ethyl}-3-(2-methanesulfonyl-ethyl)-1H-pyrimidine-2,4-dione ;
Composé 161 : N-[(S)-2-(5-(2-Chloro-3-fluoro-phenyl)-3-{2-[4-(7-fluoro-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide ;
Composé 162 : N-[(S)-2-(5-(2,3-Dichloro-phenyl)-3-{2-[4-(7-fluoro-2-oxo-1,2,4,5-tetrahydro-benzo[d] [1,3]diazepin-3-yl)-piperidin-1-yl]-2-oxo-ethyl}-2,6-dioxo-3,6-dihydro-2H-pyrimidin-1-yl)-propyl]-acetamide ;
Composé 163 : 5-(2-Chloro-3-fluoro-phenyl)-1-{2-[4-(9-fluoro-2-oxo-1,2,4,5-tetrahydro-benzo[d][1,3]diazepin-3-yl)-piperidin-1-yl]-2-oxo-ethyl}-3-isopropyl-1H-pyrimidine-2,4-dione ;
Composé 164 : 5-(2,3-difluorophenyl)-3-méthyl-1-[2-oxo-2-[4-(2-oxo-3H-imidazo[4,5-b]pyridin-1-yl)-1-piperidyl]ethyl]pyrimidine-2,4-dione ;
Composé 165 : 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide ;
Composé 166 : 5-(2-chloro-3-fluoro-phényl)-3-isopropyl-pyrimidine-2,4-dione-1-[(2R)-2'-oxospiro[1,3-dihydroindene-2,3'-1H-pyrrolo[2,3-b]pyridine]-5-yl]-acetamide ;
Composé 167 : 5-(2,3-difluorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 168 : 2-[5-(2-chloro-3-fluoro-phenyl)-2,4-dioxo-1H-pyrimidin-3-yl]acetic acid-1-[(2R)-2'-oxospiro[1,3-dihydroindene-2,3'-1H-pyrrolo[2,3-b]pyridine]-5-yl]-acetamide ;
Composé 169 : 5-(2-chloro-3-methoxy-phenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide ;
Composé 170 : 5-(2,3-dichlorophenyl)-3-(2-methylsulfonylethyl)-pyrimidine-2,4-dione-1-[(2R)-2'-oxospiro[1,3-dihydroindene-2,3'-lH-pyrrolo[2,3-b]pyridine]-5-yl]-acetamide ;
Composé 171 : 5-(2,3-dimethoxyphenyl)-3-[(1R)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 172 : 5-(2-chloro-3-fluoro-phenyl)-3-(2-methylsulfonylethyl)-pyrimidine-2,4-dione-1-[(2R)-2'-oxospiro[1,3-dihydroindene-2,3'-lH-pyrrolo[2,3-b]pyridine]-5-yl]-acetamide ;
Composé 173 : 5-(2-chloro-3-fluoro-phenyl)-3-(3-methylsulfonylpropyl)-pyrimidine-2,4-dione-1-[(2R)-2'-oxospiro[1,3-dihydroindene-2,3'-1H-pyrrolo[2,3-b]pyridine]-5-yl]-acetamide ;
Composé 174 : 5-(2,3-dichlorophenyl)-3-methyl-pyrimidine-2,4-dione-1-[(2R)-2'-oxospiro[1,3-dihydroindene-2,3'-1H-pyrrolo[2,3-b]pyridine]-5-yl]-acetamide ;
Composé 175 : 5-(2-chloro-3-fluoro-phenyl)-3-[(1S)-1-methyl-2-methylsulfonyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 176 : 5-(2-chloro-3-methoxy-phenyl)-3-[2-methoxy-1-(methoxymethyl)ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 177 : 5-(2,3-dichlorophenyl)-3-[(1R)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 178 : 5-(2,3-dichlorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 179 : 5-(2-chloro-3-fluoro-phenyl)-3-[2-methoxy-1-(methoxymethyl)ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 180 : N-[2-[5-(2-chloro-3-fluoro-phenyl)-2,4-dioxo-pyrimidin-3-yl]ethyl]acetamide-1-[(2R)-2'-oxospiro[1,3-dihydroindene-2,3'-1H-pyrrolo[2,3-b]pyridine]-5-yl]-acetamide ;
Composé 181 : 5-(2-chloro-3-fluoro-phenyl)-3-[(1R)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 182 : 5-(2,3-dichlorophenyl)-3-[2-methoxy-1-(methoxymethyl)ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 183 : 5-(2-chloro-3-fluoro-phenyl)-3-[(1R)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide ;
Composé 184 : 5-(2-Chloro-3-fluoro-phényl)-3-(2-méthanesulfonyl-éthyl)-1-{2-[4-(7-méthanesulfonyl-2-oxo-1,2,4,5-tétrahydro-benzo[d] [1,3]diazepin-3-yl)-piperidin-1-yl]-2-oxo-ethyl}-1H-pyrimidine-2,4-dione ;
Composé 185 : 5-(2-Chloro-3-fluoro-phenyl)-3-isopropyl-1-{2-[4-(7-methanesulfonyl-2-oxo-1,2,4,S-tetrahydro-benzo[d][1,3]diazepin-3-yl)-piperidin-1-yl]-2-oxo-ethyl}-1H-pyrimidine-2,4-dione ;
Composé 186 : 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d][1,3]diazepin-3-yl)-piperidin-1-yl]-2-oxo-éthyl}-1H-pyrimidine-2,4-dione ;
Composé 187 : 5-(2-Chloro-3-fluoro-phenyl)-3-((R)-2-methoxy-1-methyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
Composé 188 : 5-(2-Chloro-3-fluoro-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
Composé 189 : 5-(2-Chloro-3-fluoro-phenyl)-3-isopropyl-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione ;
Composé 190 : 3-(1-{2-[5-(2,3-difluoro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 191 : 3-(1-{2-[5-(2,3-Dichloro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 192 : 3-(1-{2-[S-(2-chloro-3-fluoro-phenyl)-3-(2-methoxy-ethyl)-2-oxo-3,4-dihydro-2H-pyrimidin-1-yl]-acetyl}-piperidin-4-yl)-1,3,4,5-tetrahydro-benzo[d][1,3]diazepin-2-one ;
Composé 193 : 3-(1-{2-[S-(2-Chloro-3-methoxy-phenyl)-3-isopropyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 194 : 3-(1-{2-[5-(2,3-dichloro-phényl)-2-oxo-3-(2,2,2-trifluoro-éthyl)-3,4-dihydro-2H-pyrimidin-1-yl]-acetyl}-piperidin-4-yl)-1,3,4,5-tetrahydro-benzo[d][1,3]diazepin-2-one ;
Composé 195 : 1'-[2-[5-(2,3-dichlorophenyl)-3-methyl-2-oxo-4H-pyrimidin-1-yl]acetyl]spiro[1H-pyrido[2,3-d][1,3]oxazine-4,4'-piperidine]-2-one ;
Composé 196 : 5-(2,3-Dichloro-phenyl)-3-methyl-1-{2-oxo-2-[4-(2-oxo-4-phenyl-2,3-dihydro-imidazol-1-yl)-piperidin-1-yl]-ethyl}-3,4-dihydro-1H-pyrimidin-2-one ;
Composé 197 : 3-(1-{2-[5-(2-Chloro-3-fluoro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 198 : 3-(1-{2-[S-(2-Chloro-3-fluoro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 199 : 3-(1-{2-[S-(2,3-Dichloro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 200 : 3-(1-{2-[5-(2-Chloro-3-fluoro-phenyl)-3-isopropyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 201 : 3-(1-{2-[5-(2,3-Dichloro-phenyl)-3-isopropyl-2-oxo-3,4-dihydro-2H-pyrimidin-1-yl]-acetyl}-piperidin-4-yl)-1,3,4,5-tetrahydro-benzo[d][1,3]diazepin-2-one ;
Composé 202 : N-[2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide ;
Composé 203 : N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 204 : N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-2,2,2-trifluoro-acétamide ;
Composé 205 : N-[(S)-2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 206 : N-[(S)-2-(5-(2-chloro-3-méthoxy-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide ;
Composé 207 : N-[2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide ;
Composé 208 : 5-(2,3-dichlorophenyl)-3-methyl-1-[2-oxo-2-[4-(2-oxo-5-phenyl-1H-imidazol-3-yl)-1-piperidyl]ethyl]pyrimidine-2,4-dione ;
Composé 209 : 2-[5-(2,3-dichlorophenyl)-3-methyl-2,4-dioxo-pyrimidin-1-yl]-N-[4-(2-oxo-4,5-dihydro-1H-1,3-benzodiazepin-3-yl)-1-piperidyl]acetamide ;
Composé 210 : 5-(2,3-dichlorophenyl)-3-isopropyl-1-[2-oxo-2-[4-(2-oxo-4,5-dihydro-1H-1,3-benzodiazepin-3-yl)-1-piperidyl]ethyl]pyrimidine-2,4-dione ;
Composé 211 : 5-(2,3-dichlorophenyl)-3-methyl-1-[2-oxo-2-(2-oxospiro[1H-pyrido[2,3-d][1,3]oxazine-4,4'-piperidine]-1'-yl)ethyl]pyrimidine-2,4-dione ;
Composé 212: 2-[3-ethyl-2,4-dioxo-1-[2-oxo-2-[4-(2-oxo-4,5-dihydro-1H-1,3-benzodiazepin-3-yl)-1-piperidyl]ethyl]pyrimidin-5-yl]benzonitrile ;
Composé 213 : 5-(2,3-dichlorophenyl)-3-methyl-1-[2-oxo-2-[4-(2-oxo-4,5-dihydro-1H-pyrido[2,3-d][1,3]diazepin-3-yl)-1-piperidyl]ethyl]pyrimidine-2,4-dione ;
Composé 214 : 5-(2,3-dimethylphenyl)-3-methyl-1-[2-oxo-2-[4-(2-oxo-5-phenyl-1H-imidazol-3-yl)-1-piperidyl]ethyl]pyrimidine-2,4-dione ;
Composé 215 : 5-(2,3-dichlorophenyl)-3-methyl-1-[2-oxo-2-[4-(2-oxo-1H-quinolin-3-yl)-1-piperidyl]ethyl]pyrimidine-2,4-dione ;
Composé 216 : 5-(2,3-dimethylphenyl)-3-isopropyl-1-[2-oxo-2-[4-(2-oxo-4,5-dihydro-1H-pyrido[4,3-d][1,3]diazepin-3-yl)-1-piperidyl]ethyl]pyrimidine-2,4-dione ;
Composé 217 : 5-(2,3-dimethylphenyl)-3-isopropyl-1-[2-oxo-2-[4-(2-oxo-4,5-dihydro-1H-pyrido[3,2-d][1,3]diazepin-3-yl)-1-piperidyl]ethyl]pyrimidine-2,4-dione ;
Composé 218 : 5-(2,3-dimethylphenyl)-3-isopropyl-1-[2-oxo-2-[4-(2-oxo-4,5-dihydro-1H-pyrido[2,3-d] [1,3]diazepin-3-yl)-1-piperidyl]ethyl]pyrimidine-2,4-dione ;
Composé 219 : 5-(2,3-dimethylphenyl)-3-methyl-1-[2-[4-(1-methyl-2-oxo-4,5-dihydro-1,3-benzodiazepin-3-yl)-1-piperidyl]-2-oxo-ethyl]pyrimidine-2,4-dione ;
Composé 220 : 5-(3,4-dichlorophenyl)-3-methyl-1-[2-oxo-2-[4-(2-oxo-4,5-dihydro-1H-1,3-benzodiazepin-3-yl)-1-piperidyl]ethyl]pyrimidine-2,4-dione ;
Composé 221 : 1-[2-[4-(7-bromo-2-oxo-4,5-dihydro-1H-1,3-benzodiazepin-3-yl)-1-piperidyl]-2-oxo-ethyl]-5-(2-chloro-3-fluoro-phenyl)-3-isopropyl-pyrimidine-2,4-dione ;
Composé 222 : 5-(2-chloro-3-fluoro-phenyl)-3-isopropyl-1-[2-[4-methyl-4-(2-oxo-4,5-dihydro-1H-1,3-benzodiazepin-3-yl)-1-piperidyl]-2-oxo-ethyl]pyrimidine-2,4-dione ;
Composé 223 : 5-(2-chloro-3-fluoro-phenyl)-3-isopropyl-1-[2-[(2S)-2-methyl-4-(2-oxo-4,5-dihydro-1H-1,3-benzodiazepin-3-yl)-1-piperidyl]-2-oxo-ethyl]pyrimidine-2,4-dione ;
Composé 224 : acide 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propanoique ;
Composé 225 : 5-(2-chloro-3-fluoro-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidine-2,4-dione ;
Composé 226 : 2-[5-(2-chloro-3-fluoro-phenyl)-2,6-dioxo-3-[2-oxo-2-[4-(2-oxo-4,S-dihydro-1H-1,3-benzodiazepin-3-yl)-1-piperidyl]ethyl]pyrimidin-1-yl]acetamide ;
Composé 227 : 5-(2-chloro-3-fluoro-phenyl)-3-(2-methylsulfinylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 228 : 5-(2-chloro-3-fluoro-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 229 : 5-(2-chloro-3-fluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione ;
Composé 230 : 5-(2-chloro-3-fluoro-phenyl)-3-isopropyl-1-[2-[4-(7-methoxy-5,5-dimethyl-2-oxo-1,4-dihydro-1,3-benzodiazepin-3-yl)-1-piperidyl]-2-oxo-ethyl]pyrimidine-2,4-dione ;
Composé 231 : 5-(2,3-difluorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 232 : 5-(2-chloro-3-fluoro-phenyl)-3-[2-méthoxy-1-(méthoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 233 : 5-(2-chloro-3-fluoro-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxo-ethyl]pyrimidine-2,4-dione ;
Composé 234 : 5-(2-chloro-3-fluoro-phenyl)-3-(2-hydroxy-2-methyl-propyl)-1-[2-oxo-2-[4-(2-oxo-4,5-dihydro-1H-1,3-benzodiazepin-3-yl)-1-piperidyl]ethyl]pyrimidine-2,4-dione ;
Composé 235 : 5-(2-chloro-3-fluoro-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrimidine-2,4-dione ;
Composé 236 : 5-(2,3-difluorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 237 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1S)-1-methylpropyl]pyrimidine-2,4-dione ;
Composé 238 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidine-2,4-dione ;
Composé 239 : 5-(2-chloro-3-fluoro-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 240 : 5-(2-chloro-3-fluoro-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 241 : 5-(2-chloro-3-methoxy-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 242 : 5-(2,3-dichlorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 243 : 5-(2-chloro-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione ;
Composé 244 : 5-(2-chloro-3-fluoro-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione ;
Composé 245 : 5-(2,3-dichlorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 246 : acide 3-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propanoique ;
Composé 247 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidine-2,4-dione ;
Composé 248 : acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]acetique ;
Composé 249 : 5-(2-chloro-3-fluoro-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione ;
Composé 250 : 5-(2-chloro-3-fluoro-phenyl)-3-(2-methylsulfonylethyl)-1-[2-oxo-2-[2-(2-oxo-4,5-dihydro-1H-1,3-benzodiazepin-3-yl)-6-azaspiro[3.3]heptan-6-yl]ethyl]pyrimidine-2,4-dione ;
Composé 251 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidine-2,4-dione ;
Composé 252 : 5-(2-chloro-3-fluoro-phenyl)-1-[2-[4-(7-fluoro-2-oxo-4,5-dihydro-1H-1,3-benzodiazepin-3-yl)-1-piperidyl]-2-oxo-ethyl]-3-[(1R)-1-methyl-2-methylsulfonyl-ethyl]pyrimidine-2,4-dione ;
Composé 253 : acide 3-[1-[2-[5-(2-chloro-3-fluoro-phenyl)-3-(2-methylsulfonylethyl)-2,4-dioxo-pyrimidin-1-yl]acetyl]-4-piperidyl]-2-oxo-4,5-dihydro-1H-1,3-benzodiazepine-7-carboxylique ;
Composé 254 : S-[2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethyl]ethanethioate ;
Composé 255 : N-[(2S)-2-[5-(2,3-dichlorophenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide ;
Composé 256 : N-[(2S)-2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide ;
Composé 257 : acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethanesulfonique ;
Composé 258 : 5-(2-chloro-3-fluoro-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione ;
Composé 259 : 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidine-2,4-dione ;
Composé 260 : N-[(2S)-2-[5-(2-chloro-3-methoxy-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propyl]propanamide ;
Composé 261 : N-[(2S)-2-[5-(2,3-dichlorophenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propyl]propanamide ;
Composé 262 : N-[(2S)-2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-4-methyl-2,6-dioxo-pyrimidin-1-yl]propyl]acetamide ;
Composé 263 : N-[(2S)-2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propyl]-2-methyl-propanamide ;
Composé 264 : N-[(2S)-2-[5-(2-chloro-3-methoxy-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propyl]-2-methyl-propanamide ;
Composé 265 : 3-[1-[2-[5-(2-chloro-3-fluoro-phenyl)-3-(2-methoxyethyl)-4,4-dimethyl-2-oxo-pyrimidin-1-yl]acetyl]-4-piperidyl]-8-methoxy-4,5-dihydro-1H-1,3-benzodiazepin-2-one ;
Composé 266 : 3-[1-[2-[4-(2-chloro-3-fluoro-phenyl)-8-[(1S)-2-methoxy-1-methyl-ethyl]-7-oxo-6,8-diazaspiro[2.5]oct-4-en-6-yl]acetyl]-4-piperidyl]-8-methoxy-4,5-dihydro-1H-1,3-benzodiazepin-2-one ;
Composé 267 : N-[(2S)-2-[5-(2-chloro-3-methoxy-phenyl)-3-[2-[4-(8-methoxy-2-oxo-4,5-dihydro-1H-1,3-benzodiazepin-3-yl)-1-piperidyl]-2-oxo-ethyl]-6,6-dimethyl-2-oxo-pyrimidin-1-yl]propyl]acetamide ;
Composé 268 : N-[(2S)-2-[4-(2-chloro-3-methoxy-phenyl)-6-[2-[4-(8-methoxy-2-oxo-4,5-dihydro-1H-1,3-benzodiazepin-3-yl)-1-piperidyl]-2-oxo-ethyl]-7-oxo-6,8-diazaspiro[2.5]oct-4-en-8-yl]propyl]acetamide ;
Composé 269:N-[(2S)-2-[5-(2-chloro-3-fluoro-phenyl)-3-[2-[4-(5,5-difluoro-7-methoxy-2-oxo-1,4-dihydro-1,3-benzodiazepin-3-yl)-1-piperidyl]-2-oxo-ethyl]-2,6-dioxo-pyrimidin-1-yl]propyl]acetamide ;
Composé 270: N-[(2S)-2-[5-(2-chloro-3-fluoro-phenyl)-3-[2-[4-(5-fluoro-7-methoxy-2-oxo-4,5-dihydro-1H-1,3-benzodiazepin-3-yl)-1-piperidyl]-2-oxo-ethyl]-2,6-dioxo-pyrimidin-1-yl]propyl]acetamide ;
Composé 271: N-[(2S)-2-[5-(2-chloro-3-fluoro-phenyl)-3-[2-[4-(4-fluoro-7-methoxy-2-oxo-4,5-dihydro-1H-1,3-benzodiazepin-3-yl)-1-piperidyl]-2-oxo-ethyl]-2,6-dioxo-pyrimidin-1-yl]propyl]acetamide ;
Composé 272: N-[(2S)-2-[5-(2-chloro-3-fluoro-phenyl)-3-[2-[2,6-dimethyl-4-(2-oxo-4,5-dihydro-1H-1,3-benzodiazepin-3-yl)-1-piperidyl]-2-oxo-ethyl]-2,6-dioxo-pyrimidin-1-yl]propyl]acetamide ;
Composé 273: N-[(2S)-2-[5-(2-chloro-3-fluoro-phenyl)-3-[2-[3,5-dimethyl-4-(2-oxo-4,5-dihydro-1H-1,3-benzodiazepin-3-yl)-1-piperidyl]-2-oxo-ethyl]-2,6-dioxo-pyrimidin-1-yl]propyl]acetamide ;
Composé 274: N-[(S)-2-(5-(2-Chloro-3-fluoro-phenyl)-3-{1-fluoro-2-[4-(7-methoxy-2-oxo-1,2,4,5-tetrahydro-benzo[d][1,3]diazepin-3-yl)-piperidin-l-yl]-2-oxo-ethyl}-2,6-dioxo-3,6-dihydro-2H-pyrimidin-1-yl)-propyl]-acetamide ;
Composé 275: N-[(S)-2-(5-(2-Chloro-3-fluoro-phenyl)-4-fluoro-3-{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide ;
Composé 276: N-[(S)-2-(5-(2-Chloro-3-fluoro-phenyl)-3-{2-[3-fluoro-4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide ;
Composé 277 : N-[(S)-2-(5-(2-Chloro-3-methoxy-phenyl)-3-{2-[4-(7-methoxy-2,5-dioxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamide.

Les composés sont encore plus préférentiellement choisis parmi les composés de formule générale (I) dans laquelle :
- Y est -C(O) ;
- R1 est 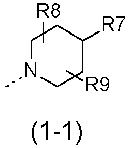
- R2, R3, R6 sont un atome d'hydrogène ;
- R4 est 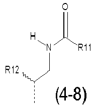
- R5 est
- R7 est 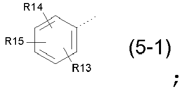 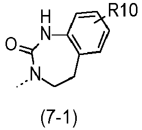
- R8, R9 sont un atome d'hydrogène ;
- R10 est -OR11;
- R11, R12 sont identiques ou différents, et choisis parmi -CH3 ou -CH2CH3;
- R13, R14, R15 sont identiques ou différents, et choisis parmi un atome d'hydrogène, -OR16, un halogène choisi parmi F et Cl; et
- R16 est un alkyle C1-C3.

A cet effet, les composés sont encore plus préférentiellement choisis parmi les composés listés dans le tableau I ci-après et pour lesquels, l'activité antagoniste au récepteur CGRP, définie par le Kd apparent ou Kdapp, est inférieure à 10nM (indiquée classe A dans le tableau I)

Parmi les composés, lorsque Y est -C(O), les composés sont avantageusement des dérivés uraciles.

Selon un mode particulièrement préféré, les composés sont choisis parmi les composés de formule générale (I) dans laquelle :
- Y est -C(O) ;
- R1 est choisi parmi les groupements suivants : 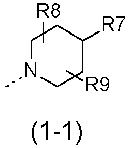
- R2, R3, R6 sont un atome d'hydrogène ;
- R4 est choisi parmi les groupements suivants : 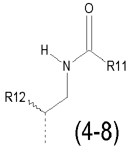
- R5 est 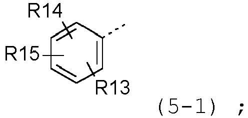
- R7 est 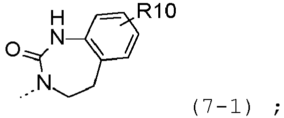
- R8, R9 sont un atome d'hydrogène ;
- R10 est -OR11;
- R11, R12 sont identiques ou différents, et choisis parmi -CH3 ou -CH2CH3 ;
- R13, R14, R15 sont identiques ou différents, et choisis parmi un atome d'hydrogène, -OR16, ou un halogène choisi parmi F et Cl ; et
- R16 est un alkyle C1-C3.

Selon l'invention, les composés sont choisis parmi les composés de formule générale (I) dans laquelle :
- Y est -C(O) ;
- R1 est 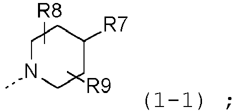
- R2, R3, R6 sont un atome d'hydrogène ;
- R4 est 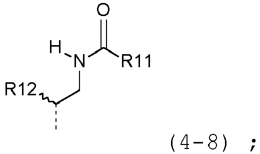
- R5 est 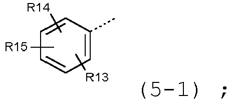
- R7 est 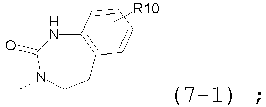
- R8, R9 sont un atome d'hydrogène ;
- R10 est -OR11 ;
- R11, R12 sont identiques ou différents, et choisis parmi -CH₃ ou -CH₂CH₃ ;
- R13, R14, R15 sont identiques ou différents, et choisis parmi un atome d'hydrogène, -OR16, ou un halogène choisi parmi F et Cl ; et
- R16 est un alkyle C1-C3.

Il va être décrit, à titre d'illustration et sans aucun caractère limitatif, divers exemples de préparation des composés décrits dans la présnete divulgation et des composés selon l'invention.

Les composés peuvent être obtenus de manière non limitative en utilisant des conditions classiques en synthèse organique selon les voies réactionnelles générales décrites dans les schémas réactionnels n°1, 2, 3 et 4 des figures respectivement 1, 2, 3 et 4. Les conditions experimentales relatives à chaque schéma de synthèse sont divulguées dans les exemples ci-dessous qui renvoient à chaque fois au schéma suivi pour leur préparation. Ainsi, l'homme du métier saura apprécier si les conditions décrites dans ces schémas sont adaptées pour l'introduction des groupements fonctionnels voulus et éventuellement il adaptera la voie de synthèse par l'utilisation de groupements protecteurs adaptés, stables dans les conditions réactionnelles. Certains intermédiaires, permettant l'introduction de certains groupements R1 envisagés dans nos composés, sont obtenus au préalable en suivant des méthodes d'obtention déjà décrites dans la littérature ou décrites dans les exemples ci-après.

Plus particulièrement, les exemples ci-dessous décrivent de façon non limitative les modes opératoires suivis pour l'obtention des composés selon les schémas de synthèse n°1, 2, 3 ou 4. Chaque composé est principalement caractérisé par son spectre de RMN ¹H réalisé sur un spectromètre à résonance magnétique nucléaire Bruker 400MHz.

### Exemple 1 : 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 1) (example illustratif)

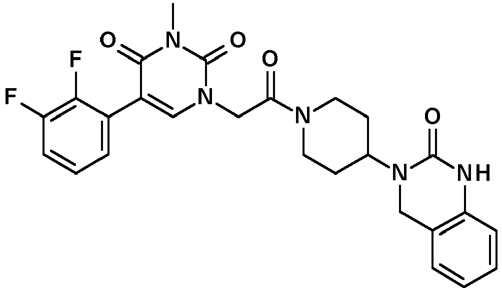

### 1.1: [2-(1-benzyl-pipéridin-4-ylcarbamoyl)-phényl]-carbamate de tert-butyle

8,9g (46,3 mmoles) de chorohydrate de 1-éthyl-3-(3-diméthylaminopropyl) carbodiimide sont additionnés à une solution de 10g (42,1 mmoles) d'acide 2-tert-butoxycarbonylamino-benzoïque, 10,3ml (50,6 mmoles) de 1-benzyl-pipéridin-4-ylamine, 6,3g (46,3 mmoles) de 1-hydroxybenzotriazole et 17,6ml (126,3 mmoles) de triéthylamine, dans 120ml de diméthylformamide. Le milieu réactionnel est ensuite agité avant l'addition de. Le milieu réactionnel est chauffé à 70°C pendant 5 heures puis hydrolysé par une solution aqueuse d'hydrogénocarbonate de sodium et dillué par de l'acétate d'éthyle. Le produit est extrait à l'acétate d'éthyle. La phase organique est lavée deux fois par une solution aqueuse saturée d'hydrogénocarbonate de sodium puis par une solution aqueuse saturée de chlorure de sodium et par de l'eau, séchée sur sulfate de magnésium et filtrée. Les solvants sont concentrés sous vide. 17g (100%) de [2-(1-benzyl-pipéridin-4-ylcarbamoyl)-phényl]-carbamate de tert-butyle sont obtenus sous la forme d'un solide beige.

### 1.2: 2-amino-N-(1-benzyl-pipéridin-4-yl)-benzamide

30mL (415 mmoles) d' acide trifluoroacétique sont ajoutés goutte à goutte à une solution de 17g (41,5 mmoles) de 2-(1-benzyl-pipéridin-4-ylcarbamoyl)-phényl]-carbamate de tert-butyle dans 170ml de dichlorométhane préalablement refroidie à 0°C. Le milieu réactionnel est agité de 0°C à température ambiante pendant 20 heures. Après concentration sous vide, le résidu est hydrolysé par une solution aqueuse d'hydrogénocarbonate de sodium puis dillué par de l'acétate d'éthyle. Le produit est extrait deux fois par de l'acétate d'éthyle. La phase organique est lavée une fois par de l'eau puis une fois par une solution aqueuse saturée de chlorure de sodium, séchée sur sulfate de magnésium, filtrée et concentrée sous vide. 14 g (100%) de 2-amino-N-(1-benzyl-pipéridin-4-yl)-benzamide sont obtenus sous la forme d'un solide beige.

### 1.3: (2-amino-benzyl)-(1-benzyl-pipéridin-4-yl)-amine

12g (38,8 mmoles) de 2-amino-N-(1-benzyl-pipéridin-4-yl)-benzamide dilués dans 72ml de dioxane sont ajoutés très lentement à une solution de 5,2g (135,7 mmoles) d'hydrure de lithium aluminium dans 260ml de dioxane, préalablement chauffé à reflux. Le milieu réactionnel (suspension grise) est ensuite agité et chauffé à reflux pendant 3 heures. Le mélange est refroidi à 0°C puis hydrolysé lentement par 5,2ml de soude aqueuse 15M et 15,5ml d'eau. Le milieu réactionnel est ensuite dillué par 240ml d'ether diéthylique et agité à température ambiante pendant 55 minutes. Après filtration des sels, le filtrat est concentré sous vide pour donner 10,7g (93%) de (2-amino-benzyl)-(1-benzyl-pipéridin-4-yl)-amine sous la forme d'une huile claire.

### 1.4: 3-(1-benzyl-pipéridin-4-yl)-3,4-dihydro-1H-quinazolin-2-one

0,9g (5,7 mmoles) de carbonyl diimidazole sont additionnés à une solution de 1,6g (5,2 mmoles) de (2-amino-benzyl)-(1-benzyl-pipéridin-4-yl)-amine dans 25ml de tétrahydrofurane. Le milieu réactionnel est agité à température ambiante pendant 5 heures. Le solvant est éliminé sous vide puis le milieu réactionnel est hydrolysé et dillué par de l'acétate d'éthyle. Après extraction à l'acétate d'éthyle, les phases organiques sont lavées deux fois par de l'eau puis par une solution aqueuse saturée de chlorure de sodium, séchées sur sulfate de magnésium, filtrées et concentrées sous vide. Le solide brut obtenu est trituré dans 15ml d'éther diéthylique puis filtré et séché sous vide pour donner 1,2g (72%) de 3-(1-benzyl-pipéridin-4-yl)-3,4-dihydro-1H-quinazolin-2-one sous la forme d'un solide blanc.

### 1.5: 3-pipéridin-4-yl-3,4-dihydro-1H-quinazolin-2-one

120mg de palladium sur charbon (10% massique) sont ajoutés à une solution de 1,2g (3,7 mmoles) de 3-(1-benzyl-pipéridin-4-yl)-3,4-dihydro-1H-quinazolin-2-one dans 30ml de méthanol, préalablement dégazée avec de l'azote. Le milieu est ensuite placé sous une atmosphère de dihydrogène pendant 48 heures puis filtré sur célite. Le filtrat est concentré sous vide pour donner 0,9g (100%) de 3-pipéridin-4-yl-3,4-dihydro-1H-quinazolin-2-one sous la forme d'un solide blanc.

### 1.6: 5-bromo-1-{2-oxo-2-[4-(2-oxo-1,4-dihydro-2H-quinazolin-3-yl)-pipéridin-1-yl]-éthyl}-1H-pyrimidine-2,4-dione

326mg (2,4 mmoles) de 1-hydroxybenzotriazole et 462mg (2,4 mmoles) de chorohydrate de 1-éthyl-3-(3-diméthylaminopropyl)carbodiimide sont additionnés à une solution de 500mg (2 mmoles) d'acide (5-bromo-2,4-dioxo-3,4-dihydro-2H-pyrimidin-1-yl)-acétique dans 12ml de diméthylformamide. Après agitation à température ambiante pendant 10 minutes, 560mg (2,4 mmoles) de 3-pipéridin-4-yl-3,4-dihydro-1H-quinazolin-2-one sont ajoutés. Le milieu réactionnel est ensuite agité à température ambiante pendant 18 heures puis hydrolysé par une solution aqueuse d'hydrogénocarbonate de sodium et dilué par 20ml d'acétate d'éthyle. Le produit est extrait à l'acétate d'éthyle puis au n-butanol. Les phases organiques sont rassemblées, lavées une fois par la solution aqueuse saturée d'hydrogénocarbonate de sodium et une fois par une solution aqueuse saturée de chlorure de sodium, séchées sur sulfate de magnésium, filtrées et concentrées sous vide. Le résidu brut est repris dans un mélange heptane/acétate d'éthyle puis filtré et séché sous azote. 900mg (97%) de 5-bromo-1-{2-oxo-2-[4-(2-oxo-1,4-dihydro-2H-quinazolin-3-yl)-pipéridin-1-yl]-éthyl}-1H-pyrimidine-2,4-dione sont obtenus sous forme d'un solide blanc cassé.

### 1.7: 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione

143mg (1mmole) de carbonate de potassium et 0,1ml (1,3 mmoles) de iodure de méthyle sont additionnés à une solution de 400mg (0,9 mmoles) de 5-bromo-1-{2-oxo-2-[4-(2-oxo-1,4-dihydro-2H-quinazolin-3-yl)-pipéridin-1-yl]-éthyl}-1H-pyrimidine-2,4-dione dans 12ml de diméthylformamide. Le milieu réactionnel est agité à température ambiante 3 heures puis hydrolysé et dilué par de l'acétate d'éthyle. Le produit est extrait à l'acétate d'éthyle. Les phases organiques sont lavées une fois par de l'eau, puis par une solution aqueuse saturée de chlorure de sodium, séchées sur sulfate de magnésium, filtrées et concentrés sous vide. Le résidu brut est purifié par chromatographie sur couche mince préparative, éluée par un mélange dichlorométhane / méthanol 90/10. 160mg (39%) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous forme d'un solide jaune pâle.

### 1.8: 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (composé 1)

19mg (0,01 mmoles) de tétrakis(triphénylphosphine)palladium(0) et 265mg (1,7 mmoles) d'acide 2,3-difluorophénylboronique sont ajoutés à une solution de 160mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione et 0,5ml (1 mmole) de solution aqueuse de carbonate de potassium 2M dans 12ml de diméthylformamide et 2ml d'eau, préalablement dégazée avec de l'azote. Le mélange réactionnel est chauffé à 90°C pendant 2 heures. La réaction est traitée par addition d'eau puis le produit est extrait trois fois à l'acétate d'éthyle. La phase organique est lavée par de l'eau puis par une solution saturée de chlorure de sodium, séchée sur sulfate de magnésium, filtrée et concentrée sous vide. Le résidu brut obtenu est purifié par chromatographie sur gel de silice élué avec un mélange dichlorométhane/méthanol 95/5. 60mg (36%) de 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous forme d'un solide blanc de point de fusion 170°C.
RMN ¹H (δ, DMSO) : 1.56 - 1.75 (m, 3H), 1.76 - 1.91 (m, 1H), 2.70 (t, J = 12.8 Hz, 1H), 3.17 (t, J = 12.8 Hz, 1H), 3.26 (s, 3H), 3.98 (d, J = 13.5 Hz, 1H), 4.30 (d, J = 3.9 Hz, 2H), 4.35 - 4.51 (m, 2H), 4.81 (s, 2H), 6.77 (d, J = 8.0 Hz, 1H), 6.86 (t, J = 7.5 Hz, 1H), 7.07 - 7.17 (m, 2H), 7.18 - 7.32 (m, 2H), 7.40 - 7.53 (m, 1H), 7.94 (s, 1H), 9.27 (s, 1H).

### Exemple 2: 5-(2-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 2, composé 2) (example illustratif)

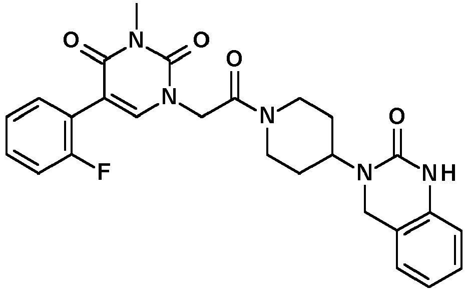

### 2.1: 1-benzhydryl-5-bromo-1H-pyrimidine-2,4-dione

6,4mL (26,2 mmoles) de N,O-bis(triméthylsilyl)acétamide sont additionnés sur une solution de 2g (10,5 mmoles) de 5-bromo-1H-pyrimidine-2,4-dione dans 40ml d' acétonitrile. Le milieu réactionnel est agité jusqu'à obtenir une solution limpide puis 3,9g (15,8 mmoles) de bromure de benzhydryle et 267mg (1,1 mmoles) d'iode sont rajoutés. Le milieu réactionnel est chauffé à reflux pendant 5 heures puis laissé à température ambiante pendant 18 heures. Les solvants sont concentrés sous vide puis le résidu est repris dans 50ml d'acétate d'éthyle et 50ml d'eau. La phase organique est lavée une fois par de l'eau puis par une solution aqueuse saturée de chlorure de sodium, séchée sur sulfate de magnésium, filtrée et concentrée sous vide.Le produit brut est chromatographié sur gel de silice élué par un mélange heptane / acétate d'éthyle 70/30 puis 50/50. 2,5g (67%) de 1-benzhydryl-5-bromo-1H-pyrimidine-2,4-dione sont obtenus sous forme d'un solide jaune.

### 2.2: 1-benzhydryl-5-bromo-3-méthyl-1H-pyrimidine-2,4-dione

290mg (2,1 mmoles) de carbonate de potassium et 0,1ml (2,1 mmoles) de iodure de méthyle sont additionnés à une solution de 500mg (1,4 mmoles) de 1-benzhydryl-5-bromo-1H-pyrimidine-2,4-dione dans 10ml de diméthylformamide. Le milieu réactionnel est agité à température ambiante pendant 2 heures puis hydrolysé et dilué par de l'acétate d'éthyle. Le produit est extrait à l'acétate d'éthyle et la phase organique est lavée une fois par de l'eau, une fois par une solution aqueuse saturée de chlorure de sodium puis séchée sur sulfate de magnésium, filtrée et concentrée sous vide. 490mg (94%) de 1-benzhydryl-5-bromo-3-méthyl-1H-pyrimidine-2,4-dione sont obtenus sous forme d'un solide jaune.

### 2.3: 5-bromo-3-méthyl-1H-pyrimidine-2,4-dione

490mg (1,3 mmoles) de 1-benzhydryl-5-bromo-3-méthyl-1H-pyrimidine-2,4-dione sont additionnés sur une solution refroidie à 0°C contenant 2ml d'acide méthanesulfonique et 4,9ml d'acide trifluoroacétique. Le milieu réactionnel est agité à 0°C puis à température ambiante pendant 43 heures. Il est versé lentement sur 50g de glace puis 150ml d'une solution aqueuse saturée d'hydrogénocarbonate de sodium sont additionnés. Le produit est extrait trois fois à l'acétate d'éthyle. Les phases organiques sont lavées une fois par de l'eau, puis par une solution aqueuse saturée de chlorure de sodium et concentrées sous vide. Le résidu brut est chromatographié sur gel de silice élué par un mélange dichlorométhane / méthanol 97/3. 195mg (72%) de 5-bromo-3-méthyl-1H-pyrimidine-2,4-dione sont obtenus sous forme d'un solide jaune pâle.

### 2.4: 3-[1-(2-hydroxy-acétyl)-pipéridin-4-yl]-3,4-dihydro-1H-quinazolin-2-one

212mg (1,6 mmoles) de 1-hydroxybenzotriazole et 307mg (1,6 mmoles) de chorohydrate de 1-éthyl-3-(3-diméthylaminopropyl)carbodiimide sont additionnés à une solution de 100mg (1,3 mmoles) d'acide hydroxyacétique dans 10ml de diméthylformamide. Le milieu réactionnel est agité pendant 5 minutes à température ambiante et 304mg (1,6 mmoles) de 3-pipéridin-4-yl-3,4-dihydro-1H-quinazolin-2-one (préparé comme décrit dans l'exemple 1.5) sont ajoutés. Après deux heures d'agitation à température ambiante, le milieu réactionnel est hydrolysé par une solution aqueuse saturée d'hydrogénocarbonate de sodium et dilué par de l'acétate d'éthyle. Le produit est extrait deux fois à l'acétate d'éthyle et une fois au n-butanol. Les phases organiques sont rassemblées, lavées une fois avec de l'eau et une fois par une solution aqueuse saturée de chlorure de sodium. Elles sont séchées sur sulfate de magnésium, filtrées et concentrées sous vide. Le résidu brut est chromatographié sur gel de silice élué par un mélange dichlorométhane méthanol 97/3. 140mg (35%) de 3-[1-(2-hydroxy-acétyl)-pipéridin-4-yl]-3,4-dihydro-1H-quinazolin-2-one sont obtenus sous forme d'un solide blanc.

### 2.5: 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione

0,2ml (1,2 mmoles) de diisopropyl azodicarboxylate sont additionnés à une solution de 100mg (0,5 mmoles) de 5-bromo-3-méthyl-1H-pyrimidine-2,4-dione, 140mg (0,5 mmoles) de 3-[1-(2-hydroxy-acétyl)-pipéridin-4-yl]-3,4-dihydro-1H-quinazolin-2-one et 321mg (1,2 mmoles) de triphénylphosphine dans 10ml de tétrahydrofurane. Le milieu réactionnel est agité à température ambiante pendant 18 heures. 15ml d'éther diéthylique sont additionnés, la suspension est agitée pendant 10 minutes puis filtrée. Le solide obtenu est rincé une fois à l'éther diéthylique puis séché à l'étuve sous vide pendant 3 heures. 160mg (74%) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc.

### 2.6: 5-(2-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (composé 2)

De manière analogue à l'exemple 1.8, à partir de 62mg (0,4 mmoles) d'acide 2-fluorophénylboronique et 140mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione, 70mg (50%) de 5-(2-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous forme d'un solide blanc.
RMN ¹H (δ, DMSO) : 1.57 - 1.70 (m, 3H), 1.83 (q, J = 12.4 Hz, 1H), 2.70 (t, J = 12.6 Hz, 1H), 3.16 (t, J = 12.6 Hz, 1H), 3.25 (s, 3H), 3.97 (d, J = 13.7 Hz, 1H), 4.30 (d, J = 4.1 Hz, 2H), 4.37 - 4.51 (m, 2H), 4.80 (s, 2H), 6.77 (dd, J = 7.9, 1.2 Hz, 1H), 6.86 (t, J = 7.5 Hz, 1H), 7.06 - 7.16 (m, 2H), 7.20 - 7.32 (m, 2H), 7.36 - 7.48 (m, 2H), 7.86 (s, 1H), 9.26 (s, 1H).

### Exemple 3: 5-(2,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 3) (example comparatif)

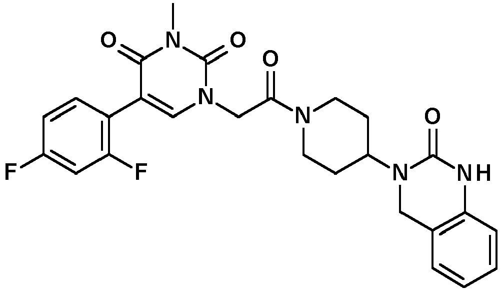

De manière analogue à l'exemple 1.8, à partir de 249mg (1,6 mmoles) d'acide 2,4-difluorophénylboronique et de 150mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5), 80mg (51%) de 5-(2,4-difluoro-phényl)-3-méthyl-1-{2-oxo-2-[4-(2-oxo-1,4-dihydro-2H-quinazolin-3-yl)-pipéridin-1-yl]-éthyl} -1H-pyrimidine-2,4-dione sont obtenus sous la forme d'un solide blanc de point de fusion 170°C.
RMN ¹H (δ, DMSO) : 1.54 - 1. 72 (m, 3H), 1. 83 (q, J = 13.7 Hz, 1H), 2.69 (t, J = 12.1 Hz, 1H), 3.16 (t, J = 12.6 Hz, 1H), 3.25 (s, 3H), 3.97 (d, J = 13.5 Hz, 1H), 4.29 (d, J = 4.0 Hz, 2H), 4.42 (m, 2 H), 4.79 (s, 2H), 6.77 (d, J = 7.8 Hz, 1H), 6.86 (t, J = 7.5 Hz, 1H), 7.04 - 7.23 (m, 3H), 7.33 (t, J = 9.8 Hz, 1H), 7.43 (q, J = 8.6 Hz, 1H), 7.85 (s, 1H), 9.26 (s, 1H).

### Exemple 4: 5-(3-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 4) (example comparatif)

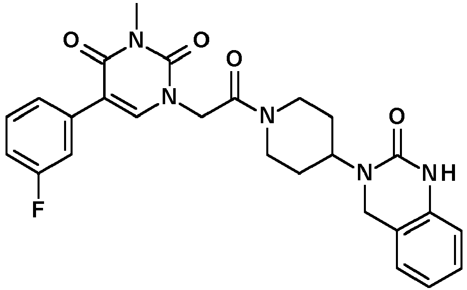

De manière analogue à l'exemple 1.8, à partir de 66mg (0,5 mmoles) d'acide 3-fluorophénylboronique et de 150mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5), 30mg (20%) de 5-(3-fluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc de point de fusion 240°C.
RMN ¹H (δ, DMSO) : 1.54 - 1.73 (m, 3H), 1.77 - 1.92 (m, 1H), 2.70 (t, J = 12.0 Hz, 1H), 3.19 (t, J = 13.0 Hz, 1H), 3.27 (s, 3H), 3.99 (d, J = 13.5 Hz, 1H), 4.31 (d, J = 4.0 Hz, 2H), 4.37 - 4.52 (m, 2H), 4.82 (d, J = 3.7 Hz, 2H), 6.77 (d, J = 7.9 Hz, 1H), 6.87 (t, J = 7.5 Hz, 1H), 7.05 - 7.23 (m, 3H), 7.39 - 7.51 (m, 3H), 8.07 (s, 1H), 9.27 (s, 1H).

### Exemple 5: 3-méthyl-1-{2-oxo-2-[4-(2-oxo-1,4-dihydro-2H-quinazolin-3-yl)-pipéridin-1-yl]-éthyl}-5-(1H-pyrazol-3-yl)-1H-pyrimidine-2,4-dione (schéma réactionnel 1, composé 5) (example comparatif)

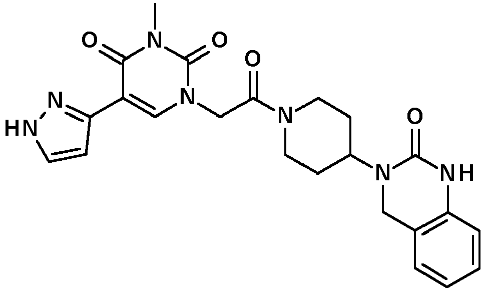

De manière analogue à l'exemple 1.8, à partir de 70mg (0,6 mmoles) d'acide 1H-pyrazol-3-ylboronique, 150mg (0,3 mmole) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5), 131mg (1,2 mmoles) de carbonate de sodium et 13mg (0,02 mmole) de complexe de dichlorométhane de 1,1'-bis-(diphénylphosphino)ferrocène-dichloropalladium(II); 10mg (7%) de 3-méthyl-1-{2-oxo-2-[4-(2-oxo-1,4-dihydro-2H-quinazolin-3-yl)-pipéridin-1-yl]-éthyl} -5-(1H-pyrazol-3-yl)-1H-pyrimidine-2,4-dione sont obtenus sous la forme d'un solide blanc.

Analyse HPLC (colonne Kinetex C18, 150x3mm, 2,6µm, éluant: eau/acétonitrile avec 0,1% acide formique, run de 30min): tr = 12.64min.

### Exemple 6: 5-(3-méthoxy-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 6) (example comparatif)

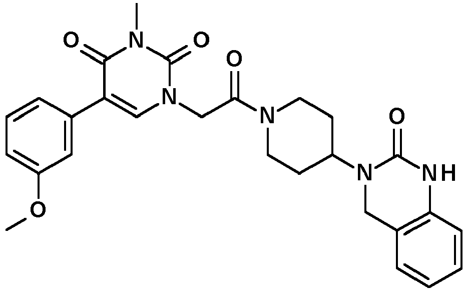

De manière analogue à l'exemple 1.8, à partir de 71mg (0,5 mmoles) d'acide 3-méthoxyphénylboronique et de 150mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5), 120mg (77%) de 5-(3-méthoxy-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc de point de fusion 251°C.
RMN ¹H (δ, DMSO) : 1.55 - 1.74 (m, 3H), 1.84 (q, J = 12.3 Hz, 1H), 2.62 - 2.76 (m, 1H), 3.19 (t, J = 13.0 Hz, 1H), 3.26 (s, 3H), 3.78 (s, 3H), 3.93 - 4.03 (m, 1H), 4.31 (d, J = 3.9 Hz, 2H), 4.42 - 4.46 (m, 2 H), 4.81 (d, J = 3.8 Hz, 2H), 6.77 (d, J = 7.8 Hz, 1H), 6.82 - 6.94 (m, 2H), 7.06 - 7.20 (m, 4H), 7.32 (t, J = 8.0 Hz, 1H), 7.96 (s, 1H), 9.27 (s, 1H).

### Exemple 7: 5-(3-hydroxy-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 7) (example comparatif)

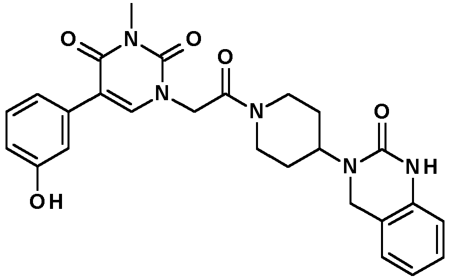

De manière analogue à l'exemple 1.8, à partir de 217mg (1,6 mmole) d'acide 3-hydroxyphénylboronique et de 150mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5), 30mg (20%) de 5-(3-hydroxy-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide gris.
RMN ¹H (δ, DMSO) : 1.56 - 1.72 (m, 3H), 1.77 - 1.89 (m, 1H), 2.64 - 2.75 (m, 1H), 3.12 - 3.23 (m, 1H), 3.26 (s, 3H), 3.98 (d, J = 13.2 Hz, 1H), 4.31 (d, J = 4.0 Hz, 2H), 4.38 - 4.49 (m, 2H), 4.81 (s, 2H), 6.69 - 6.80 (m, 2H), 6.87 (t, J = 7.4 Hz, 1H), 6.94 (d, J = 7.7 Hz, 1H), 7.02 (d, J = 2.0 Hz, 1H), 7.08 - 7.24 (m, 3H), 7.88 (s, 1H), 9.26 (s, 1H), 9.44 (s, 1H).

### Exemple 8: 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 8) (example comparatif)

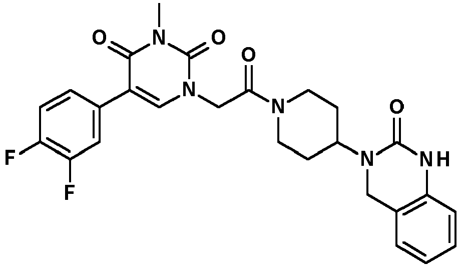

De manière analogue à l'exemple 1.8, à partir de 245mg (1,6 mmole) d'acide 3,4-difluorophénylboronique et de 150mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5). Après cristallisation dans un mélange heptane / acétate d'éthyle 70/30, filtration et séchage sous vide pendant 24 heures, 40mg (25%) de 5-(3,4-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide beige de point de fusion 153°C.
RMN ¹H (δ, DMSO) : 1.55 - 1.74 (m, 3H), 1.77 - 1.90 (m, 1H), 2.64 - 2.77 (m, 1H), 3.19 (t, J = 13.0 Hz, 1H), 3.27 (s, 3H), 3.99 (d, J = 13.1 Hz, 1H), 4.31 (d, J = 3.9 Hz, 2H), 4.36 - 4.52 (m, 2H), 4.81 (d, J = 4.5 Hz, 2H), 6.77 (d, J = 7.8 Hz, 1H), 6.87 (t, J = 7.5 Hz, 1H), 7.11 (d, J = 8.0 Hz, 2H), 7.40 - 7.56 (m, 2H), 7.60 - 7.74 (m, 1H), 8.06 (s, 1H), 9.27 (s, 1H).

### Exemple 9: 5-cyclohex-1-ényl-3-méthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 2, composé 9) (example comparatif)

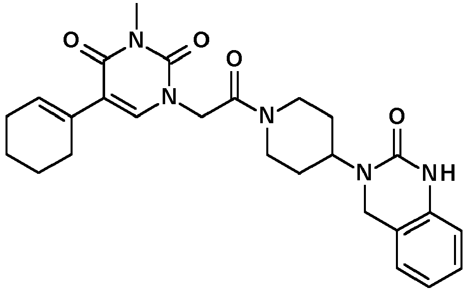

50mg (0,1 mmole) de 5-bromo-3-méthyl-1-{2-oxo-2-[4-(2-oxo-1,4-dihydro-2H-quinazolin-3-yl)-pipéridin-1-yl]-éthyl} -1H-pyrimidine-2,4-dione (préparé comme décrit dans l'exemple 2.5), 33,9µl (0,16 mmole) de 1-cyclohexen-yl-boronate de pinacole, 30,1µ1 (0,2 mmole) d'une solution 5,2M de méthoxyde de sodium et 3,7mg (0,01 mmole) de chlorure de bis(triphénylphosphine palladium(II) sont placés en solution dans 3ml de méthanol et chauffés à 70°C dans un tube scellé sous micro-ondes pendant 5 minutes. Le milieu réactionnel est hydrolysé puis dilué par de l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le produit brut est chromatographié sur gel de silice élué par un mélange dichlorométhane / méthanol 99/1 puis repris dans un mélange heptane / acétate d'éthyle 80/20 et filtré. Le solide obtenu est séché sous vide pendant 72 heures pour donner 35mg (65%) de 5-cyclohex-1-ényl-3-méthyl-1-{2-oxo-2-[4-(2-oxo-1,4-dihydro-2H-quinazolin-3-yl)-pipéridin-1-yl]-éthyl}-1H-pyrimidine-2,4-dione sous la forme d'un solide beige.
RMN ¹H (δ, DMSO) : 1.56-1.68 (m, 7H), 1.75-1.90 (m, 1H), 2.10-2.18 (m, 2H), 2.20-2.30 (m, 2H), 2.49-2.51 (m, 1H), 3.15-3.17 (m, 1H), 3.18 (s, 3H), 3.90-4.00 (m, 1H), 4.30 (d, J = 4.4 Hz, 2H), 4.35-4.48 (m, 2H), 4.74 (s, 2H), 6.17 (m, 1H), 6.77 (d, J = 7.7 Hz, 1H), 6.87 (m, 1H), 7.11 (d, J = 7.5 Hz, 2H), 7.53 (s, 1H), 9.26 (s, 1H).

### Exemple 10: 3-méthyl-1-{2-oxo-2-[4-(2-oxo-1,4-dihydro-2H-quinazolin-3-yl)-pipéridin-1-yl]-éthyl}-5-phényl-1H-pyrimidine-2,4-dione (schéma réactionnel 1, composé 10) (example comparatif)

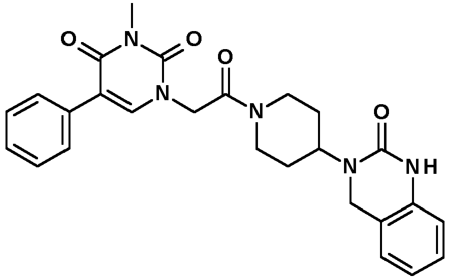

De manière analogue à l'exemple 1.8, à partir de 61mg (0,5 mmoles) d'acide phénylboronique et de 160mg (0,3 mmoles) de 5-bromo-3-méthyl-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 2.5), 110mg (70%) de 3-méthyl-1-{2-oxo-2-[4-(2-oxo-1,4-dihydro-2H-quinazolin-3-yl)-pipéridin-1-yl]-éthyl}-5-phényl-1H-pyrimidine-2,4-dione sont obtenus sous la forme d'un solide blanc de point de fusion 220°C.
RMN ¹H (δ, DMSO) : 1.57 - 1.73 (m, 3H), 1.77 - 1.90 (m, 1H), 2.70 (t, J = 12.7 Hz, 1H), 3.18 (t, J = 12.5 Hz, 1H), 3.27 (s, 3H), 3.99 (d, J = 13.5 Hz, 1H), 4.31 (d, J = 3.8 Hz, 2H), 4.37 - 4.49 (m, 2H), 4.82 (d, J = 2.5 Hz, 2H), 6.78 (d, J = 8 Hz, 1H), 6.87 (t, J = 7.4 Hz, 1H), 7.08 - 7.14 (m, 2H), 7.29 - 7.36 (m, 1H), 7.38 - 7.45 (m, 2H), 7.51 - 7.58 (m, 2H), 7.93 (s, 1H), 9.27 (s, 1H).

### Exemple 11: 5-(2,3-fluoro-phényl)-1-{2-oxo-2-[4-(2-oxo-1,4-dihydro-2H-quinazolin-3-yl)-pipéridin-1-yl]-éthyl}-3-(2,2,2-trifluoro-éthyl)-1H-pyrimidine-2,4-dione (schéma réactionnel 1, composé 11) (example comparatif)

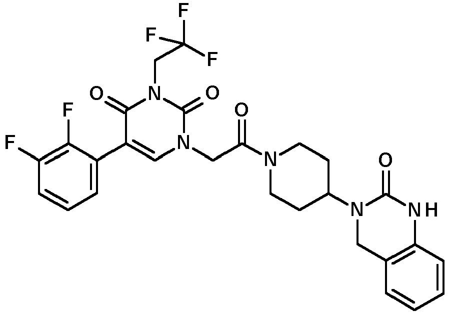

### 11.1: 5-bromo-1-{2-oxo-2-[4-(2-oxo-1,4-dihydro-2H-quinazolin-3-yl)-pipéridin-1-yl]-éthyl}-3-(2,2,2-trifluoro-éthyl)-1H-pyrimidine-2,4-dione

De manière analogue à l'exemple 1.7, à partir de 230mg (0,5 mmoles) de 5-bromo-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 1.6) et de 0,1ml (0.8 mmoles) de 1,1,1-trifluoro-2-iodo-éthane, 140mg (51%) de 5-bromo-1-{2-oxo-2-[4-(2-oxo-1,4-dihydro-2H-quinazolin-3-yl)-pipéridin-1-yl]-éthyl}-3-(2,2,2-trifluoro-éthyl)-1H-pyrimidine-2,4-dione sont obtenus sous la forme d'un solide blanc.

### 11.2: -5-(2,3-fluoro-phényl)-1-{2-oxo-2-[4-(2-oxo-1,4-dihydro 2H-quinazolin-3-yl)-pipéridin-1-yl]-éthyl}-3-(2,2,2-trifluoro-éthyl)-1H-pyrimidine-2,4-dione (composé 11)

De manière analogue à l'exemple 1.8, à partir de 203mg (1,3 mmoles) d'acide 2,3-difluorophénylboronique et de 140mg (0,3 mmoles) de 5-bromo-1-{2-oxo-2-[4-(2-oxo-1,4-dihydro-2H-quinazolin-3-yl)-pipéridin-1-yl]-éthyl}-3-(2,2,2-trifluoro-éthyl)-1H-pyrimidine-2,4-dione, 75mg (50%) de 5-(2,3-fluoro-phényl)-1-{2-oxo-2-[4-(2-oxo-1,4-dihydro-2H-quinazolin-3-yl)-pipéridin-1-yl]-éthyl}-3-(2,2,2-trifluoro-éthyl)-1H-pyrimidine-2,4-dione sont obtenus sous la forme d'un solide blanc cassé.
RMN ¹H (δ, DMSO) : 1.55 - 1.75 (m, 3H), 1.76 - 1.92 (m, 1H), 2.71 (t, J = 12.8 Hz, 1H), 3.17 (t, J = 12.8 Hz, 1H), 3.97 (d, J = 13.4 Hz, 1H), 4.30 (d, J = 3.5 Hz, 2H), 4.37 - 4.52 (m, 2H), 4.74 (q, J = 9.1 Hz, 2H), 4.85 (d, J = 2.8 Hz, 2H), 6.77 (d, J = 7.8 Hz, 1H), 6.86 (t, J = 7.2 Hz, 1H), 7.07 - 7.17 (m, 2H), 7.20 - 7.34 (m, 2H), 7.43 - 7.55 (m, 1H), 8.04 (s, 1H), 9.26 (s, 1H).

### Exemple 12: 5-(2,3-difluoro-phényl)-3-éthyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel 1, composé 12) (example comparatif)

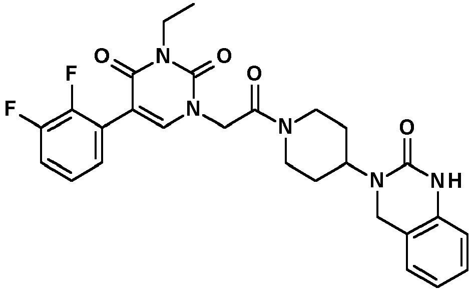

### 12.1: 5-bromo-3-éthyl-1-{2-oxo-2-[4-(2-oxo-1,4-dihydro-2H-quinazolin-3-yl)-pipéridin-1-yl]-éthyl}-1H-pyrimidine-2,4-dione

De manière analogue à l'exemple 1.7, à partir de 240mg (0,5 mmoles) de 5-bromo-1-{2-oxo-2-[4-(2-oxo-1,4-dihydro-2H-quinazolin-3-yl)-pipéridin-1-yl]-éthyl}-1H-pyrimidine-2,4-dione (préparé comme décrit dans l'exemple 1.6) et de 86□L (1.1 mmoles) de iodoéthane, 150mg (59%) de 5-bromo-3-éthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc.

### 12.2: 5-(2,3-difluoro-phényl)-3-éthyl-1-{2-oxo-2-[4-(2-oxo-1,4-dihydro-2H-quinazolin-3-yl)-pipéridin-1-yl]-éthyl} -1H-pyrimidine-2,4-dione (composé 12)

De manière analogue à l'exemple 1.8, à partir de 241mg (1,5 mmoles) d'acide 2,3-difluorophénylboronique et de 150mg (0,3 mmoles) de 5-bromo-3-éthyl-1- {2-oxo-2- [4- (2-oxo-1, 4-dihydro-2H-quinazolin-3-yl)-pipéridin-1-yl]-éthyl}-1H-pyrimidine-2,4-dione, 75mg (48%) de 5-(2,3-difluoro-phényl)-3-éthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc cassé.
RMN ¹H (δ, DMSO) : 1.14 (t, J = 7.0 Hz, 3H), 1.54 - 1.75 (m, 3H), 1.75 - 1.93 (m, 1H), 2.70 (t, J = 12.8, 3.2 Hz, 1H), 3.17 (t, J = 12.9 Hz, 1H), 3.82 - 4.02 (m, 3H), 4.30 (d, J = 4.1 Hz, 2H), 4.37 - 4.53 (m, 2H), 4.80 (s, 2H), 6.77 (d, J = 7.8 Hz, 1H), 6.86 (t, J = 7.4 Hz, 1H), 7.05 - 7.19 (m, 2H), 7.18 - 7.35 (m, 2H), 7.39 - 7.51 (m, 1H), 7.92 (s, 1H), 9.27 (s, 1H) .

### Exemple 13: 5-(2-chloro-3-fluoro-phényl)-3-isopropyl-1-{2-oxo-2-[4-(2-oxo-1,4-dihydro-2H-quinazolin-3-yl)-pipéridin-1-yl]-éthyl}-1H-pyrimidine-2,4-dione (schéma réactionnel n°1, composé 13) (example comparatif)

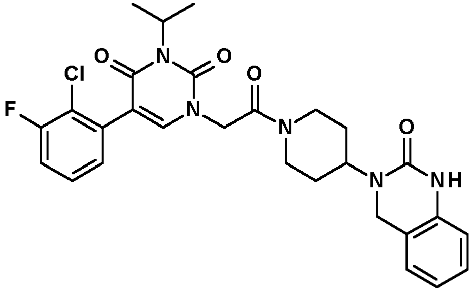

### 13.1: 2-(5-bromo-2,4-dioxo-3,4-dihydro-2H-pyrimidin-1-yl)-acétate de méthyle

12ml (128 mmoles) de bromoacétate de méthyle sont additionnés à une solution de 25g (128 mmoles) de 5-bromouracile et 17,7g (128 mmoles) de carbonate de potassium dans 441ml de diméthylformamide. Le milieu réactionnel est agité à température ambiante pendant 3 heures. Il est hydrolysé et dilué par de l'acétate d'éthyle. La phase aqueuse est neutralisée avec une solution aqueuse d'acide citrique 5% puis le produit est extrait une fois à l'acétate d'éthyle et trois fois au n-butanol. Les phases organiques sont rassemblées, lavées deux fois par de l'eau puis par une solution aqueuse saturée de chlorure de sodium et concentrées sous vide. Le produit brut obtenu est repris dans 150mL d'acétate d'éthyle et chauffé à 80°C pendant 10 minutes. Après avoir été ramenée à température ambiante, la suspension est filtrée et le solide obtenu est rincé à l'éther diéthylique puis séché à l'étuve sous vide pendant 20h. 17g (50%) de (5-bromo-2,4-dioxo-3,4-dihydro-2H-pyrimidin-1-yl)-acétate de méthyle est obtenu sous la forme d'un solide blanc.

### 13.2: (5-bromo-3-isopropyl-2,4-dioxo-3,4-dihydro-2H-pyrimidin-1-yl)-acétate de méthyle

1g (30 mmoles) d'hydrure de sodium est additionné par portions sur une solution refroidie à 0°C de 6,5g (24,7 mmoles) de (5-bromo-2,4-dioxo-3,4-dihydro-2H-pyrimidin-1-yl)-acétate de méthyle dans 130ml de diméthylformamide. Le milieu réactionnel est agité pendant 5 minutes puis 3,5ml (37 mmoles) de 2-bromopropane sont additionnés. Après 30 minutes d'agitation à température ambiante puis 4 heures à 50°C, 2,8ml (30 mmoles) de 2-bromopropane sont additionnés et le milieu réactionnel est chauffé à 50°C pendant 2 heures supplémentaires et 20 heures à température ambiante. Le milieu réactionnel est hydrolysé puis dilué par de l'acétate d'éthyle. La phase organique est lavée une fois par de l'eau et une fois par une solution aqueuse saturée de chlorure de sodium, séchée sur sulfate de magnésium, filtrée et concentrée sous vide. Le résidu brut est chromatographié sur gel de silice élué par un mélange heptane / acétate d'éthyle 50/50. 3,5g (46%) de 2-(5-bromo-3-isopropyl-2,4-dioxo-3,4-dihydro-2H-pyrimidin-1-yl)-acétate de méthyle sont obtenus sous la forme d'un solide blanc cassé.

### 13.3: acide [5-(2-chloro-3-fluoro-phényl)-3-isopropyl-2,4-dioxo-3,4-dihydro-2H-pyrimidin-1-yl]-acétique

80mg (0,1 mmole) de complexe de dichlorométhane de 1,1'-bis-(diphénylphosphino)ferrocene-dichloropalladium(II) sont additionnés sur une solution, préalablement dégazée à l'azote pendant 5 minutes, contenant 600mg (2 mmoles) de 2-(5-bromo-3-isopropyl-2,4-dioxo-3,4-dihydro-2H-pyrimidin-1-yl)-acétate de méthyle, 625mg (5,9 mmoles) de carbonate de sodium et 411mg (2,4 mmoles) d'acide 2-chloro-3-fluorophényl boronique, dans 60ml de 1,4-dioxane et 6ml d'eau. Le milieu réactionnel est chauffé à 100°C pendant 30 minutes puis 343mg (2 mmoles) d'acide 2-chloro-3-fluorophényl boronique sont additionnés. Le mélange est chauffé à 100°C pendant 1 heure supplémentaire. 3ml (3 mmoles) d'une solution aqueuse d'hydroxyde de lithium 1M et 3ml d'eau sont additionnés. Le milieu réactionnel est ramené à température ambiante et agité pendant 1 heure. Il est hydrolysé puis dilué par de l'acétate d'éthyle. La phase aqueuse est amenée à pH acide par une solution aqueuse d'acide chlorhydrique 1N. Le produit est extrait à l'acétate d'éthyle. La phase organique est lavée une fois par de l'eau et une fois par une solution aqueuse saturée de chlorure de sodium, puis séchée sur sulfate de magnésium, filtrée et concentrée sous vide. Le produit est repris dans l'éther diéthylique et concentré sous vide. 680mg (100%) d'acide [5-(2-chloro-3-fluoro-phényl)-3-isopropyl-2,4-dioxo-3,4-dihydro-2H-pyrimidin-1-yl]-acétique sont obtenus sous la forme d'un solide beige.

### 13.4: 5-(2-chloro-3-fluoro-phényl)-3-isopropyl-1-{2-oxo-2-[4-(2-oxo-1,4-dihydro-2H-quinazolin-3-yl)-pipéridin-1-yl]-éthyl}-1H-pyrimidine-2,4-dione (composé 13)

112mg (0,5 mmoles) de 3-pipéridin-4-yl-3,4-dihydro-1H-quinazolin-2-one (préparé comme décrit dans l'exemple 1.5) et 0,1ml (0,5 mmoles) de triéthylamine sont additionnés sur une solution préalablement agitée pendant 5 minutes contenant 150mg (0,4 mmoles) d'acide [5-(2-chloro-3-fluoro-phényl)-3-isopropyl-2,4-dioxo-3,4-dihydro-2H-pyrimidin-1-yl]-acétique , 70mg (0,5 mmoles) de 1-hydroxybenzotriazole et 101,3mg (0,5 mmoles) de chorohydrate de 1-éthyl-3-(3-diméthylaminopropyl)carbodiimide dans 6ml de diméthylformamide. Le milieu réactionnel est agité à température ambiante pendant 14 heures. Le milieu réactionnel est hydrolysé par une solution aqueuse saturée d'hydrogénocarbonate de sodium puis dilué par de l'acétate d'éthyle. La phase organique est lavée une fois par une solution aqueuse saturée d'hydrogénocarbonate de sodium, une fois par de l'eau et une fois par une solution aqueuse saturée de chlorure de sodium, séchée sur sulfate de magnésium puis filtrée et concentrée sous vide. Le résidu brut est purifié par chromatographie sur gel de silice élué par un mélange dichlorométhane / méthanol, 95/5, puis recristallisé dans l'acétate d'éthyle. 39mg (16%) de 5-(2-chloro-3-fluoro-phényl)-3-isopropyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous la forme d'un solide blanc cassé.
RMN ¹H (δ, DMSO) : 1.41 (d, J = 6.9 Hz, 6 H); 1.64-1.68 (m, 3 H); 1.82-1.85 (m, 1 H); 2.67-2.73 (m, 1 H); 3.17 (t, J = 13.1 Hz, 1 H); 3.96 (d, J = 13.6 Hz, 1 H); 4.30 (d, J = 3.3 Hz, 2 H); 4.39-4.48 (m, 2 H); 4.76 (s, 2 H); 5.07-5.14 (m, 1 H); 6.77-6.79 (m, 1 H); 6.84-6.88 (m, 1 H); 7.09-7.15 (m, 2 H); 7.21-7.23 (m, 1 H); 7.43-7.46 (m, 2 H); 7.79 (s, 1 H); 9.24 (s, 1 H) .

### Exemple 14: 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione (schéma réactionnel n°1, composé 14) (example comparatif)

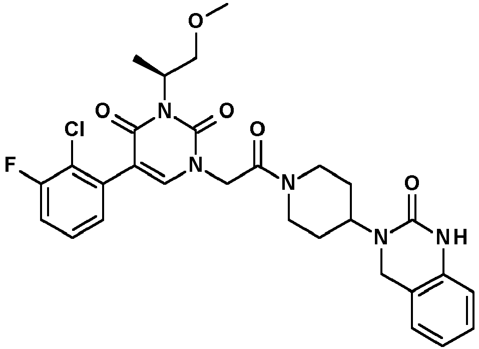

### 14.1: acétate de [5-bromo-3-((S)-2-méthoxy-1-méthyl-éthyl)-2,4-dioxo-3,4-dihydro-2H-pyrimidin-1-yl]-méthyle

De manière analogue à l'exemple 2.5, à partir de 1g (3,8 mmoles) de (5-bromo-2,4-dioxo-3,4-dihydro-2H-pyrimidin-1-yl)-acétate de méthyle de 0,4ml (4 mmoles) de (R)-1-méthoxy-propan-2-ol et après purification par chromatographie sur gel de silice élué par un mélange heptane / acétate d'éthyle 60/40, 1,2g (78%) d'acétate de [5-bromo-3-((S)-2-méthoxy-1-méthyl-éthyl)-2,4-dioxo-3,4-dihydro-2H-pyrimidin-1-yl]-méthyle sont obtenus sous la forme d'une huile incolore.

### 14.2: acide [5-(2-chloro-3-fluoro-phényl)-3-((S)-2-méthoxy-1-méthyl-éthyl)-2,4-dioxo-3,4-dihydro-2H-pyrimidin-1-yl]-acétique

De manière analogue à l'exemple 1.8, à partir de 250mg (1,4 mmoles) d'acide 2-chloro-3-fluorophénylboronique et de 400mg (1.2 mmoles) d' acétate de [5-bromo-3-((S)-2-méthoxy-1-méthyl-éthyl)-2,4-dioxo-3,4-dihydro-2H-pyrimidin-1-yl]-méthyle, 345mg (78 %) d'acide [5-(2-chloro-3-fluoro-phényl)-3-((S)-2-méthoxy-1-méthyl-éthyl)-2,4-dioxo-3,4-dihydro-2H-pyrimidin-1-yl]-acétique sont obtenus sous forme d'huile marron.

### 14.3: 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl}-1H-pyrimidine-2,4-dione (composé 14)

De manière analogue à l'exemple 13.4, à partir de 345mg (0,9 mmoles) d'acide [5-(2-chloro-3-fluoro-phényl)-3-((S)-2-méthoxy-1-méthyl-éthyl)-2,4-dioxo-3,4-dihydro-2H-pyrimidin-1-yl]-acétique, 170mg (30%) de 5-(2-chloro-3-fluoro-phényl)-3-((S)-2-méthoxy-1-méthyl-éthyl)-1-{2-oxo-2-[4-(2-oxo-1,4-dihydro-2H-quinazolin-3-yl)-pipéridin-1-yl]-éthyl} -1H-pyrimidine-2,4-dione sont obtenus sous la forme d'un solide blanc.
RMN ¹H (δ, DMSO) : 1.34 (d, J = 7.0 Hz, 3H); 1.66 (m, 3H); 1.82-1.85 (m, 1H); 2.68-2.74 (m, 1H); 3.17 (t, J = 13.0 Hz, 1H); 3.24 (s, 3H); 3.53-3.57 (m, 1H); 3.90-3.98 (m, 2H); 4.29-4.30 (m, 2H); 4.42-4.48 (m, 2H); 4.76 (s, 2H); 5.14-5.16 (m, 1H); 6.77-6.79 (m, 1H); 6.84-6.88 (m, 1H); 7.09-7.15 (m, 2H); 7.21-7.23 (m, 1H); 7.44-7.47 (m, 2H); 7.81 (s, 1H); 9.24 (s, 1H).

### Exemple 15 : 5-(2,3-difluoro-phényl)-3-méthyl-1-[1-méthyl-2-oxo-2-[4-(2-oxo-1,4-dihydroquinazolin-3-yl)-1-pipéridyl]éthyl]pyrimidine-2,4-dione (schéma réactionnel n° 2, composé 15) (example comparatif)

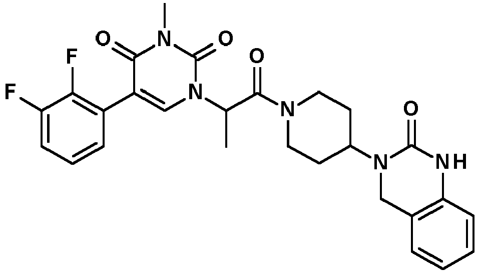

### 15.1: 1-benzhydryl-5-(2,3-difluoro-phényl)-3-méthyl-1H-pyrimidine-2,4-dione

De manière analogue à l'exemple 1.8, à partir de 4,3g (27 mmoles) d'acide 2,3-difluorophénylboronique et de 2g (5,4 mmoles) de 1-benzhydryl-5-bromo-3-méthyl-1H-pyrimidine-2,4-dione (préparé comme décrit dans l'exemple 2.2), 1,6g (73%) de 1-benzhydryl-5-(2,3-difluoro-phényl)-3-méthyl-1H-pyrimidine-2,4-dione sont obtenus sous forme d'un solide blanc.

### 15.2: 5-(2,3-difluoro-phényl)-3-méthyl-1H-pyrimidine-2,4-dione

De manière analogue à l'exemple 2.3, à partir de 1,6g (4 mmoles) de 1-benzhydryl-5-(2,3-difluoro-phényl)-3-méthyl-1H-pyrimidine-2,4-dione, 900mg (96%) de 5-(2,3-difluoro-phényl)-3-méthyl-1H-pyrimidine-2,4-dione sont obtenus sous la forme d'un solide crème.

### 15.3: 3-[1-(2-bromo-propionyl)-pipéridin-4-yl]-3,4-dihydro-1H-quinazolin-2-one

De manière analogue à l'exemple 2.4, à partir de 0,2ml (2,7 mmoles) d'acide 2-bromo-propionique et 300mg (1,2 mmoles) de 3-pipéridin-4-yl-3,4-dihydro-1H-quinazolin-2-one (préparé comme décrit dans l'exemple 1.5), 400mg (94%) de 3-[1-(2-bromo-propionyl)-pipéridin-4-yl]-3,4-dihydro-1H-quinazolin-2-one sont obtenus sous la forme d'un solide blanc cassé.

### 15.4: 5-(2,3-difluorophényl)-3-méthyl-1-[1-méthyl-2-oxo-2-[4-(2-oxo-1,4-dihydroquinazolin-3-yl)-1-pipéridyl]éthyl]pyrimidine-2,4-dione (composé 15)

De manière analogue à l'exemple 2.2, à partir de 50mg (0,2 mmoles) de 5-(2,3-difluoro-phényl)-3-méthyl-1H-pyrimidine-2,4-dione et 72mg (0,2 mmoles) de 3-[1-(2-bromo-propionyl)-pipéridin-4-yl]-3,4-dihydro-1H-quinazolin-2-one et après purification du résidu brut par chromatographie sur gel de silice élué par un mélange dichlorométhane / méthanol 98/2, 20mg (18%) de 5-(2,3-difluorophényl)-3-méthyl-1-[1-méthyl-2-oxo-2-[4-(2-oxo-1,4-dihydroquinazolin-3-yl)-1-pipéridyl]éthyl]pyrimidine-2,4-dione sont obtenus sous la forme d'un solide blanc.
RMN ¹H (δ, DMSO) : 1.43 - 1.73 (m, 7H), 2.62 - 2.78 (m, 1H), 3.25 (s, 1H), 3.29 (s, 3H) ; 4.06 - 4.21 (m, 1H), 4.22 - 4.36 (m, 2H), 4.36 - 4.51 (m, 2H), 5.64 - 5.76 (m, 1H), 6.77 (d, J = 7.8 Hz, 1H), 6.86 (t, J = 7.6 Hz, 1H), 7.02 - 7.17 (m, 2H), 7.20 - 7.32 (m, 2H), 7.38 - 7.51 (m, 1H), 7.92 (s, 1H), 9.26 (s, 1H).

### Exemple 16 : 5-(2,3-difluoro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 16) (example comparatif)

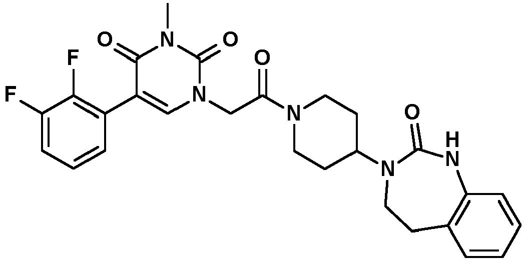

### 16.1: (1-benzyl-pipéridin-4-yl)-[2-(2-nitro-phényl)-éthyl]-amine

8ml (40 mmoles) de 1-benzyl-pipéridin-4-ylamine sont additionnés sur 4g (17,4 mmoles) de 1-(2-bromo-éthyl)-2-nitro-benzene et le mélange est chauffé à 100°C pendant 18 heures. Après refroidissement, de l'éther diéthylique est rajouté, le milieu est filtré et le filtrat est concentré. Le résidu brut est purifié par chromatographie sur gel de silice élué par un mélange acétate d'éthyle / heptane, 60/40 puis dichlorométhane / méthanol / ammoniaque, 95/3/2. 4,9g (82%) de (1-benzyl-pipéridin-4-yl)-[2-(2-nitro-phényl)-éthyl]-aminé sont obtenus sous la forme d'une huile orangée.

### 16.2: [2-(2-amino-phényl)-éthyl]-(1-benzyl-pipéridin-4-yl)-amine

De manière analogue à l'exemple 1.5, à partir de 4,8g (14 mmoles) de (1-benzyl-pipéridin-4-yl)-[2-(2-nitro-phényl)-éthyl]-amine, 140mg d'oxyde de platine (10% molaire) et après purification du résidu par chromatographie sur gel de silice élué par un mélange dichlorométhane / méthanol / ammoniaque 90/8/2, 4,6g (100%) de [2-(2-amino-phényl)-éthyl]-(1-benzyl-pipéridin-4-yl)-amine sont obtenus sous la forme d'une huile brune.

### 16.3: 3-(1-benzyl-pipéridin-4-yl)-1,3,4,5-tétrahydro-benzo[d][1,3]diazépin-2-one

De manière analogue à l'exemple 1.4, à partir de 1g (6,5 mmoles) de [2-(2-amino-phényl)-éthyl]-(1-benzyl-pipéridin-4-yl)-amine, 3,9g (78%) de 3-(1-benzyl-pipéridin-4-yl)-1,3,4,5-tetrahydro-benzo[d][1,3]diazépin-2-one sont obtenus sous la forme d'une poudre blanche.

### 16.4: 3-pipéridin-4-yl-1,3,4,5-tétrahydro-benzo[d][1,3]diazépin-2-one

De manière analogue à l'exemple 1.5, à partir de 3,7g (11 mmoles) de 3-(1-benzyl-pipéridin-4-yl)-1,3,4,5-tetrahydro-benzo[d][1,3]diazépin-2-one, le milieu réactionnel étant placé sous 5bar de dihydrogène, 2,6g (96%) de 3-pipéridin-4-yl-1,3,4,5-tétrahydro-benzo[d][1,3]diazépin-2-one sont obtenus sous la forme d'une poudre blanche.

### 16.5: 1-benzhydryl-5-(2,3-difluoro-phényl)-3-méthyl-1H-pyrimidine-2,4-dione

De manière analogue à l'exemple 1.8, à partir de 2g (5,4 mmoles) de 1-benzhydryl-5-bromo-3-méthyl-1H-pyrimidine-2,4-dione (préparé comme décrit dans l'exemple 2.2) et 4,3g (27 mmoles) d'acide 2,3-difluorophénylboronique, et après purification par chromatographie sur gel de silice élué avec un mélange heptane / acétate d'éthyle 80/20, 1,6g (73%) de 1-benzhydryl-5-(2,3-difluoro-phényl)-3-méthyl-1H-pyrimidine-2,4-dione sont obtenus sous la forme d'un solide blanc.

### 16.6: 5-(2,3-difluoro-phényl)-3-méthyl-1H-pyrimidine-2,4-dione

De manière analogue à l'exemple 2.3, à partir de 1,6g (4 mmoles) de 1-benzhydryl-5-(2,3-difluoro-phényl)-3-méthyl-1H-pyrimidine-2,4-dione et après trituration du résidu brut dans l'éther diéthylique, filtration et séchage sous azote; 900mg (96%) de 5-(2,3-difluoro-phényl)-3-méthyl-1H-pyrimidine-2,4-dione sont obtenus sous la forme d'un solide beige.

### 16.7: [5-(2,3-difluoro-phényl)-3-méthyl-2,4-dioxo-3,4-dihydro-2H-pyrimidin-l-yl]-acétate de méthyle

De manière analogue à l'exemple 13.1, à partir de 450mg (1,9 mmoles) de 5-(2,3-difluoro-phényl)-3-méthyl-1H-pyrimidine-2,4-dione et 0,2ml (2,1 mmoles) de chloroacétate de méthyle, 475mg (81%) de [5-(2,3-difluoro-phényl)-3-méthyl-2,4-dioxo-3,4-dihydro-2H-pyrimidin-1-yl]-acétate de méthyle sont obtenus sous la forme d'un solide blanc.

### 16.8: acide [5-(2,3-difluoro-phényl)-3-méthyl-2,4-dioxo-3,4-dihydro-2H-pyrimidin-1-yl]-acétique

2,3ml (2,3 mmoles) d'une solution aqueuse d'hydroxyde de lithium 1N sont ajoutés à une solution de 475mg (1,5 mmoles) de [5-(2,3-difluoro-phényl)-3-méthyl-2,4-dioxo-3,4-dihydro-2H-pyrimidin-1-yl]-acétate de méthyle dans 15ml de tétrahydrofurane et 3mL d'eau. Le milieu réactionnel est agité à température ambiante pendant 2 heures, puis amené à pH6 par ajout de 4ml d'une solution aqueuse d'acide acétique 1N. Le produit est extrait à l'acétate d'éthyle. La phase organique est lavée par de l'eau puis par une solution aqueuse saturée de chlorure de sodium, séchée sur sulfate de magnésium, filtrée et concentrée sous vide. 400mg (88%) d'acide [5-(2,3-difluoro-phényl)-3-méthyl-2,4-dioxo-3,4-dihydro-2H-pyrimidin-1-yl]-acétique sont obtenus sous la forme d'un solide blanc.

### 16.9: 5-(2,3-difluoro-phényl)-3-méthyl-1-{2-oxo-2-[4-(2-oxo-1,2, 4,5-tétrahydro-benzo [d][1,3]diazépin-3-yl)-pipéridin-1-yl]-éthyl}-1H-pyrimidine-2,4-dione (composé 16)

De manière analogue à l'exemple 1.6, à partir de 100mg (0,3 mmoles) d'acide [5-(2,3-difluoro-phényl)-3-méthyl-2,4-dioxo-3,4-dihydro-2H-pyrimidin-1-yl]-acétique et 100mg (0,4 mmoles) de 3-pipéridin-4-yl-1,3,4,5-tétrahydro-benzo[d][1,3]diazépin-2-one (préparé comme décrit dans l'exemple 16.4) et après cristallisation dans un mélange heptane / acétate d'éthyle, 70/30; 120mg (67%) de 5-(2,3-difluoro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 160°C.
RMN¹H (δ, DMSO) : 1.46 - 1.62 (m, 1H), 1.62 - 1.82 (m, 3H), 2.67 (t, J = 16 Hz, 1H), 2.89 (t, J = 4.9 Hz, 2H), 3.15 (t, J = 16 Hz, 1H), 3.25 (s, 3H), 3.33 - 3.41 (m, 2H), 3.95 (d, J = 13.5 Hz, 1H), 4.27 - 4.37 (m, 1H), 4.41 (d, J = 8 Hz, 1H), 4.79 (s, 2H), 6.73 - 6.85 (m, 1H), 6.96 - 7.10 (m, 3H), 7.16 - 7.34 (m, 2H), 7.38 - 7.55 (m, 1H), 7.93 (s, 1H), 8.54 (s, 1H).

### Exemple 17 : 5-(2,3-dichloro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 17) (example comparatif)

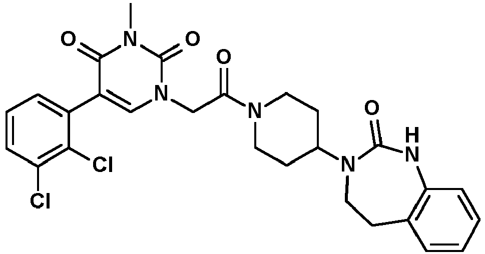

### 17.1: (5-bromo-3-méthyl-2,4-dioxo-3,4-dihydro-2H-pyrimidin-1-yl)-acétate de méthyle

De manière analogue à l'exemple 13.1, à partir de 1,5g (7,1 mmoles) de 5-bromo-3-méthyl-1H-pyrimidine-2,4-dione (préparé comme décrit dans l'exemple 2.3) et 0,7ml (7,8 mmoles) de chloro-acétate de méthyle, 1,6g (79%) de (5-bromo-3-méthyl-2,4-dioxo-3,4-dihydro-2H-pyrimidin-1-yl)-acétate de méthyle sont obtenus sous la forme d'un solide blanc.

### 17.2: acide (5-bromo-3-méthyl-2,4-dioxo-3,4-dihydro-2H-pyrimidin-1-yl)-acétique

De manière analogue à l'exemple 16.8, à partir de 1,6g (5,6 mmoles) de (5-bromo-3-méthyl-2,4-dioxo-3,4-dihydro-2H-pyrimidin-1-yl)-acétate de méthyle, 1,1g (76%) d'acide (5-bromo-3-méthyl-2,4-dioxo-3,4-dihydro-2H-pyrimidin-1-yl)-acétique sont obtenus sous la forme d'un solide blanc.

### 17.3: 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione

De manière analogue à l'exemple 1.6, à partir de 960mg (3,7 mmoles) d'acide (5-bromo-3-méthyl-2,4-dioxo-3,4-dihydro-2H-pyrimidin-1-yl)-acétique et 895mg (3,7 mmoles) de 3-pipéridin-4-yl-1,3,4,5-tetrahydro-benzo[d][1,3]diazépin-2-one (préparé comme décrit dans l'exemple 16.4), 1,7g (95%) de 5-bromo-3-méthyl-1-{2-oxo-2-[4-(2-oxo-1,2,4,5-tétrahydro-benzo[d] [1,3]diazépin-3-yl)-pipéridin-l-yl]-éthyl}-1H-pyrimidine-2,4-dione sont obtenus sous la forme d'un solide blanc.

### 17.4: 5-(2,3-dichloro-phényl)-3-méthyl-l-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 17)

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione et 58mg (0,3 mmoles) d'acide 2,3-dichlorophénylboronique, et après purification par chromatographie sur gel de silice élué par un mélange dichlorométhane / méthanol 99/1, 40mg (35%) de 5-(2,3-dichlorophényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 170°C.

RMN ¹H (δ, DMSO) : 1.39 - 1.53 (m, 1H), 1.55 - 1.72 (m, 3H), 2.61 (t, J = 12.4 Hz, 1H), 2.77 - 2.88 (m, 2H), 3.08 (t, J = 12.7 Hz, 1H), 3.18 (s, 3H), 3.27 - 3.34 (m, 2H), 3.87 (d, J = 13.6 Hz, 1H), 4.24 - 4.34 (m, 1H), 4.35 (d, J = 12.9 Hz, 1H), 4.70 (d, J = 3.4 Hz, 2H), 6.66 - 6.80 (m, 1H), 6.89 - 7.04 (m, 3H), 7.26 (dd, J = 7.7, 1.6 Hz, 1H), 7.36 (t, J = 7.8 Hz, 1H), 7.62 (dd, J = 8.1, 1.6 Hz, 1H), 7.76 (s, 1H), 8.48 (s, 1H).

### Exemple 18 : 5-(2-chloro-phényl)-3-méthyl-1-{2-oxo-2-[4-(2-oxo-1,2,4,5-tétrahydro benzo[d][1,3]diazépin-3-yl)-pipéridin-1-yl]-éthyl}-1H-pyrimidine-2,4-dione (schéma réactionnel n°3, composé 18) (example comparatif)

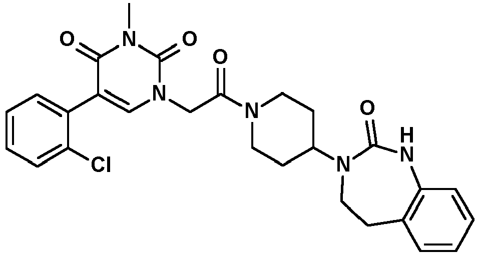

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 48mg (0,3 mmoles) d'acide 2-chlorophénylboronique, 40mg (38%) de 5-(2-chloro-phényl)-3-méthyl-1-{2-oxo-2-[4-(2-oxo-1,2,4,5 tétrahydrobenzo[d][1,3]diazépin-3-yl)-pipéridin-1-yl]-éthyl}-1H-pyrimidine-2,4-dione sont obtenus sous la forme d'un solide beige de point de fusion 175°C.
RMN ¹H (δ, DMSO) : 1.40-1.55 (m, 1H), 1.58-1.70 (m, 3H), 2.55-2.68 (m, 1H), 2.82 (t, J = 4.7 Hz, 2H), 3.05-3.12 (m, 1H), 3.18 (s, 3H), 3.30 (t, J = 4.4 Hz, 2H), 3.88 (m, 1H), 4.20-4.30 (m, 1H), 4.38 (m, 1H), 4.70 (d, J = 4.2 Hz, 2H), 6.72-6.75 (m, 1H), 6.95-6.98 (m, 3H), 7.26-7.28 (m, 1H), 7.31-7.36 (m, 2H), 7.46-7.48 (m, 1H), 7.71 (s, 1H), 8.47 (s, 1H).

### Exemple 19 : 3-méthyl-1-{2-oxo-2-[4-(2-oxo-1,2,4,5-tétrahydro-benzo[d][1,3]diazépin-3-yl)-pipéridin-1-yl]-éthyl}-5-(2-trifluorométhoxy-phényl)-1H-pyrimidine-2,4-dione (schéma réactionnel n°3, composé 19) (example comparatif)

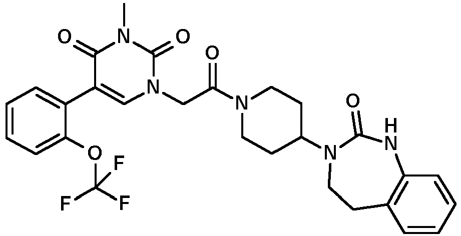

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 63mg (0,3 mmoles) d'acide 2-(trifluorométhoxy)benzène boronique, 40mg (34%) de 3-méthyl-1-{2-oxo-2-[4-(2-oxo-1,2,4,5-tétrahydro-benzo[d] [1,3]diazépin-3-yl)-pipéridin-1-yl]-éthyl}-5-(2-trifluorométhoxy-phényl)-1H-pyrimidine-2,4-dione sont obtenus sous la forme d'un solide rose de point de fusion 235°C.
RMN ¹H (δ, DMSO) : 1.40-1.55 (m, 1H), 1.58-1.72 (m, 3H), 2.55-2.68 (m, 1H), 2.82 (t, J = 4.5 Hz, 2H), 3.05-3.15 (m, 1H), 3.18 (s, 3H), 3.30 (t, J = 4.5 Hz, 2H), 3.90 (m, 1H), 4.20-4.30 (m, 1H), 4.35 (m, 1H), 4.71 (s, 2H), 6.71-6.75 (m, 1H), 6.95-6.98 (m, 3H), 7.33-7.40 (m, 3H), 7.44-7.48 (m, 1H), 7.74 (s, 1H), 8.47 (s, 1H).

### Exemple 20 : 3-méthyl-1-{2-oxo-2-[4-(2-oxo-1,2,4,5-tétrahydro-benzo[d][1,3]diazépin-3-yl)-pipéridin-1-yl]-éthyl}-5-thiophen-3-yl-1H-pyrimidine-2,4-dione (schéma réactionnel n°3, composé 20) (example comparatif)

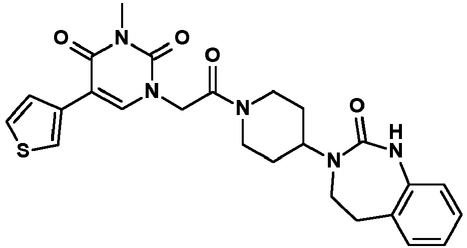

De manière analogue à l'exemple 9, à partir de 150mg (0,3 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 59mg (0,5 mmoles) d'acide thiophène-3-boronique, 70mg (46%) de 3-méthyl-1-{2-oxo-2-[4-(2-oxo-1,2,4,5-tétrahydro-benzo[d] [1,3]diazépin-3-yl)-pipéridin-1-yl]-éthyl}-5-thiophen-3-yl-1H-pyrimidine-2,4-dione sont obtenus sous la forme d'un solide rose de point de fusion 283°C.
RMN 1H (δ, DMSO) : 1.40-1.55 (m, 1H), 1.58-1.70 (m, 3H), 2.60-2.68 (m, 1H), 2.83 (t, J = 4.5 Hz, 2H), 3.05-3.15 (m, 1H), 3.20 (s, 3H), 3.32 (t, J = 4.6 Hz, 2H), 3.85-3.95 (m, 1H), 4.20-4.30 (m, 1H), 4.35-4.40 (m, 1H), 4.72 (d, J = 4.3 Hz, 2H), 6.72-6.76 (m, 1H), 6.95-6.98 (m, 3H), 7.37 (dd, J = 1.2-5 Hz, 1H), 7.52 (dd, J = 3.0-5.1 Hz, 1H), 7.91 (dd, J = 1.2-3 Hz, 1H), 8.10 (s, 1H), 8.48 (s, 1H).

### Exemple 21 : 5-(2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 21) (example comparatif)

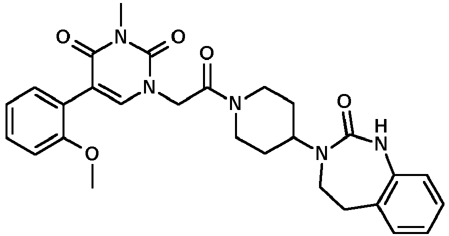

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 47mg (0,3 mmoles) d'acide 2-méthoxyphényl boronique, 25mg (24%) de 5-(2-méthoxy-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide beige de point de fusion 250°C.
RMN ¹H (δ, DMSO) : 1.40-1.55 (m, 1H), 1.56-1.72 (m, 3H), 2.55-2.65 (m, 1H), 2.82 (t, J = 4.6 Hz, 2H), 3.05-3.14 (m, 1H), 3.15 (s, 3H), 3.29 (t, J = 4.9 Hz, 2H), 3.67 (s, 3H), 3.88 (m, 1H), 4.20-4.30 (m, 1H), 4.35 (m, 1H), 4.68 (d, J = 5.8 Hz, 2H), 6.70-6.78 (m, 1H), 6.90 (m, 1H), 6.95-7.00 (m, 4H), 7.10 (dd, J = 1.72-7.4 Hz, 1H), 7.30 (m, 1H), 7.57 (s, 1H), 8.47 (s, 1H).

### Exemple 22 : 5-(3-chloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 22) (example comparatif)

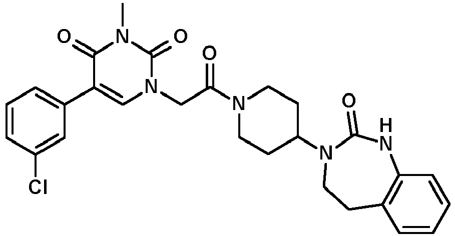

De manière analogue à l'exemple 1.8, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 48mg (0,3 mmoles) d'acide 3-chlorophénylboronique, 55mg (52%) de 5-(3-chloro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 220°C.
RMN ¹H (δ, DMSO) : 1.50-1.60 (m, 1H), 1.62-1.70 (m, 3H), 2.69 (m, 1H), 2.90 (t, J = 4.8 Hz, 2H), 3.26 (m, 1H), 3.33 (s, 3H), 3.38 (t, J = 4.6 Hz, 2H), 3.95 (m, 1H), 4.30-4.40 (m, 1H), 4.45 (m, 1H), 4.81 (s, 2H), 6.79-6.82 (m, 1H), 7.03-7.05 (m, 3H), 7.38-7.41 (m, 1H), 7.45 (t, J = 7.9 Hz, 1H), 7.55-7.65 (m, 1H), 7.65 (d, J = 1.8 Hz, 1H), 8.05 (s, 1H), 8.55 (s, 1H).

### Exemple 23 : 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 23) (example comparatif)

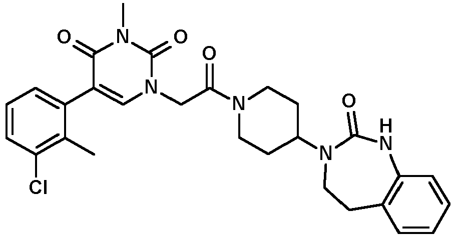

De manière analogue à l'exemple 9, à partir de 150mg (0,3 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 78mg (0,5 mmoles) d'acide 3-chloro-2-méthylphénylboronique, 75mg (46%) de 5-(3-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide gris de point de fusion 190°C.
RMN ¹H (δ, DMSO) : 1.45-1.60 (m, 1H), 1.62-1.80 (m, 3H), 2.20 (s, 3H), 2.70 (m, 1H), 2.89 (t, J = 4.5 Hz, 2H), 3.15 (m, 1H), 3.25 (s, 3H), 3.37 (t, J = 4.8 Hz, 2H), 3.95 (m, 1H), 4.35 (m, 1H), 4.45 (m, 1H), 4.76 (d, J = 2.6 Hz, 2H), 6.78-6.85 (m, 1H), 7.02-7.05 (m, 3H), 7.13 (d, J = 6.8 Hz, 1H), 7.26 (t, J = 7.8 Hz, 1H), 7.47 (d, J = 7.2 Hz, 1H), 7.72 (s, 1H), 8.54 (s, 1H) .

### Exemple 24 : 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 24) (example comparatif)

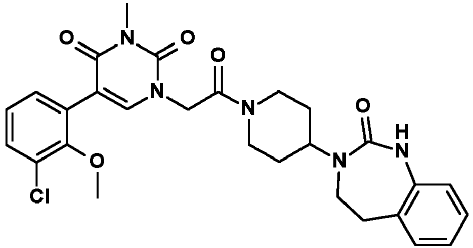

De manière analogue à l'exemple 9, à partir de 150mg (0,3 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 86mg (0,5 mmoles) d'acide 3-chloro-2-méthoxyphényl boronique, 60mg (36%) de 5-(3-chloro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl} -1H-pyrimidine-2,4-dione sont obtenus sous la forme d'un solide beige de point de fusion 260°C.
RMN ¹H (δ, DMSO) : 1.50-1.60 (m, 1H), 1.62-1.80 (m, 3H), 2.68 (m, 1H), 2.89 (t, J = 4.4 Hz, 2H), 3.15 (m, 1H), 3.26 (s, 3H), 3.37 (t, J = 4.5 Hz, 2H), 3.65 (s, 3H), 3.95 (m, 1H), 4.30 (m, 1H), 4.45 (m, 1H), 4.78 (d, J = 2.44 Hz, 2H), 6.78-6.85 (m, 1H), 7.02-7.05 (m, 3H), 7.18 (t, J = 7.8 Hz, 1H), 7.27 (dd, J = 1.7-7.7 Hz, 1H), 7.49 (dd, J = 1.7-8.0 Hz, 1H), 7.80 (s, 1H), 8.54 (s, 1H).

### Exemple 25 : 5-(2-méthoxy-3-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 25) (example comparatif)

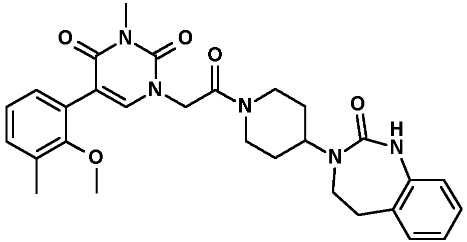

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 51mg (0,3 mmoles) d'acide 2-méthoxy-3-méthyl phénylboronique, 20mg (17%) de 5-(2-méthoxy-3-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 148°C.
RMN ¹H (δ, DMSO) : 1.48-1.60 (m, 1H), 1.62-1.80 (m, 3H), 2.26 (s, 3H), 2.70 (m, 1H), 2.90 (m, 2H), 3.15 (m, 1H), 3.25 (s, 3H), 3.40 (m, 2H), 3.53 (s, 3H), 3.95 (m, 1H), 4.35 (m, 1H), 4.45 (m, 1H), 4.77 (d, J = 3.92 Hz, 2H), 6.85 (m, 1H), 7.00-7.08 (m, 4H), 7.10 (m, 1H), 7.20 (m, 1H), 7.72 (s, 1H), 8.54 (s, 1H).

### Exemple 26 : 5-(3-chloro-2-hydroxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 26) (example comparatif)

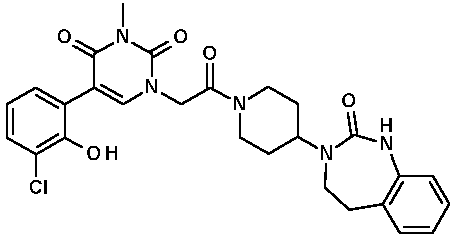

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 53mg (0,3 mmoles) d'acide 3-chloro-2-hydroxy phényl boronique, 45mg (41%) de 5-(3-chloro-2-hydroxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide gris de point de fusion 270°C.
RMN ¹H (δ, DMSO) : 1.45-1.58 (m, 1H), 1.65-1.76 (m, 3H), 2.68 (t, J = 12.4 Hz, 1H), 2.90 (d, J = 3.4 Hz, 2H), 3.12-3.18 (m, 1H), 3.24 (s, 3H), 3.36 (d, J = 4.0 Hz, 2H), 3.96 (d, J = 12.6 Hz, 1H), 4.30-4.35 (m, 1H), 4.42 (d, J = 12.2 Hz, 1H), 4.71-4.83 (m, 2H), 6.52-6.78 (m, 1H), 6.86 (t, J = 7.8 Hz, 1H), 7.02-7.09 (m, 4H), 7.37 (dd, J = 1.2-7.9 Hz, 1H), 7.76 (s, 1H), 8.54 (s, 1H), 9.34 (s, 1H) .

### Exemple 27 : 5-(2,3-diméthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 27) (example comparatif)

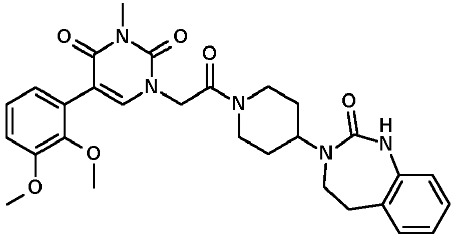

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 56mg (0,3 mmoles) d'acide 2,3-diméthoxy-phényl boronique, 54mg (60%) de 5-(2,3-diméthoxy-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide gris de point de fusion 236°C.
RMN ¹H (δ, DMSO) : 1.51-1.55 (m, 1H), 1.64-1.76 (m, 3H), 2.64-2.70 (m, 1H), 2.89 (t, J = 3.5 Hz, 2H), 3.14-3.19 (m, 1H), 3.24 (s, 3H), 3.37 (t, J = 4.1 Hz, 2H), 3.66 (s, 3H), 3.83 (s, 3H), 3.94 (d, J = 12.9 Hz, 1H), 4.30-4.35 (m, 1H), 4.42 (d, J = 12.8 Hz, 1H), 4.76 (d, J = 3.2 Hz, 2H), 6.78-6.82 (m, 2H), 7.02-7.08 (m, 5H), 7.64 (s, 1H), 8.54 (s, 1H).

### Exemple 28 : 5-(3-fluoro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 28) (example comparatif)

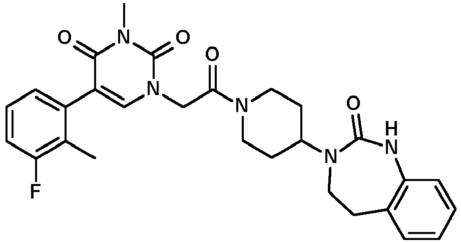

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 47mg (0,3 mmoles) d'acide 3-fluoro-2-méthyl-phényl boronique, 50mg (47%) de 5-(3-fluoro-2-méthyl-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide gris de point de fusion 160°C.
RMN ¹H (δ, DMSO) : 1.50-1.60 (m, 1H), 1.55-1.74 (m, 3H), 2.08 (d, J = 2 Hz, 3H), 2.55-2.70 (m, 1H), 2.89 (t, J = 4.5 Hz, 2H), 3.10-3.20 (m, 1H), 3.25 (s, 3H), 3.37 (d, J = 4.4 Hz, 2H), 3.95 (d, J = 13.7 Hz, 1H), 4.30-4.40 (m, 1H), 4.42 (d, J = 12.5 Hz, 1H), 4.77 (d, J = 2 Hz, 2H), 6.78-6.82 (m, 1H), 7.00-7.05 (m, 4H), 7.18 (t, J = 8.8 Hz, 1H), 7.24-7.27 (m, 1H), 7.71 (s, 1H), 8.54 (s, 1H).

### Exemple 29 : 5-(3-chloro-2-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 29) (example comparatif)

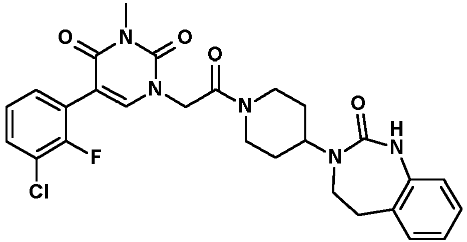

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 53mg (0,3 mmoles) d'acide 3-chloro-2-fluoro-phényl boronique, 65mg (57%) de 5-(3-chloro-2-fluoro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide gris de point de fusion 206°C.
RMN ¹H (δ, DMSO) : 1.50-1.60 (m, 1H), 1.65-1.80 (m, 3H), 2.69 (t, J = 12 Hz, 1H), 2.90 (m, 2H), 3.16 (t, J = 10 Hz, 1H), 3.26 (s, 3H), 3.37 (m, 2H), 3.95 (d, J = 12.6 Hz, 1H), 4.30-4.40 (m, 1H), 4.42 (d, J = 11.7 Hz, 1H), 4.80 (s, 2H), 6.81 (d, J = 3.7 Hz, 1H), 7.03-7.04 (m, 3H), 7.29 (t, J = 7.8 Hz, 1H), 7.37 (t, J = 6.5 Hz, 1H), 7.61 (t, J = 7.2 Hz, 1H), 7.93 (s, 1H), 8.54 (s, 1H).

### Exemple 30 : 5-(2,3-diméthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 30) (example comparatif)

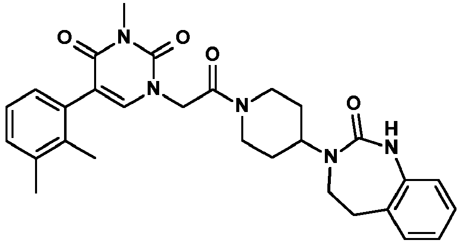

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 46mg (0,3 mmoles) d'acide 2,3-diméthyl-phényl boronique, 50mg (48%) de 5-(2,3-diméthyl-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc cassé de point de fusion 253°C.
RMN ¹H (δ, DMSO) : 1.50-1.60 (m, 1H), 1.55-1.80 (m, 3H), 2.06 (s, 3H), 2.28 (s, 3H), 2.68 (t, J = 12 Hz, 1H), 2.90 (t, J = 4 Hz, 2H), 3.15 (t, J = 12 Hz, 1H), 3.25 (s, 3H), 3.39 (t, J = 4 Hz, 2H), 3.95 (d, J = 12 Hz, 1H), 4.30-4.40 (m, 1H), 4.44 (d, J = 12 Hz, 1H), 4.77 (d, J = 4 Hz, 2H), 6.79-6.83 (m, 1H), 6.96 (d, J = 8 Hz, 1H), 7.02-7.05 (m, 3H), 7.11 (t, J = 8 Hz, 1H), 7.18 (d, J = 8 Hz, 1H), 7.60 (s, 1H), 8.52 (s, 1H).

### Exemple 31 : 5-(2-chloro-3-fluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 31) (example comparatif)

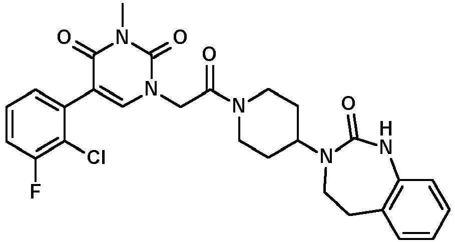

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 53mg (0,3 mmoles) d'acide 2-chloro-3-fluoro-phényl-phénylboronique, 75mg (68%) de 5-(2-chloro-3-fluoro-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2, 4-dione sont obtenus sous la forme d'un solide gris de point de fusion 162°C.
RMN ¹H (δ, DMSO) : 1.50-1.60 (m, 1H), 1.65-1.75 (m, 3H), 2.68 (t, J = 12 Hz, 1H), 2.89 (t, J = 4.4 Hz, 2H), 3.15 (t, J = 12 Hz, 1H), 3.25 (s, 3H), 3.37 (t, J = 4.3 Hz, 2H), 3.94 (d, J = 12 Hz, 1H), 4.31-4.34 (m, 1H), 4.42 (d, J = 12 Hz, 1H), 4.78 (d, J = 1.96 Hz, 2H), 6.78-6.82 (m, 1H), 7.02-7.05 (m, 3H), 7.20-7.22 (m, 1H), 7.43-7.47 (m, 2H), 7.84 (s, 1H), 8.54 (s, 1H) .

### Exemple 32 : 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 32) (example comparatif)

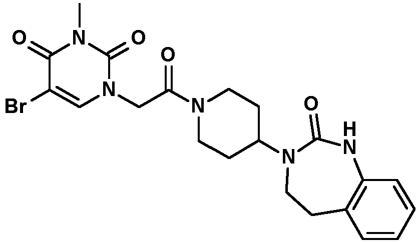

De manière analogue à l'exemple 1.6, à partir de 930mg (3,5 mmoles) d'acide (5-bromo-3-méthyl-2,4-dioxo-3,4-dihydro-2H-pyrimidin-1-yl) -acétique (préparé comme décrit dans l'exemple 17.2) et 1g (4,2 mmoles) de 3-pipéridin-4-yl-1,3,4,5-tetrahydro-benzo[d][1,3]diazépin-2-one (préparé comme décrit dans l'exemple 16.4), 1,4g (80%) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.50-1.60 (m, 1H), 1.65-1.80 (m, 3H), 2.70 (t, J = 12 Hz, 1H), 2.90 (t, J = 4 Hz, 2H), 3.15 (t, J = 12 Hz, 1H), 3.23 (s, 3H), 3.40 (t, J = 4 Hz, 2H), 4.95 (d, J = 12 Hz, 1H), 4.30-4.38 (m, 1H), 4.40 (d, J = 12 Hz, 1H), 4.73 (s, 2H), 6.80-6.85 (m, 1H), 7.04 (m, 3H), 8.20 (s, 1H), 8.54 (s, 1H).

### Exemple 33 : 5-(3-fluoro-2-méthoxy-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 33) (example comparatif)

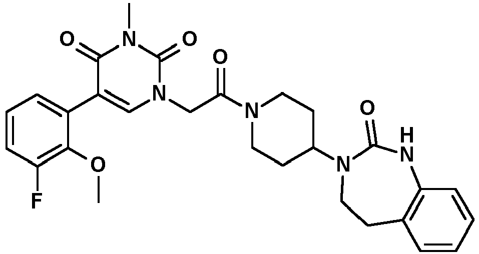

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 52mg (0,3 mmoles) d'acide 3-fluoro-2-méthoxy-phényl boronique, 75mg (68%) de 5-(3-fluoro-2-méthoxy-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide gris de point de fusion 152°C.
RMN ¹H (δ, DMSO) : 1.50-1.60 (m, 1H), 1.55-1.80 (m, 3H), 2.68 (d, J = 12 Hz, 1H), 2.89 (t, J = 4 Hz, 2H), 3.15 (d, J = 12 Hz, 1H), 3.25 (s, 3H), 3.37 (t, J = 4 Hz, 2H), 3.78 (d, J = 1.4 Hz, 3H), 3.94 (d, J = 12 Hz, 1H), 4.30-4.40 (m, 1H), 4.42 (d, J = 12 Hz, 1H), 4.77 (d, J = 2.3 Hz, 2H), 6.78-6.82 (m, 1H), 7.02-7.07 (m, 4H), 7.11-7.16 (m, 1H), 7.26-7.31 (m, 1H), 7.73 (s, 1H), 8.54 (s, 1H).

### Exemple 34 : 5-(2,6-difluoro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 34) (example comparatif)

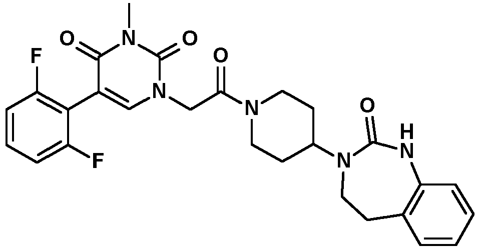

2,3mg (0,01 mmoles) d' acétate de palladium(II), 7,2mg (0,02 mmole) de biphényle de 2-dicyclohexylphosphine et 164,3mg (1 mmole) d'acide 2,6-difluorophénylboronique sont ajoutés à une solution de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3) et 0,5ml (1 mmole) de solution aqueuse de phosphate de potassium tribasique 2M dans 5ml de diméthylformamide, préalablement dégazée avec de l'azote. Le mélange réactionnel est chauffé à 90°C pendant 2 heures. La réaction est traitée par addition d'eau puis le produit est extrait trois fois à l'acétate d'éthyle. La phase organique est lavée par de l'eau puis par une solution saturée de chlorure de sodium, séchée sur sulfate de magnésium, filtrée et concentrée sous vide. Le résidu brut obtenu est purifié par chromatographie sur gel de silice élué avec un mélange dichlorométhane/méthanol 96/4. 8mg (6%) de 5-(2,6-difluoro-phényl)-3-méthyl-1-{2-oxo-2-[4-(2-oxo-1,4-dihydro-2H-quinazolin-3-yl)-pipéridin-1-yl]-éthyl}-1H-pyrimidine-2,4-dione sont obtenus sous forme d'un solide blanc.
RMN ¹H (δ, DMSO) : 1.55-1.65 (m, 1H), 1.70-1.85 (m, 3H), 2.74 (t, J = 12 Hz, 1H), 2.95 (m, 2H), 3.24 (t, J = 12 Hz, 1H), 3.31 (s, 3H), 3.45 (m, 2H), 3.99 (d, J = 12 Hz, 1H), 4.30-4.40 (m, 1H), 4.47 (d, J = 12 Hz, 1H), 4.84 (s, 2H), 6.84-6.88 (m, 1H), 7.08-7.10 (m, 3H), 7.25 (t, J = 8.6 Hz, 2H), 7.54-7.58 (m, 1H), 7.96 (s, 1H), 8.59 (s, 1H).

### Exemple 35 : 5-(3,5-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 35) (example comparatif)

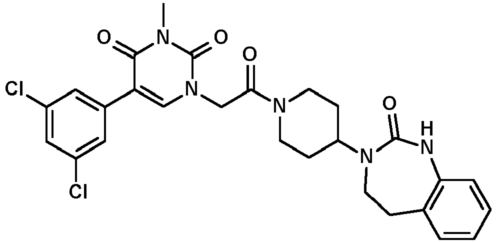

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 78mg (0,4 mmoles) d'acide 3,5-dichlorophényl boronique, et après cristallisation dans un mélange heptane / acétate d'éthyle 70/30, 50mg (44%) de 5-(3,5-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 170°C.
RMN ¹H (δ, DMSO) : 1.40-1.55 (m, 1H), 1.58-1.70 (m, 3H), 2.55 (m, 1H), 2.83 (t, J = 4.4 Hz, 2H), 3.10 (m, 1H), 3.20 (s, 3H), 3.31 (t, J = 4.7 Hz, 2H), 3.90 (m, 1H), 4.30 (m, 1H), 4.35 (m, 1H), 4.74 (s, 2H), 6.71-6.76 (m, 1H), 6.96-6.98 (m, 3H), 7.49-7.63 (m, 3H), 8.11 (s, 1H), 8.47 (s, 1H).

### Exemple 36 : 4-(3-méthyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzonitrile (schéma réactionnel n°3, composé 36) (example comparatif)

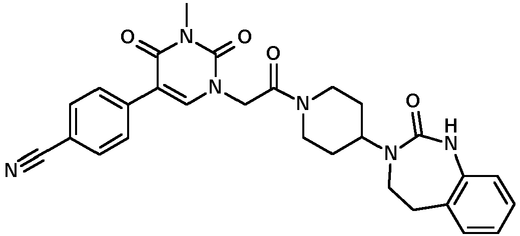

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 45mg (0,3 mmoles) d'acide 4-(cyanophényl)-boronique, 13mg (11%) de 4-(3-méthyl-2,4-dioxo-1-{2-oxo-2-[4-(2-oxo-1,2, 4,5-tétrahydro-benzo[d][1,3]diazépin-3-yl)-pipéridin-1-yl]-éthyl}-1,2,3,4-tétrahydro-pyrimidin-5-yl)-benzonitrile sont obtenus sous la forme d'un solide blanc.

RMN ¹H (δ, DMSO) : 1.50-1.60 (m, 1H), 1.62-1.80 (m, 3H), 2.7 (m, 1H), 2.90 (t, 2H), 3.15 (m, 1H), 3.27 (s, 3H), 3.40 (t, 2H), 3.95 (d, 1H), 4.30-4.40 (m, 1H), 4.42 (d, 1H), 4.83 (s, 2H), 6.80 (m, 1H), 7.00-7.05 (m, 3H), 7.80 (d, J = 8.6 Hz, 2H), 7.88 (d, J = 8.5 Hz, 2H), 8.15 (s, 1H), 8.54 (s, 1H).

### Exemple 37 : 3-(3-méthyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzonitrile (schéma réactionnel n°3, composé 37) (example comparatif)

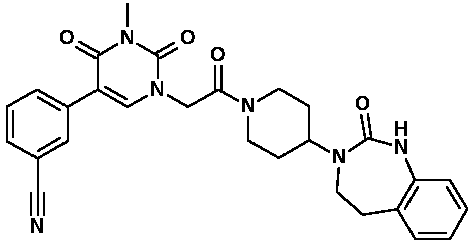

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 17.3), et 45mg (0,3 mmoles) d'acide 3-(cyanophényl)-boronique, 50mg (46%) de 3-(3-méthyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzonitrile sont obtenus sous la forme d'un solide blanc de point de fusion 258°C.
RMN ¹H (δ, DMSO) : 1.50-1.60 (m, 1H), 1.62-1.80 (m, 3H), 2.7 (m, 1H), 2.90 (t, 2H), 3.15 (m, 1H), 3.27 (s, 3H), 3.40 (t, 2H), 3.95 (d, 1H), 4.30-4.40 (m, 1H), 4.42 (d, 1H), 4.83 (s, 2H), 6.80 (m, 1H), 7.00-7.05 (m, 3H), 7.64 (t, J = 8.0 Hz, 1H), 7.79 (d, J = 7.6 Hz, 1H), 7.95 (d, J = 8.0 Hz, 1 H), 8.01 (s, 1 H), 8.13 (s, 1H), 8.54 (s, 1H).

### Exemple 38: 2-(3-méthyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzonitrile (schéma réactionnel n°3, composé 38) (example comparatif)

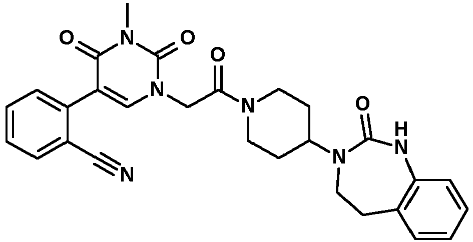

De manière analogue à l'exemple 9, à partir de 100mg (0,2 mmoles) de 5-bromo-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (préparé comme décrit dans l'exemple 17.3), et 45mg (0,3 mmoles) d'acide 2-(cyanophényl)-boronique, 60mg (57%) de 2-(3-méthyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzonitrile sont obtenus sous la forme d'un solide beige de point de fusion 196°C.
RMN ¹H (δ, DMSO) : 1.50-1.60 (m, 1H), 1.62-1.80 (m, 3H), 2.70 (t, J = 12 Hz, 1H), 2.89 (t, J = 4.6 Hz, 2H), 3.15 (t, J = 12 Hz, 1H), 3.27 (s, 3H), 3.37 (t, J = 4.6 Hz, 2H), 3.95 (d, J = 12 Hz, 1H), 4.30-4.40 (m, 1H), 4.42 (d, J = 12 Hz, 1H), 4.81 (s, 2H), 7.02-7.05 (m, 1H), 7.02-7.08 (m, J = 0.6-7.8 Hz, 3H), 7.50 (dd, J = 06-7.8 Hz, 1H), 7.59 (dd, J = 1.2-7.6 Hz, 1H), 7.76 (dd, J = 1.4-7.7 Hz, 1H), 7.92 (dd, J = 0.9-7.7 Hz, 1H), 7.98 (s, 1H), 8.53 (s, 1H).

### Exemple 39 : 3-méthyl-5-(3-méthyl-pyridin-4-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 39) (example comparatif)

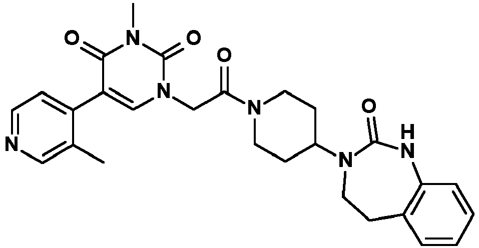

### 39.1: 3-[1-(2-chloro-acétyl)-pipéridin-4-yl]-1,3,4,5-tétrahydro-benzo[d][1,3]diazépin-2-one

De manière analogue à l'exemple 2.4, à partir de 300mg (1,2 mmoles) de 3-pipéridin-4-yl-1,3,4,5-tétrahydro-benzo[d][1,3]diazépin-2-one (préparé comme décrit dans l'exemple 16.4) et 254mg (2,7 mmoles) d'acide chloroacétique, 350mg (89%) de 3-[1-(2-chloro-acétyl)-pipéridin-4-yl]-1,3,4,5-tétrahydro-benzo[d][1,3]diazépin-2-one sont obtenus sous la forme d'un solide blanc.

### 39.2: 1-benzhydryl-3-méthyl-5-(3-méthyl-pyridin-4-yl)-1H-pyrimidine-2,4-dione

De manière analogue à l'exemple 1.8, à partir de 280mg (0,8 mmoles) de 1-benzhydryl-5-bromo-3-méthyl-1H-pyrimidine-2,4-dione (préparé comme décrit dans l'exemple 2.2), et 207mg (1,5 mmoles) d'acide 3-méthylpyridine-4-boronique, 250mg (86%) de 1-benzhydryl-3-méthyl-5-(3-méthyl-pyridin-4-yl)-1H-pyrimidine-2,4-dione sont obtenus sous la forme d'une huile incolore.

### 39.3: 3-méthyl-5-(3-méthyl-pyridin-4-yl)-1H-pyrimidine-2,4-dione

De manière analogue à l'exemple 2.3, à partir de 250mg (0,7 mmoles) de 1-benzhydryl-3-méthyl-5-(3-méthyl-pyridin-4-yl)-1H-pyrimidine-2,4-dione, 50mg (35%) de 3-méthyl-5-(3-méthyl-pyridin-4-yl)-1H-pyrimidine-2,4-dione sont obtenus sous la forme d'un solide blanc.

### 39.4: 3-méthyl-5-(3-méthyl-pyridin-4-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 39)

De manière analogue à l'exemple 13.1, à partir de 82mg (0,3 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et 50mg (0,2 mmoles) de 3-méthyl-5-(3-méthyl-pyridin-4-yl)-1H-pyrimidine-2,4-dione, et après purification par chromatographie sur gel de silice élué avec un mélange dichlorométhane / méthanol 97/3, 60mg (52%) de 3-méthyl-5-(3-méthyl-pyridin-4-yl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 178°C.
RMN ¹H (δ, DMSO) : 1.40-1.55 (m, 1H), 1.58-1.70 (m, 3H), 2.13 (s, 3H), 2.61 (t, J = 12 Hz, 1H), 2.82 (t, J = 4.5 Hz, 2H), 3.08 (t, J = 12 Hz, 1H), 3.18 (s, 3H), 3.30 (t, J = 4.6 Hz, 2H), 3.87 (d, J = 13 Hz, 1H), 4.20-4.30 (m, 1H), 4.35 (d, J = 13 Hz, 1H), 4.71 (s, 2H), 6.71-6.75 (m, 1H), 6.95-6.98 (m, 3H), 7.10 (d, J = 4.9 Hz, 1H), 7.72 (s, 1H), 8.34 (d, J = 4.8 Hz, 1H), 8.41 (s, 1H), 8.45 (s, 1H).

### Exemple 40 : 3-méthyl-5-(2-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 40) (example comparatif)

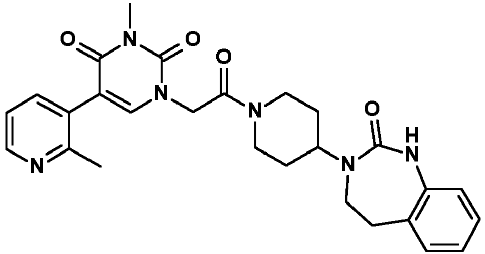

### 40.1: 1-benzhydryl-3-méthyl-5-(2-méthyl-pyridin-3-yl)-1H-pyrimidine-2,4-dione

De manière analogue à l'exemple 1.8, à partir de 280mg (0,8 mmoles) de 1-benzhydryl-5-bromo-3-méthyl-1H-pyrimidine-2,4-dione (préparé comme décrit dans l'exemple 2.2), et 207mg (1,5 mmoles) d'acide 2-méthylpyridine-3-boronique, 40mg (14%) de 1-benzhydryl-3-méthyl-5-(2-méthyl-pyridin-3-yl)-1H-pyrimidine-2,4-dione sont obtenus sous la forme d'une huile incolore.

### 40.2: 3-méthyl-5-(2-méthyl-pyridin-3-yl)-1H-pyrimidine-2,4-dione

De manière analogue à l'exemple 2.3, à partir de 40mg (0,1 mmoles) de 1-benzhydryl-3-méthyl-5-(2-méthyl-pyridin-3-yl)-1H-pyrimidine-2,4-dione, 23mg (100%) de 3-méthyl-5-(2-méthyl-pyridin-3-yl)-1H-pyrimidine-2,4-dione sont obtenus sous la forme d'un solide jaune.

### 40.3: 3-méthyl-5-(2-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 40)

De manière analogue à l'exemple 13.1, à partir de 37mg (0,1 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et 23mg (0,1 mmoles) de 3-méthyl-5-(2-méthyl-pyridin-3-yl)-1H-pyrimidine-2,4-dione, 15mg (28%) de 3-méthyl-5-(2-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 163°C.

RMN ¹H (δ, DMSO) : 1.48-1.60 (m, 1H), 1.62-1.80 (m, 3H), 2.38 (s, 3H), 2.70 (t, J = 12 Hz, 1H), 2.89 (t, J = 4.5 Hz, 2H), 3.15 (t, J = 12 Hz, 1H), 3.25 (s, 3H), 3.37 (t, J = 4.5 Hz, 2H), 3.95 (d, J = 12 Hz, 1H), 4.28-4.38 (m, 1H), 4.42 (d, J = 12 Hz, 1H), 4.77 (s, 2H), 6.78-6.83 (m, 1H), 7.02-7.04 (m, 3H), 7.27 (m, 1H), 7.53 (dd, J = 1.7-7.7 Hz, 1H), 7.75 (s, 1H), 8.45 (q, J = 1.7 Hz, 1H), 5.84 (s, 1H).

### Exemple 41 : 3-méthyl-5-(4-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 41) (example comparatif)

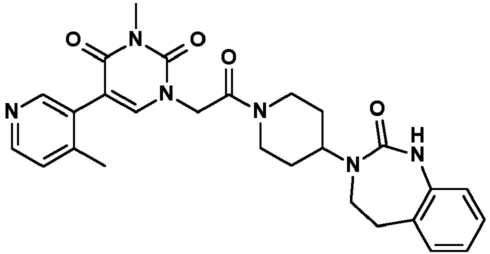

### 41.1: 1-benzhydryl-3-méthyl-5-(4-méthyl-pyridin-3-yl)-1H-pyrimidine-2,4-dione

De manière analogue à l'exemple 1.8, à partir de 280mg (0,8 mmoles) de 1-benzhydryl-5-bromo-3-méthyl-1H-pyrimidine-2,4-dione (préparé comme décrit dans l'exemple 2.2), et 207mg (1,5 mmoles) d'acide 4-méthylpyridine-3-boronique, 270mg (93%) de 1-benzhydryl-3-méthyl-5-(4-méthyl-pyridin-3-yl)-1H-pyrimidine-2,4-dione sont obtenus sous la forme d'une huile orange.

### 41.2: 3-méthyl-5-(4-méthyl-pyridin-3-yl)-1H-pyrimidine-2,4-dione

De manière analogue à l'exemple 2.3, à partir de 270mg (0,7 mmoles) de 1-benzhydryl-3-méthyl-5-(4-méthyl-pyridin-3-yl)-1H-pyrimidine-2,4-dione, 153mg (100%) de 3-méthyl-5-(4-méthyl-pyridin-3-yl)-1H-pyrimidine-2,4-dione sont obtenus sous la forme d'un solide jaune.

### 41.3: 3-méthyl-5-(4-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 41)

De manière analogue à l'exemple 13.1, à partir de 140mg (0,4 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et 86mg (0,4 mmoles) de 3-méthyl-5-(4-méthyl-pyridin-3-yl)-1H-pyrimidine-2,4-dione, 20mg (10%) de 3-méthyl-5-(4-méthyl-pyridin-3-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 170°C.

RMN ¹H (δ, DMSO) : 1.48-1.60 (m, 1H), 1.62-1.80 (m, 3H), 2.21 (s, 3H), 2.7 (t, J = 12 Hz, 1H), 2.88 (t, J = 4.3 Hz, 2H), 3.15 (t, J = 12 Hz, 1H), 3.25 (s, 3H), 3.36 (t, J = 4.4 Hz, 2H), 3.95 (d, J = 12 Hz, 1H), 4.28-4.38 (m, 1H), 4.45 (d, J = 12 Hz, 1H), 4.78 (s, 2H), 6.78-6.82 (m, 1H), 7.01-7.04 (m, 3H), 7.31 (d, J = 5 Hz, 1H), 7.79 (s, 1H), 8.28 (s, 1H), 8.43 (d, J = 5 Hz, 1H), 8.54 (s, 1H).

### Exemple 42 : 3-méthyl-5-(6-méthyl-pyridin-2-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 42) (example comparatif)

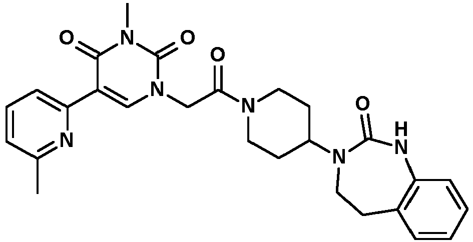

### 42.1: 1-benzhydryl-3-méthyl-5-(6-méthyl-1-oxy-pyridin-2-yl)-1H-pyrimidine-2,4-dione

15mg (0,1 mmoles) d'acétate de palladium(II) et 20mg (0,1 mmoles) de tétrafluoroborate de tri-tert-butylphosphonium sont ajoutés à une solution de 500mg (1,4 mmoles) de 1-benzhydryl-5-bromo-3-méthyl-1H-pyrimidine-2,4-dione (préparé comme décrit dans l'exemple 2.2), 294mg (2,7 mmoles) de 2-picoline-N-oxide, et 279mg (2 mmoles) de carbonate de potassium dans 10ml de toluène, préalablement dégazée avec de l'azote pendant 10 minutes. Le milieu réactionnel est chauffé à 110°C dans un tube scellé pendant 40 heures. Il est filtré sur célite et le filtrat est concentré sous vide. Le résidu brut est chromatographié sur gel de silice élué par un mélange dichlorométhane / acétone 70/30. 220mg (41%) de 1-benzhydryl-3-méthyl-5-(6-méthyl-1-oxypyridin-2-yl)-1H-pyrimidine-2,4-dione sont obtenus sous la forme d'un solide blanc cassé.

### 42.2: 1-benzhydryl-3-méthyl-5-(6-méthyl-pyridin-2-yl)-1H-pyrimidine-2,4-dione

87mg (2 mmoles) de fer sont ajoutés à une solution de 125mg (0,3 mmoles) de 1-benzhydryl-3-méthyl-5-(6-méthyl-1-oxy-pyridin-2-yl)-1H-pyrimidine-2,4-dione dans 9,4ml d'acide acétique. Le milieu réactionnel est chauffé à 70°C dans un tube scellé pendant 4 heures. Il est filtré sur célite puis le filtrat est hydrolysé par une solution aqueuse saturée d'hydrogénocarbonate de sodium. Le produit est extrait à l'acétate d'éthyle. La phase organique est lavée par une solution aqueuse saturée d'hydrogénocarbonate de sodium, puis par de l'eau et par une solution aqueuse saturée de chlorure de sodium, séchée sur sulfate de magnésium, filtrée et concentrée sous vide. 120mg (100%) de 1-benzhydryl-3-méthyl-5-(6-méthyl-pyridin-2-yl)-1H-pyrimidine-2,4-dione sont obtenus sous la forme d'un solide beige.

### 42.3: 3-méthyl-5-(6-méthyl-pyridin-2-yl)-1H-pyrimidine-2,4-dione

De manière analogue à l'exemple 2.3, à partir de 135mg (0,4 mmoles) de 1-benzhydryl-3-méthyl-5-(6-méthyl-pyridin-2-yl)-1H-pyrimidine-2,4-dione, 76mg (100%) de 3-méthyl-5-(6-méthyl-pyridin-2-yl)-1H-pyrimidine-2,4-dione sont obtenus sous la forme d'un solide jaune.

### 42.4: 3-méthyl-5-(6-méthyl-pyridin-2-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 42)

De manière analogue à l'exemple 13.1, à partir de 136mg (0.4 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et 66mg (0,4 mmoles) de 3-méthyl-5-(6-méthyl-pyridin-2-yl)-1H-pyrimidine-2,4-dione, 105mg (59%) de 3-méthyl-5-(6-méthyl-pyridin-2-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 248°C.
RMN ¹H (δ, DMSO) : 1.65-1.71 (m, 4 H); 2.50 (s, 3 H); 2.69 (t, J = 12.3 Hz, 1 H); 2.88-2.93 (m, 2 H); 3.18 (t, J = 13.1 Hz, 1 H); 3.28 (s, 3 H); 3.38-3.41 (m, 2 H); 3.95 (d, J = 13.6 Hz, 1 H); 4.29-4.36 (m, 1 H); 4.43 (d, J = 13.0 Hz, 1 H) ; 4.94 (s, 2 H); 6.80-6.82 (m, 1 H); 7.03-7.05 (m, 3 H); 7.16 (d, J = 7.6 Hz, 1 H); 7.71 (t, J = 7.8 Hz, 1 H); 8.08 (d, J = 8.0 Hz, 1 H); 8.45 (s, 1 H); 8.54 (s, 1 H).

### Exemple 43 : 3-méthyl-5-(6-méthyl-1-oxy-pyridin-2-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 43) (example comparatif)

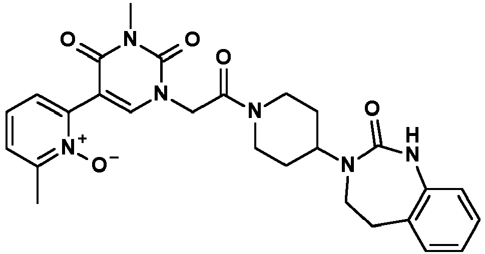

### 43.1: 3-méthyl-5-(6-méthyl-1-oxy-pyridin-2-yl)-1H-pyrimidine-2,4-dione

De manière analogue à l'exemple 2.3, à partir de 63mg (0,2 mmoles) de 1-benzhydryl-3-méthyl-5-(6-méthyl-1-oxy-pyridin-2-yl)-1H-pyrimidine-2,4-dione (préparé comme décrit dans l'exemple 42.1), 37mg (100%) de 3-méthyl-5-(6-méthyl-1-oxypyridin-2-yl)-1H-pyrimidine-2,4-dione sont obtenus sous la forme d'un solide blanc.

### 43.2: 3-méthyl-5-(6-méthyl-1-oxy-pyridin-2-yl)-1-{2-οxο-2-[4-(2-oxo-1,2,4,5-tétrahydro-benzo[d][1,3]diazépin-3-yl)-pipéridin-1-yl]-éthyl}-1H-pyrimidine-2,4-dione (composé 43)

De manière analogue à l'exemple 13.1, à partir de 61mg (0,2 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et 37mg (0,2 mmoles) de 3-méthyl-5-(6-méthyl-pyridin-2-yl)-1H-pyrimidine-2,4-dione, 15mg (18%) de 3-méthyl-5-(6-méthyl-1-oxy-pyridin-2-yl)-1-{2-oxo-2-[4-(2-oxo-1,2, 4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.62-1.69 (m, 4H); 2.38 (s, 3H); 2.68 (m, 1H); 2.88-2.90 (m, 2H); 3.15 (m, 1H); 3.24 (s, 3 H); 3.36-3.38 (m, 2H); 3.94 (d, J = 13.7 Hz, 1H); 4.32 (m, 1H); 4.42 (d, J = 13.0 Hz, 1H); 4.81 (s, 2H); 6.80-6.82 (m, 1H); 7.03-7.05 (m, 3H); 7.27 (t, J = 7.8 Hz, 1H); 7.47-7.50 (m, 2H); 8.29 (s, 1H); 8.54 (s, 1H).

### Exemple 44 : 3-méthyl-5-(1-méthyl-1H-pyrazol-4-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 44) (example comparatif)

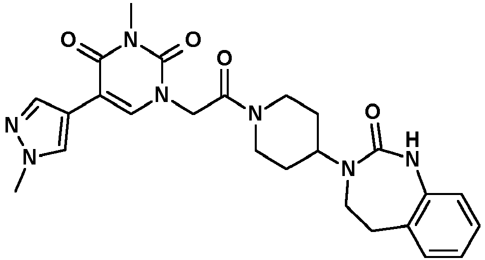

### 44.1: [3-méthyl-5-(1-méthyl-1H-pyrazol-4-yl)-2,4-dioxo-3,4-dihydro-2H-pyrimidin-1-yl]-acétate de méthyle

44,2mg (50 µmoles) de complexe de dichlorométhane de 1,1'-bis-(diphénylphosphino)ferrocene-dichloropalladium(II) sont ajoutés à une solution contenant 300mg (1,1 mmoles) de (5-bromo-3-méthyl-2,4-dioxo-3,4-dihydro-2H-pyrimidin-1-yl)-acétate de méthyle (préparé comme décrit dans l'exemple 17.1), 338mg (1,6 mmoles) de 1-méthyl-4-(4,4,5,5-tétraméthyl-[1,3,2]dioxaborolan-2-yl)-1H-pyrazole et 344mg (3,3 mmoles) de carbonate de sodium dans 30ml de 1,4-dioxane et 3ml d'eau, préalablement dégazée avec de l'azote pendant 5 minutes. Le milieu réactionnel est ensuite chauffé à 100°C pendant 1 heure. Il est hydrolysé puis dilué par de l'acétate d'éthyle. Le produit est extrait à l'acétate d'éthyle, les phases organiques sont rassemblées, lavées une fois par de l'eau et une fois par une solution aqueuse saturée de chlorure de sodium, puis séchées sur sulfate de magnésium, filtrées et concentrées sous vide. Le résidu brut est chromatographié sur gel de silice élué par un mélange dichlorométhane / méthanol 97/3. 60mg (20%) de [3-méthyl-5-(1-méthyl-1H-pyrazol-4-yl)-2,4-dioxo-3,4-dihydro-2H-pyrimidin-1-yl]-acétate de méthyle sont obtenus sous la forme d'un solide beige.

### 44.2: acide [3-méthyl-5-(1-méthyl-1H-pyrazol-4-yl)-2,4-dioxo-3,4-dihydro-2H-pyrimidin-1-yl]-acétique

De manière analogue à l'exemple 16.8, à partir de 60mg (0,2 mmoles) de [3-méthyl-5-(1-méthyl-1H-pyrazol-4-yl)-2,4-dioxo-3,4-dihydro-2H-pyrimidin-1-yl]-acétate de méthyle, 57mg (100%) d'acide [3-méthyl-5-(1-méthyl-1H-pyrazol-4-yl)-2,4-dioxo-3,4-dihydro-2H-pyrimidin-1-yl]-acétique sont obtenus sous la forme d'un solide beige.

### 44.3: 3-méthyl-5-(1-méthyl-1H-pyrazol-4-yl)-1-{2-οxο-2-[4-(2-oxo-1,2,4,5-tétrahydro-benzo[d][1,3]diazépin-3-yl)-pipéridin-1-yl]-éthyl}-1H-pyrimidine-2,4-dione (composé 44)

De manière analogue à l'exemple 1.6, à partir de 67mg (0,2 mmoles) d'acide [3-méthyl-5-(1-méthyl-1H-pyrazol-4-yl)-2,4-dioxo-3,4-dihydro-2H-pyrimidin-1-yl]-acétique et 58mg (0,2 mmoles) de 3-pipéridin-4-yl-1,3,4,5-tétrahydro-benzo[d] [1,3]diazépin-2-one (préparé comme décrit dans l'exemple 16.4), et après recristallisation dans l'éthanol, 45mg (42%) de 3-méthyl-5-(1-méthyl-1H-pyrazol-4-yl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 290°C.
RMN ¹H (δ, DMSO) : 1.61-1.70 (m, 4H); 2.69 (t, J = 12.8 Hz, 1H); 2.91 (m, 2H); 3.10-3.26 (m, 2H); 3.25 (s, 3H); 3.36-3.41 (m, 2H); 3.86 (s, 3H); 3.98 (d, J = 13.6 Hz, 1H); 4.28-4.38 (m, 1H); 4.43 (d, J = 13.0 Hz, 1H); 4.75 (d, J = 6.0 Hz, 2H); 6.80-6.82 (m, 1H); 7.04-7.05 (m, 3H); 7.74 (s, 1H); 8.03 (s, 1H); 8.10 (s, 1H); 8.55 (s, 1H).

### Exemple 45 : 5-(5-chloro-pyridin-3-yl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 45) (example comparatif)

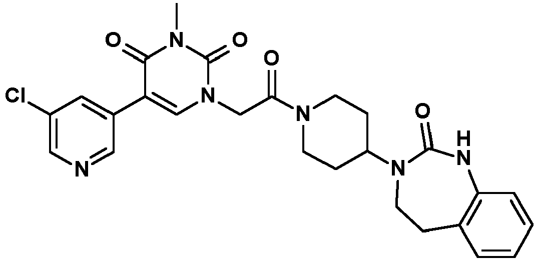

### 45.1: 1-benzhydryl-5-(5-chloro-pyridin-3-yl)-3-méthyl-1H-pyrimidine-2,4-dione

De manière analogue à l'exemple 44.1, à partir de 191mg (1,2 mmoles) d'acide 5-chloropyridine-3- boronique et 300mg (0,8 mmoles) de 1-benzhydryl-5-bromo-3-méthyl-1H-pyrimidine-2,4-dione (préparé comme décrit dans l'exemple 2.2), 340mg (96%) de 1-benzhydryl-5-(5-chloro-pyridin-3-yl)-3-méthyl-1H-pyrimidine-2,4-dione sont obtenus sous la forme d'un solide blanc.

### 45.2: 5-(5-chloro-pyridin-3-yl)-3-méthyl-1H-pyrimidine-2,4-dione

De manière analogue à l'exemple 2.3, à partir de 326mg (0,8 mmoles) de 1-benzhydryl-5-(5-chloro-pyridin-3-yl)-3-méthyl-1H-pyrimidine-2,4-dione, 190mg (99%) de 5-(5-chloro-pyridin-3-yl)-3-méthyl-1H-pyrimidine-2,4-dione sont obtenus sous la forme d'un solide blanc.

### 45.3: 5-(5-chloro-pyridin-3-yl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 45)

De manière analogue à l'exemple 13.1, à partir de 190mg (0,8 mmoles) de 5-(5-chloro-pyridin-3-yl)-3-méthyl-1H-pyrimidine-2,4-dione et 283mg (0,9 mmoles) de 3-[1-(2-chloro-acétyl)-pipéridin-4-yl]-1,3,4,5-tétrahydro-benzo[d] [1,3]diazépin-2-one (préparé comme décrit dans l'exemple 39.1), après chauffage à 80°C pendant 1h30 et purification par chromatographie sur gel de silice élué par un mélange dichlorométhane / méthanol 95/5, 180mg (43%) de 5-(5-chloro-pyridin-3-yl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 250°C.
RMN ¹H (δ, DMSO) : 1.56-1.65 (m, 4H); 2.69 (t, J = 12.2 Hz, 1H); 2.87-2.92 (m, 2H); 3.16-3.21 (m, 1H); 3.27 (s, 3H); 3.36-3.39 (m, 2H); 3.97 (d, J = 13.6 Hz, 1H); 4.27-4.40 (m, 1H); 4.42 (d, J = 13.1 Hz, 1H); 4.81 (s, 2H); 6.80-6.81 (m, 1H); 7.02-7.05 (m, 3H); 8.14 (t, J = 2.1 Hz, 1H); 8.22 (s, 1H); 8.55 (s, 1H); 8.58 (d, J = 2.4 Hz, 1H); 8.75 (d, J = 1.9 Hz, 1H).

### Exemple 46 : 5-benzyl-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 46) (example comparatif)

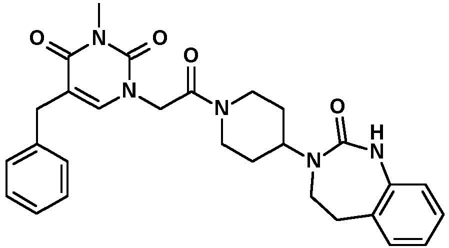

### 46.1: 5-benzyl-1H-pyrimidine-2,4-dione

20ml de benzène sont ajoutés à une solution de 2g (14 mmoles) de 5-hydroxyméthyl-1H-pyrimidine-2,4-dione dans 40ml d'acide trifluoroacétique. Le mélange est chauffé à 130°C pendant 20 minutes dans des tubes scellés. Après refroidissement, les solvants sont évaporés et le résidu est repris dans du dichlorométhane. Le produit précipite, il est filtré et séché sous vide. 2,4g (84%) de 5-benzyl-1H-pyrimidine-2,4-dione sont obtenus sous la forme d'un solide blanc.

### 46.2: (5-benzyl-2,4-dioxo-3,4-dihydro-2H-pyrimidin-1-yl)-acétate de méthyle

De manière analogue à l'exemple 13.1, à partir de 200mg (1 mmole) de 5-benzyl-1H-pyrimidine-2,4-dione, 105mg (39%) de (5-benzyl-2,4-dioxo-3,4-dihydro-2H-pyrimidin-1-yl)-acétate de méthyle sont obtenus sous la forme d'un solide blanc.

### 46.3: acide (5-benzyl-3-méthyl-2,4-dioxo-3,4-dihydro-2H-pyrimidin-1-yl)-acétique

75,6mg (0,6 mmoles) de carbonate de potassium et 27µl (0,4 mmoles) de iodométhane sont ajoutés à une solution de (5-benzyl-2,4-dioxo-3,4-dihydro-2H-pyrimidin-1-yl)-acétate de méthyle dans 5ml de diméthylformamide. Le milieu réactionnel est chauffé à 50°C pendant 1h30. 0,6ml (0,6 mmoles) d'une solution aqueuse d'hydroxyde de lithium 1M sont rajoutés avec 1ml d'eau et 2,5ml de tétrahydrofurane. Après 20 heures d'agitation à température ambiante, le mélange est hydrolysé par une solution aqueuse d'acide chlorhydrique 1N puis dilué par de l'acétate d'éthyle. Le produit est extrait à l'acétate d'éthyle. Les phases organiques sont rassemblées, lavées deux fois par de l'eau et une fois par une solution aqueuse saturée de chlorure de sodium, puis séchées sur sulfate de magnésium, filtrées et concentrées sous vide. 100mg (100%) d'acide (5-benzyl-3-méthyl-2,4-dioxo-3,4-dihydro-2H-pyrimidin-1-yl)-acétique sont obtenus sous la forme d'une huile incolore.

### 46.4: 5-benzyl-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 46)

De manière analogue à l'exemple 1.6, à partir de 100mg (0,4 mmoles) d'acide (5-benzyl-3-méthyl-2,4-dioxo-3,4-dihydro-2H-pyrimidin-1-yl)-acétique et 107mg (0,4 mmoles) de 3-pipéridin-4-yl-1,3,4,5-tétrahydro-benzo[d][1,3]diazépin-2-one (préparé comme décrit dans l'exemple 16.4), 150mg (79%) de 5-benzyl-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 266°C.
RMN ¹H (δ, DMSO) : 1.56-1.68 (m, 4H); 2.67 (t, J = 12.6 Hz, 1H); 2.87-2.92 (m, 2H); 3.11-3.14 (m, 1H); 3.18 (m, 3H); 3.36-3.39 (m, 2H); 3.59 (s, 2H); 3.93 (d, J = 13.6 Hz, 1H); 4.32 (m, 1H); 4.42 (d, J = 13.0 Hz, 1H); 4.71 (d, J = 2.6 Hz, 2H); 6.80-6.81 (m, 1H); 7.04-7.05 (m, 3H); 7.24-7.26 (m, 5H); 7.53 (s, 1H); 8.54 (s, 1H).

### Exemple 47 : 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 47) (example comparatif)

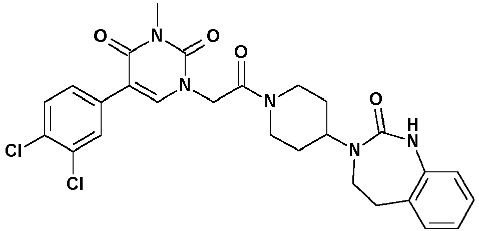

### 47.1: acide [5-(3,4-dichloro-phényl)-3-méthyl-2,4-dioxo-3,4-dihydro-2H-pyrimidin-1-yl]-acétique

De manière analogue à l'exemple 44.1, à partir de 450mg (1,6 mmoles) de (5-bromo-3-méthyl-2,4-dioxo-3,4-dihydro-2H-pyrimidin-1-yl)-acétate de méthyle (préparé comme décrit dans l'exemple 17.1) et 620mg (3,3 mmoles) d'acide 3,4-dichlorophénylboronique, et après ajout de 5ml d'une solution de soude aqueuse 1N et chauffage à 100°C pendant 1 heure, 520mg (97%) d' acide [5-(3,4-dichloro-phényl)-3-méthyl-2,4-dioxo-3,4-dihydro-2H-pyrimidin-1-yl]-acétique sont obtenus sous la forme d'un solide beige.

### 47.2: 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 47)

De manière analogue à l'exemple 1.6, à partir de 400mg (1,2 mmoles) d' acide [5-(3,4-dichloro-phényl)-3-méthyl-2,4-dioxo-3,4-dihydro-2H-pyrimidin-1-yl]-acétique et 358mg (1,5 mmoles) de 3-pipéridin-4-yl-1,3,4,5-tétrahydro-benzo[d][1,3]diazépin-2-one (préparé comme décrit dans l'exemple 16.4), 320mg (47%) de 5-(3,4-dichloro-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.52-1.61 (m, 4 H). 2.63 (t, J = 10.6 Hz, 1 H); 2.84 (m, 2 H); 3.10 (m, 1 H); 3.19 (s, 3 H), 3.30-3.33 (m, 2 H); 3.90 (d, J = 13.6 Hz, 1 H); 4.26 (m, 1 H); 4.35 (d, J = 12.5 Hz, 1 H); 4.74 (s, 2 H); 6.73-6.74 (m, 1 H); 6.96-6.97 (m, 3 H); 7.55 (dd, J = 8.5, 2.1 Hz, 1 H); 7.62 (d, J = 8.5 Hz, 1 H); 7.81 (d, J = 2.1 Hz, 1 H); 8.05 (s, 1 H); 8.47 (s, 1H).

### Exemple 48 : 5-(5-chloro-2-méthyl-phényl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 48) (example comparatif)

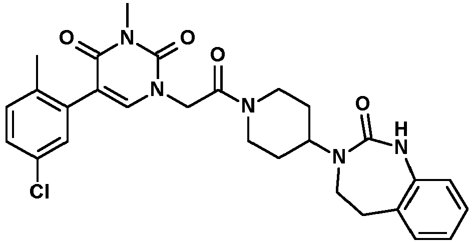

### 48.1: acide [5-(5-chloro-2-méthyl-phényl)-3-méthyl-2,4-dioxo-3,4-dihydro-2H-pyrimidin-1-yl]-acétique

De manière analogue à l'exemple 47.1, à partir de 400mg (1,4 mmoles) de (5-bromo-3-méthyl-2,4-dioxo-3,4-dihydro-2H-pyrimidin-1-yl)-acétate de méthyle (préparé comme décrit dans l'exemple 17.1) et 492mg (2,9 mmoles) d'acide (5-chloro-2-méthyl-phényl)boronique, et après ajout de 5ml d'une solution de soude aqueuse 1N et chauffage à 100°C pendant 1 heure, 440mg (99%) d' acide [5-(5-chloro-2-méthyl-phényl)-3-méthyl-2,4-dioxo-3,4-dihydro-2H-pyrimidin-1-yl]-acétique sont obtenus sous la forme d'un solide beige.

### 48.2: 5-(5-chloro-2-méthyl-phényl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 48)

De manière analogue à l'exemple 1.6, à partir de 445mg (1,4 mmoles) d'acide [5-(5-chloro-2-méthyl-phényl)-3-méthyl-2,4-dioxo-3,4-dihydro-2H-pyrimidin-1-yl]-acétique et 424mg (1,7 mmoles) de 3-pipéridin-4-yl-1,3,4,5-tétrahydro-benzo[d] [1,3]diazépin-2-one (préparé comme décrit dans l'exemple 16.4), 162mg (19%) de 5-(5-chloro-2-méthyl-phényl)-3-méthyl-1-{2-oxo-2-[4-(2-oxo-1,2,4,5-tétrahydro-benzo[d] [1,3]diazépin-3-yl)-pipéridin-1-yll-éthyl}-1H-pyrimidine-2,4-dione sont obtenus sous la forme d'un solide blanc.
RMN ¹H (δ, DMSO) : 1.57-1.66 (m, 4 H); 2.16 (s, 3 H); 2.68 (t, J = 12.6 Hz, 1 H); 2.89 (d, J = 6.7 Hz, 2 H); 3.15 (t, J = 12.3 Hz, 1 H); 3.24 (s, 3 H); 3.35-3.38 (m, 2 H); 3.94 (d, J = 13.7 Hz, 1 H); 4.26-4.39 (m, 1 H); 4.42 (d, J = 12.9 Hz, 1 H); 4.76 (d, J = 2.6 Hz, 2 H); 6.79-6.80 (m, 1 H); 7.03-7.05 (m, 3 H); 7.19 (d, J = 2.3 Hz, 1 H); 7.29 (d, J = 8.3 Hz, 1 H); 7.34 (dd, J = 8.2, 2.3 Hz, 1 H); 7.73 (s, 1 H); 8.52 (s, 1 H).

### Exemple 49 : 5-(4,5-diméthyl-pyridin-3-yl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 49) (example comparatif)

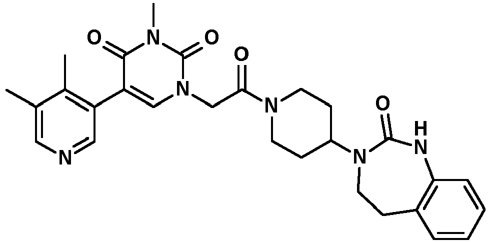

### 49.1: 1-benzhydryl-5-(4,5-diméthyl-pyridin-3-yl)-3-méthyl-1H-pyrimidine-2,4-dione

De manière analogue à l'exemple 44.1, à partir de 244mg (1,6 mmoles) d'acide 4,5-diméthyl-pyridine boronique et 400mg (1,1 mmoles) de 1-benzhydryl-5-bromo-3-méthyl-1H-pyrimidine-2,4-dione (préparé comme décrit dans l'exemple 2.2), 290mg (68%) de 1-benzhydryl-5-(4,5-diméthyl-pyridin-3-yl)-3-méthyl-1H-pyrimidine-2,4-dione sont obtenus sous la forme d'une huile jaune clair.

### 49.2: 5-(4,5-diméthyl-pyridin-3-yl)-3-méthyl-1H-pyrimidine-2,4-dione

De manière analogue à l'exemple 2.3, à partir de 290mg (0,7 mmoles) de 1-benzhydryl-5-(4,5-diméthyl-pyridin-3-yl)-3-méthyl-1H-pyrimidine-2,4-dione, 120mg (71%) de 5-(4,5-diméthyl-pyridin-3-yl)-3-méthyl-1H-pyrimidine-2,4-dione sont obtenus sous la forme d'un solide blanc.

### 49.3: 5-(4,5-diméthyl-pyridin-3-yl)-3-méthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 49)

De manière analogue à l'exemple 13.1, à partir de 120mg (0,5 mmoles) de 5-(4,5-diméthyl-pyridin-3-yl)-3-méthyl-1H-pyrimidine-2,4-dione et 184mg (0,6 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), après chauffage à 50°C pendant 18 heures et purification par cristallisation dans l'éthanol, 120mg (45%) de 5-(4,5-diméthyl-pyridin-3-yl)-3-méthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 225°C.
RMN ¹H (δ, DMSO) : 1.58-1.70 (m, 4H); 2.10 (s, 3H); 2.28 (s, 3H); 2.69 (t, J = 12.5 Hz, 1H); 2.87-2.92 (m, 2H); 3.16 (t, J = 12.2 Hz, 1H); 3.26 (s, 3H); 3.36-3.39 (m, 2H); 3.96 (d, J = 13.6 Hz, 1H); 4.33 (m, 1H); 4.43 (d, J = 13.0 Hz, 1H); 4.78 (s, 2H); 6.80-6.81 (m, 1H); 7.03-7.04 (m, 3H); 7.75 (s, 1H); 8.12 (s, 1H); 8.33 (s, 1H); 8.54 (s, 1H).

### Exemple 50 : 3-méthyl-1-{2-oxo-2-[4-(2-oxo-1,2,4,S-tétrahydro-benzo[d][1,3]diazépin-3-yl)-pipéridin-1-yl]-éthyl}-5-phénoxy-1H-pyrimidine-2,4-dione (schéma réactionnel n°3, composé 50) (example comparatif)

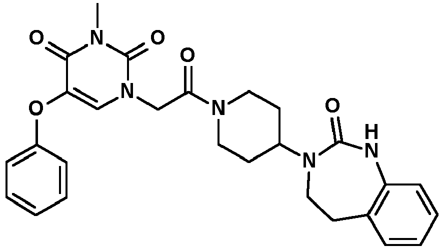

### 50.1: 1-benzhydryl-3-méthyl-5-phénoxy-1H-pyrimidine-2,4-dione

254mg (2,7 mmoles) de phénol, 67mg (0,7 mmoles) de chlorure de cuivre, 25mg (0,1 mmoles) de 2,2,6,6-tétraméthyl-3,5-heptanedione et 878mg (2,7 mmoles) de carbonate de césium sont ajoutés à une solution de 500mg (1,4 mmoles) de 1-benzhydryl-5-bromo-3-méthyl-1H-pyrimidine-2,4-dione dans 2,5ml de 1-méthyl-2-pyrrolidinone, préalablement dégazée à l'azote. Le mélange réactionnel est chauffé à 120°C pendant 22 heures puis dilué dans 6ml d'acétate d'éthyle et filtré sur célite. Les solvants sont évaporés, le résidu est lavé une fois par une solution aqueuse d'acide chlorhydrique 1N puis par une solution aqueuse d'hydroxyde de sodium 1N et par de l'eau. La phase organique est séchée sur sulfate de magnésium, filtrée et concentrée sous vide. Le résidu brut est chromatographié sur gel de silice élué par un mélange heptane / acétate d'éthyle 80/20. 105mg (20%) de 1-benzhydryl-3-méthyl-5-phénoxy-1H-pyrimidine-2,4-dione sont obtenus sous la forme d'un solide jaune.

### 50.2: 3-méthyl-5-phénoxy-1H-pyrimidine-2,4-dione

De manière analogue à l'exemple 2.3, à partir de 145mg (0,4 mmoles) de 1-benzhydryl-3-méthyl-5-phénoxy-1H-pyrimidine-2,4-dione, 85mg (100%) de 3-méthyl-5-phénoxy-1H-pyrimidine-2,4-dione sont obtenus sous la forme d'une huile jaune.

### 50.3: 3-méthyl-1-{2-oxo-2-[4-(2-oxo-1,2,4,S-tétrahydro-benzo[d][1,3]diazépin-3-yl)-pipéridin-1-yl]-éthyl}-5-phénoxy-1H-pyrimidine-2,4-dione (composé 50)

De manière analogue à l'exemple 13.1, à partir de 85mg (0,4 mmoles) de 3-méthyl-5-phénoxy-1H-pyrimidine-2,4-dione, 125mg (0,4 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et après chauffage à 50°C pendant 18 heures, 65mg (32%) de 3-méthyl-1-{2-oxo-2-[4-(2-oxo-1,2,4,5-tétrahydro-benzo[d][1,3]diazépin-3-yl)-pipéridin-1-yl]-éthyl}-5-phénoxy-1H-pyrimidine-2,4-dione sont obtenus sous la forme d'un solide jaune pâle de point de fusion 163°C.
RMN ¹H (δ, DMSO) : 1.55-1.68 (m, 4H); 2.69 (t, J = 12.5 Hz, 1H); 2.90 (m, 2H); 3.15 (m, 1H); 3.21 (s, 3H); 3.38 (m, 2H); 3.94 (d, J = 13.6 Hz, 1H); 4.33 (m, 1H); 4.44 (d, J = 13.0 Hz, 1H); 4.74 (s, 2H); 6.80-6.83 (m, 1H); 7.01-7.03 (m, 6H); 7.33 (t, J = 7.8 Hz, 2H); 7.92 (s, 1H); 8.54 (s, 1H).

### Exemple 51 : 5-(2,3-diméthyl-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 51) (example comparatif)

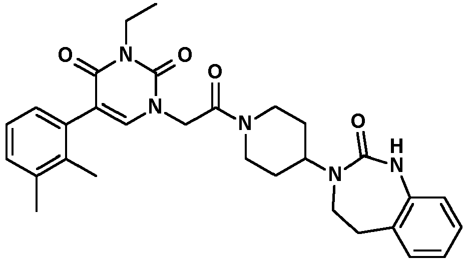

### 51.1: (5-bromo-3-éthyl-2,4-dioxo-3,4-dihydro-2H-pyrimidin-1-yl)-acétate de méthyle

2,4g (17 mmoles) de carbonate de potassium et 1,4ml (17 mmoles) de iodoéthane sont ajoutés à une solution de 3g (11 mmoles) de (5-bromo-2,4-dioxo-3,4-dihydro-2H-pyrimidin-1-yl)-acétate de méthyle (préparé comme décrit dans l'exemple 13.1) dans 60ml de diméthylformamide. Le milieu réactionnel est chauffé à 50°C pendant 2 heures puis hydrolysé et extrait par de l'acétate d'éthyle. La phase organique est lavée une fois par de l'eau et une fois par une solution aqueuse saturée de chlorure de sodium, puis séchée sur sulfate de magnésium, filtrée et concentrée sous vide. Le résidu brut est repris dans de l'éther diéthylique puis filtré et séché sous azote. 1,7g (50%) de (5-bromo-3-éthyl-2,4-dioxo-3,4-dihydro-2H-pyrimidin-1-yl)-acétate de méthyle sont obtenus sous la forme d'un solide crème.

### 51.2: [5-(2,3-diméthyl-phényl)-3-éthyl-2,4-dioxo-3,4-dihydro-2H-pyrimidin-1-yl]-acétate de méthyle

De manière analogue à l'exemple 44.1, à partir de 350mg (1,2 mmoles) de (5-bromo-3-éthyl-2,4-dioxo-3,4-dihydro-2H-pyrimidin-1-yl)-acétate de méthyle et 289mg (1,9 mmoles) d'acide 2,3-diméthylphénylboronique, et après purification par chromatographie sur gel de silice élué avec un mélange heptane / acétate d'éthyle 70/30, 255mg (67%) de [5-(2,3-diméthyl-phényl)-3-éthyl-2,4-dioxo-3,4-dihydro-2H-pyrimidin-1-yl]-acétate de méthyle sont obtenus sous la forme d'un solide blanc.

### 51.3: acide [5-(2,3-diméthyl-phényl)-3-éthyl-2,4-dioxo-3,4-dihydro-2H-pyrimidin-1-yl]-acétique

De manière analogue à l'exemple 16.8, à partir de 255mg (0,8 mmoles) de [5-(2,3-diméthyl-phényl)-3-éthyl-2,4-dioxo-3,4-dihydro-2H-pyrimidin-1-yl]-acétate de méthyle, 220mg (90%) d' acide [5-(2,3-diméthyl-phényl)-3-éthyl-2,4-dioxo-3,4-dihydro-2H-pyrimidin-1-yl]-acétique sont obtenus sous la forme d'un solide blanc.

### 51.4: 5-(2,3-diméthyl-phényl)-3-éthyl-1-{2-oxo-2-[4-(2-oxo-1,2, 4,5-tétrahydro-benzo[d][1,3]diazépin-3-yl)-pipéridin-1-yl]-éthyl}-1H-pyrimidine-2,4-dione (composé 51)

De manière analogue à l'exemple 1.6, à partir de 220mg (0,7 mmoles) d' acide [5-(2,3-diméthyl-phényl)-3-éthyl-2,4-dioxo-3,4-dihydro-2H-pyrimidin-1-yl]-acétique, 196mg (0,8 mmoles) de 3-pipéridin-4-yl-1,3,4,5-tétrahydro-benzo[d][1,3]diazépin-2-one (préparé comme décrit dans l'exemple 16.4) et 0,1ml (0,9 mmoles) de triéthylamine et après purification par chromatographie sur gel de silice élué avec un mélange dichlorométhane / méthanol 97/3 suivie d'une cristallisation dans un mélange heptane / acétate d'éthyle 70/30, 280mg (72%) de 5-(2,3-diméthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 170°C.
RMN ¹H (δ, DMSO) : 1.13 (t, J = 7 Hz, 3H) 1.48-1.60 (m, 1H), 1.62-1.80 (m, 3H), 2.05 (t, 3H), 2.30 (t, 3H), 2.68 (t, J = 11.7 Hz, 1H), 2.89 (t, J = 4.6 Hz, 2H), 3.15 (t, J = 11.7 Hz, 1H), 3.37 (t, J = 4.6 Hz, 2H), 3.88-3.95 (m, 3H), 4.28-4.36 (m, 1H), 4.43 (d, J = 12.7 Hz, 1H), 4.75 (d, J = 6.4 Hz, 2H), 6.77-6.83 (m, 1H), 6.96 (d, J = 7 Hz, 1H), 7.02-7.06 (m, 3H), 7.10 (t, J = 7.5 Hz, 1H), 7.17 (d, J = 7.3 Hz, 1H), 7.58 (s, 1H), 8.52 (s, 1H) .

### Exemple 52 : 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 52) (example comparatif)

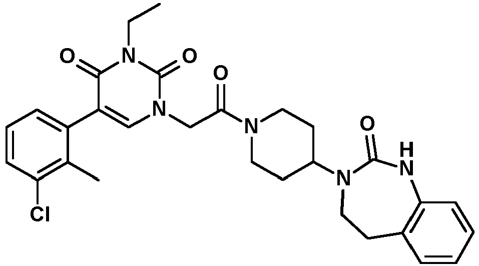

### 52.1: acide [5-(3-chloro-2-méthyl-phényl)-3-éthyl-2,4-dioxo-3,4-dihydro-2H-pyrimidin-1-yl]-acétique

De manière analogue à l'exemple 13.3, à partir de 350mg (1,2 mmoles) de (5-bromo-3-éthyl-2,4-dioxo-3,4-dihydro-2H-pyrimidin-1-yl)-acétate de méthyle (préparé comme décrit dans l'exemple 51.1), et 328mg (1,9 mmoles) d'acide 3-chloro-2-méthyl-phényl boronique, 330mg (85%) d'acide [5-(3-chloro-2-méthyl-phényl)-3-éthyl-2,4-dioxo-3,4-dihydro-2H-pyrimidin-1-yl]-acétique sont obtenus sous la forme d'un solide beige.

### 52.2: 5-(3-chloro-2-méthyl-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 52)

De manière analogue à l'exemple 51.4, à partir de 330mg (1 mmole) d' acide [5-(3-chloro-2-méthyl-phényl)-3-éthyl-2,4-dioxo-3,4-dihydro-2H-pyrimidin-1-yl]-acétique et 276mg (1,1 mmoles) de 3-pipéridin-4-yl-1,3,4,5-tétrahydro-benzo[d] [1,3]diazépin-2-one (préparé comme décrit dans l'exemple 16.4), 290mg (51%) de 5-(3-chloro-2-méthyl-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 247°C.
RMN ¹H (δ, DMSO) : 1.13 (t, J = 7 Hz, 3H), 1.50-1.62 (m, 1H), 1.52-1.80 (m, 3H), 2.19 (s, 3H), 2.68 (t, J = 12.2 Hz, 1H), 2.89 (t, J = 4.1 Hz, 2H), 3.15 (t, J = 12.2 Hz, 1H), 3.37 (t, J = 4.1 Hz, 2H), 3.88-3.96 (m, 3H), 4.29-4.35 (m, 1H), 4.43 (d, J = 12.7 Hz, 1H), 4.76 (d, J = 4.2 Hz, 2H) 6.78-6.83 (m, 1H), 7.02-7.05 (m, 3H), 7.14 (d, J = 6.8 Hz, 1H), 7.26 (t, J = 7.8 Hz, 1H), 7.46 (d, J = 7.2 Hz, 1H), 7.70 (s, 1H), 8.52 (s, 1H).

### Exemple 53 : 5-(2,3-dichloro-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 53) (example comparatif)

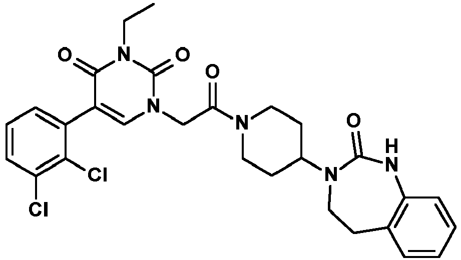

### 53.1: acide [5-(2,3-dichloro-phényl)-3-éthyl-2,4-dioxo-3,4-dihydro-2H-pyrimidin-1-yl]-acétique

De manière analogue à l'exemple 13.3, à partir de 350mg (1,2 mmoles) de (5-bromo-3-éthyl-2,4-dioxo-3,4-dihydro-2H-pyrimidin-1-yl)-acétate de méthyle (préparé comme décrit dans l'exemple 51.1), et 367mg (1,9 mmoles) d'acide 2,3-dichlorophénylboronique, 120mg (29%) d'acide [5-(2,3-dichloro-phényl)-3-éthyl-2,4-dioxo-3,4-dihydro-2H-pyrimidin-1-yl]-acétique sont obtenus sous la forme d'un solide beige.

### 53.2: 5-(2,3-dichloro-phényl)-3-éthyl-1-{2-oxo-2-[4-(2-oxo-1,2, 4,5-tétrahydro-benzo [d] [1,3]diazépin-3-yl)-pipéridin-1-yl]-éthyl}-1H-pyrimidine-2,4-dione (composé 53)

De manière analogue à l'exemple 51.4, à partir de 120mg (0,4 mmoles) d' acide [5-(2,3-dichloro-phényl)-3-éthyl-2,4-dioxo-3,4-dihydro-2H-pyrimidin-1-yl]-acétique et 94mg (0,4 mmoles) 3-pipéridin-4-yl-1,3,4,5-tétrahydro-benzo[d] [1,3]diazépin-2-one (préparé comme décrit dans l'exemple 16.4), 130mg (63%) de 5-(2,3-dichloro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 170°C.
RMN ¹H (δ, DMSO) : 1.13 (t, J = 6.9 Hz, 3H), 1.48-1.60 (m, 1H), 1.62-1.80 (m, 3H), 2.68 (t, J = 11 Hz, 1H), 2.88 (t, J = 4.4 Hz, 2H), 3.15 (t, J = 11 Hz, 1H), 3.37 (t, J = 4.0 Hz, 2H), 3.89-3.91 (m, 2H), 3.95 (d, J = 13 Hz, 1H), 4.28-4.38 (m, 1H), 4.45 (d, J = 13 Hz, 1H) 4.76 (d, J = 5.2 Hz, 2H), 6.75-6.83 (m, 1H), 7.02-7.04 (m, 3H), 7.33 (dd, J = 1.6-7.6 Hz, 1H), 7.42 (t, J = 7.8 Hz, 1H), 7.68 (dd, J = 1.6-8.1 Hz, 1H) 7.80 (s, 1H), 8.54 (s, 1H).

### Exemple 54 : 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 54) (example comparatif)

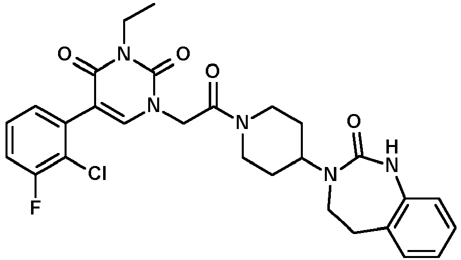

### 54.1: acide [5-(2-chloro-3-fluoro-phényl)-3-éthyl-2,4-dioxo-3,4-dihydro-2H-pyrimidin-1-yl]-acétique

De manière analogue à l'exemple 13.3, à partir de 300mg (1 mmole) de (5-bromo-3-éthyl-2,4-dioxo-3,4-dihydro-2H-pyrimidin-1-yl)-acétate de méthyle (préparé comme décrit dans l'exemple 51.1), et 270mg (1,6 mmoles) d'acide 2-chloro-3-fluoro-phényl boronique, 255mg (76%) d'acide [5-(2-chloro-3-fluoro -phényl)-3-éthyl-2,4-dioxo-3,4-dihydro-2H-pyrimidin-1-yl]-acétique sont obtenus sous la forme d'un solide marron.

### 54.2: 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 54)

De manière analogue à l'exemple 51.4, à partir de 255mg (0,8 mmoles) d' acide [5-(2-chloro-3-fluoro-phényl)-3-éthyl-2,4-dioxo-3,4-dihydro-2H-pyrimidin-1-yl]-acétique et 211mg (0,9 mmoles) de 3-pipéridin-4-yl-1,3,4,5-tétrahydro-benzo[d] [1,3]diazépin-2-one (préparé comme décrit dans l'exemple 16.4), 190mg (44%) de 5-(2-chloro-3-fluoro-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 248°C.
RMN ¹H (δ, DMSO) : 1.13 (t, J = 6.9 Hz, 3H), 1.45-1.60 (m, 1H), 1.62-1.80 (m, 3H), 2.68 (t, J = 12.1 Hz, 1H), 2.89 (t, J = 4.2 Hz, 2H), 3.15 (t, J = 10.3 Hz, 1H), 3.37 (t, J = 4.2 Hz, 2H), 3.88-3.95 (m, 3H), 4.3-4.35 (m, 1H), 4.43 (d, J = 12.6 Hz, 1H), 4.77 (d, J = 4.4 Hz, 2H), 6.78-6.82 (m, 1H), 7.02-7.05 (m, 3H), 7.21-7.23 (m, 1H), 7.43-7.48 (m, 2H), 7.82 (s, 1H), 8.54 (s, 1H) .

### Exemple 55 : 5-(3-chloro-2-méthoxy-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 55) (example comparatif)

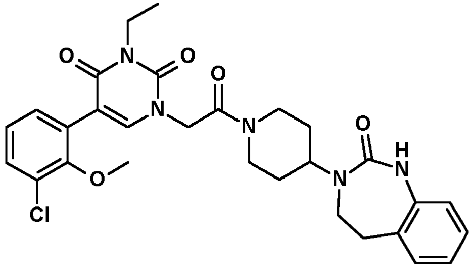

### 55.1: acide [5-(3-chloro-2-méthoxy-phényl)-3-éthyl-2,4-dioxo-3,4-dihydro-2H-pyrimidin-1-yl]-acétique

De manière analogue à l'exemple 13.3, à partir de 400mg (1,4 mmoles) de (5-bromo-3-éthyl-2,4-dioxo-3,4-dihydro-2H-pyrimidin-1-yl)-acétate de méthyle (préparé comme décrit dans l'exemple 51.1), et 410mg (2,2 mmoles) d'acide 3-chloro-2-méthoxy-phényl boronique, 307mg (65%) d'acide [5-(3-chloro-2-méthoxy-phényl)-3-éthyl-2,4-dioxo-3,4-dihydro-2H-pyrimidin-1-yl]-acétique sont obtenus sous la forme d'un solide blanc.

### 55.2: 5-(3-chloro-2-méthoxy-phényl)-3-éthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 55)

De manière analogue à l'exemple 51.4, à partir de 300mg (0,9 mmoles) d'acide [5-(3-chloro-2-méthoxy-phényl)-3-éthyl-2,4-dioxo-3,4-dihydro-2H-pyrimidin-l-yl]-acétique et 239mg (1 mmole) de 3-pipéridin-4-yl-1,3,4,5-tétrahydro-benzo[d] [1,3]diazépin-2-one (préparé comme décrit dans l'exemple 16.4), 395mg (78%) de 5-(3-chloro-2-méthoxy-phényl)-3-éthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 219°C.
RMN ¹H (δ, DMSO) : 1.14 (t, J = 7.0 Hz, 3H); 1.45-1.80 (m, 4H); 2.68 (t, J = 12.5 Hz, 1H); 2.86-2.91 (m, 2H); 3.15 (t, J = 12.5 Hz, 1H); 3.34-3.39 (m, 2H); 3.67 (s, 3H); 3.90-3.93 (m, 3H); 4.28-4.35 (m, 1H); 4.43 (d, J = 13.0 Hz, 1H); 4.77-4.79 (m, 2H); 6.79-6.80 (m, 1H); 7.03-7.05 (m, 3H); 7.18 (t, J = 7.8 Hz, 1H) ; 7.27 (dd, J = 7.7, 1.7 Hz, 1H); 7.49 (dd, J = 8.0, 1.7 Hz, 1H); 7.77 (s, 1H); 8.54 (s, 1H).

### Exemple 56 : 5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-3-propyl-1H-pyrimidine-2,4-dione (schéma réactionnel n°3, composé 56) (example comparatif)

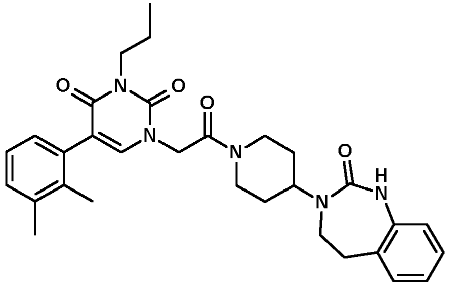

### 56.1: (5-bromo-2,4-dioxo-3-propyl-3,4-dihydro-2H-pyrimidin-1-yl)-acétate de méthyle

De manière analogue à l'exemple 51.1, à partir de 4g (20 mmoles) de (5-bromo-2,4-dioxo-3,4-dihydro-2H-pyrimidin-1-yl)-acétate de méthyle (préparé comme décrit dans l'exemple 13.1) et 2,1ml (20 mmoles) de 1-bromopropane, 2,9g (61%) de (5-bromo-2,4-dioxo-3-propyl-3,4-dihydro-2H-pyrimidin-1-yl)-acétate de méthyle sont obtenus sous la forme d'un solide blanc.

### 56.2: [5-(2,3-diméthyl-phényl)-2,4-dioxo-3-propyl-3,4-dihydro-2H-pyrimidin-1-yl]-acétate de méthyle

De manière analogue à l'exemple 44.1, à partir de 300mg (1 mmole) de (5-bromo-2,4-dioxo-3-propyl-3,4-dihydro-2H-pyrimidin-1-yl)-acétate de méthyle et 221mg (1,5 mmoles) d'acide 2,3-diméthyl-phényl boronique, 260mg (80%) de [5-(2,3-diméthyl-phényl)-2,4-dioxo-3-propyl-3,4-dihydro-2H-pyrimidin-1-yl]-acétate de méthyle sont obtenus sous la forme d'un solide blanc.

### 56.3: acide [5-(2,3-diméthyl-phényl)-2,4-dioxo-3-propyl-3,4-dihydro-2H-pyrimidin-1-yl]-acétique

De manière analogue à l'exemple 16.8, à partir de 260mg (0,8 mmoles) de [5-(2,3-diméthyl-phényl)-2,4-dioxo-3-propyl-3,4-dihydro-2H-pyrimidin-1-yl]-acétate de méthyle, 230mg (92%) d' acide [5-(2,3-diméthyl-phényl)-2,4-dioxo-3-propyl-3,4-dihydro-2H-pyrimidin-1-yl]-acétique sont obtenus sous la forme d'un solide blanc.

### 56.4: 5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-3-propyl-1H-pyrimidine-2,4-dione (composé 56)

De manière analogue à l'exemple 51.4, à partir de 230mg (0,7 mmoles) d'acide [5-(2,3-diméthyl-phényl)-2,4-dioxo-3-propyl-3,4-dihydro-2H-pyrimidin-1-yl]-acétique et 196mg (0,8 mmoles) de 3-pipéridin-4-yl-1,3,4,5-tétrahydro-benzo[d][1,3]diazépin-2-one (préparé comme décrit dans l'exemple 16.4), 250mg (63%) de 5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-3-propyl-1H-pyrimidine-2,4-dione sont obtenus sous la forme d'un solide blanc de point de fusion 194°C.
RMN ¹H (δ, DMSO) : 0.86 (t, J = 3.7 Hz, 3H), 1.52-1.60 (m, 3H), 1.62-1.80 (m, 3H), 2.04 (s, 3H), 2.27 (s, 3H), 2.67 (t, J = 11 Hz, 1H), 2.89 (t, J = 4.4 Hz, 2H), 3.14 (t, J = 11 Hz, 1H), 3.37 (t, J = 4.4 Hz, 2H), 3.83 (t, J = 7 Hz, 2H), 3.93 (d, J = 13.7 Hz, 1H), 4.28-4.38 (m, 1H), 4.43 (d, J = 12.6 Hz, 1H), 4.75 (d, J = 6.2 Hz, 2H), 6.78-6.82 (m, 1H), 6.96 (d, J = 7.2 Hz, 1H), 7.01-7.05 (m, 3H), 7.10 (t, J = 7.5 Hz, 1H), 7.17 (d, J = 7.3 Hz, 1H), 7.59 (s, 1H), 8.54 (s, 1H).

### Exemple 57 : 5-(2-chloro-3-fluoro-phényl)-1-{2-oxo-2-[4-(2-oxo-1,2,4,5-tétrahydro-benzo[d][1,3]diazépin-3-yl)-pipéridin-1-yl]-éthyl}-3-propyl-1H-pyrimidine-2,4-dione (schéma réactionnel n°3, composé 57) (example comparatif)

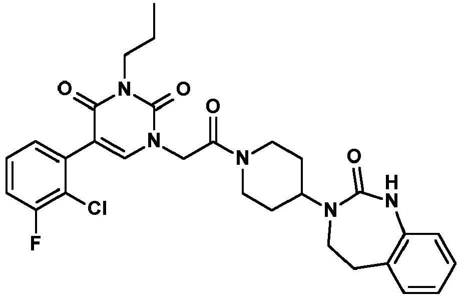

### 57.1: acide [5-(2-chloro-3-fluoro-phényl)-2,4-dioxo-3-propyl-3,4-dihydro-2H-pyrimidin-1-yl]-acétique

De manière analogue à l'exemple 13.3, à partir de 300mg (1 mmole) de (5-bromo-2,4-dioxo-3-propyl-3,4-dihydro-2H-pyrimidin-1-yl)-acétate de méthyle (préparé comme décrit dans l'exemple 56.1) et 257mg (1,5 mmoles) d'acide 2-chloro-3-fluoro-phényl boronique, 200mg (60%) d'acide [5-(2-chloro-3-fluoro-phényl)-2,4-dioxo-3-propyl-3,4-dihydro-2H-pyrimidin-1-yl]-acétique sont obtenus sous la forme d'un solide beige.

### 57.2: 5-(2-chloro-3-fluoro-phényl)-1-{2-oxo-2-[4-(2-oxo-1,2, 4,5-tétrahydro-benzo[d][1,3]diazépin-3-yl)-pipéridin-1-yl]-éthyl}-3-propyl-1H-pyrimidine-2,4-dione (composé 57)

De manière analogue à l'exemple 51.4, à partir de 200mg (0,6 mmoles) d'acide [5-(2-chloro-3-fluoro-phényl)-2,4-dioxo-3-propyl-3,4-dihydro-2H-pyrimidin-1-yl]-acétique et 158mg (0,7 mmoles) de 3-pipéridin-4-yl-1,3,4,5-tétrahydro-benzo[d] [1,3]diazépin-2-one (préparé comme décrit dans l'exemple 16.4), et après recristallisation dans l'éthanol, 240mg (71%) de 5-(2-chloro-3-fluoro-phényl)-1-{2-oxo-2-[4-(2-oxo-1,2,4,5-tétrahydro-benzo[d] [1,3]diazépin-3-yl)-pipéridin-1-yl]-éthyl}-3-propyl-1H-pyrimidine-2,4-dione sont obtenus sous la forme d'un solide blanc de point de fusion 242°C.

RMN ¹H (δ, DMSO) : 0.88 (t, J = 7.4 Hz, 3 H); 1.54-1.85 (m, 6 H); 2.68 (t, J = 12.6 Hz, 1 H); 2.89 (m, 2 H); 3.15 (t, J = 12.6 Hz, 1 H); 3.35-3.38 (m, 2 H); 3.84 (t, J = 7.3 Hz, 2 H); 3.94 (d, J = 13.6 Hz, 1 H); 4.28-4.36 (m, 1H); 4.42 (d, J = 13.6 Hz, 1 H); 4.77 (d, J = 3.5 Hz, 2 H); 6.79-6.81 (m, 1 H); 7.02-7.05 (m, 3 H); 7.21 (dd, J = 6.6, 2.5 Hz, 1 H); 7.43-7.47 (m, 2 H); 7.82 (s, 1 H); 8.54 (s, 1 H).

### Exemple 58 : 1-{2-oxo-2-[4-(2-oxo-1,2,4,5-tétrahydro-benzo[d][1,3]diazépin-3-yl)-pipéridin-1-yl]-éthyl}-3-propyl-5-pyridazin-3-yl-1H-pyrimidine-2,4-dione (schéma réactionnel n°3, composé 58) (example comparatif)

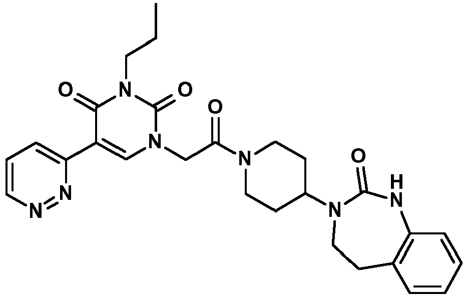

### 58.1: 1-benzhydryl-5-bromo-3-propyl-1H-pyrimidine-2,4-dione

De manière analogue à l'exemple 2.2, à partir de 3g (8,4 mmoles) de 1-benzhydryl-5-bromo-1H-pyrimidine-2,4-dione (préparé comme décrit dans l'exemple 2.1) et 1,2ml (12,6 mmoles) de 1-bromopropane, 2,5g (73%) de 1-benzhydryl-5-bromo-3-propyl-1H-pyrimidine-2,4-dione sont obtenus sous la forme d'un solide beige.

### 58.2: 1-benzhydryl-5-(2-oxy-pyridazin-3-yl)-3-propyl-1H-pyrimidine-2,4-dione

De manière analogue à l'exemple 42.1, à partir de 500mg (1,3 mmoles) de 1-benzhydryl-5-bromo-3-propyl-1H-pyrimidine-2,4-dione et 241mg (2,5 mmoles) de N-oxyde-pyridazine, dans 5ml de dioxane, 250mg (48%) de 1-benzhydryl-5-(2-oxy-pyridazin-3-yl)-3-propyl-1H-pyrimidine-2,4-dione sont obtenus sous la forme d'un solide beige.

### 58.3: 1-benzhydryl-3-propyl-5-pyridazin-3-yl-1H-pyrimidine-2,4-dione

De manière analogue à l'exemple 42.2, à partir de 250mg (0,6 mmoles) de 1-benzhydryl-5-(2-oxy-pyridazin-3-yl)-3-propyl-1H-pyrimidine-2,4-dione, 120mg (50%) de 1-benzhydryl-3-propyl-5-pyridazin-3-yl-1H-pyrimidine-2,4-dione sont obtenus sous la forme d'un solide beige.

### 58.4: 3-propyl-5-pyridazin-3-yl-1H-pyrimidine-2,4-dione

De manière analogue à l'exemple 42.3, à partir de 120mg (0,3 mmoles) de 1-benzhydryl-3-propyl-5-pyridazin-3-yl-1H-pyrimidine-2,4-dione, 70mg (100%) de 3-propyl-5-pyridazin-3-yl-1H-pyrimidine-2,4-dione sont obtenus sous la forme d'un solide jaune.

### 58.5: 1-{2-oxo-2-[4-(2-oxo-1,2,4,5-tetrahydro-benzo[d][1,3]diazepin-3-yl)-piperidin-1-yl]-ethyl}-3-propyl-5-pyridazin-3-yl-1H-pyrimidine-2,4-dione (composé 58)

De manière analogue à l'exemple 13.1, à partir de 70mg (0,3 mmoles) de 3-propyl-5-pyridazin-3-yl-1H-pyrimidine-2,4-dione et 107mg (0,3 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), et après purification par chromatographie flash sur gel de silice élué par un mélange dichlorométhane / méthanol 95/5 et recristallisation dans l'éthanol, 60mg (38%) de 1-{2-oxo-2-[4-(2-oxo-1,2,4,5-tetrahydro-benzo[d][1,3]diazepin-3-yl)-piperidin-1-yl]-ethyl}-3-propyl-5-pyridazin-3-yl-1H-pyrimidine-2,4-dione sont obtenus sous forme de solide blanc de point de fusion 210°C.
RMN¹H (δ, DMSO) : 0.89 (t, J = 7.4 Hz, 3H); 1.50-1.90 (m, 6H); 2.70 (t, J = 11.7 Hz, 1H); 2.91 (m, 2H); 3.17 (t, J = 12.8 Hz, 1H); 3.38-3.41 (m, 2H); 3.89-3.92 (m, 3H); 4.30-4.37 (m, 1H); 4.43 (d, J = 13.0 Hz, 1H); 4.96 (d, J = 4.5 Hz, 2H); 6.80-6.82 (m, 1H); 7.03-7.05 (m, 3H); 7.73 (dd, J = 8.7, 4.9 Hz, 1H); 8.37 (dd, J = 8.7, 1.6 Hz, 1H); 8.55 (s, 1H); 8.75 (s, 1H); 9.16 (dd, J = 4.9, 1.6 Hz, 1H).

### Exemple 59 : 5-(3-chloro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 59) (example comparatif)

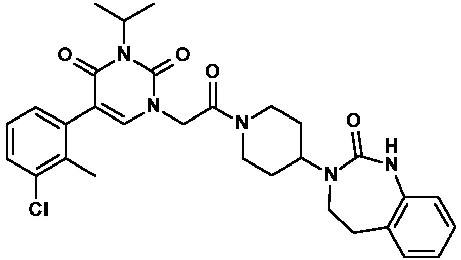

### 59.1: (5-bromo-3-isopropyl-2,4-dioxo-3,4-dihydro-2H-pyrimidin-1-yl)-acétate de méthyle

De manière analogue à l'exemple 51.1, à partir de 5g (19 mmoles) de (5-bromo-2,4-dioxo-3,4-dihydro-2H-pyrimidin-1-yl)-acétate de méthyle (préparé comme décrit dans l'exemple 13.1) et 2,7ml (29 mmoles) de 2-bromopropane, 3,4g (59%) de (5-bromo-3-isopropyl-2,4-dioxo-3,4-dihydro-2H-pyrimidin-1-yl)-acétate de méthyle sont obtenus sous la forme d'un solide blanc.

### 59.2: [5-(3-chloro-2-méthyl-phényl)-3-isopropyl-2,4-dioxo-3,4-dihydro-2H-pyrimidin-1-yl]-acétate de méthyle

De manière analogue à l'exemple 44.1, à partir de 300mg (1 mmole) de (5-bromo-3-isopropyl-2,4-dioxo-3,4-dihydro-2H-pyrimidin-1-yl)-acétate de méthyle et 275mg (1,5 mmoles) d'acide 3-chloro-2-méthyl-phényl boronique, 190mg (55%) de [5-(3-chloro-2-méthyl-phényl)-3-isopropyl-2,4-dioxo-3,4-dihydro-2H-pyrimidin-1-yl]-acétate de méthyle sont obtenus sous la forme d'un solide blanc.

### 59.3: acide [5-(3-chloro-2-méthyl-phényl)-3-isopropyl-2,4-dioxo-3,4-dihydro-2H-pyrimidin-1-yl]-acétique

De manière analogue à l'exemple 16.8, à partir de 190mg (0,5 mmoles) de [5-(3-chloro-2-méthyl-phényl)-3-isopropyl-2,4-dioxo-3,4-dihydro-2H-pyrimidin-1-yl]-acétate de méthyle, 180mg (99%) d' acide [5-(3-chloro-2-méthyl-phényl)-3-isopropyl-2,4-dioxo-3,4-dihydro-2H-pyrimidin-1-yl]-acétique sont obtenus sous la forme d'un solide blanc.

### 59.4: 5-(3-chloro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 59)

De manière analogue à l'exemple 51.4, à partir de 180mg (0,5 mmoles) d' acide [5-(3-chloro-2-méthyl-phényl)-3-isopropyl-2,4-dioxo-3,4-dihydro-2H-pyrimidin-1-yl]-acétique et 144mg (0,6 mmoles) de 3-pipéridin-4-yl-1,3,4,5-tétrahydro-benzo[d] [1,3]diazépin-2-one (préparé comme décrit dans l'exemple 16.4), 250mg (83%) de 5-(3-chloro-2-méthyl-phényl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 166°C.
RMN¹H (δ, DMSO) : 1.41 (d, J = 6.9 Hz, 6H) 1.51-1.59 (m, 1H), 1.68-1.79 (m, 3H), 2.19 (s, 3H), 2.68 (t, J = 12.9 Hz, 1H), 2.89 (t, J = 4.3 Hz, 2H), 3.15 (t, J = 12 Hz, 1H), 3.38 (t, J = 4.4 Hz, 2H) 3.93 (d, J = 13 Hz, 1H), 4.29-4.36 (m, 1H), 4.43 (d, J = 13 Hz, 1H), 4.73 (dd, J = 16.3-20.2 Hz, 2H), 5.08-5.15 (m, 1H), 7.78-6.84 (m, 1H), 7.02-7.06 (m, 3H), 7.13 (dd, J = 1-7.7 Hz, 1H), 7.25 (t, J = 7.7 Hz, 1H), 7.45 (d, J = 7.2 Hz, 1H), 7.65 (s, 1H), 8.50 (s, 1H).

### Exemple 60 : 5-(3-chloro-2-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 60) (example comparatif)

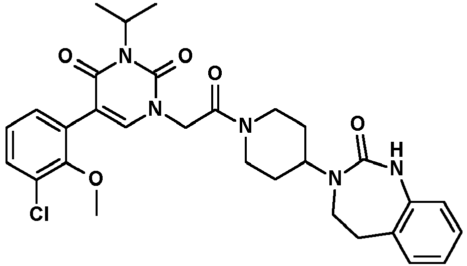

### 60.1: [5-(3-chloro-2-méthoxy-phényl)-3-isopropyl-2,4-dioxo-3,4-dihydro-2H-pyrimidin-1-yl]-acétate de méthyle

De manière analogue à l'exemple 44.1, à partir de 300mg (1 mmole) de (5-bromo-3-isopropyl-2,4-dioxo-3,4-dihydro-2H-pyrimidin-1-yl)-acétate de méthyle (préparé comme décrit dans l'exemple 59.1) et 293mg (1,6 mmoles) d'acide 3-chloro-2-méthoxy-phényl boronique, 325mg (90%) de [5-(3-chloro-2-méthoxy-phényl)-3-isopropyl-2,4-dioxo-3,4-dihydro-2H-pyrimidin-1-yl]-acétate de méthyle sont obtenus sous la forme d'un solide blanc.

### 60.2: acide [5-(3-chloro-2-méthoxy-phényl)-3-isopropyl-2,4-dioxo-3,4-dihydro-2H-pyrimidin-1-yl]-acétique

De manière analogue à l'exemple 16.8, à partir de 325mg (0,9 mmoles) de [5-(3-chloro-2-méthoxy-phényl)-3-isopropyl-2,4-dioxo-3,4-dihydro-2H-pyrimidin-1-yl]-acétate de méthyle, 300mg (96%) d' acide [5-(3-chloro-2-méthoxy-phényl)-3-isopropyl-2,4-dioxo-3,4-dihydro-2H-pyrimidin-1-ylJ-acétique sont obtenus sous la forme d'un solide blanc.

### 60.3: 5-(3-chloro-2-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 60)

De manière analogue à l'exemple 51.4, à partir de 180mg (0,5 mmoles) d' acide [5-(3-chloro-2-méthoxy-phényl)-3-isopropyl-2,4-dioxo-3,4-dihydro-2H-pyrimidin-1-yl]-acétique et 230mg (0,9 mmoles) de 3-pipéridin-4-yl-1,3,4,5-tétrahydro-benzo[d] [1,3]diazépin-2-one (préparé comme décrit dans l'exemple 16.4), 400mg (81%) de 5-(3-chloro-2-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 155°C.
RMN ¹H (δ, DMSO) : 1.40 (d, J = 7 Hz, 6H), 1.53-1.60 (m, 1H), 1.65-1.78 (m, 3H), 2.67 (t, J = 12 Hz, 1H), 2.89 (t, J = 4.7 Hz, 2H), 3.15 (t, J = 12 Hz, 1H), 3.37 (t, J = 4.6 Hz, 2H), 3.68 (s, 3H), 3.94 (d, J = 12.7 Hz, 1H), 4.29-4.35 (m, 1H), 4.43 (d, J = 12.6 Hz, 1H), 4.73 (dd, J = 16.2-19.6 Hz, 2H), 5.09-5.16 (m, 1H), 6.79-6.83 (m, 1H), 7.02-7.06 (m, 3H), 7.17 (t, J = 7.9 Hz, 1H), 7.25 (dd, J = 1.6-7.6 Hz, 1H), 7.48 (dd, J = 1.6-8 Hz, 1H), 7.71 (s, 1H), 8.50 (s, 1H).

### Exemple 61 : 5-(3-fluoro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 61) (example comparatif)

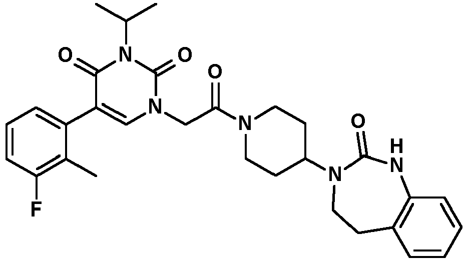

### 61.1: acide [5-(3-fluoro-2-méthyl-phényl)-3-isopropyl-2,4-dioxo-3,4-dihydro-2H-pyrimidin-1-yl]-acétique

De manière analogue à l'exemple 44.1, à partir de 200mg (0,7 mmoles) de (5-bromo-3-isopropyl-2,4-dioxo-3,4-dihydro-2H-pyrimidin-1-yl)-acétate de méthyle (préparé comme décrit dans l'exemple 59.1) et 161mg (1,1 mmoles) d'acide 3-fluoro-2-méthyl-phényl boronique, et après purification par chromatographie flash sur gel de silice élué par un mélange dichlorométhane / méthanol 94/6 avec 0,1% d'acide acétique; 175mg (83%) d'acide [5-(3-fluoro-2-méthyl-phényl)-3-isopropyl-2,4-dioxo-3,4-dihydro-2H-pyrimidin-1-yl]-acétique sont obtenus sous la forme d'un solide beige.

### 61.2: 5-(3-fluoro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 61)

De manière analogue à l'exemple 51.4, à partir de 180mg (0,5 mmoles) d' acide [5-(3-fluoro-2-méthyl-phényl)-3-isopropyl-2,4-dioxo-3,4-dihydro-2H-pyrimidin-1-yl]-acétique et 134mg (0,6 mmoles) de 3-pipéridin-4-yl-1,3,4,5-tétrahydro-benzo[d][1,3]diazépin-2-one (préparé comme décrit dans l'exemple 16.4), 240mg (79%) de 5-(3-fluoro-2-méthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 244°C.
RMN ¹H (δ, DMSO) : 1.40 (d, J = 6.8 Hz, 6H), 1.52-1.57 (m, 1H), 1.64-1.73 (m, 3H), 2.06 (d, J = 2.1 Hz, 3H), 2.68 (d, J = 11 Hz, 1H), 2.88 (t, J = 4.4 Hz, 2H), 3.14 (d, J = 11 Hz, 1H), 3.37 (t, J = 4.4 Hz, 2H), 3.93 (d, J = 13 Hz, 1H), 4.31-4.33 (m, 1H), 4.43 (d, J = 13 Hz, 1H), 4.73 (d, J = 6.6 Hz, 2H), 5.11 (q, J = 6.9 Hz, 1H), 6.78-6.82 (m, 1H), 7.00-7.05 (m, 4H), 7.15-7.19 (m, 1H), 7.23-7.28 (m, 1H), 7.66 (s, 1H), 8.54 (s, 1H).

### Exemple 62 : 5-(2,3-difluoro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 62) (example comparatif)

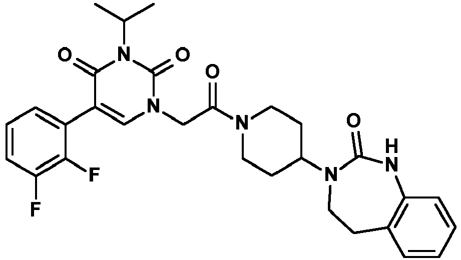

### 62.1: [5-(2,3-difluoro-phényl)-3-isopropyl-2,4-dioxo-3,4-dihydro-2H-pyrimidin-1-yl]-acétate de méthyle

De manière analogue à l'exemple 44.1, à partir de 200mg (0,7 mmoles) de (5-bromo-3-isopropyl-2,4-dioxo-3,4-dihydro-2H-pyrimidin-1-yl)-acétate de méthyle (préparé comme décrit dans l'exemple 59.1) et 422mg (2,6 mmoles) d'acide 2,3-difluoro-phényl boronique, 110mg (50%) de [5-(2,3-difluoro-phényl)-3-isopropyl-2,4-dioxo-3,4-dihydro-2H-pyrimidin-1-yl]-acétate de méthyle sont obtenus sous la forme d'une huile incolore.

### 62.2: acide [5-(2,3-difluoro-phényl)-3-isopropyl-2,4-dioxo-3,4-dihydro-2H-pyrimidin-1-yl]-acétique

De manière analogue à l'exemple 16.8, à partir de 135mg (0,4 mmoles) de [5-(2,3-difluoro-phényl)-3-isopropyl-2,4-dioxo-3,4-dihydro-2H-pyrimidin-1-yl]-acétate de méthyle, 130mg (100%) d' acide [5-(2,3-difluoro-phényl)-3-isopropyl-2,4-dioxo-3,4-dihydro-2H-pyrimidin-1-yl]-acétique sont obtenus sous la forme d'une huile incolore.

### 62.3: 5-(2,3-difluoro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 62)

De manière analogue à l'exemple 51.4, à partir de 130mg (0,4 mmoles) d' acide [5-(2,3-difluoro-phényl)-3-isopropyl-2,4-dioxo-3,4-dihydro-2H-pyrimidin-1-yl]-acétique et 98mg (0,4 mmoles) de 3-pipéridin-4-yl-1,3,4,5-tétrahydro-benzo[d] [1,3]diazépin-2-one (préparé comme décrit dans l'exemple 16.4), 160mg (72%) de 5-(2,3-difluoro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 242°C.
RMN ¹H (δ, DMSO) : 1.40 (d, J = 6.9 Hz, 6H), 1.45-1.60 (m, 1H), 1.62-1.80 (m, 3H), 2.68 (t, J = 12 Hz, 1H), 2.89 (t, J = 4.5 Hz, 2H), 3.15 (t, J = 12 Hz, 1H), 3.38 (t, J = 4.4 Hz, 2H), 3.95 (d, J = 12 Hz, 1H), 4.30-4.40 (m, 1H), 4.45 (d, J = 12 Hz, 1H), 4.75 (d, J = 2 Hz, 2H), 5.11 (q, J = 6.9 Hz, 1H), 6.78-6.82 (m, 1H), 7.02-7.04 (m, 3H), 7.19-7.26 (m, 2H), 7.44 (dd, J = 1.9-10.2 Hz, 1H), 7.86 (s, 1H), 8.55 (s, 1H).

### Exemple 63 : 5-(2-chloro-3-fluoro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 63) (example comparatif)

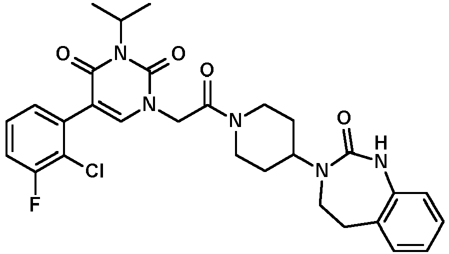

### 63.1: [5-(2-chloro-3-fluoro-phényl)-3-isopropyl-2,4-dioxo-3,4-dihydro-2H-pyrimidin-1-yl]-acétate de méthyle

De manière analogue à l'exemple 44.1, à partir de 200mg (0,7 mmoles) de (5-bromo-3-isopropyl-2,4-dioxo-3,4-dihydro-2H-pyrimidin-1-yl)-acétate de méthyle (préparé comme décrit dans l'exemple 59.1) et 183mg (1.1 mmoles) d'acide 2-chloro-3-fluoro-phényl boronique, 130mg (56%) de [5-(2-chloro-3-fluoro-phényl)-3-isopropyl-2,4-dioxo-3,4-dihydro-2H-pyrimidin-1-yl]-acétate de méthyle sont obtenus sous la forme d'un solide beige.

### 63.2: acide [5-(2-chloro-3-fluoro-phényl)-3-isopropyl-2,4-dioxo-3,4-dihydro-2H-pyrimidin-1-yl]-acétique

De manière analogue à l'exemple 16.8, à partir de 165mg (0,5 mmoles) de [5-(2-chloro-3-fluoro-phényl)-3-isopropyl-2,4-dioxo-3,4-dihydro-2H-pyrimidin-1-yl]-acétate de méthyle, 160mg (100%) d' acide [5-(2-chloro-3-fluoro-phényl)-3-isopropyl-2,4-dioxo-3,4-dihydro-2H-pyrimidin-1-yl]-acétique sont obtenus sous la forme d'une huile brune.

### 63.3: 5-(2-chloro-3-fluoro-phényl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 63)

De manière analogue à l'exemple 51.4, à partir de 160mg (0,5 mmoles) d' acide [5-(2-chloro-3-fluoro-phényl)-3-isopropyl-2,4-dioxo-3,4-dihydro-2H-pyrimidin-1-yl]-acétique et 115mg (0,5 mmoles) de 3-pipéridin-4-yl-1,3,4,5-tétrahydro-benzo[d][1,3]diazépin-2-one (préparé comme décrit dans l'exemple 16.4), 170mg (63%) de 5-(2-chloro-3-fluoro-phényl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide beige de point de fusion 254°C.
RMN ¹H (δ, DMSO) : 1.40 (d, J = 6.8 Hz, 6H), 1.48-1.60 (m, 1H), 1.62-1.80 (m, 3H), 2.68 (t, J = 12 Hz, 1H), 2.89 (t, J = 4.5 Hz, 2H), 3.14 (t, J = 12 Hz, 1H), 3.37 (t, J = 4.5 Hz, 2H), 3.93 (d, J = 13.9 Hz, 1H), 4.28-4.38 (m, 1H), 4.43 (d, J = 13.9 Hz, 1H), 4.74 (d, J = 6.2 Hz, 2H), 5.10 (q, J = 6.9 Hz, 1H), 6.78-6.82 (m, 1H), 7.02-7.04 (m, 3H), 7.20-7.22 (m, 1H), 7.41-7.47 (m, 2H), 7.77 (s, 1H), 8.54 (s, 1H).

### Exemple 64 : 3-isopropyl-5-(2-méthoxy-3-méthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 64) (example comparatif)

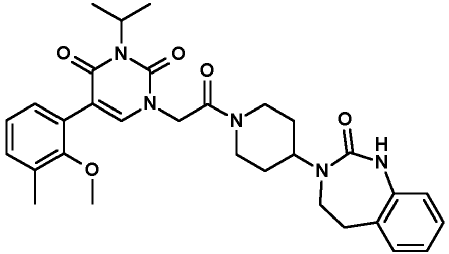

### 64.1: acide [3-isopropyl-5-(2-méthoxy-3-méthyl-phényl)-2,4-dioxo-3,4-dihydro-2H-pyrimidin-1-yl]-acétique

De manière analogue à l'exemple 13.3, à partir de 500mg (1,6 mmoles) de (5-bromo-3-isopropyl-2,4-dioxo-3,4-dihydro-2H-pyrimidin-1-yl)-acétate de méthyle (préparé comme décrit dans l'exemple 59.1) et 408mg (2,5 mmoles) d'acide 2-méthoxy-3-méthyl-phényl boronique, 495mg (91%) d' acide [3-isopropyl-5-(2-méthoxy-3-méthyl-phényl)-2,4-dioxo-3,4-dihydro-2H-pyrimidin-1-yl]-acétique sont obtenus sous la forme d'un solide beige.

### 64.2: 3-isopropyl-5-(2-méthoxy-3-méthyl-phényl)-1-{2-oxo-2-[4-(2-oxo-1,2,4,5-tétrahydro-benzo[d][1,3]diazépin-3-yl)-pipéridin-1-yl)-éthyl}-1H-pyrimidine-2,4-dione (composé 64)

De manière analogue à l'exemple 51.4, à partir de 495mg (1,5 mmoles) d'acide [3-isopropyl-5-(2-méthoxy-3-méthyl-phényl)-2,4-dioxo-3,4-dihydro-2H-pyrimidin-1-yl]-acétique et 384mg (1,6 mmoles) de 3-pipéridin-4-yl-1,3,4,5-tétrahydro-benzo[d][1,3]diazépin-2-one (préparé comme décrit dans l'exemple 16.4), et après recristallisation dans le n-butanol, 285mg (34%) de 3-isopropyl-5-(2-méthoxy-3-méthyl-phényl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 260°C.
RMN ¹H (δ, DMSO) : 1.34 (d, J = 6.9 Hz, 6H); 1.52-1.59 (m, 4H); 2.19 (s, 3H); 2.60 (t, J = 12.6 Hz, 1H); 2.79-2.84 (m, 2H); 3.07 (t, J = 12.7 Hz, 1H); 3.29-3.32 (m, 2H); 3.47 (s, 3H); 3.86 (d, J = 13.5 Hz, 1H); 4.24-4.28 (m, 1H); 4.36 (d, J = 12.9 Hz, 1H); 4.66 (d, J = 7.7 Hz, 2H); 5.05-5.07 (m, 1H); 6.72-6.74 (m, 1H); 6.94-7.02 (m, 5H); 7.12 (d, J = 7.3 Hz, 1H); 7.56 (s, 1H); 8.47 (s, 1H).

### Exemple 65 : 5-(3-chloro-4-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 65) (example comparatif)

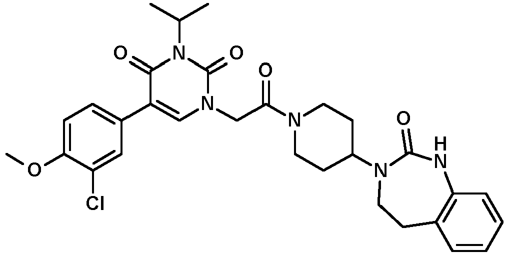

### 65.1: acide [5-(3-chloro-4-méthoxy-phényl)-3-isopropyl-2,4-dioxo-3,4-dihydro-2H-pyrimidin-1-yl]-acétique

De manière analogue à l'exemple 13.3, à partir de 300mg (1 mmole) de (5-bromo-3-isopropyl-2,4-dioxo-3,4-dihydro-2H-pyrimidin-1-yl)-acétate de méthyle (préparé comme décrit dans l'exemple 59.1) et 367mg (2 mmoles) d'acide 3-chloro-4-méthoxy-phényl boronique, 160mg (46%) d' acide [5-(3-chloro-4-méthoxy-phényl)-3-isopropyl-2,4-dioxo-3,4-dihydro-2H-pyrimidin-1-yl]-acétique sont obtenus sous la forme d'un solide jaune.

### 65.2: 5-(3-chloro-4-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 65)

De manière analogue à l'exemple 51.4, à partir de 150mg (0,4 mmoles) d' acide [5-(3-chloro-4-méthoxy-phényl)-3-isopropyl-2,4-dioxo-3,4-dihydro-2H-pyrimidin-1-yl]-acétique et 125mg (0,5 mmoles) de 3-pipéridin-4-yl-1,3,4,5-tétrahydro-benzo[d][1,3]diazépin-2-one (préparé comme décrit dans l'exemple 16.4), 138mg (55%) de 5-(3-chloro-4-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 250°C.
RMN ¹H (δ, DMSO) : 1.41 (d, J = 6.9 Hz, 6 H); 1.57 (d, J = 13.0 Hz, 1 H); 1.63-1.76 (m, 3 H); 2.69 (t, J = 12.5 Hz, 1 H) ; 2.91 (s, 2 H); 3.16 (t, J = 12.1 Hz, 1 H); 3.40 (d, J = 6.6 Hz, 2 H); 3.89 (s, 3 H); 3.96 (d, J = 13.5 Hz, 1 H); 4.33 (m, 1H); 4.44 (d, J = 12.9 Hz, 1 H); 4.76 (s, 2 H); 5.14-5.16 (m, 1 H); 6.80-6.82 (m, 1 H); 7.04-7.06 (m, 3 H); 7.19 (d, J = 8.7 Hz, 1 H); 7.51 (dd, J = 8.6, 2.3 Hz, 1 H); 7.62 (d, J = 2.2 Hz, 1 H); 7.89 (s, 1 H); 8.54 (s, 1 H).

### Exemple 66 : 5-(2-fluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 66) (example comparatif)

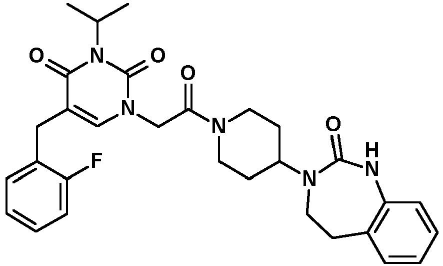

### 66.1: acide [5-(2-fluoro-benzyl)-3-isopropyl-2,4-dioxo-3,4-dihydro-2H-pyrimidin-1-yl]-acétique

De manière analogue à l'exemple 44.1, à partir de 100mg (0,3 mmoles) de (5-bromo-3-isopropyl-2,4-dioxo-3,4-dihydro-2H-pyrimidin-1-yl)-acétate de méthyle (préparé comme décrit dans l'exemple 59.1) et 186mg (0,8 mmoles) d'ester de 2-fluoro-benzyl pinacol boronique, 35mg (33%) d' acide [5-(2-fluoro-benzyl)-3-isopropyl-2,4-dioxo-3,4-dihydro-2H-pyrimidin-1-yl]-acétique sont obtenus sous la forme d'un solide beige.

### 66.2: 5-(2-fluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 66)

De manière analogue à l'exemple 51.4, à partir de 35mg (0,1 mmoles) d'acide [5-(2-fluoro-benzyl)-3-isopropyl-2,4-dioxo-3,4-dihydro-2H-pyrimidin-1-yl]-acétique et 32mg (0,1 mmoles) de 3-pipéridin-4-yl-1,3,4,5-tétrahydro-benzo[d] [1,3]diazépin-2-one (préparé comme décrit dans l'exemple 16.4), 20mg (29%) de 5-(2-fluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.35 (d, J = 6.9 Hz, 6H); 1.54-1.69 (m, 4H); 2.60-2.73 (m, 1H); 2.90 (m, 2H); 3.13 (t, J = 12.6 Hz, 1H); 3.33 (m, 2H); 3.59 (m, 2H); 3.91 (d, J = 13.8 Hz, 1H); 4.31 (m, 1H); 4.42 (d, J = 12.9 Hz, 1H); 4.68 (s, 2H); 5.05-5.08 (m, 1H); 6.80-6.83 (m, 1H); 7.02-7.05 (m, 3H); 7.15-7.17 (m, 2H); 7.29 (m, 2H); 7.36 (s, 1H); 8.53 (s, 1H).

### Exemple 67 : 5-(3,4-dichloro-phényl)-3-isopropyl -1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 67) (example comparatif)

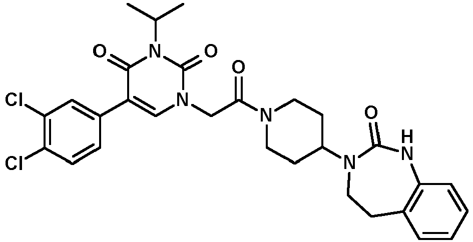

### 67.1: acide [5-(3,4-dichloro-phényl)-3-isopropyl-2,4-dioxo-3,4-dihydro-2H-pyrimidin-1-yl]-acétique

De manière analogue à l'exemple 13.3, à partir de 650mg (2 mmoles) de (5-bromo-3-isopropyl-2,4-dioxo-3,4-dihydro-2H-pyrimidin-1-yl)-acétate de méthyle (préparé comme décrit dans l'exemple 59.1), et 691mg (3,6 mmoles) d'acide 3,4-dichloro-phényl boronique, 700mg (92%) d'acide [5-(3,4-dichloro-phényl)-3-isopropyl-2,4-dioxo-3,4-dihydro-2H-pyrimidin-1-yl]-acétique sont obtenus sous la forme d'un solide beige.

### 67.2: 5-(3,4-dichloro-phényl)-3-isopropyl -1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 67)

De manière analogue à l'exemple 51.4, à partir de 760mg (2 mmoles) d'acide [5-(3,4-dichloro-phényl)-3-isopropyl-2,4-dioxo-3,4-dihydro-2H-pyrimidin-1-yl]-acétique et 627mg (2,6 mmoles) de 3-pipéridin-4-yl-1,3,4,5-tétrahydro-benzo[d][1,3]diazépin-2-one (préparé comme décrit dans l'exemple 16.4), et après cristallisation dans l'isopropanol, 1g (88%) de 5-(3,4-dichloro-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 237°C.
RMN ¹H (δ, DMSO) : 1.42 (d, J = 6.9 Hz, 6 H); 1.71-1.60 (m, 4 H); 2.69 (t, J = 12.5 Hz, 1 H); 2.89-2.92 (m, 2 H); 3.24-3.11 (m, 1 H); 3.96 (d, J = 13.6 Hz, 2 H); 4.27-4.38 (m, 1 H); 4.29-4.45 (m, J = 12.9 Hz, 1 H); 4.78 (s, 2 H); 5.14-5.15 (m, 1 H); 6.80-6.81 (m, 1 H); 7.04-7.05 (m, 3 H); 7.59 (dd, J = 8.5, 2.1 Hz, 1 H); 7.68 (d, J = 8.5 Hz, 1 H); 7.85 (d, J = 2.1 Hz, 1 H); 8.54 (s, 1 H); 8.05 (s, 1 H).

### Exemple 68 : 5-(2,3-diméthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 68) (example comparatif)

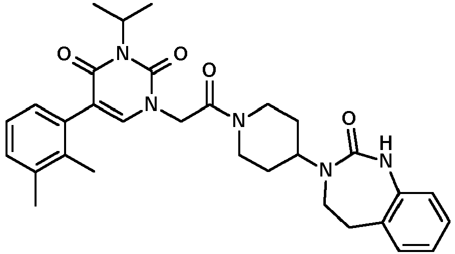

### 68.1: [5-(2,3-diméthyl-phényl)-3-isopropyl-2,4-dioxo-3,4-dihydro-2H-pyrimidin-1-yl]-acétate de méthyle

De manière analogue à l'exemple 44.1, à partir de 300mg (1 mmole) de (5-bromo-3-isopropyl-2,4-dioxo-3,4-dihydro-2H-pyrimidin-1-yl)-acétate de méthyle (préparé comme décrit dans l'exemple 59.1) et 236mg (1,6 mmoles) d'acide 2,3-diméthyl-phényl boronique, 200mg (62%) de [5-(2,3-diméthyl-phényl)-3-isopropyl-2,4-dioxo-3,4-dihydro-2H-pyrimidin-1-yl]-acétate de méthyle sont obtenus sous la forme d'une huile incolore.

### 68.2: acide [5-(2,3-diméthyl-phényl)-3-isopropyl-2,4-dioxo-3,4-dihydro-2H-pyrimidin-1-yl]-acétique

De manière analogue à l'exemple 16.8, à partir de 200mg (0,6 mmoles) de [5-(2,3-diméthyl-phényl)-3-isopropyl-2,4-dioxo-3,4-dihydro-2H-pyrimidin-1-yl]-acétate de méthyle, 190mg (99%) d' acide [5-(2,3-diméthyl-phényl)-3-isopropyl-2,4-dioxo-3,4-dihydro-2H-pyrimidin-1-yl]-acétique sont obtenus sous la forme d'un solide blanc.

### 68.3: 5-(2,3-diméthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 68)

De manière analogue à l'exemple 51.4, à partir de 190mg (0,6 mmoles) d'acide [5-(2,3-diméthyl-phényl)-3-isopropyl-2,4-dioxo-3,4-dihydro-2H-pyrimidin-1-yl]-acétique et 162mg (0,7 mmoles) de 3-pipéridin-4-yl-1,3,4,5-tétrahydro-benzo[d] [1,3]diazépin-2-one (préparé comme décrit dans l'exemple 16.4), 200mg (61%) de 5-(2,3-diméthyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 269°C.
RMN ¹H (δ, DMSO) : 1.40 (d, J = 6.8 Hz, 6H), 1.52-1.56 (m, 1H), 1.64-1.76 (m, 3H), 2.04 (s, 3H), 2.27 (s, 3H), 2.67 (t, J = 12.5 Hz, 1H), 2.88 (t, J = 4.4 Hz, 2H), 3.14 (t, J = 12.5 Hz, 1H), 3.37 (t, J = 4.6 Hz, 2H), 3.93 (d, J = 13.3 Hz, 1H), 4.31-4.33 (m, 1H), 4.43 (d, J = 13.3 Hz, 1H), 4.71 (d, J = 8.5 Hz, 2H), 5.09-5.13 (m, 1H), 6.78-6.82 (m, 1H), 6.95 (d, J = 7.1 Hz, 1H), 7.01-7.05 (m, 3H), 7.09 (t, J = 7.5 Hz, 1H), 7.16 (d, J = 7.4 Hz, 1H), 7.54 (s, 1H), 8.53 (s, 1H).

### Exemple 69 : 5-(2-chloro-3-méthoxy-phényl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 69) (example comparatif)

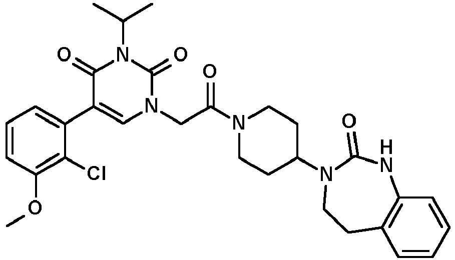

### 69.1: acide [5-(2-chloro-3-méthoxy-phényl)-3-isopropyl-2,4-dioxo-3,4-dihydro-2H-pyrimidin-1-yl]-acétique

De manière analogue à l'exemple 13.3, à partir de 300mg (1 mmole) de (5-bromo-3-isopropyl-2,4-dioxo-3,4-dihydro-2H-pyrimidin-1-yl)-acétate de méthyle (préparé comme décrit dans l'exemple 59.1) et 275mg (1,5 mmoles) d'acide 2-chloro-3-méthoxy-phényl boronique, 115mg (33%) d' acide [5-(2-chloro-3-méthoxy-phényl)-3-isopropyl-2,4-dioxo-3,4-dihydro-2H-pyrimidin-1-yl]-acétique sont obtenus sous la forme d'une huile brune.

### 69.2: 5-(2-chloro-3-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 69)

De manière analogue à l'exemple 51.4, à partir de 105mg (0,3 mmoles) d'acide [5-(2-chloro-3-méthoxy-phényl)-3-isopropyl-2,4-dioxo-3,4-dihydro-2H-pyrimidin-1-yl]-acétique et 88mg (0,4 mmoles) de 3-pipéridin-4-yl-1,3,4,5-tétrahydro-benzo[d] [1,3]diazépin-2-one (préparé comme décrit dans l'exemple 16.4), 85mg (48%) de 5-(2-chloro-3-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.40 (d, J = 6.9 Hz, 6 H); 1.53-1.56 (m, 1H); 1.65-1.70 (m, 3H); 2.60-2.75 (m, 1H); 2.90 (m, 2H); 3.10-3.20 (m, 1H); 3.38 (m, 2 H); 3.89 (s, 3 H); 3.90 (m, 1H); 4.20-4.40 (m, 1H); 4.40-4.50 (m, 1H); 4.73 (d, J = 6.0 Hz, 2 H); 5.10 (t, J = 6.9 Hz, 1 H); 6.80-6.82 (m, 1 H); 6.91 (dd, J = 7.6, 1.4 Hz, 1 H); 7.04-7.06 (m, 3 H); 7.17-7.18 (m, 1 H); 7.34 (t, J = 8.0 Hz, 1 H); 7.67 (s, 1 H); 8.53 (s, 1 H).

### Exemple 70 : 5-(5-chloro-2-méthoxy-phényl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 70) (example comparatif)

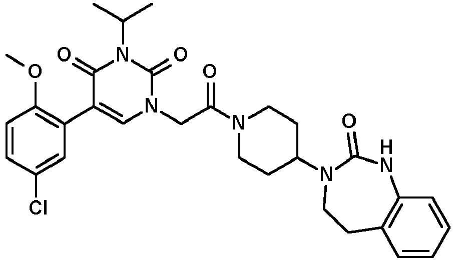

### 70.1: [5-(5-chloro-2-méthoxy-phényl)-3-isopropyl-2,4-dioxo-3,4-dihydro-2H-pyrimidin-1-yl]-acétate de méthyle

De manière analogue à l'exemple 44.1, à partir de 500mg (1,6 mmoles) de (5-bromo-3-isopropyl-2,4-dioxo-3,4-dihydro-2H-pyrimidin-1-yl)-acétate de méthyle (préparé comme décrit dans l'exemple 59.1) et 458mg (2,5 mmoles) d'acide 5-chloro-2-méthoxy-phényl boronique, 275mg (46%) de [5-(5-chloro-2-méthoxy-phényl)-3-isopropyl-2,4-dioxo-3,4-dihydro-2H-pyrimidin-1-yl]-acétate de méthyle sont obtenus sous la forme d'une huile brune.

### 70.2: acide [5-(5-chloro-2-méthoxy-phényl)-3-isopropyl-2,4-dioxo-3,4-dihydro-2H-pyrimidin-1-yl]-acétique

De manière analogue à l'exemple 16.8, à partir de 270mg (0,7 mmoles) de [5-(5-chloro-2-méthoxy-phényl)-3-isopropyl-2,4-dioxo-3,4-dihydro-2H-pyrimidin-1-yl]-acétate de méthyle, 241mg (93%) d' acide [5-(5-chloro-2-méthoxy-phényl)-3-isopropyl-2,4-dioxo-3,4-dihydro-2H-pyrimidin-1-yl]-acétique sont obtenus sous la forme d'un solide blanc.

### 70.3: 5-(5-chloro-2-méthoxy-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 70)

De manière analogue à l'exemple 51.4, à partir de 230mg (0,7 mmoles) d'acide [5-(5-chloro-2-méthoxy-phényl)-3-isopropyl-2,4-dioxo-3,4-dihydro-2H-pyrimidin-1-yl]-acétique et 192mg (0,8 mmoles) de 3-pipéridin-4-yl-1,3,4,5-tétrahydro-benzo[d] [1,3]diazépin-2-one (préparé comme décrit dans l'exemple 16.4), 308mg (81%) de 5-(2-chloro-3-méthoxy-phényl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.40 (d, J = 6.9 Hz, 6 H); 1.59-1.71 (m, 4 H); 2.66 (d, J = 12.7 Hz, 1 H); 2.90 (s, 2 H); 3.13 (d, J = 12.8 Hz, 1 H); 3.38 (t, J = 4.7 Hz, 2H); 3.75 (s, 3 H); 3.94 (d, J = 13.3 Hz, 1 H); 4.33 (t, J = 9.9 Hz, 1 H); 4.43 (d, J = 13.0 Hz, 1 H); 4.72 (d, J = 5.1 Hz, 2 H); 5.09-5.10 (m, 1 H); 6.80-6.81 (m, 1 H); 7.05-7.06 (m, 4 H); 7.25 (d, J = 2.7 Hz, 1 H); 7.39 (dd, J = 8.8, 2.7 Hz, 1 H); 7.68 (s, 1 H) ; 8.53 (s, 1 H).

### Exemple 71 : 5-(2,3-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 71) (example comparatif)

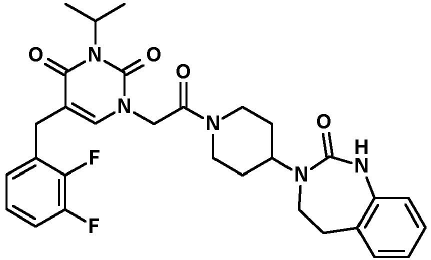

### 71.1: 2-(2,3-difluoro-benzyl)-4,4,5,5-tétraméthyl-[1,3,2]dioxaborolane

De manière analogue à l'exemple 1.8, à partir de 2ml (16 mmoles) de bromure de 2,3-difluorobenzyle et 4,8g (19 mmoles) de bis pinacol borane, 2,5g (62 %) de 2-(2,3-difluoro-benzyl)-4,4,5,5-tétraméthyl-[1,3,2]dioxaborolane sont obtenus sous la forme d'une huile incolore.

### 71.2: acide [5-(2,3-difluoro-benzyl)-3-isopropyl-2,4-dioxo-3,4-dihydro-2H-pyrimidin-1-yl]-acétique

De manière analogue à l'exemple 44.1, à partir de 300mg (1 mmole) de (5-bromo-3-isopropyl-2,4-dioxo-3,4-dihydro-2H-pyrimidin-1-yl)-acétate de méthyle (préparé comme décrit dans l'exemple 59.1) et 1g (4 mmoles) de 2-(2,3-difluoro-benzyl)-4,4,5,5-tétraméthyl-[1,3,2]dioxaborolane, 270mg (32%) d'acide [5-(2,3-difluoro-benzyl)-3-isopropyl-2,4-dioxo-3,4-dihydro-2H-pyrimidin-1-yl]-acétique sont obtenus sous la forme d'une huile brune.

### 71.3: 5-(2,3-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 71)

De manière analogue à l'exemple 51.4, à partir de 270mg (0,8 mmoles) d'acide [5-(2,3-difluoro-benzyl)-3-isopropyl-2,4-dioxo-3,4-dihydro-2H-pyrimidin-1-yl]-acétique et 235mg (1 mmole) de 3-pipéridin-4-yl-1,3,4,5-tétrahydro-benzo[d] [1,3]diazépin-2-one (préparé comme décrit dans l'exemple 16.4), 60mg (13%) de 5-(2,3-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 230°C.
RMN ¹H (δ, DMSO) : 1.35 (d, J = 6.9 Hz, 6H); 1.50-1.80 (m, 4H); 2.67 (t, J = 12.5 Hz, 1H) ; 2.90 (m, 2H); 3.13 (t, J = 10.5 Hz, 1H); 3.39 (m, 2H); 3.63 (s, 2H); 3.92 (d, J = 13.6 Hz, 1H); 4.31 (m, 1H); 4.42 (d, J = 12.9 Hz, 1H); 4.68 (d, J = 2.9 Hz, 2H); 5.05-5.06 (m, 1H); 6.80-6.82 (m, 1H); 7.03-7.05 (m, 3H); 7.12-7.14 (m, 2H); 7.28-7.31 (m, 1H); 7.42 (s, 1H); 8.54 (s, 1H).

De manière analogue à l'exemple 70, en utilsant les réactifs appropriés commerciaux ou préalablement préparés, les composés suivants sont obtenus:

### composé 72: (5-(2-Chloro-3-fluoro-phenyl)-2,6-dioxo-3-{2-oxo-2-[4-(2-oxo-1,2,4,5-tetrahydro-benzo[d][1,3]diazepin-3-yl)-piperidin-1-yl]-ethyl}-3,6-dihydro-2H-pyrimidin-1-yl)-acetonitrile (example comparatif)

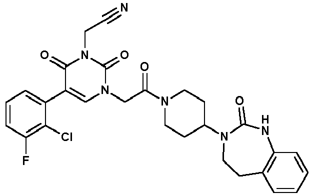

RMN ¹H (400 MHz, DMSO): δ (ppm) 1.56 (d, J = 13.8 Hz, 1H); 1.65-1.77 (m, 3H); 2.70 (t, J = 12.5 Hz, 1H); 2.90 (t, J = 4.9 Hz, 2H); 3.17 (t, J= 12.6 Hz, 1H); 3.38 (s, 2H); 3.93-3.96 (m, 1H); 4.33 (t, J = 11.4 Hz, 1H); 4.43 (d, J = 12.9 Hz, 1H); 4.85 (s, 2H); 4.94 (s, 2H); 6.79-6.83 (m, 1H); 7.04 (t, J = 4.5 Hz, 3H); 7.24-7.29 (m, 4H); 7.44-7.49 (m, 2H); 7.98 (s, 1H); 8.53 (s, 1H).

### composé 73: Acide 3-(5-(2-Chloro-3-fluoro-phenyl)-2,6-dioxo-3-{2-oxo-2-[4-(2-oxo-1,2,4,5-tetrahydro-benzo[d] [1,3]diazepin-3-yl)-piperidin-1-yl]-ethyl}-3,6-dihydro-2H-pyrimidin-1-yl)-propanoïque (example comparatif)

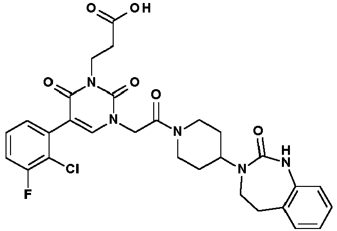

RMN ¹H (400 MHz, DMSO): δ (ppm) 1.55 (d, J = 12.9 Hz, 1H); 1.72 (t, J = 21.2 Hz, 3H); 2.50 (m, 2H); 2.69 (t, J = 12.4 Hz, 1H); 2.90 (m, 2H); 3.16 (m, 1H); 3.38 (m, 2H); 3.95 (d, J = 13.6 Hz, 1H); 4.09 (t, J = 7.7 Hz, 2H); 4.33 (s, 1H); 4.43 (d, J = 12.8 Hz, 1H); 4.78 (s, 2H); 6.81 (dt, J = 7.8, 4.2 Hz, 1H); 7.04 (t, J = 4.0 Hz, 3H); 7.22 (dd, J = 6.3, 2.8 Hz, 1H); 7.43-7.46 (m, 2H); 7.84 (s, 1H); 8.53 (s, 1H).

### Exemple 74 : 3-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoic acétate de méthyle (schéma réactionnel n°3, composé 74) (example comparatif)

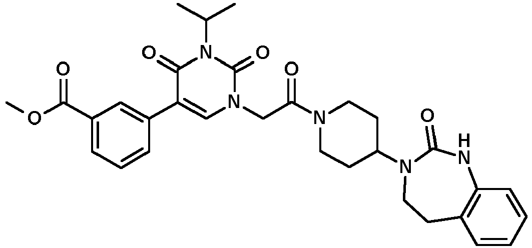

### 74.1: acide (5-bromo-3-isopropyl-2,4-dioxo-3,4-dihydro-2H-pyrimidin-1-yl)-acétique

De manière analogue à l'exemple 16.8, à partir de 1,9g (10 mmoles) de (5-bromo-3-isopropyl-2,4-dioxo-3,4-dihydro-2H-pyrimidin-1-yl)-acétate de méthyle (préparé comme décrit dans l'exemple 59.1), 1,8g (100%) d'acide (5-bromo-3-isopropyl-2,4-dioxo-3,4-dihydro-2H-pyrimidin-1-yl)-acétique sont obtenus sous la forme d'un solide blanc.

### 74.2 : 5-bromo-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione

De manière analogue à l'exemple 1.6, à partir de 1g (3 mmoles) d'acide (5-bromo-3-isopropyl-2,4-dioxo-3,4-dihydro-2H-pyrimidin-1-yl)-acétique et 1g (4 mmoles) de 3-pipéridin-4-yl-1,3,4,5-tetrahydro-benzo[d][1,3]diazépin-2-one (préparé comme décrit dans l'exemple 16.4), 1,8g (100%) de 5-bromo-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide beige.

### 74.3 : 3-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïc acétate de méthyle (composé 74)

De manière analogue à l'exemple 44.1, à partir de 300mg (0,6 mmoles) de 5-bromo-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione et 146mg (0,8 mmoles) d'acide 3-méthoxycarbonyl phényl boronique, 51mg (15%) de 3-(3-isopropyl-2,4-dioxo-1-{2-oxo-2-[4-(2-oxo-1,2,4,5-tétrahydro-benzo[d] [1,3]diazépin-3-yl)-pipéridin-1-yl]-éthyl}-1,2,3,4-tétrahydro-pyrimidin-5-yl)-benzoic acétate de méthyle sont obtenus sous la forme d'un solide blanc.
RMN ¹H (δ, DMSO) : 1.43 (d, J = 6.9 Hz, 6 H) ; 1.55-1.61 (m, 1 H); 1.62-1.82 (m , 3H); 2.68 (m, 1 H); 2.91 (m, 2 H); 3.17 (t , J = 12Hz, 1 H); 3.41 (m, 2 H); 3.89 (s, 3 H); 4.03 (d, J = 8 Hz; 1 H); 4.34 (m, 1 H); 4.45 (d, J = 12 Hz, 1 H); 4.80 (s, 2 H); 5.15-5.18 (m, 1 H); 6.81-6.83 (m, 1 H); 7.04-7.06 (m, 3 H); 7.52 (t, J = 7.8 Hz, 1 H); 7.78 (d, J = 7.5 Hz, 1 H); 7.89 (d, J = 7.7 Hz, 1 H); 7.95 (s, 1 H); 8.12 (s, 1 H); 8.53 (s, 1 H) .

### Exemple 75 : acide 3-(3-isopropyl-2,4-dioxo-1-{2-oxo-2-[4-(2-oxo-1,2,4,5-tétrahydro-benzo[d] [1,3]diazépin-3-yl)-pipéridin-1-yl]-éthyl}-1,2,3,4-tétrahydro-pyrimidin-5-yl)-benzoïque (schéma réactionnel n°3, composé 75) (example comparatif)

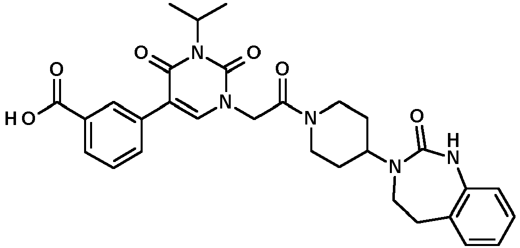

De manière analogue à l'exemple 44.1, à partir de 300mg (0,6 mmoles) de 5-bromo-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione et 146mg (0,8 mmoles) d'acide 3-méthoxycarbonyl phényl boronique, et après purification par chromatographie liquide haute pression préparative (colonne Xbridge C18, isochratique, 41% acétonitrile dans eau), 92mg (28%) d'acide 3-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïque sont obtenus sous la forme d'un solide blanc.
RMN ¹H (δ, DMSO) : 1.43 (d, J = 6.9 Hz, 6 H) ; 1.55-1.61 (m, 1 H); 1.62-1.82 (m , 3H); 2.68 (m, 1 H); 2.91 (m, 2 H); 3.17 (t, J = 12Hz, 1 H) ; 3.41 (m, 1 H) ; 3.96 (d, J = 12 Hz; 1 H) ; 4.03 (s, 1 H); 4.34 (m, 1 H); 4.45 (d, J = 12 Hz, 1 H); 4.80 (s, 2 H); 5.15-5.18 (m, 1 H); 6.81-6.83 (m, 1 H); 7.04-7.06 (m, 3 H); 7.52 (t, J = 7.8 Hz, 1 H); 7.78 (d, J = 7.5 Hz, 1 H); 7.89 (d, J = 7.7 Hz, 1 H); 7.95 (s, 1 H); 8.12 (s, 1 H); 8.53 (s, 1 H).

### Exemple 76 : 5-(2-chloro-3-méthylsulfanyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 76) (example comparatif)

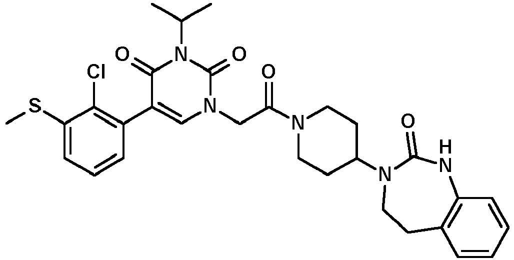

### 76.1 : 1-bromo-2-chloro-3-méthylsulfanyl-benzène

1,6ml (4 mmoles) d'une solution de n-butyllithium 2,5M dans l'hexane sont additionnés sur une solution refroidie à -78°C de 1g (3,7mmoles) de 1,3-dibromo-2-chloro-benzène dans 20ml d'éther diéthylique. Le mélange est agité à -78°C pendant 30 minutes, puis 0,4ml (4 mmoles) de diméthyl disulfide est additionné goutte à goutte. Le mileu réactionnel est agité pendant 15 minutes à -78°C puis 30 minutes à température ambiante. Il est lentement hydrolysé puis extrait par de l'acétate d'éthyle. La phase organique est lavée une fois par de l'eau et une fois par une solution aqueuse saturée de chlorure de sodium, puis séchée sur sulfate de magnésium, filtrée et concentrée sous vide.
Le résidu brut est chromatographié sur gel de silice élué par de l'heptane pur. 0,4g (43%) de 1-bromo-2-chloro-3-méthylsulfanyl-benzène sont obtenus sous la forme d'une huile incolore.

### 76.2 : [5-(2-chloro-3-méthylsulfanyl-phényl)-3-isopropyl-2,4-dioxo-3,4-dihydro-2H-pyrimidin-1-yl]-acétate de méthyle

65mg (0,1 mmoles) de complexe de dichlorométhane de 1,1'-bis-(diphénylphosphino)ferrocène-dichloropalladium(II) sont additionnés à une solution préalablement dégazée avec de l'azote de 380mg (1,6 mmoles) de 1-bromo-2-chloro-3-méthylsulfanylbenzène, 447mg (1,8 mmoles) de bis pinacol borane et 471mg (4,8 mmoles) d'acétate de potassium, dans 15ml de diméthylformamide. Le milieu réactionnel est chauffé à 90°C pendant 2 heures. Après refroidissement, 565mg (5,3 mmoles) de carbonate de sodium, 488mg (1,6 mmoles) de (5-bromo-3-isopropyl-2,4-dioxo-3,4-dihydro-2H-pyrimidin-1-yl)-acétate de méthyle (préparé comme décrit dans l'exemple 59.1), 65mg (0,1 mmoles) de complexe de dichlorométhane de 1,1'-bis-(diphénylphosphino)ferrocènedichloropalladium(II) et 1,5ml d'eau sont rajoutés. Après 4 heures de chauffage à 90°C, le milieu réactionnel est hydrolysé puis dilué par de l'acétate d'éthyle. Le produit est extrait à l'acétate d'éthyle. Les phases organiques sont rassemblées, lavées par de l'eau puis par une solution aqueuse saturée de chlorure de sodium, séchées sur sulfate de magnésium, filtrées et concentrées sous vide. Le résidu brut est chromatographié sur gel de silice élué par un mélange heptane / acétate d'éthyle 70/30. 80mg (13%) de [5-(2-chloro-3-méthylsulfanyl-phényl)-3-isopropyl-2,4-dioxo-3,4-dihydro-2H-pyrimidin-1-yl]-acétate de méthyle sont obtenus sous la forme d'une huile incolore.

### 76.3 : acide [5-(2-chloro-3-méthylsulfanyl-phényl)-3-isopropyl-2,4-dioxo-3,4-dihydro-2H-pyrimidin-1-yl]-acétique

De manière analogue à l'exemple 16.8, à partir de 80mg (0,2 mmoles) de [5-(2-chloro-3-méthylsulfanyl-phényl)-3-isopropyl-2,4-dioxo-3,4-dihydro-2H-pyrimidin-1-yl]-acétate de méthyle, 70mg (91%) d'acide [5-(2-chloro-3-méthylsulfanyl-phényl)-3-isopropyl-2,4-dioxo-3,4-dihydro-2H-pyrimidin-1-yl]-acétique sont obtenus sous la forme d'un solide jaune.

### 76.4 : 5-(2-chloro-3-méthylsulfanyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 76)

64mg (0,3 mmoles) de 3-pipéridin-4-yl-1,3,4,5-tétrahydro-benzo[d] [1,3]diazépin-2-one (préparé comme décrit dans l'exemple 16.4) et 40µl (0,3 mmoles) de triéthylamine sont ajoutés à une solution de 80mg (0,2 mmoles) d'acide [5-(2-chloro-3-méthylsulfanyl-phényl)-3-isopropyl-2,4-dioxo-3,4-dihydro-2H-pyrimidin-1-yl]-acétique, 29mg (0,3 mmoles) de 1-oxy-pyridin-2-ol et 50mg (0,3 mmoles) de chlorure de 1-éthyl-3-(3-diméthylaminopropyl)carbodiimide, dans 3,2ml de diméthylformamide. Le milieu réactionnel est agité à température ambiante pendant 18 heures. Il est hydrolysé par une solution aqueuse saturée d'hydrogénocarbonate de sodium puis dilué par de l'acétate d'éthyle. Le produit est extrait à l'acétate d'éthyle. Les phases organiques sont rassemblées, lavées par une solution aqueuse saturée d'hydrogénocarbonate de sodium, par de l'eau puis par une solution aqueuse saturée de chlorure de sodium, séchées sur sulfate de magnésium, filtrées et concentrées sous vide. Le résidu brut est chromatographié sur gel de silice élué par un mélange dichlorométhane / méthanol 97/3. Le produit est cristallisé dans l'acétate d'éthyle. 70mg (54%) de 5-(2-chloro-3-méthylsulfanyl-phényl)-3-isopropyl-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione sont obtenus sous la forme d'un solide blanc de point de fusion 261°C.
RMN ¹H (δ, DMSO) : 1.40 (d, J = 6.9 Hz, 6H); 1.50-1.80 (m, 4H); 2.55 (s, 3H); 2.68 (t, J = 12.4 Hz, 1H); 2.90 (m, 2H); 3.15 (t, J = 12.3 Hz, 1H); 3.38 (m, 2H); 3.94 (d, J = 13.5 Hz, 1H); 4.33 (m, 1H); 4.44 (d, J = 12.8 Hz, 1H); 4.73 (d, J = 5.4 Hz, 2H); 5.08-5.11 (m, 1H); 6.79-6.83 (m, 1H); 7.02-7.05 (m, 3H); 7.11 (dd, J = 7.5, 1.6 Hz, 1H); 7.31-7.33 (m, 1H); 7.40 (t, J = 7.7 Hz, 1H); 7.70 (s, 1H); 8.53 (s, 1H).

### Exemple 77 : 5-(2-chloro-3-méthanesulfinyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 77) (example comparatif)

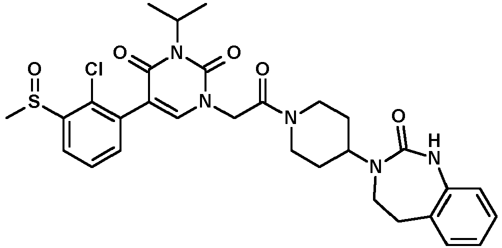

7,5mg (0,03 mmoles) d'acide 3-chloropéroxybenzoïque dans 0,2ml de dichlorométhane sont ajoutés sur une solution de 20mg (0,03 mmoles) de 5-(2-chloro-3-méthylsulfanyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 76.4) dans 0,2ml de dichlorométhane. Le mélange réactionnel est agité à température ambiante pendant 30 minutes. Il est dilué par du dichlorométhane. La phase organique est lavée une fois par une solution aqueuse saturée d'hydrogénocarbonate de sodium, une fois par une solution aqueuse 10% de thiosulfate de sodium, séchée sur sulfate de magnésium, filtrée et concentrée sous vide. Le résidu brut est chromatographié sur gel de silice élué par un mélange dichlorométhane / méthanol 97/3. 12mg (58%) de 5-(2-chloro-3-méthanesulfinyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.41 (d, J = 6.9 Hz, 6H); 1.53-1.76 (m, 4H); 2.66-2.72 (m, 1H); 2.83 (s, 3H); 2.90 (m, 2H); 3.15 (t, J = 11.9 Hz, 1H); 3.38 (m, 2H); 3.94 (d, J = 13.6 Hz, 1H); 4.33 (m, 1H); 4.43 (d, J = 12.9 Hz, 1H); 4.75 (m, 2H); 5.07-5.14 (m, 1H); 6.79-6.83 (m, 1H); 7.02-7.05 (m, 3H); 7.56 (dd, J = 7.5, 1.7 Hz, 1H); 7.71 (t, J = 7.7 Hz, 1H); 7.80 (s, 1H); 7.87 (dd, J = 7.8, 1.6 Hz, 1H); 8.53 (s, 1H).

### Exemple 78 : 5-(2-chloro-3-méthanesulfonyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 78) (example comparatif)

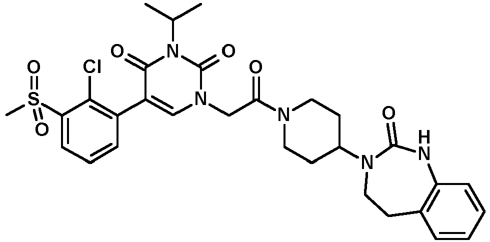

Une solution contenant 2mg (2 µmoles) de molybdate d'ammonium tétrahydrate dans 76µl (0,7 mmoles) de péroxyde d'hydrogène est additionnée sur 22mg (40 µmoles) de 5-(2-chloro-3-méthylsulfanyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 76.4), dans 0,9ml d'éthanol. Le milieu réactionnel est agité à température ambiante pendant 2 heures, il est hydrolysé par une solution aqueuse saturée d'hydrogénocarbonate de sodium puis extrait à l'acétate d'éthyle. La phase organique est lavée deux fois par une solution aqueuse à 10% de thiosulfate de sodium, une fois par de l'eau, séchée sur sulfate de magnésium, filtrée et concentrée sous vide. Le produit précipite dans l'éther diéthylique puis le solvant est éliminé sous azote et le solide blanc obtenu est séché sous vide. 18mg (76%) de 5-(2-chloro-3-méthanesulfonyl-phényl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.42 (d, J = 6.9 Hz, 6H); 1.51-1.74 (m, 4H); 2.69 (t, J = 12.4 Hz, 1H); 2.89-2.91 (m, 2H); 3.15 (t, J = 12.3 Hz, 1H); 3.37-3.39 (m, 2H); 3.42 (s, 3H); 3.94 (d, J = 13.6 Hz, 1H); 4.30-4.35 (m, 1H); 4.44 (d, J = 12.9 Hz, 1H); 4.71-4.81 (m, 2H); 5.08-5.15 (m, 1H); 6.79-6.83 (m, 1H); 7.02-7.05 (m, 3H); 7.65-7.74 (m, 2H); 7.81 (s, 1H); 8.11 (dd, J = 7.7, 1.9 Hz, 1H); 8.53 (s, 1H).

### Exemple 79 : 2-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïc acétate de méthyle (schéma réactionnel n°3, composé 79) (example comparatif)

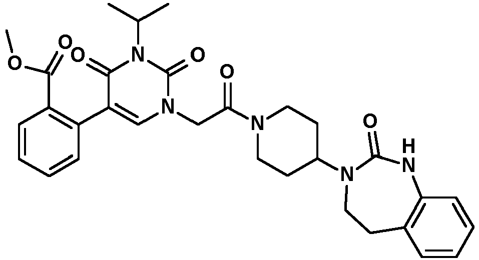

De manière analogue à l'exemple 44.1, à partir de 400mg (0,8 mmoles) de 5-bromo-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (préparé comme décrit dans l'exemple 74.2) et 278mg (1,5 mmoles) d'acide 2-méthoxycarbonyl phényl boronique et après purification par chromatographie liquide haute pression préparative (colonne Xbridge C18, isochratique, 46% acétonitrile dans eau), 150mg (33%) de 2-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïc acétate de méthyle sont obtenus sous la forme d'un solide blanc.
RMN ¹H (δ, DMSO) : 1.39 (d, J = 6.9 Hz, 6 H); 1.62-1.51 (m, 1 H); 1.80-1.63 (m, 3 H); 2.70 (t, J = 12.1 Hz, 1 H); 2.90 (m, 2 H); 3.16 (t, J = 12.6 Hz, 1 H); 3.39 (m, 2 H); 3.68 (s, 3 H); 3.96 (d, J = 13.5 Hz, 1 H); 4.34 (m, 1 H); 4.46 (d, J = 12.9 Hz, 1 H); 4.77 (s, 2 H); 5.11-5.04 (m, 1 H); 6.83-6.79 (m, 1 H); 7.05 (t, J = 3.6 Hz, 3 H); 7.32-7.30 (m, 1 H); 7.49 (td, J = 7.6, 1.3 Hz, 1 H); 7.63 (td, J = 7.6, 1.5 Hz, 1 H); 7.74 (s, 1 H) ; 7.80 (dd, J = 7.7, 1.4 Hz, 1 H) ; 8.54 (s, 1 H) .

### Exemple 80 : acide 2-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïque (schéma réactionnel n°3, composé 80) (example comparatif)

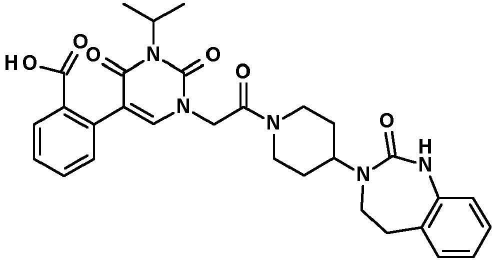

371mg (1,2 mmoles) d'hydroxyde de baryum octahydrate sont additionnés à une solution de 45mg (78 µmoles) de 2-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïc acétate de méthyle dans 2ml de méthanol. Le mélange est agité à température ambiante pendant 25 heures. De l'eau est rajoutée dans le milieu. La phase aqueuse est amenée à pH 5 avec une solution d'acide chlorhydrique aqueuse 1N, puis après extraction avec de l'acétate d'éthyle, la phase organique est séchée sur sulfate de sodium anhydre puis filtrée et concentrée à sec. Le résidu brut est purifié par chromatographie liquide haute pression préparative (colonne Xbridge C18, isochratique, 37% acétonitrile dans eau). 12mg (27%) d'acide 2-(3-isopropyl-2,4-dioxo-1-{2-oxo-2-[4-(2-oxo-1,2,4,5-tétrahydro-benzo[d][1,3]diazépin-3-yl)-pipéridin-1-yl]-éthyl}-1,2,3,4-tétrahydro-pyrimidin-5-yl)-benzoïque sont obtenus sous la forme d'un solide blanc.
RMN ¹H (δ, DMSO) : 0.67 (d, J = 6.9 Hz, 6H), 0.86-1.20 (m, 4H), 1.96 (t, J = 13.0 Hz, 1H), 2.14-2.23 (m, 2H), 2.41-2.44 (m, 1H), 2.63-2.79 (m, 3H), 3.25 (d, J = 13.7 Hz, 1H), 3.54-3.69 (m, 1H), 3.79-3.96 (m, 2H), 4.32-4.45 (m, 1H), 6.00-6.16 (m, 2H), 6.26 (d, J = 7.7 Hz, 2H) , 6.53 (d, J = 7.5 Hz, 1H), 6.59-6.70 (m, 2H), 6.74 (t, J = 7.4 Hz, 1H), 7.12 (d, J = 7.8 Hz, 1H).

### Exemple 81 : 5-(3,4-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 81) (example comparatif)

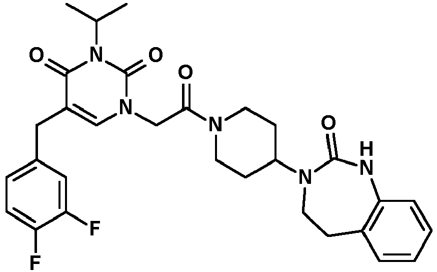

### 81.1 : 5-(tert-butyl-diméthyl-silanyloxyméthyl)-1H-pyrimidine-2,4-dione

2,6g (20 mmoles) de tert-butyl-diméthyl-chlorosilane sont additionnés sur une solution de 2g (10 mmoles) de 5-hydroxyméthyl-1H-pyrimidine-2,4-dione et 2,4g (40 mmoles) d'imidazole dans 20ml de diméthylformamide. Le milieu réactionnel est chauffé à 60°C pendant 1h30. Il est hydrolysé puis dilué par de l'acétate d'éthyle. La phase organique est lavée par de l'eau puis séchée sur sulfate de magnésium, filtrée et concentrée sous vide. La pâte blanche obtenue est reprise dans 10ml de méthanol et 100ml d'éther diéthylique. La suspension est filtrée pour donner 2,8g (78%) de 5-(tert-butyl-diméthyl-silanyloxyméthyl)-1H-pyrimidine-2,4-dione sous la forme d'un solide blanc.

### 81.2 : [5-(tert-butyl-diméthyl-silanyloxyméthyl)-2,4-dioxo-3,4-dihydro-2H-pyrimidin-1-yl]-acétate de méthyle

De manière analogue à l'exemple 13.1, à partir de 2,8g (11 mmoles) de 5-(tert-butyl-diméthyl-silanyloxyméthyl)-1H-pyrimidine-2,4-dione et de 1ml (11 mmoles) de bromoacétate de méthyle, 3,5g (96%) de [5-(tert-butyl-diméthylsilanyloxyméthyl)-2,4-dioxo-3,4-dihydro-2H-pyrimidin-1-yl]-acétate de méthyle sont obtenus sous la forme d'un solide blanc.

### 81.3 : [5-(tert-butyl-diméthyl-silanyloxyméthyl)-3-isopropyl-2,4-dioxo-3,4-dihydro-2H-pyrimidin-1-yl]-acétate de méthyle

De manière analogue à l'exemple 1.7, à partir de 3,5g (11 mmoles) de [5-(tert-butyl-diméthyl-silanyloxyméthyl)-2,4-dioxo-3,4-dihydro-2H-pyrimidin-1-yl]-acétate de méthyle et de 2ml (21 mmoles) de 2-bromopropane, et après purification par chromatographie flash sur gel de silice élué par un mélange heptane / acétate d'éthyle 80/20, 2,7g (68%) de [5-(tert-butyl-diméthyl-silanyloxyméthyl)-3-isopropyl-2,4-dioxo-3,4-dihydro-2H-pyrimidin-1-yl]-acétate de méthyle sont obtenus sous la forme d'une huile incolore.

### 81.4 : (5-hydroxyméthyl-3-isopropyl-2,4-dioxo-3,4-dihydro-2H-pyrimidin-1-yl)-acétate de méthyle

6,1ml (6 mmoles) d'une solution de fluorure de tétrabutylammonium 1M sont ajoutés à une solution de 1,5g (4 mmoles) de [5-(tert-butyl-diméthyl-silanyloxyméthyl)-3-isopropyl-2,4-dioxo-3,4-dihydro-2H-pyrimidin-1-yl]-acétate de méthyle dans 15ml de tétrahydrofurane, refroidie à 0°C. Le milieu réactionnel est agité à température ambiante pendant 45 minutes. Le milieu réactionnel est hydrolysé par une solution aqueuse saturée de chlorure d'ammonium puis dilué par de l'acétate d'éthyle. La phase organique est lavée par de l'eau puis par une solution aqueuse saturée de chlorure de sodium, séchée sur sulfate de magnésium, filtrée et concentrée sous vide. Le résidu brut est chromatographié sur gel de silice élué par un mélange dichlorométhane / méthanol 98/2. 0,75g (72%) de (5-hydroxyméthyl-3-isopropyl-2,4-dioxo-3,4-dihydro-2H-pyrimidin-1-yl)-acétate de méthyle sont obtenus sous la forme d'une huile incolore.

### 81.5 : (5-bromométhyl-3-isopropyl-2,4-dioxo-3,4-dihydro-2H-pyrimidin-1-yl)-acétate de méthyle

0,2ml (2,5 mmoles) d'une solution de tribromure de phosphore dans 4ml de dichlorométhane sont ajoutés à une solution de 0,6g (2,3 mmoles) de (5-hydroxyméthyl-3-isopropyl-2,4-dioxo-3,4-dihydro-2H-pyrimidin-1-yl)-acétate de méthyle dans 12ml de dichlorométhane, refroidie à 0°C. Le mélange est agité à 0°C pendant 30 minutes. Après retour à température ambiante, le milieu est hydrolysé par de l'eau puis par une solution aqueuse saturée d'hydrogénocarbonate de sodium. Le produit est extrait au dichlorométhane. La phase organique est lavée une fois par de l'eau puis séchée sur sulfate de magnésium, filtrée et concentrée sous vide. Le résidu brut est purifié par chromatographie sur gel de silice élué par un mélange dichlorométhane / méthanol 99/1. 0,41g (29%) de (5-bromométhyl-3-isopropyl-2,4-dioxo-3,4-dihydro-2H-pyrimidin-1-yl)-acétate de méthyle sont obtenus sous la forme d'un solide blanc.

### 81.6 : acide [5-(3,4-difluoro-benzyl)-3-isopropyl-2,4-dioxo-3,4-dihydro-2H-pyrimidin-1-yl]-acétique

De manière analogue à l'exemple 34, à partir de 210mg (0,7 mmoles) de (5-bromométhyl-3-isopropyl-2,4-dioxo-3,4-dihydro-2H-pyrimidin-1-yl)-acétate de méthyle et de 156mg (1 mmole) d'acide 3,4-difluorophénylboronique, 45mg (20%) d' acide [5-(3,4-difluoro-benzyl)-3-isopropyl-2,4-dioxo-3,4-dihydro-2H-pyrimidin-1-yl]-acétique sont obtenus sous la forme d'une huile jaune.

### 81.7 : 5-(3,4-difluoro-benzyl)-3-isopropyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 81)

De manière analogue à l'exemple 13.4, à partir de 45mg (0,1 mmoles) d'acide [5-(3,4-difluoro-benzyl)-3-isopropyl-2,4-dioxo-3,4-dihydro-2H-pyrimidin-1-yl]-acétique et de 39mg (0,2 mmoles) de 3-pipéridin-4-yl-1,3,4,5-tétrahydro-benzo[d][1,3]diazépin-2-one (préparé comme décrit dans l'exemple 16.4), et après purification par chromatographie sur gel de silice élué par un mélange dichlorométhane / méthanol 97/3, 35mg (46%) de 5-(3,4-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 224 °C.
RMN ¹H (δ, DMSO) : 1.35 (d, J = 6.9 Hz, 6H); 1.56-1.68 (m, 4H); 2.67 (t, J = 12.6 Hz, 1H); 2.90 (m, 2H); 3.09-3.15 (m, 1H); 3.36-3.41 (m, 2H); 3.56 (s, 2H); 3.92 (d, J = 13.5 Hz, 1H); 4.31 (m, 1H); 4.43 (d, J = 13.0 Hz, 1H); 4.67 (d, J = 3.5 Hz, 2H); 5.05-5.06 (m, 1H); 6.80-6.81 (m, 1H); 7.04-7.05 (m, 4H); 7.32-7.33 (m, 2H); 7.44 (s, 1H); 8.54 (s, 1H).

### Exemple 82 : 5-(3,5-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 82) (example comparatif)

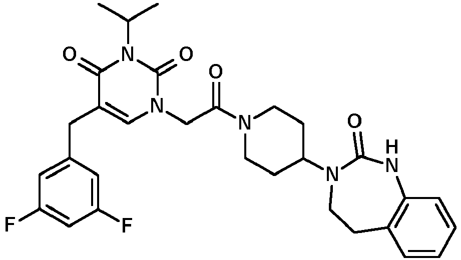

### 82.1 : acide [5-(3,5-difluoro-benzyl)-3-isopropyl-2,4-dioxo-3,4-dihydro-2H-pyrimidin-1-yl]-acétique

De manière analogue à l'exemple 34, à partir de 200mg (0,6 mmoles) de (5-bromométhyl-3-isopropyl-2,4-dioxo-3,4-dihydro-2H-pyrimidin-1-yl)-acétate de méthyle (préparé comme décrit dans l'exemple 81.5) et de 158mg (1 mmole) d'acide 3,5-difluorophénylboronique, et après ajout de 0,9ml (0,9 mmoles) d'une solution aqueuse d'hydroxyde de lithium 1M, 60mg (28%) d' acide [5-(3,5-difluoro-benzyl)-3-isopropyl-2,4-dioxo-3,4-dihydro-2H-pyrimidin-1-yl]-acétique sont obtenus sous la forme d'une solide blanc.

### 82.2 : 5-(3,5-difluoro-benzyl)-3-isopropyl-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione (composé 82)

De manière analogue à l'exemple 13.4, à partir de 60mg (0,2 mmoles) d'acide [5-(3,5-difluoro-benzyl)-3-isopropyl-2,4-dioxo-3,4-dihydro-2H-pyrimidin-1-yl]-acétique et de 52mg (0,2 mmoles) de 3-pipéridin-4-yl-1,3,4,5-tétrahydro-benzo[d][1,3]diazépin-2-one (préparé comme décrit dans l'exemple 16.4), et après purification par chromatographie sur gel de silice élué par un mélange dichlorométhane / méthanol 97/3 et précipitation du produit dans l'éther diéthylique, filtration et séchage, 55mg (54%) de 5-(3,5-difluoro-benzyl)-3-isopropyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 238°C.
RMN ¹H (δ, DMSO) : 1.35 (d, J = 6.9 Hz, 6H); 1.50-1.80 (m, 4H); 2.68 (m, 1H); 2.90 (m, 2H); 3.14 (m, 1H); 3.39 (m, 2H); 3.59 (s, 2H); 3.93 (d, J = 13.6 Hz, 1H); 4.32 (m, 1H); 4.43 (d, J = 12.9 Hz, 1H); 4.68 (d, J = 3.4 Hz, 2H); 5.05-5.06 (m, 1H); 6.80-6.81 (m, 1H); 6.96-6.99 (m, 2H); 7.04-7.06 (m, 4H); 7.49 (s, 1H) ; 8.54 (s, 1H).

### Exemple 83 : 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 83) (example comparatif)

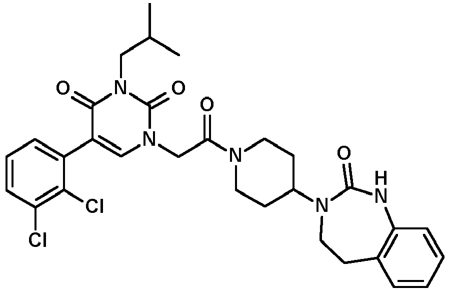

### 83.1 : (5-bromo-3-isobutyl-2,4-dioxo-3,4-dihydro-2H-pyrimidin-1-yl)-acétate de méthyle

De manière analogue à l'exemple 1.7, à partir de 2g (8 mmoles) de (5-bromo-2,4-dioxo-3,4-dihydro-2H-pyrimidin-1-yl)-acétate de méthyle (préparé comme décrit dans l'exemple 13.1), et de 1,2ml (11 mmoles) de 1-bromo-2-méthyl propane, et après chauffage du mélange à 50°C pendant 4 heures, 1,8g (74%) de (5-bromo-3-isobutyl-2,4-dioxo-3,4-dihydro-2H-pyrimidin-1-yl)-acétate de méthyle sont obtenus sous la forme d'un solide blanc.

### 83.2 : [5-(2,3-dichloro-phényl)-3-isobutyl-2,4-dioxo-3,4-dihydro-2H-pyrimidin-1-yl]-acétate de méthyle

De manière analogue à l'exemple 44.1, à partir de 400mg (1,3 mmoles) de (5-bromo-3-isobutyl-2,4-dioxo-3,4-dihydro-2H-pyrimidin-1-yl)-acétate de méthyle et de 717mg (3,8 mmoles) d'acide 2,3-dichlorophénylboronique et après purification par chromatographie sur gel de silice élué par un mélange heptane / acétate d'éthyle 80/20, 260mg (54%) de [5-(2,3-dichlorophényl)-3-isobutyl-2,4-dioxo-3,4-dihydro-2H-pyrimidin-1-yl]-acétate de méthyle sont obtenus sous la forme d'un solide jaune pâle.

### 83.3 : acide [5-(2,3-dichloro-phényl)-3-isobutyl-2,4-dioxo-3,4-dihydro-2H-pyrimidin-1-yl]-acétique

De manière analogue à l'exemple 16.8, à partir de 260mg (0,7 mmoles) de de [5-(2,3-dichloro-phényl)-3-isobutyl-2,4-dioxo-3,4-dihydro-2H-pyrimidin-1-yl]-acétate de méthyle, 250mg (100%) d'acide [5-(2,3-dichloro-phényl)-3-isobutyl-2,4-dioxo-3,4-dihydro-2H-pyrimidin-1-yl]-acétique sont obtenus sous la forme d'un solide beige.

### 83.4 : 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 83)

De manière analogue à l'exemple 13.4, à partir de 250mg (0,7 mmoles) d'acide [5-(2,3-dichloro-phényl)-3-isopropyl-2,4-dioxo-3,4-dihydro-2H-pyrimidin-1-yl]-acétique et de 192mg (0,7 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 290mg (71%) de 5-(2,3-dichloro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 248 °C.
RMN ¹H (δ, DMSO) : 0.86 (d, J = 6.7 Hz, 6H), 1.48-1.60 (m, 1H), 1.62-1.80 (m, 3H), 2.03 (m, 1H), 2.67 (t, J = 12 Hz, 1H), 2.89 (t, J = 4.5 Hz, 2H), 3.14 (t, J = 12 Hz, 1H), 3.36 (t, J = 4.5 Hz, 2H), 3. 71 (d, J = 7.3 Hz, 2H), 3.92 (d, J = 13 Hz, 1H), 4.28-4.38 (m, 1H), 4.42 (d, J = 13 Hz, 1H), 4.76 (d, J = 2.2 Hz, 2H), 6.75-6.82 (m, 1H), 7.01-7.04 (m, 3H), 7.32 (dd, J = 1.5-7.6 Hz, 1H), 7.42 (t, J = 7.8 Hz, 1H), 7.68 (dd, J = 1.48 Hz, 1H), 7.81 (s, 1H), 8.53 (s, 1H).

### Exemple 84 : 5-(2-chloro-3-fluoro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 84) (example comparatif)

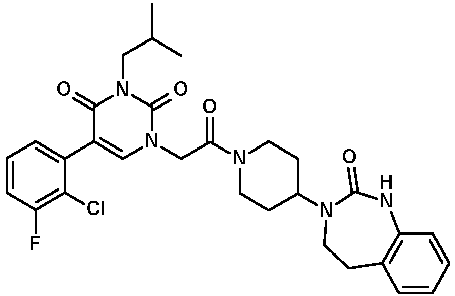

### 84.1 : acide [5-(2-chloro-3-fluoro-phényl)-3-isobutyl-2,4-dioxo-3,4-dihydro-2H-pyrimidin-1-yl]-acétique

De manière analogue à l'exemple 13.3, à partir de 300mg (0,9 mmoles) de (5-bromo-3-isobutyl-2,4-dioxo-3,4-dihydro-2H-pyrimidin-1-yl)-acétate de méthyle (préparé comme décrit dans l'exemple 83.1) et de 246mg (1,4 mmoles) d'acide 2-chloro-3-fluorophénylboronique, 215mg (64%) d' acide [5-(2-chloro-3-fluoro-phényl)-3-isobutyl-2,4-dioxo-3,4-dihydro-2H-pyrimidin-1-yl]-acétique sont obtenus sous la forme d'une huile jaune.

### 84.2 : 5-(2-chloro-3-fluoro-phényl)-3-isobutyl-1-{2-oxo-2-[4-(2-oxo-1,2,4,5-tétrahydrobenzo[d][1,3]diazépin-3-yl)-pipéridin-1-yl]-éthyl}-1H-pyrimidine-2,4-dione (composé 84)

De manière analogue à l'exemple 13.4, à partir de 215mg (0,6 mmoles) d'acide [5-(2-chloro-3-fluoro-phényl)-3-isopropyl-2,4-dioxo-3,4-dihydro-2H-pyrimidin-1-yl]-acétique et de 164mg (0,7 mmoles) de 3-pipéridin-4-yl-1,3,4,5-tétrahydro-benzo[d][1,3]diazépin-2-one (préparé comme décrit dans l'exemple 16.4), et après recristallisation dans l'acétone, 140mg (38%) de 5-(2-chloro-3-fluoro-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 248°C.
RMN ¹H (δ, DMSO) : 0.87 (d, J = 6.7 Hz, 6H), 1.48-1.60 (m, 1H), 1.62-1.80 (m, 3H), 2.04 (m, 1H), 2.68 (t, J = 11 Hz, 1H), 2.89 (t, J = 4.3 Hz, 2H), 3.15 (t, J = 11 Hz, 1H), 3.37 (t, J = 4.3 Hz, 2H), 3.72 (d, J = 7.3 Hz, 2H), 3.94 (d, J = 13.4 Hz, 1H), 4.32 (m, 1H), 4.42 (d, J = 13.1 Hz, 1H), 4.77 (d, J = 2.2 Hz, 2H), 6.78-6.82 (m, 1H), 7.02-7.05 (m, 3H), 7.20-7.22 (m, 1H), 7.43-7.47 (m, 2H), 7.83 (s, 1H), 8.54 (s, 1H).

### Exemple 85 : 5-(2,3-diméthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 85) (example comparatif)

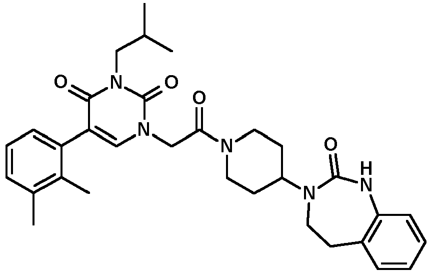

### 85.1 : [5-(2,3-diméthyl-phényl)-3-isobutyl-2,4-dioxo-3,4-dihydro-2H-pyrimidin-1-yl]-acétate de méthyle

De manière analogue à l'exemple 44.1, à partir de 300mg (0,9 mmoles) de (5-bromo-3-isobutyl-2,4-dioxo-3,4-dihydro-2H-pyrimidin-1-yl)-acétate de méthyle (préparé comme décrit dans l'exemple 83.1) et de 211mg (1,4 mmoles) d'acide 2,3-diméthylphénylboronique, 255mg (79%) de [5-(2,3-diméthyl-phényl)-3-isobutyl-2,4-dioxo-3,4-dihydro-2H-pyrimidin-1-yl]-acétate de méthyle sont obtenus sous la forme d'une huile jaune.

### 85.2 : acide [5-(2,3-diméthyl-phényl)-3-isobutyl-2,4-dioxo-3,4-dihydro-2H-pyrimidin-1-yl]-acétique

De manière analogue à l'exemple 16.8, à partir de 255mg (0,7 mmoles) de [5-(2,3-diméthyl-phényl)-3-isobutyl-2,4-dioxo-3,4-dihydro-2H-pyrimidin-1-yl]-acétate de méthyle, 230mg (94%) d'acide [5-(2,3-diméthyl-phényl)-3-isobutyl-2,4-dioxo-3,4-dihydro-2H-pyrimidin-1-yl]-acétique sont obtenus sous la forme d'un solide blanc.

### 85.3 : 5-(2,3-diméthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 85)

De manière analogue à l'exemple 13.4, à partir de 230mg (0,7 mmoles) d'acide [5-(2,3-diméthyl-phényl)-3-isopropyl-2,4-dioxo-3,4-dihydro-2H-pyrimidin-1-yl]-acétique et de 188mg (0,8 mmoles) de 3-pipéridin-4-yl-1,3,4,5-tétrahydro-benzo[d][1,3]diazépin-2-one (préparé comme décrit dans l'exemple 16.4), et après recristallisation dans un mélange acétone / heptane 50/50, 170mg (43%) de [5-(2,3-diméthylphényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 270°C.

RMN ¹H (δ, DMSO) : 0.86 (d, J = 6.7 Hz, 6H), 1.48-1.60 (m, 1H), 1.62-1.80 (m, 3H), 2.03 (s, 3H), 2.06 (m, 1H), 2.27 (s, 3H), 2.67 (t, J = 11 Hz, 1H), 2.89 (t, J = 4.5 Hz, 2H), 3.15 (t, J = 11 Hz, 1H), 3.37 (t, J = 4.4 Hz, 2H), 3.72 (d, J = 7.2 Hz, 2H), 3.95 (d, J = 13 Hz, 1H), 4.28-4.38 (m, 1H), 3.95 (d, J = 13 Hz, 1H), 4.75 (d, J = 5.2 Hz, 2H), 6.80-6.82 (m, 1H), 6.96 (d, J = 7.1 Hz, 1H), 7.02 (s, 1H), 7.04 (d, J = 3.7 Hz, 2H), 7.10 (t, J = 7.5 Hz, 1H), 7.17 (d, J = 7.4 Hz, 1H), 7.60 (s, 1H), 8.54 (s, 1H).

### Exemple 86 : 5-(3-fluoro-2-méthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 86) (example comparatif)

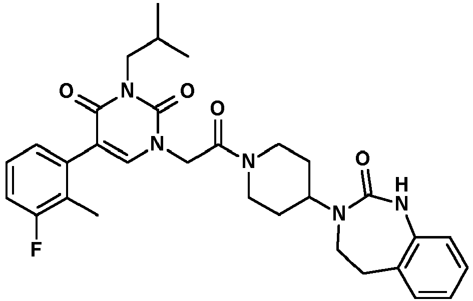

### 86.1 : [5-(3-fluoro-2-méthyl-phényl)-3-isobutyl-2,4-dioxo-3,4-dihydro-2H-pyrimidin-1-yl]-acétate de méthyle

De manière analogue à l'exemple 44.1, à partir de 500mg (1,6 mmoles) de (5-bromo-3-isobutyl-2,4-dioxo-3,4-dihydro-2H-pyrimidin-1-yl)-acétate de méthyle (préparé comme décrit dans l'exemple 83.1) et de 386mg (2,5 mmoles) d'acide 3-fluoro-2-méthyl-phénylboronique, et après purification par chromatographie sur gel de silice élué par un mélange heptane / acétate d'éthyle 80/20, 620mg (100%) de [5-(3-fluoro-2-méthylphényl)-3-isobutyl-2,4-dioxo-3,4-dihydro-2H-pyrimidin-1-yl]-acétate de méthyle sont obtenus sous la forme d'une huile jaune.

### 86.2 : acide [5-(3-fluoro-2-méthyl-phényl)-3-isobutyl-2,4-dioxo-3,4-dihydro-2H-pyrimidin-1-yl]-acétique

De manière analogue à l'exemple 16.8, à partir de 545mg (1,6 mmoles) de [5-(3-fluoro-2-méthyl-phényl)-3-isobutyl-2,4-dioxo-3,4-dihydro-2H-pyrimidin-1-yl]-acétate de méthyle, 440mg (84%) d'acide [5-(3-fluoro-2-méthyl-phényl)-3-isobutyl-2,4-dioxo-3,4-dihydro-2H-pyrimidin-1-yl]-acétique sont obtenus sous la forme d'un solide beige.

### 86.3 : 5-(3-fluoro-2-méthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 86)

De manière analogue à l'exemple 13.4, à partir de 440mg (1,3 mmoles) d'acide [5-(3-fluoro-2-méthyl-phényl)-3-isopropyl-2,4-dioxo-3,4-dihydro-2H-pyrimidin-1-yl]-acétique et de 355mg (1,5 mmoles) de 3-pipéridin-4-yl-1,3,4,5-tétrahydro-benzo[d][1,3]diazépin-2-one (préparé comme décrit dans l'exemple 16.4), et après recristallisation dans l'éthanol, 530mg (71%) de [5-(3-fluoro-2-méthyl-phényl)-3-isobutyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 251 °C.

RMN ¹H (δ, DMSO) : 0.87 (d, J = 6.7 Hz, 6H); 1.52-1.73 (m, 4H); 2.01-2.07 (m, 4H); 2.68 (t, J = 12.6 Hz, 1H); 2.86-2.91 (m, 2H); 3.15 (t, J = 12.4 Hz, 1H); 3.34-3.39 (m, 2H); 3.73 (d, J = 7.3 Hz, 2H); 3.94 (d, J = 13.7 Hz, 1H); 4.28-4.35 (m, 1H); 4.43 (d, J = 12.9 Hz, 1H) ; 4.77 (d, J = 3.2 Hz, 2H) ; 6.79-6.80 (m, 1H); 7.00-7.05 (m, 4H); 7.16-7.27 (m, 2H); 7.72 (s, 1H) ; 8.54 (s, 1H).

### Exemple 87 : [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamic acid tert-butyl ester (schéma réactionnel n°3, composé 87) (example comparatif)

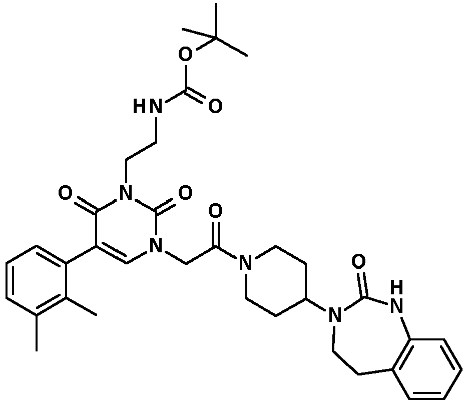

### 87.1 : [5-bromo-3-(2-tert-butoxycarbonylamino-éthyl)-2,4-dioxo-3,4-dihydro-2H-pyrimidin-1-yl]-acétate de méthyle

De manière analogue à l'exemple 1.7, à partir de 0,5g (2 mmoles) de (5-bromo-2,4-dioxo-3,4-dihydro-2H-pyrimidin-1-yl)-acétate de méthyle (préparé comme décrit dans l'exemple 13.1), et de 511mg (2 mmoles) de tert-butyl-N-(2-bromo-éthyl)carbamate, et après chauffage du mélange à 50°C pendant 3 heures, 0,63mg (82%) de [5-bromo-3-(2-tert-butoxycarbonylamino-éthyl)-2,4-dioxo-3,4-dihydro-2H-pyrimidin-1-yl]-acétate de méthyle sont obtenus sous la forme d'une huile incolore.

### 87.2 : acide [3-(2-tert-butoxycarbonylamino-éthyl)-5-(2,3-diméthyl-phényl)-2,4-dioxo-3,4-dihydro-2H-pyrimidin-1-yl]-acétique

De manière analogue à l'exemple 44.1, à partir de 632mg (1,6 mmoles) de [5-bromo-3-(2-tert-butoxycarbonylamino-éthyl)-2,4-dioxo-3,4-dihydro-2H-pyrimidin-1-yl]-acétate de méthyle et de 350mg (2,3 mmoles) d'acide 2,3-diméthylphénylboronique, 380mg (59%) d' acide [3-(2-tert-butoxycarbonylamino-éthyl)-5-(2,3-diméthyl-phényl)-2,4-dioxo-3,4-dihydro-2H-pyrimidin-1-yl]-acétique sont obtenus sous la forme d'un solide beige.

### 87.3 : [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert-butyle (composé 87)

De manière analogue à l'exemple 13.4, à partir de 380mg (0,9 mmoles) d'acide [3-(2-tert-butoxycarbonylamino-éthyl)-5-(2,3-diméthyl-phényl)-2,4-dioxo-3,4-dihydro-2H-pyrimidin-1-yl]-acétique et de 246mg (1 mmole) de 3-pipéridin-4-yl-1,3,4,5-tétrahydro-benzo[d][1,3]diazépin-2-one (préparé comme décrit dans l'exemple 16.4), et après cristallisation dans un mélange heptane / acétate d'éthyle 70/30, 325mg (55%) de [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert-butyle sont obtenus sous la forme d'un solide beige de point de fusion 169°C.

RMN ¹H (δ, DMSO) : 1.36 (s, 9H); 1.55-1.67 (m, 4H); 2.07 (s, 3H); 2.27 (s, 3H); 2.68 (t, J = 12.5 Hz, 1H); 2.89 (m, 2H); 3.11-3.19 (m, 3H); 3.35-3.40 (m, 2H); 3.94 (m, 3H); 4.30 (m, 1H); 4.43 (d, J = 12.9 Hz, 1H); 4.74 (s, 2H); 6.80-6.83 (m, 2H); 6.95 (d, J = 7.5 Hz, 1H); 7.02-7.04 (m, 3H); 7.10 (t, J = 7.5 Hz, 1H); 7.17 (d, J = 7.6 Hz, 1H); 7.57 (s, 1H); 8.53 (s, 1H).

### Exemple 88 : 3-(2-amino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 88) (example comparatif)

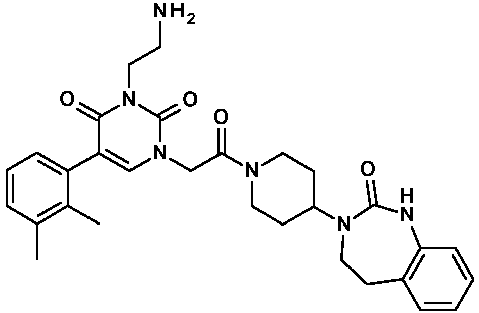

2ml (5 mmoles) d'une solution d'acide chlorhydrique 2.5M dans l'éthanol sont additionnés à 100mg (0,2 mmoles) de [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert-butyle (préparé comme décrit dans l'exemple 87.3). Le milieu réactionnel est agité pendant 3 heures à température ambiante puis concentré sous vide. Le mélange est hydrolysé par une solution aqueuse saturée d'hydrogénocarbonate de sodium puis dilué par de l'acétate d'éthyle. La phase organique est lavée avec une solution aqueuse saturée d'hydrogénocarbonate de sodium, puis par de l'eau et par une solution aqueuse saturée de chlorure de sodium; séchée sur sulfate de magnésium, filtrée et concentrée sous vide. Le solide blanc obtenu est repris dans de l'éther diéthylique puis filtré et séché sous vide. 80mg (93%) de 3-(2-amino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 230°C.
RMN ¹H (δ, DMSO) : 1.55-1.80 (m, 6H); 2.05 (s, 3H); 2.27 (s, 3H); 2.64-2.71 (m, 3H); 2.89 (m, 2H); 3.14 (t, J = 12.5 Hz, 1H); 3.37 (m, 2H); 3.87 (t, J = 7.0 Hz, 2H); 3.94 (d, J = 13.5 Hz, 1H); 4.32 (m, 1H); 4.43 (d, J = 13.0 Hz, 1H); 4.75 (d, J = 6.1 Hz, 2H); 6.79-6.81 (m, 1H); 6.95-7.04 (m, 4H); 7.08-7.18 (m, 2H); 7.58 (s, 1H); 8.54 (s, 1H).

### Exemple 89 : 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 89) (example comparatif)

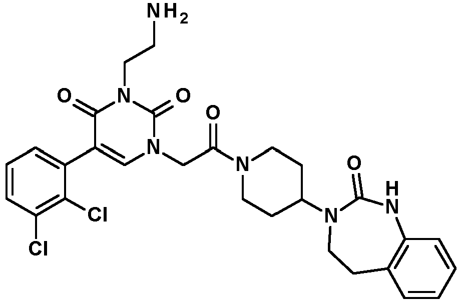

### 89.1 : acide [3-(2-tert-butoxycarbonylamino-éthyl)-5-(2,3-dichloro-phényl)-2,4-dioxo-3,4-dihydro-2H-pyrimidin-1-yl]-acétique

De manière analogue à l'exemple 44.1, à partir de 700mg (1,7 mmoles) de [5-bromo-3-(2-tert-butoxycarbonylamino-éthyl)-2,4-dioxo-3,4-dihydro-2H-pyrimidin-1-yl]-acétate de méthyle (préparé comme décrit dans l'exemple 87.1) et 526mg (2,8 mmoles) d'acide 2,3-dichlorophénylboronique, 210mg (27%) d'acide [3-(2-tert-butoxycarbonylamino-éthyl)-5-(2,3-dichlorophényl)-2,4-dioxo-3,4-dihydro-2H-pyrimidin-1-yl]-acétique sont obtenus sous la forme d'une huile orange.

### 89.2 : [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert-butyle

De manière analogue à l'exemple 13.4, à partir de 210mg (0,5 mmoles) d'acide [3-(2-tert-butoxycarbonylamino-éthyl)-5-(2,3-dichloro-phényl)-2,4-dioxo-3,4-dihydro-2H-pyrimidin-1-yl]-acétique et de 112mg (0,5 mmoles) de 3-pipéridin-4-yl-1,3,4,5-tétrahydro-benzo[d][1,3]diazépin-2-one (préparé comme décrit dans l'exemple 16.4), 190mg (60%) de [2-(5-(2,3-dichlorophényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert butyle sont obtenus sous la forme d'un solide blanc.

### 89.3 : 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 89)

De manière analogue à l'exemple 88, à partir de 190mg (0,3 mmoles) de [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-carbamate de tert butyle, 145mg (88%) de 3-(2-amino-éthyl)-5-(2,3-dichloro-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 250°C.
RMN ¹H (δ, DMSO) : 1.48-1.85 (m, 4H); 2.65-2.73 (m, 3H); 2.89 (m, 2H); 3.11-3.16 (m, 1H); 3.35-3.50 (m, 2H); 3.89-3.93 (m, 3H); 4.32 (m, 1H); 4.42 (d, J = 12.7 Hz, 1H); 4.79 (s, 2H); 6.79-6.81 (m, 1H); 7.02-7.04 (m, 3H); 7.34 (dd, J = 7.6, 1.6 Hz, 1H); 7.43 (t, J = 7.9 Hz, 1H); 7.69 (dd, J = 8.0, 1.6 Hz, 1H); 7.85 (s, 1H); 8.55 (s, 1H).

### Exemple 90 : 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 90) (example comparatif)

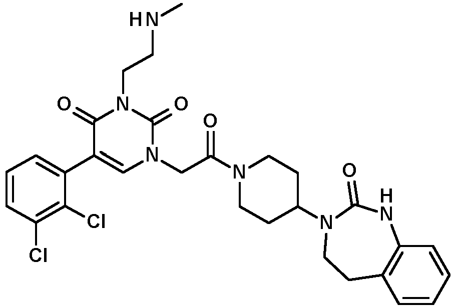

### 90.1 : [3-(2-tert-butoxycarbonylamino-éthyl)-5-(2,3-dichlorophényl)-2,4-dioxo-3,4-dihydro-2H-pyrimidin-1-yl]-acétate de méthyle

De manière analogue à l'exemple 44.1, à partir de 700mg (1,7 mmoles) de [5-bromo-3-(2-tert-butoxycarbonylamino-éthyl)-2,4-dioxo-3,4-dihydro-2H-pyrimidin-1-yl]-acétate de méthyle (préparé comme décrit dans l'exemple 87.1) et 526mg (2,8 mmoles) d'acide 2,3-dichlorophénylboronique, 130mg (16%) de [3-(2-tert-butoxycarbonylamino-éthyl)-5-(2,3-dichloro-phényl)-2,4-dioxo-3,4-dihydro-2H-pyrimidin-1-yl]-acétate de méthyle sont obtenus sous la forme d'un solide beige.

### 90.2 : acide[3-[2-(tert-butoxycarbonyl-méthyl-amino)-éthyl]-5-(2,3-dichloro-phényl)-2,4-dioxo-3,4-dihydro-2H-pyrimidin-1-yl]-acétique

De manière analogue à l'exemple 13.2, à partir de 130mg (0,3 mmoles) de [3-(2-tert-butoxycarbonylamino-éthyl)-5-(2,3-dichloro-phényl)-2,4-dioxo-3,4-dihydro-2H-pyrimidin-1-yl]-acétate de méthyle et 26µl (0,4 mmoles) de iodométhane, 100mg (77%) d'acide[3-[2-(tert-butoxycarbonyl-méthyl-amino)-éthyl]-5-(2,3-dichloro-phényl)-2,4-dioxo-3,4-dihydro-2H-pyrimidin-1-yl]-acétique sont obtenus sous la forme d'un solide jaune pâle.

### 90.3 : [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tert-butyle

De manière analogue à l'exemple 13.4, à partir de 100mg (0,2 mmoles) d'acide [3-[2-(tert-butoxycarbonyl-méthyl-amino)-éthyl]-5-(2,3-dichloro-phényl)-2,4-dioxo-3,4-dihydro-2H-pyrimidin-1-yl]-acétique et de 52mg (0,2 mmoles) de 3-pipéridin-4-yl-1,3,4,5-tétrahydro-benzo[d][1,3]diazépin-2-one (préparé comme décrit dans l'exemple 16.4), 95mg (64%) de [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tert-butyle sont obtenus sous la forme d'un solide blanc.

### 90.4 : 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 90)

De manière analogue à l'exemple 88, à partir de 95mg (0,1 mmoles) [2-(5-(2,3-dichloro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tert-butyle, et après recristallisation dans l'éthanol, 55mg (66%) de 5-(2,3-dichloro-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 225°C.
RMN ¹H (δ, DMSO) : 1.45-1.69 (m, 4H); 2.29 (s, 3H); 2.65-2.71 (m, 3H); 2.90 (m, 2H); 3.14-3.20 (m, 1H); 3.38 (m, 2H); 3.95 (t, J = 7.0 Hz, 2H); 4.33 (m, 1H); 4.43 (d, J = 12.9 Hz, 1H); 4.77 (d, J = 2.9 Hz, 2H); 6.80-6.81 (m, 1H); 7.03-7.05 (m, 3H); 7.34 (dd, J = 7.7, 1.6 Hz, 1H); 7.43 (t, J = 7.9 Hz, 1H); 7.69 (dd, J = 8.0, 1.6 Hz, 1H); 7.81 (s, 1H); 8.53 (s, 1H).

### Exemple 91 : 3-((R)-2,3-dihydroxy-propyl)-5-(2,3-diméthylphényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 91) (example comparatif)

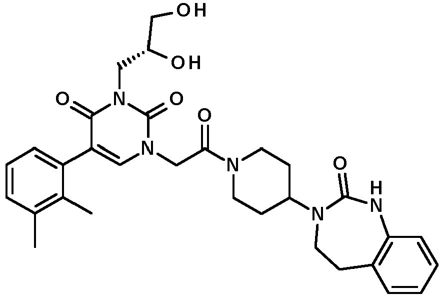

### 91.1 : acide [5-bromo-3-((R)-2,2-diméthyl-[1,3]dioxolan-4-ylméthyl)-2,4-dioxo-3,4-dihydro-2H-pyrimidin-1-yl]-acétique

0,8g (5,7 mmoles) de carbonate de potassium et 0,6ml (4,6 mmoles) de (S)-(-)-4-(chlorométhyl)-2,2-diméthyl-1,3-dioxolane sont ajoutés à une solution de 1g (3,8 mmoles) de (5-bromo-2,4-dioxo-3,4-dihydro-2H-pyrimidin-1-yl)-acétate de méthyle (préparé comme décrit dans l'exemple 13.1), dans 25ml de diméthylformamide. Le milieu réactionnel est chauffé à 70°C pendant 2h30. 1,5g (4 mmoles) de iodure de tétrabutylammonium sont ajoutés ainsi que 0,5ml (4 mmoles) de (S)-(-)-4-(chlorométhyl)-2,2-diméthyl-1,3-dioxolane. Après 20 heures de chauffage à 130°C, le milieu réactionnel est hydrolysé puis dilué par de l'acétate d'éthyle. La phase aqueuse est amenée à pH=5 par une solution aqueuse d'acide chlorhydrique 1N. Le produit est extrait à l'acétate d'éthyle. La phase organique est lavée par de l'eau et par une solution aqueuse saturée de chlorure de sodium, puis séchée sur sulfate de magnésium, filtrée et concentrée sous vide. Le résidu brut est chromatographié sur gel de silice élué par un mélange dichlorométhane / méthanol 97/3. 115mg (8%) d'acide [5-bromo-3-((R)-2,2-diméthyl-[1,3]dioxolan-4-ylméthyl)-2,4-dioxo-3,4-dihydro-2H-pyrimidin-1-yl]-acétique sont obtenus sous la forme d'une huile orange.

### 91.2 : 5-bromo-3-((R)-2,3-dihydroxy-propyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione

De manière analogue à l'exemple 13.4, à partir de 115mg (0,3 mmoles) d'acide [5-bromo-3-((R)-2,2-diméthyl-[1,3]dioxolan-4-ylméthyl)-2,4-dioxo-3,4-dihydro-2H-pyrimidin-1-yl]-acétique et de 82mg (0,3 mmoles) de 3-pipéridin-4-yl-1,3,4,5-tétrahydro-benzo[d] [1,3]diazépin-2-one (préparé comme décrit dans l'exemple 16.4), 16mg (9%) de 5-bromo-3-((R)-2,3-dihydroxypropyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige.

### 91.3 : 3-((R)-2,3-dihydroxy-propyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 91)

De manière analogue à l'exemple 44.1, à partir de 16mg (30 µmoles) de 5-bromo-3-((R)-2,3-dihydroxy-propyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione et de 7mg (40 µmoles) d'acide 2,3-diméthylphénylboronique, 4mg (20%) de 3-((R)-2,3-dihydroxy-propyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide jaune clair.

Analyse HPLC (colonne Kinetex C18, 150x2,1mm, 1,7µm, éluant: eau/acétonitrile avec 0,1% acide formique, run de 30min): tr = 9,37min.

### Exemple 92 : 3-(3,4-dihydroxy-butyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 92) (example comparatif)

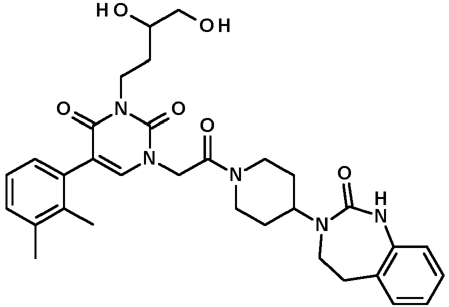

### 92.1 : [5-bromo-3-((S)-2-oxiranyl-éthyl)-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1) et 0,6g (3,8 mmoles) de (S)-2-(2-bromo-éthyl)-oxirane, 0,8g (66%) de [5-bromo-3-((S)-2-oxiranyl-éthyl)-2,4-dioxo-3,4-dihydro-2H-pyrimidin-1-yl]-acétate de méthyle sont obtenus sous la forme d'un solide blanc.

### 92.2 : acide [3-(3,4-dihydroxy-butyl)-5-(2,3-diméthyl-phényl)-2,4-dioxo-3,4-dihydro-2H-pyrimidin-1-yl]-acétique

De manière analogue à l'exemple 13.3, à partir de 400mg (1,2 mmoles) de [5-bromo-3-((S)-2-oxiranyl-éthyl)-2,4-dioxo-3,4-dihydro-2H-pyrimidin-1-yl]-acétate de méthyle et 270mg (1,8 mmoles) d'acide 2,3-diméthylphénylboronique, 280mg (64%) d'acide [3-(3,4-dihydroxy-butyl)-5-(2,3-diméthyl-phényl)-2,4-dioxo-3,4-dihydro-2H-pyrimidin-1-yl]-acétique sont obtenus sous la forme d'un solide rose.

### 92.3 : 3-(3,4-dihydroxy-butyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 92)

De manière analogue à l'exemple 13.4, à partir de 280mg (0,8 mmoles) d'acide [3-(3,4-dihydroxy-butyl)-5-(2,3-diméthylphényl)-2,4-dioxo-3,4-dihydro-2H-pyrimidin-1-yl]-acétique et 227mg (0,9 mmoles) de 3-pipéridin-4-yl-1,3,4,5-tétrahydro-benzo[d][1,3]diazépin-2-one (préparé comme décrit dans l'exemple 16.4), 75mg (16%) de 3-(3,4-dihydroxy-butyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide rose de point de fusion 175°C.

RMN ¹H (δ, DMSO) : 1.49-1.80 (m, 6H); 2.04 (s, 3H); 2.27 (s, 3H); 2.68 (t, J = 12.4 Hz, 1H); 2.89 (m, 2H); 3.14 (t, J = 12.4 Hz, 1H); 3.22-3.24 (m, 1H); 3.30-3.32 (m, 1H); 3.35-3.39 (m, 2H); 3.45-3.48 (m, 1H); 3.83-3.88 (m, 1H); 3.94 (d, J = 13.7 Hz, 1H); 4.05-4.08 (m, 1H); 4.28-4.36 (m, 1H); 4.43 (d, J = 12.9 Hz, 1H); 4.53-4.54 (m, 2H); 4.75 (d, J = 3.5 Hz, 2H); 6.79-6.80 (m, 1H); 6.96 (d, J = 7.5 Hz, 1H); 7.03-7.05 (m, 3H); 7.10 (t, J = 7.5 Hz, 1H); 7.17 (d, J = 7.5 Hz, 1H); 7.59 (s, 1H); 8.55 (s, 1H).

### Exemple 93 : 5-(2,3-diméthyl-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 93) (example comparatif)

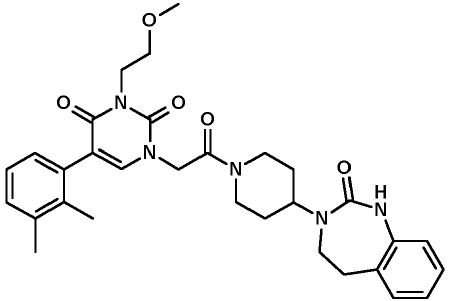

### 93.1 : [5-bromo-3-(2-méthoxy-éthyl)-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,8g (5,7 mmoles) de 2-bromoéthyl méthyl éther, 0,8g (62%) de [5-bromo-3-(2-méthoxy-éthyl)-2,4-dioxo-3,4-dihydro-2H-pyrimidin-1-yl]-acétate de méthyle sont obtenus sous la forme d'un solide blanc.

### 93.2 : acide [5-(2,3-diméthyl-phényl)-3-(2-méthoxy-éthyl)-2,4-dioxo-3,4-dihydro-2H-pyrimidin-1-yl]-acétique

De manière analogue à l'exemple 13.3, à partir de 400mg (1,3 mmoles) de [5-bromo-3-(2-méthoxy-éthyl)-2,4-dioxo-3,4-dihydro-2H-pyrimidin-1-yl]-acétate de méthyle et 280mg (1,9 mmoles) d'acide 2,3-diméthylphénylboronique, 410mg (99%) d'acide [5-(2,3-diméthyl-phényl)-3-(2-méthoxy-éthyl)-2,4-dioxo-3,4-dihydro-2H-pyrimidin-1-yl]-acétique sont obtenus sous la forme d'une huile orange.

### 93.3 : 5-(2,3-diméthyl-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 93)

De manière analogue à l'exemple 13.4, à partir de 410mg (1,2 mmoles) d'acide [5-(2,3-diméthyl-phényl)-3-(2-méthoxy-éthyl)-2,4-dioxo-3,4-dihydro-2H-pyrimidin-1-yl]-acétique et 363mg (1,5 mmoles) de 3-pipéridin-4-yl-1,3,4,5-tétrahydro-benzo[d][1,3]diazépin-2-one (préparé comme décrit dans l'exemple 16.4), et après recristallisation dans l'éthanol, 400mg (57%) de 5-(2,3-diméthyl-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 227 °C.
RMN ¹H (δ, DMSO) : 1.50-1.80 (m, 4H); 2.05 (s, 3H); 2.28 (s, 3H); 2.68 (t, J = 12.6 Hz, 1H); 2.86-2.91 (m, 2H); 3.15 (t, J = 12.5 Hz, 1H); 3.26 (s, 3H); 3.36-3.39 (m, 2H); 3.52 (t, J = 6.1 Hz, 2H); 3.94 (d, J = 13.6 Hz, 1H); 4.07 (t, J = 6.1 Hz, 2H); 4.33 (m, 1H); 4.43 (d, J = 12.9 Hz, 1H); 4.76 (d, J = 4.9 Hz, 2H); 6.80-6.81 (m, 1H); 6.97 (d, J = 7.5 Hz, 1H); 7.04-7.06 (m, 3H); 7.11 (t, J = 7.5 Hz, 1H); 7.18 (d, J = 7.5 Hz, 1H); 7.60 (s, 1H); 8.53 (s, 1H).

### Exemple 94 : 5-(3,5-dichloro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 94) (example comparatif)

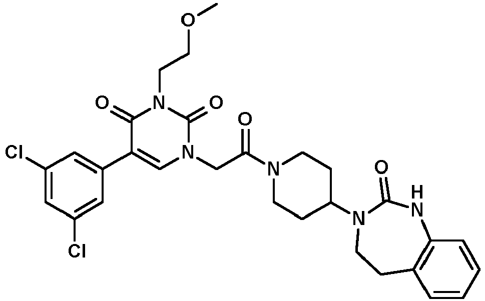

### 94.1 : acide [5-(3,5-dichloro-phényl)-3-(2-méthoxy-éthyl)-2,4-dioxo-3,4-dihydro-2H-pyrimidin-1-yl]-acétique

De manière analogue à l'exemple 13.3, à partir de 430mg (1,3 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 383mg (2 mmoles) d'acide 3,5-dichlorophénylboronique, 580mg (100%) d'acide [5-(3,5-dichlorophényl)-3-(2-méthoxy-éthyl)-2,4-dioxo-3,4-dihydro-2H-pyrimidin-1-yl]-acétique sont obtenus sous la forme d'un solide beige.

### 94.2 : 5-(3,5-dichloro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 94)

De manière analogue à l'exemple 13.4, à partir de 500mg (1,3 mmoles) d'acide [5-(3,5-dichloro-phényl)-3-(2-méthoxy-éthyl)-2,4-dioxo-3,4-dihydro-2H-pyrimidin-1-yl]-acétique et 394mg (1,6 mmoles) de 3-pipéridin-4-yl-1,3,4,5-tétrahydro-benzo[d][1,3]diazépin-2-one (préparé comme décrit dans l'exemple 16.4), et après recristallisation dans l'éthanol, 350mg (43%) de 5-(3,5-dichloro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 148°C.
RMN ¹H (δ, DMSO) : 1.56-1.65 (m, 4H); 2.67-2.71 (m, 1H); 2.87-2.92 (m, 2H); 3.13-3.20 (m, 1H); 3.25 (s, 3H); 3.36-3.39 (m, 2H); 3.52 (t, J = 6.0 Hz, 2H); 3.95 (d, J= 12.9Hz, 1H); 4.08 (t, J = 6.1 Hz, 2H); 4.28-4.40 (m, 1H); 4.42 (d, J = 12.9 Hz, 1H); 4.81 (s, 2H); 6.79-6.80 (m, 1H); 7.03-7.05 (m, 3H); 7.57 (s, 1H); 7.69 (d, J = 1.9 Hz, 2H); 8.17 (s, 1H); 8.55 (s, 1H).

### Exemple 95 : 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 95) (example comparatif)

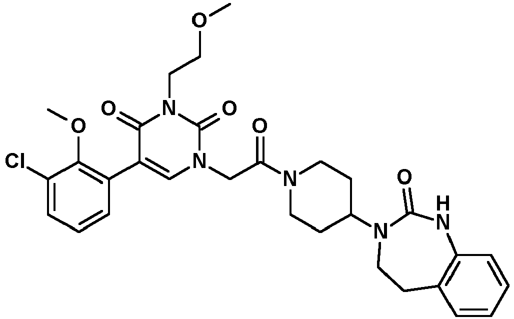

### 95.1 : acide [5-(3-chloro-2-méthoxy-phényl)-3-(2-méthoxyéthyl)-2,4-dioxo-3,4-dihydro-2H-pyrimidin-1-yl]-acétique

De manière analogue à l'exemple 13.3, à partir de 300mg (0,9 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 261mg (1,4 mmoles) d'acide 3-chloro-2-méthoxyphénylboronique, 340mg (98%) d'acide [5-(3-chloro-2-méthoxy-phényl)-3-(2-méthoxy-éthyl)-2,4-dioxo-3,4-dihydro-2H-pyrimidin-1-yl]-acétique sont obtenus sous la forme d'une huile marron.

### 95.2 : 5-(3-chloro-2-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 95)

De manière analogue à l'exemple 13.4, à partir de 340mg (0,9 mmoles) d' acide [5-(3-chloro-2-méthoxy-phényl)-3-(2-méthoxyéthyl)-2,4-dioxo-3,4-dihydro-2H-pyrimidin-1-yl]-acétique et 271mg (1,1 mmoles) de 3-pipéridin-4-yl-1,3,4,5-tétrahydro-benzo[d] [1,3]diazépin-2-one (préparé comme décrit dans l'exemple 16.4), et après recristallisation dans l'éthanol, 360mg (63%) de 5-(3-chloro-2-méthoxy-phényl)-3-(2-méthoxyéthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 206°C.
RMN ¹H (δ, DMSO) : 1.61-1.69 (m, 4H); 2.68 (t, J = 12.6 Hz, 1H); 2.90 (m, 2H); 3.15 (t, J = 12.6 Hz, 1H); 3.26 (s, 3H); 3.36-3.39 (m, 2H); 3.53 (t, J = 6.1 Hz, 2H); 3.67 (s, 3H); 3.95 (d, J = 13.6 Hz, 1H); 4.08 (t, J = 6.1 Hz, 2H); 4.29-4.36 (m, 1H); 4.43 (d, J = 12.9 Hz, 1H); 4.79 (s, 2H); 6.80-6.81 (m, 1H); 7.03-7.05 (m, 3H); 7.19 (t, J = 7.8 Hz, 1H); 7.27 (dd, J = 7.7, 1.7 Hz, 1H); 7.50 (dd, J = 7.9, 1.7 Hz, 1H); 7.78 (s, 1H); 8.53 (s, 1H).

### Exemple 96 : 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 96) (example comparatif)

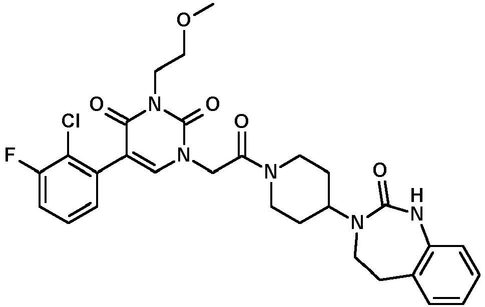

### 96.1 : acide [5-(2-chloro-3-fluoro-phényl)-3-(2-méthoxy-éthyl)-2,4-dioxo-3,4-dihydro-2H-pyrimidin-1-yl]-acétique

De manière analogue à l'exemple 13.3, à partir de 300mg (0,9 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 244mg (1,4 mmoles) d'acide 2-chloro-3-fluorophénylboronique, 300mg (90%) d'acide [5-(2-chloro-3-fluoro-phényl)-3-(2-méthoxy-éthyl)-2,4-dioxo-3,4-dihydro-2H-pyrimidin-1-yl]-acétique sont obtenus sous la forme d'une huile marron.

### 96.2 : 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 96)

De manière analogue à l'exemple 13.4, à partir de 300mg (0,8 mmoles) d'acide [5-(2-chloro-3-fluoro-phényl)-3-(2-méthoxyéthyl)-2,4-dioxo-3,4-dihydro-2H-pyrimidin-1-yl]-acétique et 248mg (1 mmole) de 3-pipéridin-4-yl-1,3,4,5-tétrahydro-benzo[d][1,3]diazépin-2-one (préparé comme décrit dans l'exemple 16.4), et après recristallisation dans un mélange n-butanol / éthanol, 140mg (27%) de 5-(2-chloro-3-fluoro-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 232°C.

RMN ¹H (δ, DMSO) : 1.59-1.71 (m, 4H); 2.69 (t, J = 12.6 Hz, 1H); 2.90 (m, 2H); 3.15 (t, J = 12.3 Hz, 1H); 3.26 (s, 3H); 3.36-3.39 (m, 2H); 3.53 (t, J = 6.0 Hz, 2H); 3.95 (d, J = 13.6 Hz, 1H); 4.07 (t, J = 6.1 Hz, 2H); 4.26-4.37 (m, 1H); 4.43 (d, J = 13.0 Hz, 1H); 4.78 (d, J = 2.7 Hz, 2H); 6.80-6.81 (m, 1H); 7.03-7.05 (m, 3H); 7.21-7.22 (m, 1H); 7.45-7.46 (m, 2H); 7.84 (s, 1H); 8.54 (s, 1H).

### Exemple 97 : 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 97) (example comparatif)

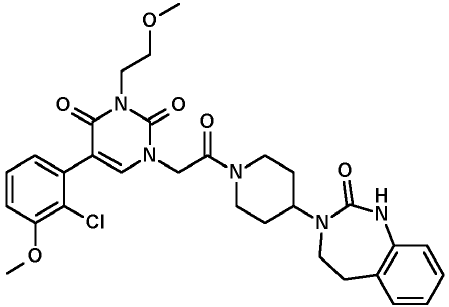

### 97.1 : acide [5-(2-chloro-3-méthoxy-phényl)-3-(2-méthoxyéthyl)-2,4-dioxo-3,4-dihydro-2H-pyrimidin-1-yl]-acétique

De manière analogue à l'exemple 13.3, à partir de 200mg (0,6 mmoles) de [5-bromo-3-(2-méthoxy-éthyl)-2,4-dioxo-3,4-dihydro-2H-pyrimidin-1-yl]-acétate de méthyle (préparé comme décrit dans l'exemple 93.1) et 232mg (1,3 mmoles) d'acide 2-chloro-3-méthoxyphénylboronique, 158mg (69%) d' acide [5-(2-chloro-3-méthoxy-phényl)-3-(2-méthoxy-éthyl)-2,4-dioxo-3,4-dihydro-2H-pyrimidin-1-yl]-acétique sont obtenus sous la forme d'une huile jaune.

### 97.2 : 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 97)

De manière analogue à l'exemple 13.4, à partir de 150mg (0,4 mmoles) d' acide [5-(2-chloro-3-méthoxy-phényl)-3-(2-méthoxyéthyl)-2,4-dioxo-3,4-dihydro-2H-pyrimidin-1-yl]-acétique et 120mg (0,5 mmoles) de 3-pipéridin-4-yl-1,3,4,5-tétrahydro-benzo[d][1,3]diazépin-2-one (préparé comme décrit dans l'exemple 16.4), 180mg (72%) de 5-(2-chloro-3-méthoxy-phényl)-3-(2-méthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc.
RMN ¹H (δ, DMSO) : 1.55 (m, 1 H); 1.71 (m, 3 H); 2.68-2.70 (m, 1 H); 2.90 (m, 2 H); 3.13-3.17 (m, 1 H); 3.26 (s, 3 H); 3.38 (m, 2 H); 3.51 (t, J = 6.1 Hz, 2 H); 3.89 (s, 3 H); 3.94 (d, J = 14.0 Hz, 1 H); 4.06 (t, J = 6.1 Hz, 2 H); 4.31-4.33 (m, 1 H); 4.43 (d, J = 12.8 Hz, 1 H); 4.76-4.78 (m, 2 H); 6.80-6.81 (m, 1 H); 6.92 (dd, J = 7.6, 1.4 Hz, 1 H); 7.04-7.06 (m, 3 H); 7.19 (dd, J = 8.4, 1.4 Hz, 1 H); 7.35 (t, J = 8.0 Hz, 1 H); 7.73 (s, 1 H); 8.54 (s, 1 H).

### Exemple 98 : 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 98) (example comparatif)

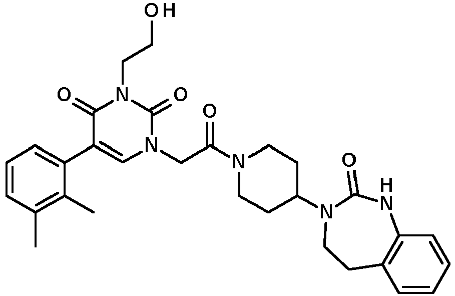

### 98.1 : [3-(2-benzyloxy-éthyl)-5-bromo-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,7ml (4,2 mmoles) de benzyl-2-bromoéthyl éther, 0,9g (60%) de [3-(2-benzyloxy-éthyl)-5-bromo-2,4-dioxo-3,4-dihydro-2H-pyrimidin-1-yl]-acétate de méthyle sont obtenus sous la forme d'un solide beige.

### 98.2 : acide [3-(2-benzyloxy-éthyl)-5-(2,3-diméthyl-phényl)-2,4-dioxo-3,4-dihydro-2H-pyrimidin-1-yl]-acétique

De manière analogue à l'exemple 13.3, à partir de 400mg (1 mmole) de [3-(2-benzyloxy-éthyl)-5-bromo-2,4-dioxo-3,4-dihydro-2H-pyrimidin-1-yl]-acétate de méthyle et 242mg (1,6 mmoles) d'acide 2,3-diméthylphénylboronique, 410mg (100%) d' acide [3-(2-benzyloxy-éthyl)-5-(2,3-diméthyl-phényl)-2,4-dioxo-3,4-dihydro-2H-pyrimidin-1-yl]-acétique sont obtenus sous la forme d'une huile orange.

### 98.3 : 3-(2-benzyloxy-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione

De manière analogue à l'exemple 13.4, à partir de 410mg (1 mmole) d' acide [3-(2-benzyloxy-éthyl)-5-(2,3-diméthyl-phényl)-2,4-dioxo-3,4-dihydro-2H-pyrimidin-1-yl]-acétique et 296mg (1,2 mmoles) de 3-pipéridin-4-yl-1,3,4,5-tétrahydro-benzo[d][1,3]diazépin-2-one (préparé comme décrit dans l'exemple 16.4), 320mg (50%) de 3-(2-benzyloxy-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide rose pâle.

### 98.4 : 5-(2,3-diméthyl-phényl)-3-(2-hydroxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 98)

De manière analogue à l'exemple 1.5, à partir de 320mg (0,5 mmoles) de 3-(2-benzyloxy-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione et 256mg (0,1 mmoles) de palladium sur charbon à 10%, le milieu réactionnel étant placé sous 3bar de dihydrogène pendant 24 heures, 180mg (66%) de 5-(2,3-diméthyl-phényl)-3-(2-hydroxyéthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 237°C.
RMN ¹H (δ, DMSO) : 1.58-1.70 (m, 4H); 2.05 (s, 3H); 2.28 (s, 3H); 2.68 (t, J = 12.5 Hz, 1H); 2.89 (m, 2H); 3.15 (t, J = 12.3 Hz, 1H); 3.36-3.39 (m, 2H); 3.53 (q, J = 6.4 Hz, 2H); 3.94-3.97 (m, 3H); 4.33-4.34 (m, 1H); 4.43 (d, J = 12.9 Hz, 1H); 4.70-4.85 (m, 3H); 6.80-6.81 (m, 1H); 6.97 (d, J = 7.5 Hz, 1H); 7.04-7.06 (m, 3H); 7.11 (t, J = 7.5 Hz, 1H); 7.18 (d, J = 7.5 Hz, 1H); 7.59 (s, 1H); 8.53 (s, 1H).

### Exemple 99 : 3-(2-diméthylamino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 99) (example comparatif)

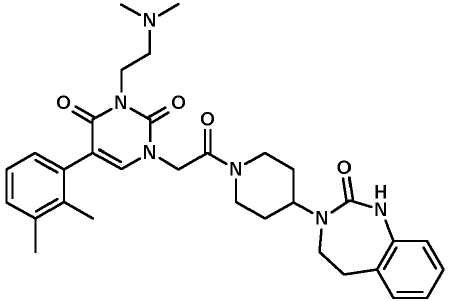

### 99.1 : [5-bromo-3-(2-diméthylamino-éthyl)-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), 0,7g (4,6 mmoles) de chlorhydrate de 2-diméthylaminoéthyl chlorure et 0,6ml (4,6 mmoles) de triéthylamine, 0,7g (55%) de [5-bromo-3-(2-diméthylamino-éthyl)-2,4-dioxo-3,4-dihydro-2H-pyrimidin-1-yl]-acétate de méthyle sont obtenus sous la forme d'un solide blanc.

### 99.2 : acide 3-(2-diméthylamino-éthyl)-5-(2,3-diméthyl-phényl)-2,4-dioxo-3,4-dihydro-2H-pyrimidin-1-yl]-acétique

De manière analogue à l'exemple 13.3, à partir de 400mg (1,2 mmoles) de [5-bromo-3-(2-diméthylamino-éthyl)-2,4-dioxo-3,4-dihydro-2H-pyrimidin-1-yl]-acétate de méthyle et de 269mg (1,8 mmoles) d'acide 2,3-diméthylphénylboronique, 60mg (15%) d'acide 3-(2-diméthylamino-éthyl)-5-(2,3-diméthyl-phényl)-2,4-dioxo-3,4-dihydro-2H-pyrimidin-1-yl]-acétique sont obtenus sous la forme d'un solide blanc.

### 99.3 : 3-(2-diméthylamino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 99)

De manière analogue à l'exemple 13.4, à partir de 60mg (0,2 mmoles) d'acide 3-(2-diméthylamino-éthyl)-5-(2,3-diméthylphényl)-2,4-dioxo-3,4-dihydro-2H-pyrimidin-1-yl]-acétique et de 51mg (0,2 mmoles) de 3-pipéridin-4-yl-1,3,4,5-tétrahydro-benzo[d][1,3]diazépin-2-one (préparé comme décrit dans l'exemple 16.4), 50mg (50%) de 3-(2-diméthylamino-éthyl)-5-(2,3-diméthyl-phényl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 170°C.
RMN ¹H (δ, DMSO) : 1.55-1.68 (m, 4H); 2.05 (s, 3H); 2.23 (s, 6H); 2.28 (s, 3H); 2.49-2.51 (m, 2H); 2.68 (m, 1H); 2.89 (m, 2H); 3.15 (t, J = 9.7 Hz, 1H); 3.38 (m, 2H); 3.96-4.00 (m, 3H); 4.33 (m, 1H); 4.43 (d, J = 12.9 Hz, 1H); 4.76 (d, J = 4.2 Hz, 2H); 6.81 (m, 1H); 6.97 (d, J = 7.5 Hz, 1H); 7.04-7.06 (m, 3H); 7.11 (t, J = 7.5 Hz, 1H); 7.18 (d, J = 7.5 Hz, 1H); 7.60 (s, 1H); 8.53 (s, 1H).

### Exemple 100 : 5-(2,3-diméthyl-phényl)-3-(2-méthoxyméthoxyéthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 100) (example comparatif)

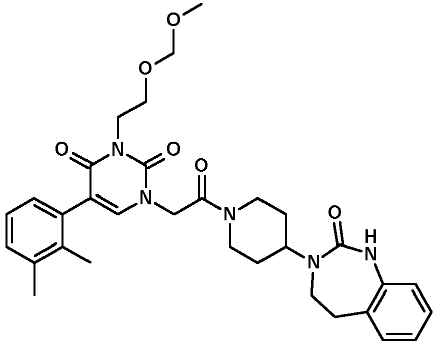

### 100.1 : [5-bromo-3-(2-méthoxyméthoxy-éthyl)-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), 0 et 0,5ml (4,6 mmoles) de 1-bromo-2-méthoxyméthoxy-éthane, 0,6g (43%) de [5-bromo-3-(2-méthoxyméthoxy-éthyl)-2,4-dioxo-3,4-dihydro-2H-pyrimidin-1-yl]-acétate de méthyle sont obtenus sous la forme d'un solide blanc.

### 100.2 : acide [5-(2,3-diméthyl-phényl)-3-(2-méthoxyméthoxyéthyl)-2,4-dioxo-3,4-dihydro-2H-pyrimidin-1-yl]-acétique

De manière analogue à l'exemple 13.3, à partir de 300mg (0,9 mmoles) de [5-bromo-3-(2-méthoxyméthoxy-éthyl)-2,4-dioxo-3,4-dihydro-2H-pyrimidin-1-yl]-acétate de méthyle et 205mg (1,4 mmoles) d'acide 2,3-diméthylphénylboronique, 250mg (81%) d'acide [5-(2,3-diméthyl-phényl)-3-(2-méthoxyméthoxy-éthyl)-2,4-dioxo-3,4-dihydro-2H-pyrimidin-1-yl]-acétique sont obtenus sous la forme d'une huile brune.

### 100.3 : 5-(2,3-diméthyl-phényl)-3-(2-méthoxyméthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 100)

De manière analogue à l'exemple 13.4, à partir de 250mg (0,7 mmoles) d'acide [5-(2,3-diméthyl-phényl)-3-(2-méthoxyméthoxyéthyl)-2,4-dioxo-3,4-dihydro-2H-pyrimidin-1-yl]-acétique et 203mg (0,8 mmoles) de 3-pipéridin-4-yl-1,3,4,5-tétrahydro-benzo[d] [1,3]diazépin-2-one (préparé comme décrit dans l'exemple 16.4), et après recrsitallisation dans un mélange heptane / acétone 50/50; 200mg (49%) de 5-(2,3-diméthyl-phényl)-3-(2-méthoxyméthoxy-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 160°C.
RMN ¹H (δ, DMSO) : 1.50-1.80 (m, 4H); 2.05 (s, 3H); 2.28 (s, 3H); 2.68 (m, 1H); 2.89 (m, 2H); 3.16 (m, 1H); 3.23 (s, 3H); 3.38 (m, 2H); 3.65 (t, J = 6.1 Hz, 2H); 4.95 (d, J=12.9 Hz, 1H); 4.10 (t, J = 6.2 Hz, 2H); 4.33 (m, 1H); 4.43 (d, J = 12.9 Hz, 1H); 4.54 (s, 2H); 4.76 (d, J = 3.8 Hz, 2H); 6.80-6.81 (m, 1H); 6.96 (d, J = 7.5 Hz, 1H); 7.03-7.05 (m, 3H); 7.11 (t, J = 7.5 Hz, 1H); 7.18 (d, J = 7.5 Hz, 1H); 7.61 (s, 1H); 8.53 (s, 1H).

### Exemple 101 : N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide (schéma réactionnel n°3, composé 101) (example comparatif)

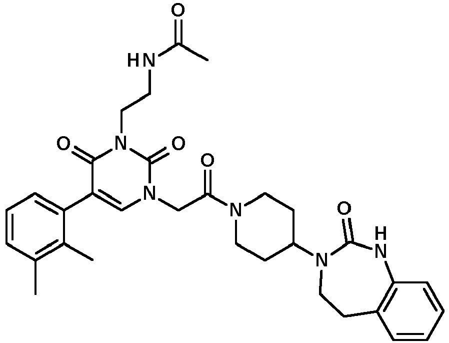

### 101.1 : [3-(2-acétylamino-éthyl)-5-bromo-2,4-dioxo-3,4-dihydro-2H-pyrimidin-1-yl]-acétate de méthyle

De manière analogue à l'exemple 83.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,4ml (3,8 mmoles) de N-(2-chloro-éthyl)-acétamide, 170mg (13%) de [3-(2-acétylamino-éthyl)-5-bromo-2,4-dioxo-3,4-dihydro-2H-pyrimidin-1-yl]-acétate de méthyle sont obtenus sous la forme d'un solide jaune pâle.

### 101.2 : acide [3-(2-acétylamino-éthyl)-5-(2,3-diméthyl-phényl)-2,4-dioxo-3,4-dihydro-2H-pyrimidin-1-yl]-acétique

De manière analogue à l'exemple 44.1, à partir de 170mg (0,5 mmoles) de [3-(2-acétylamino-éthyl)-5-bromo-2,4-dioxo-3,4-dihydro-2H-pyrimidin-1-yl]-acétate de méthyle et 117mg (0,8 mmoles) d'acide 2,3-diméthylphénylboronique, 35mg (20%) d'acide [3-(2-acétylamino-éthyl)-5-(2,3-diméthyl-phényl)-2,4-dioxo-3,4-dihydro-2H-pyrimidin-1-yl]-acétique sont obtenus sous la forme d'un solide rose.

### 101.3 : N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-acétamide (composé 101)

De manière analogue à l'exemple 13.4, à partir de 35mg (0,1 mmoles) d' acide [3-(2-acétylamino-éthyl)-5-(2,3-diméthylphényl)-2,4-dioxo-3,4-dihydro-2H-pyrimidin-1-yl]-acétique et 29mg (0,1 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 8mg (14%) de N-[2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyll-3,6-dihydro-2H-pyrimidin-1-yl)-éthyl]-acétamide sont obtenus sous la forme d'un solide blanc de point de fusion 267°C.
RMN ¹H (δ, DMSO) : 1.56-1.68 (m, 4 H), 1.76 (s, 3 H), 2.07 (s, 3 H), 2.28 (s, 3 H), 2.68-2.72 (m, 1 H), 2.90 (m, 2 H), 3.14 (t, 1 H), 3.25-3.28 (m, 2 H), 3.36-3.38 (m, 2 H), 3.95 (m, 3 H), 4.32 (m, 1 H), 4.44 (m, 1 H), 4.76 (s, 2 H), 6.81 (m, 1 H), 6.96 (d, J=7.5 Hz, 1 H), 7.05 (m, 3 H), 7.11 (t, J=7.5 Hz, 1 H), 7.18 (d, J=7.5 Hz, 1 H), 7.58 (s, 1 H), 7.92 (t, J=5.9 Hz, 1 H), 8.52 (s, 1 H).

### Exemple 102 : 5-(2,3-diméthyl-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 102) (example comparatif)

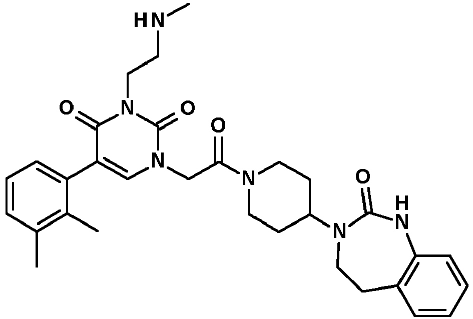

### 102.1 : acide [3-(2-tert-butoxycarbonylamino-éthyl)-5-(2,3-diméthyl-phényl)-2,4-dioxo-3,4-dihydro-2H-pyrimidin-1-yl]-acétique

De manière analogue à l'exemple 44.1, à partir de 500mg (1,2 mmoles) de [5-bromo-3-(2-tert-butoxycarbonylamino-éthyl)-2,4-dioxo-3,4-dihydro-2H-pyrimidin-1-yl]-acétate de méthyle (préparé comme décrit dans l'exemple 87.1), et 295mg (2 mmoles) d'acide 2,3-diméthylphénylboronique, 460mg (90%) d'acide [3-(2-tert-butoxycarbonylamino-éthyl)-5-(2,3-diméthyl-phényl)-2,4-dioxo-3,4-dihydro-2H-pyrimidin-1-yl]-acétique sont obtenus sous la forme d'un solide beige.

### 102.2 : acide [3-[2-(tert-butoxycarbonyl-méthyl-amino)-éthyl]-5-(2,3-diméthyl-phényl)-2,4-dioxo-3,4-dihydro-2H-pyrimidin-1-yl]-acétique

De manière analogue à l'exemple 90.2, à partir de 100mg (0,2 mmoles) de d'acide [3-(2-tert-butoxycarbonylamino-éthyl)-5-(2,3-diméthyl-phényl)-2,4-dioxo-3,4-dihydro-2H-pyrimidin-1-yl]-acétique et 93µl (1,5 mmoles) de iodométhane, et après 4 heures d'agitation dans 5ml de tétrahydrofurane, 85mg (82%) d' acide [3-[2-(tert-butoxycarbonyl-méthyl-amino)-éthyl]-5-(2,3-diméthyl-phényl)-2,4-dioxo-3,4-dihydro-2H-pyrimidin-1-yl]-acétique sont obtenus sous la forme d'une huile incolore.

### 102.3 : [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tertbutyle

De manière analogue à l'exemple 13.4, à partir de 85mg (0,2 mmoles) d' acide [3-[2-(tert-butoxycarbonyl-méthyl-amino)-éthyl]-5-(2,3-diméthyl-phényl)-2,4-dioxo-3,4-dihydro-2H-pyrimidin-1-yl]-acétique et 58mg (0,2 mmoles) de 3-pipéridin-4-yl-1,3,4,5-tétrahydro-benzo[d][1,3]diazépin-2-one (préparé comme décrit dans l'exemple 16.4), 110mg (85%) de [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tertbutyle sont obtenus sous la forme d'un solide blanc.

### 102.4 : 5-(2,3-diméthyl-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 102)

De manière analogue à l'exemple 88, à partir de 110mg (0,2 mmoles) de [2-(5-(2,3-diméthyl-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-éthyl]-méthyl-carbamate de tertbutyle, et après recristallisation dans un mélange acétone / heptane 50/50, 50mg (48%) de 5-(2,3-diméthyl-phényl)-3-(2-méthylamino-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide beige de point de fusion 203°C.
RMN ¹H (δ, DMSO) : 1.53-1.67 (m, 4H); 2.04 (s, 3H); 2.26-2.27 (m, 6H); 2.63-2.66 (m, 3H); 2.88 (m, 2H); 3.14 (t, J = 12.4 Hz, 1H); 3.36 (m, 2H); 3.92-3.96 (m, 3H); 4.32 (m, 1H); 4.42 (d, J = 12.9 Hz, 1H); 4.74 (d, J = 6.4 Hz, 2H); 6.80 (m, 1H); 6.96 (d, J = 7.7 Hz, 1H); 7.03-7.05 (m, 3H); 7.10 (t, J = 7.5 Hz, 1H); 7.17 (d, J = 7.6 Hz, 1H); 7.58 (s, 1H); 8.54 (s, 1H) .

### Exemple 103 : (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle (schéma réactionnel n°3, composé 103) (example comparatif)

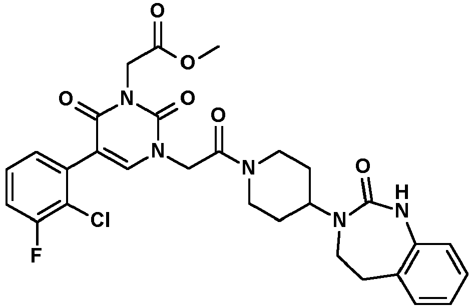

### 103.1 : (3-benzhydryl-5-bromo-2,6-dioxo-3,6-dihydro-2H-pyrimidin-1-yl)-acétate de méthyle

De manière analogue à l'exemple 2.2, à partir de 1g (2,8 mmoles) de 1-benzhydryl-5-bromo-1H-pyrimidine-2,4-dione (préparé comme décrit dans l'exemple 2.1) et 346µl (3,6 mmoles) de bromoacétate de méthyle, 960mg (80%) de (3-benzhydryl-5-bromo-2,6-dioxo-3,6-dihydro-2H-pyrimidin-1-yl)-acétate de méthyle sont obtenus sous la forme d'une pâte blanche.

### 103.2 : [3-benzhydryl-5-(2-chloro-3-fluoro-phényl)-2,6-dioxo-3,6-dihydro-2H-pyrimidin-1-yl]-acétate de méthyle

De manière analogue à l'exemple 44.1, à partir de 500mg (1,2 mmoles) de (3-benzhydryl-5-bromo-2,6-dioxo-3,6-dihydro-2H-pyrimidin-1-yl)-acétate de méthyle et 305mg (1,8 mmoles) d'acide 2-chloro-3-fluorophénylboronique, et après purification par chromatographie sur gel de silice élué par un mélange heptane / acétate d'éthyle 80/20, 208mg (37%) de [3-benzhydryl-5-(2-chloro-3-fluoro-phényl)-2,6-dioxo-3,6-dihydro-2H-pyrimidin-1-yl]-acétate de méthyle sont obtenus sous la forme d'un solide beige.

### 103.3 : [5-(2-chloro-3-fluoro-phényl)-2,6-dioxo-3,6-dihydro-2H-pyrimidin-1-yl]-acétate de méthyle

De manière analogue à l'exemple 2.3, à partir de 200mg (0,4 mmoles) de [3-benzhydryl-5-(2-chloro-3-fluoro-phényl)-2,6-dioxo-3,6-dihydro-2H-pyrimidin-1-yl]-acétate de méthyle et après 10 jours d'agitation à température ambiante, 75mg (57%) de [5-(2-chloro-3-fluoro-phényl)-2,6-dioxo-3,6-dihydro-2H-pyrimidin-1-yl]-acétate de méthyle sont obtenus sous la forme d'une huile brune.

### 103.4 : (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle (composé 103)

De manière analogue à l'exemple 39.4, à partir de 74mg (0,2 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1) et 65mg (0,2 mmoles) de [5-(2-chloro-3-fluoro-phényl)-2,6-dioxo-3,6-dihydro-2H-pyrimidin-1-yl]-acétate de méthyle, 72mg (57%) de (5-(2-chloro-3-fluorophényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle sont obtenus sous la forme d'un solide blanc
RMN ¹H (δ, DMSO) : 1.51-1.59 (m, 1 H); 1.65-1.75 (m, J = 22.0 Hz, 3 H); 2.69 (t, J = 12.6 Hz, 1 H); 2.88-2.90 (m, 2 H) ; 3.15 (t, J = 12.0 Hz, 1 H) ; 3.36-3.38 (m, 2 H); 3.69 (s, 3 H); 3.95 (d, J = 13.5 Hz, 1 H); 4.30-4.33 (m, 1 H); 4.43 (d, J = 12.9 Hz, 1 H); 4.66 (s, 2 H); 4.82 (s, 2 H); 6.81 (dt, J = 7.7, 4.2 Hz, 1 H); 7.06-7.04 (m, 3 H); 7.24 (dd, J = 6.5, 2.7 Hz, 1 H); 7.48-7.46 (m, 2 H); 7.93 (s, 1 H); 8.53 (s, 1 H) .

### Exemple 104 : acide (5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétique (schéma réactionnel n°3, composé 104) (example comparatif)

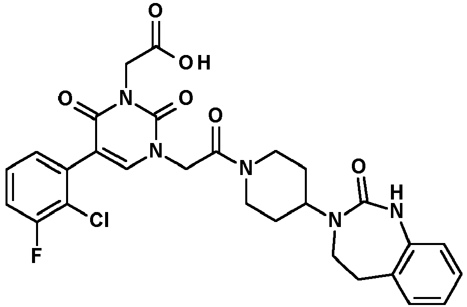

De manière analogue à l'exemple 16.8, à partir de 50mg (80 µmoles) de (5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-acétate de méthyle, et après purification par chromatographie sur gel de silice élué par un mélange dichlorométhane / méthanol 98/2, 10mg (20%) d'acide (5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-acétique sont obtenus sous la forme d'un solide blanc.
RMN ¹H (δ, DMSO) : 1.51-1.59 (m, 1 H); 1.65-1.75 (m, J = 22.0 Hz, 3 H); 2.69 (t, J = 12.6 Hz, 1 H); 2.88-2.90 (m, 2 H) ; 3.15 (t, J = 12.0 Hz, 1 H); 3.36-3.38 (m, 2 H); 3.95 (d, J = 13.5 Hz, 1 H); 4.30-4.33 (m, 1 H); 4.43 (d, J = 12.9 Hz, 1 H); 4.66 (s, 2 H); 4.82 (s, 2 H); 6.81 (dt, J = 7.7, 4.2 Hz, 1 H); 7.06-7.04 (m, 3 H); 7.24 (dd, J = 6.5, 2.7 Hz, 1 H) ; 7.48-7.46 (m, 2 H); 7.93 (s, 1 H); 8.53 (s, 1 H).

### Exemple 105: 5-(2-chloro-3-fluoro-phényl)-3-oxétan-3-ylméthyl-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (schéma réactionnel n°3, composé 105) (example comparatif)

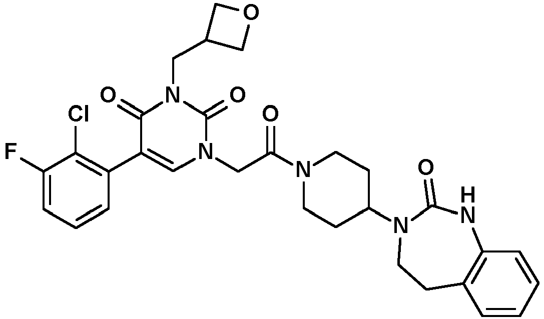

### 105.1: (5-bromo-3-oxétan-3-ylméthyl-2,4-dioxo-3,4-dihydro-2H-pyrimidin-1-yl)-acétate de méthyle

De manière analogue à l'exemple 13.2, à partir de 500mg (1,9 mmoles) de (5-bromo-2,4-dioxo-3,4-dihydro-2H-pyrimidin-1-yl)-acétate de méthyle (préparé comme décrit dans l'exemple 13.1), et 0,15 ml (2,9 mmoles) de 3-bromométhyl-oxétane, 310mg (49%) de (5-bromo-3-oxétan-3-ylméthyl-2,4-dioxo-3,4-dihydro-2H-pyrimidin-1-yl)-acétate de méthyle sont obtenus sous la forme d'un solide blanc.

### 105.2: acide [5-(2-chloro-3-fluoro-phényl)-3-oxétan-3-ylméthyl-2,4-dioxo-3,4-dihydro-2H-pyrimidin-1-yl]-acétique

De manière analogue à l'exemple 44.1, à partir de 310mg (0,9 mmoles) de (5-bromo-3-oxétan-3-ylméthyl-2,4-dioxo-3,4-dihydro-2H-pyrimidin-1-yl)-acétate de méthyle et 243mg (1,4 mmoles) d'acide 2-chloro-3-fluorophénylboronique, 80mg (23%) d'acide [5-(2-chloro-3-fluoro-phényl)-3-oxétan-3-ylméthyl-2,4-dioxo-3,4-dihydro-2H-pyrimidin-1-yl]-acétique sont obtenus sous la forme d'une huile marron.

### 105.3: 5-(2-chloro-3-fluoro-phényl)-3-oxétan-3-ylméthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 105)

De manière analogue à l'exemple 13.4, à partir de 80mg (0,2 mmoles) d'acide [5-(2-chloro-3-fluoro-phényl)-3-oxétan-3-ylméthyl-2,4-dioxo-3,4-dihydro-2H-pyrimidin-1-yl]-acétique et 59mg (0,2 mmoles) de 3-pipéridin-4-yl-1,3,4,5-tétrahydro-benzo[d][1,3]diazépin-2-one (préparé comme décrit dans l'exemple 16.4), 30mg (22%) de 5-(2-chloro-3-fluoro-phényl)-3-oxétan-3-ylméthyl-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 244°C.

RMN ¹H (δ, DMSO) : 1.53-1.56 (m, 1 H); 1.66-1.72 (m, 3 H); 2.69 (t, J = 12.3 Hz, 1 H); 2.90 (m, 2 H); 3.15 (t, J = 12.2 Hz, 1 H); 3.25-3.30 (m, 1 H); 3.37-3.39 (m, 2 H); 3.92-3.95 (m, 1H); 4.19 (d, J = 6.7 Hz, 2 H) ; 4.33 (m, 1H); 4.40-4.43 (m, 3 H); 4.60 (dd, J = 7.9, 6.0 Hz, 2 H); 4.78 (s, 2 H); 6.79-6.83 (m, 1 H); 7.02-7.05 (m, 3 H); 7.21-7.23 (m, 1 H); 7.44-7.48 (m, 2H); 7.85 (s, 1H); 8.53 (s, 1H).

### Exemple 106: 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 106) (example comparatif)

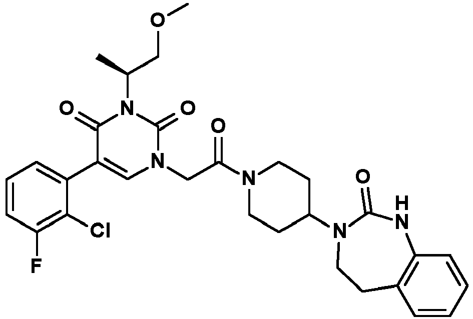

### 106.1: [5-bromo-3-((S)-2-méthoxy-1-méthyl-éthyl)-2,4-dioxo-3,4-dihydro-2H-pyrimidin-1-yl]-acétate de méthyle

De manière analogue à l'exemple 2.5, à partir de 1g (3,8 mmoles) de (5-bromo-2,4-dioxo-3,4-dihydro-2H-pyrimidin-1-yl)-acétate de méthyle (préparé comme décrit dans l'exemple 13.1), 0,8ml (7,8 mmoles) de (R)-1-méthoxy-propan-2-ol et 1,2ml (7,6 mmoles) de diéthyl azodicarboxylate rajoutés à 0°C, et après purification par chromatographie sur gel de silice élué par un mélange heptane / acétate d'éthyle 60/40, 1,2g (78%) de [5-bromo-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 106.2: [5-(2-chloro-3-fluoro-phényl)-3-((S)-2-méthoxy-1-méthyl-éthyl)-2,4-dioxo-3,4-dihydro-2H-pyrimidin-1-yl]-acétate de méthyle

De manière analogue à l'exemple 44.1, à partir de 300mg (0,9 mmoles) de [5-bromo-3-((S)-2-méthoxy-1-méthyl-éthyl)-2,4-dioxo-3,4-dihydro-2H-pyrimidin-1-yl]-acétate de méthyle, 234mg (1,3 mmoles) d'acide 2-chloro-3-fluoro-phénylboronique, et 1,3ml (2,7 mmoles) d'une solution aqueuse de carbonate de potassium 2M, dans 8ml de toluène, 130mg (38%) de [5-(2-chloro-3-fluoro-phényl)-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 106.3: acide [5-(2-chloro-3-fluoro-phényl)-3-((S)-2-méthoxy-1-méthyl-éthyl)-2,4-dioxo-3,4-dihydro-2H-pyrimidin-1-yl]-acétique

De manière analogue à l'exemple 16.8, à partir de 130mg (0,3 mmoles) [5-(2-chloro-3-fluoro-phényl)-3-((S)-2-méthoxy-1-méthyl-éthyl)-2,4-dioxo-3,4-dihydro-2H-pyrimidin-1-yl]-acétate de méthyle, 125mg (100%) d' acide [5-(2-chloro-3-fluoro-phényl)-3-((S)-2-méthoxy-1-méthyl-éthyl)-2,4-dioxo-3,4-dihydro-2H-pyrimidin-1-yl]-acétique sont obtenus sous la forme d'une huile incolore.

### 106.4: 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 106)

De manière analogue à l'exemple 13.4, à partir de 125mg (0,3 mmoles) d' acide [5-(2-chloro-3-fluoro-phényl)-3-((S)-2-méthoxy-1-méthyl-éthyl)-2,4-dioxo-3,4-dihydro-2H-pyrimidin-1-yl]-acétique et 99mg (0,4 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 150mg (73%) de 5-(2-chloro-3-fluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 228°C.
RMN ¹H (δ, DMSO) : 1.34 (d, J = 7.0 Hz, 3H); 1.50-1.80 (m, 4H); 2.68 (t, J = 12.5 Hz, 1H); 2.90 (m, 2H); 3.15 (m, 1H); 3.33 (s, 3H); 3.38 (m, 2H); 3.54 (dd, J = 9.9, 5.9 Hz, 1H); 3.90-3.95 (m, 2H); 4.33 (m, 1H); 4.43 (d, J = 12.9 Hz, 1H); 4.75 (m, 2H); 5.15 (m, 1H); 6.80-6.81 (m, 1H); 7.04-7.06 (m, 3H); 7.20-7.21 (m, 1H); 7.44-7.45 (m, 2H); 7.80 (s, 1H); 8.53 (s, 1H) .

### Exemple 107: (5-bromo-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle (schéma réactionnel n°3, composé 107) (example comparatif)

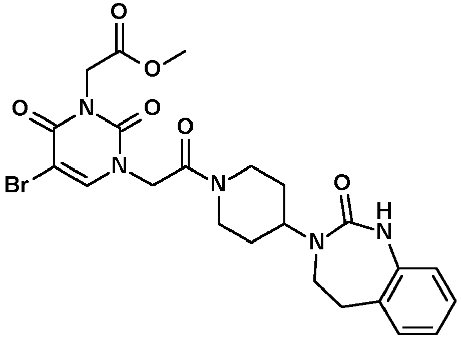

### 107.1: (5-bromo-2,6-dioxo-3,6-dihydro-2H-pyrimidin-1-yl)-acétate de méthyle

De manière analogue à l'exemple 2.3, à partir de 400mg (0,9 mmoles) de (3-benzhydryl-5-bromo-2,6-dioxo-3,6-dihydro-2H-pyrimidin-1-yl)-acétate de méthyle (préparé comme décrit dans l'exemple 103.1), 172mg (70%) de (5-bromo-2,6-dioxo-3,6-dihydro-2H-pyrimidin-1-yl)-acétate de méthyle sont obtenus sous la forme d'un solide blanc.

### 107.2: (5-bromo-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-acétate de méthyle (composé 107)

De manière analogue à l'exemple 83.1, à partir de 160mg (0,6 mmoles) de (5-bromo-2,6-dioxo-3,6-dihydro-2H-pyrimidin-1-yl)-acétate de méthyle et 215mg (0,7 mmoles) de 3-[1-(2-chloro-acétyl)-pipéridin-4-yl]-1,3,4,5-tétrahydro-benzo[d][1,3]diazépin-2-one (préparé comme décrit dans l'exemple 39.1), 300mg (89%) de (5-bromo-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-acétate de méthyle sont obtenus sous la forme d'un solide blanc.
RMN ¹H (δ, DMSO) : 1.54 (m, 1H); 1.71 (m, 3 H); 2.69 (m, 1 H); 2.91 (s, 2 H); 3.15 (m, 1 H); 3.39 (s, 2 H); 3.69 (s, 3 H); 3.90 (m, 1H); 4.33 (m, 1 H); 4.42 (m, 1 H); 4.64 (s, 2 H) ; 4.78 (s, 2 H); 6.82 (s, 1 H); 7.06-7.04 (m, 3 H); 8.29 (s, 1 H) ; 8.53 (s, 1 H).

### Exemple 108: 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 108) (example comparatif)

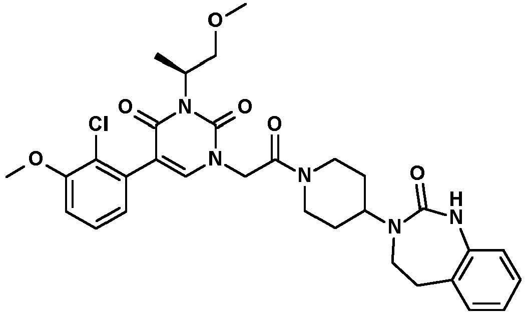

### 108.1: acide [5-(2-chloro-3-méthoxy-phényl)-3-((S)-2-méthoxy-1-méthyl-éthyl)-2,4-dioxo-3,4-dihydro-2H-pyrimidin-1-yl]-acétique

De manière analogue à l'exemple 13.3, à partir de 800mg (2,4 mmoles) de [5-bromo-3-((S)-2-méthoxy-1-méthyl-éthyl)-2,4-dioxo-3,4-dihydro-2H-pyrimidin-1-yl]-acétate de méthyle (préparé comme décrit dans l'exemple 106.1) et 534mg (2,9 mmoles) de 2-chloro-3-méthoxy-phénylboronique, 912mg (100%) d'acide [5-(2-chloro-3-méthoxy-phényl)-3-((S)-2-méthoxy-1-méthyl-éthyl)-2,4-dioxo-3,4-dihydro-2H-pyrimidin-1-yl]-acétique sont obtenus sous la forme d'une huile marron.

### 108.2: 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 108)

De manière analogue à l'exemple 13.4, à partir de 450mg (1,2 mmoles) d'acide [5-(2-chloro-3-méthoxy-phényl)-3-((S)-2-méthoxy-1-méthyl-éthyl)-2,4-dioxo-3,4-dihydro-2H-pyrimidin-1-yl]-acétique et 346mg (1,4 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 250mg (35%) de 5-(2-chloro-3-méthoxy-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 274°C.
RMN ¹H (δ, DMSO) : 1.33 (d, J = 6.9 Hz, 3H); 1.53-1.56 (m, 1H); 1.65-1.70 (m, 3H); 2.68 (t, J = 12.4 Hz, 1H); 2.88-2.90 (m, 2H); 3.14 (t, J = 12.5 Hz, 1H); 3.23 (s, 3H); 3.37-3.39 (m, 2H); 3.54 (dd, J = 9.9, 5.9 Hz, 1H); 3.89 (s, 3H); 3.91-3.95 (m, 2H); 4.26-4.36 (m, 1H); 4.42-4.45 (m, 1H); 4.73-4.77 (m, 2H); 5.09-5.19 (m, 1H); 6.79-6.83 (m, 1H); 6.91 (dd, J= 7.6, 1.4 Hz, 1H) ; 7.02-7.05 (m, 3H) ; 7.18 (dd, J = 8.4, 1.4 Hz, 1H); 7.33-7.37 (m, 1H); 7.69 (s, 1H); 8.53 (s, 1H).

### Exemple 109: 5-(2,3-difluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 109) (example comparatif)

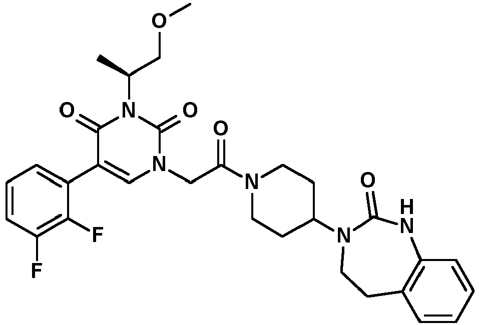

### 109.1: [5-(2,3-difluoro-phényl)-3-((S)-2-méthoxy-1-méthyl-éthyl)-2,4-dioxo-3,4-dihydro-2H-pyrimidin-1-yl]-acétate de méthyle

De manière analogue à l'exemple 44.1, à partir de 1,4g (4,2 mmoles) de [5-bromo-3-((S)-2-méthoxy-1-méthyl-éthyl)-2,4-dioxo-3,4-dihydro-2H-pyrimidin-1-yl]-acétate de méthyle (préparé comme décrit dans l'exemple 106.1) et 2,7g (16,7 mmoles) d'acide 2,3-difluoro-phénylboronique, et après purification par chromatographie sur gel de silice élué par un mélange heptane / acétate d'éthyle 60/40, 0,9g (60%) de [5-(2,3-difluoro-phényl)-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 109.2: acide [5-(2,3-difluoro-phényl)-3-((S)-2-méthoxy-1-méthyl-éthyl)-2,4-dioxo-3,4-dihydro-2H-pyrimidin-1-yl]-acétique

De manière analogue à l'exemple 16.8, à partir de 920mg (2,5 mmoles) de [5-(2,3-difluoro-phényl)-3-((S)-2-méthoxy-1-méthyl-éthyl)-2,4-dioxo-3,4-dihydro-2H-pyrimidin-1-yl]-acétate de méthyle, 840mg (95%) d'acide [5-(2,3-difluoro-phényl)-3-((S)-2-méthoxy-1-méthyl-éthyl)-2,4-dioxo-3,4-dihydro-2H-pyrimidin-1-yl]-acétique sont obtenus sous la forme d'un solide blanc.

### 109.3: 5-(2,3-difluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 109)

De manière analogue à l'exemple 76.4, à partir de 150mg (0,4 mmoles) d'acide [5-(2,3-difluoro-phényl)-3-((S)-2-méthoxy-1-méthyl-éthyl)-2,4-dioxo-3,4-dihydro-2H-pyrimidin-1-yl]-acétique et 125mg (0,5 mmoles) de 3-pipéridin-4-yl-1,3,4,5-tétrahydro-benzo[d][1,3]diazépin-2-one (préparé comme décrit dans l'exemple 16.4),150mg (59%) de 5-(2,3-difluoro-phényl)-3-((S)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 234°C.
RMN ¹H (δ, DMSO) : 1.34 (d, J = 7.0 Hz, 3H); 1.53-1.80 (m, 4H); 2.69 (t, J = 12.4 Hz, 1H) ; 2.90 (m, 2H); 3.15 (t, J = 12.3 Hz, 1H); 3.23 (s, 3H); 3.38-3.40 (m, 2H); 3.54 (dd, J = 9.9, 5.9 Hz, 1H); 3.90-3.95 (m, 2H); 4.28-4.38 (m, 1H); 4.43 (d, J = 12.9 Hz, 1H); 4.77 (s, 2H); 5.15-5.17 (m, 1H); 6.79-6.83 (m, 1H); 7.02-7.05 (m, 3H); 7.19-7.29 (m, 2H); 7.42-7.49 (m, 1H); 7.89 (s, 1H); 8.53 (s, 1H).

### Exemple 110: 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 110) (example comparatif)

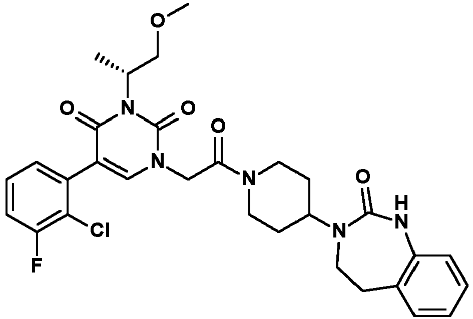

### 110.1: [5-bromo-3-((R)-2-méthoxy-1-méthyl-éthyl)-2,4-dioxo-3,4-dihydro-2H-pyrimidin-1-yl]-acétate de méthyle

De manière analogue à l'exemple 106.1, à partir de 1g (3,8 mmoles) de (5-bromo-2,4-dioxo-3,4-dihydro-2H-pyrimidin-1-yl)-acétate de méthyle (préparé comme décrit dans l'exemple 13.1), et 0,4ml (4,2 mmoles) de (S)-1-méthoxy-propan-2-ol, 0,8g (63%) de [5-bromo-3-((R)-2-méthoxy-1-méthyl-éthyl)-2,4-dioxo-3,4-dihydro-2H-pyrimidin-1-yl]-acétate de méthyle sont obtenus sous la forme d'une huile incolore.

### 110.2: acide [5-(2-chloro-3-fluoro-phényl)-3-((R)-2-méthoxy-1-méthyl-éthyl)-2,4-dioxo-3,4-dihydro-2H-pyrimidin-1-yl]-acétique

De manière analogue à l'exemple 13.3, à partir de 300mg (0,9 mmoles) de [5-bromo-3-((R)-2-méthoxy-1-méthyl-éthyl)-2,4-dioxo-3,4-dihydro-2H-pyrimidin-1-yl]-acétate de méthyle et 156mg (0,9 mmoles) d'acide 2-chloro-3-fluoro-phénylboronique, 180mg (54%) d' acide [5-(2-chloro-3-fluoro-phényl)-3-((R)-2-méthoxy-1-méthyl-éthyl)-2,4-dioxo-3,4-dihydro-2H-pyrimidin-1-yl]-acétique sont obtenus sous la forme d'une huile brune.

### 110.3: 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 110)

De manière analogue à l'exemple 13.4, à partir de 180mg (0,5 mmoles) d'acide [5-(2-chloro-3-fluoro-phényl)-3-((R)-2-méthoxy-1-méthyl-éthyl)-2,4-dioxo-3,4-dihydro-2H-pyrimidin-1-yl]-acétique et 143mg (0,6 mmoles) de 3-pipéridin-4-yl-1,3,4,5-tétrahydro-benzo[d][1,3]diazépin-2-one (préparé comme décrit dans l'exemple 16.4), et après cristallisation dans l'acétate d'éthyle, 105mg (36%) de 5-(2-chloro-3-fluoro-phényl)-3-((R)-2-méthoxy-1-méthyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 225°C.
RMN ¹H (δ, DMSO): 1.34 (d, J = 7.0 Hz, 3H); 1.50-1.80 (m, 4H); 2.68 (t, J = 12.5 Hz, 1H); 2.90 (m, 2H); 3.15 (m, 1H); 3.33 (s, 3H); 3.38 (m, 2H); 3.54 (dd, J = 9.9, 5.9 Hz, 1H); 3.90-3.95 (m, 2H); 4.33 (m, 1H); 4.43 (d, J = 12.9 Hz, 1H); 4.75 (m, 2H); 5.15 (m, 1H); 6.80-6.81 (m, 1H); 7.04-7.06 (m, 3H); 7.20-7.21 (m, 1H); 7.44-7.45 (m, 2H); 7.80 (s, 1H); 8.53 (s, 1H) .

### Exemple 111: 2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle (schéma réactionnel n°3, composé 111) (example comparatif)

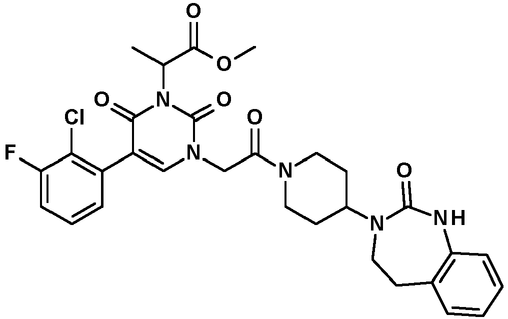

### 111.1: (3-benzhydryl-5-bromo-2,6-dioxo-3,6-dihydro-2H-pyrimidin-1-yl)-acétate de méthyle

De manière analogue à l'exemple 2.2, à partir de 10g (30 mmoles) de 1-benzhydryl-5-bromo-1H-pyrimidine-2,4-dione (préparé comme dans l'exemple 2.1) et 3,5ml (40 mmoles) de bromoacétate de méthyle, 11,1g (92%) de (3-benzhydryl-5-bromo-2,6-dioxo-3,6-dihydro-2H-pyrimidin-1-yl)-acétate de méthyle sont obtenus sous la forme d'un solide blanc.

### 111.2: 2-(3-benzhydryl-5-bromo-2,6-dioxo-3,6-dihydro-2H-pyrimidin-1-yl)-propanoate de méthyle

3,7g (20 mmoles) de bis(triméthylsilyl)amide de potassium et 1,7ml (20 mmoles) d'iodométhane sont ajoutés à une solution contenant 1g (2 mmoles) de (3-benzhydryl-5-bromo-2,6-dioxo-3,6-dihydro-2H-pyrimidin-1-yl)-acétate de méthyle dans 20 ml de tétrahydrofuranne préalablement refroidie à -78°C. Le mélange est agité à -78°C pendant 2 heures puis versé sur une solution saturée de chlorure d'ammonium et extrait par du dichlorométhane. La phase organique est séchée sur du sulfate de sodium anhydre puis filtrée et concentrée à sec. 640mg (62%) 2-(3-benzhydryl-5-bromo-2,6-dioxo-3,6-dihydro-2H-pyrimidin-1-yl)-propanoate de méthyle sont obtenus sous forme d'un solide jaune.

### 111.3: 2-[3-benzhydryl-5-(2-chloro-3-fluoro-phényl)-2,6-dioxo-3,6-dihydro-2H-pyrimidin-1-yl]-propanoate de méthyle

De manière analogue à l'exemple 44.1, à partir de 450mg (1 mmole) de 2-(3-benzhydryl-5-bromo-2,6-dioxo-3,6-dihydro-2H-pyrimidin-1-yl)-propanoate de méthyle et 354mg (2 mmoles) d'acide 2-chloro-3-fluorophénylboronique , et après purification par chromatographie sur gel de silice élué par un mélange d'acétate d'éthyle dans l'heptane, suivant un gradient de polarité (de 5% à 30% d'acétate d'éthyle dans l'heptane), 150mg (30%) de 2-[3-benzhydryl-5-(2-chloro-3-fluoro-phényl)-2,6-dioxo-3,6-dihydro-2H-pyrimidin-1-yl]-propanoate de méthyle sont obtenus sous la forme d'un solide jaune.

### 111.4: 2-[5-(2-chloro-3-fluoro-phényl)-2,6-dioxo-3,6-dihydro-2H-pyrimidin-1-yl]-propanoate de méthyle

De manière analogue à l'exemple 2.3, à partir de 150mg (0,3 mmoles) de 2-[3-benzhydryl-5-(2-chloro-3-fluoro-phényl)-2,6-dioxo-3,6-dihydro-2H-pyrimidin-1-yl]-propanoate de méthyle, 125mg (126%) de 2-[5-(2-chloro-3-fluoro-phényl)-2,6-dioxo-3,6-dihydro-2H-pyrimidin-1-yl]-propanoate de méthyle sont obtenus sous forme d'une huile marron.

### 111.5: 3-[1-(2-chloro-acétyl)-pipéridin-4-yl]-1,3,4,5-tétrahydro-benzo[d][1,3]diazépin-2-one

De manière analogue à l'exemple 2.4, à partir de 5g (20 mmoles) de 3-pipéridin-4-yl-1,3,4,5-tétrahydro-benzo[d][1,3]diazépin-2-one (préparé comme dans l'exemple 16.4) et 4,2g (44,8 mmoles) d'acide chloroacétique, 7g (107%) de 3-[1-(2-chloro-acétyl)-pipéridin-4-yl]-1,3,4,5-tétrahydro-benzo[d] [1,3]diazépin-2-one sont obtenus sous la forme d'un solide blanc.

### 111.6: 2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle (composé 111)

Une solution contenant 125mg (0,4 mmoles) de 2-[5-(2-chloro-3-fluoro-phényl)-2,6-dioxo-3,6-dihydro-2H-pyrimidin-1-yl]-propanoate de méthyle dans 2,5ml N,N-diméthylformamide est additionnée sur 148mg (0,5 mmoles) de 3-[1-(2-chloro-acétyl)-pipéridin-4-yl]-1,3,4,5-tétrahydro-benzo[d] [1,3]diazépin-2-one, 63mg (0,5 mmoles) de carbonate de potassium et 13mg (0,1 mmoles) d'iodure de potassium. Le mélange est agité pendant 45 minutes à 50°C. Il est hydrolysé puis extrait par de l'acétate d'éthyle. La phase organique est lavée à l'eau puis séchée sur sulfate de sodium anhydre, filtrée et concentrée à sec. L'huile jaune obtenue est purifiée par HPLC préparative. Après évaporation, 95mg (40%) de 2-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle sont obtenus sous forme d'un solide blanc.
RMN ¹H (400 MHz, MeOD-d4) δ: 1.59 (d, J = 6.9 Hz, 3H), 1.68-2.02 (m, 4H), 2.79 (m, 1H), 2.95-3.08 (m, 2H), 3.19-3.31 (m, 1H), 3.48-3.62 (m, 2H), 3.72 (s, 3H), 4.05 (m, 1H), 4.36-4.51 (m, 1H), 4.56-4.69 (m, 1H), 4.71-5.03 (m, 2H), 5.55 (q, J = 6.9 Hz, 1H), 6.86-6.97 (m, 2H), 7.09 (m, 2H), 7.24 (m, 1H), 7.30 (m, 1H), 7.39 (m, 1H), 7.71 (s, 1H); NH non observé dans le méthanol.

### Exemple 112: 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 112) (example comparatif)

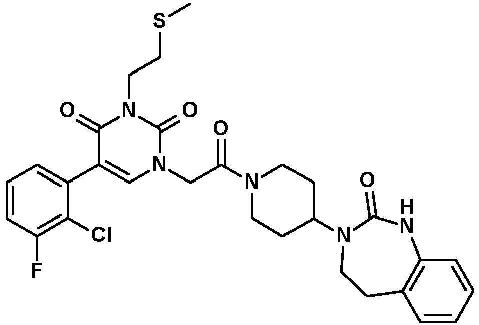

### 112.1: [5-bromo-3-(2-méthylsulfanyl-éthyl)-2,4-dioxo-3,4-dihydro-2H-pyrimidin-1-yl]-acétate de méthyle

De manière analogue à l'exemple 1.7, à partir de 0,5g (1,9 mmoles) de 5-bromo-2,4-dioxo-3,4-dihydro-2H-pyrimidin-1-yl)-acétate de méthyle (préparé comme dans l'exemple 13.1) et de 420mg (3,8 mmoles) de 2-chloroéthyl méthyl sulfide, et après chauffage du mélange à 50 °C pendant 5 heures, 520mg (81%) de [5-bromo-3-(2-méthylsulfanyl-éthyl)-2,4-dioxo-3,4-dihydro-2H-pyrimidin-1-yl]-acétate de méthyle sont obtenus sous la forme d'un solide blanc.

### 112.2: acide [5-(2-chloro-3-fluoro-phényl)-3-(2-méthylsulfanyl-éthyl)-2,4-dioxo-3,4-dihydro-2H-pyrimidin-1-yl]-acétique

De manière analogue à l'exemple 13.3, à partir de 520mg (1,5 mmoles) de [5-bromo-3-(2-méthylsulfanyl-éthyl)-2,4-dioxo-3,4-dihydro-2H-pyrimidin-1-yl]-acétate de méthyle et 296mg (1,7 mmoles) d'acide 2-chloro-3-fluorophénylboronique, 490mg (85%) d'acide [5-(2-chloro-3-fluoro-phényl)-3-(2-méthylsulfanyl-éthyl)-2,4-dioxo-3,4-dihydro-2H-pyrimidin-1-yl]-acétique sont obtenus sous la forme d'un solide beige.

### 112.3 : 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (composé 112)

De manière analogue à l'exemple 13.4, à partir de 490mg (1,3 mmoles) d'acide [5-(2-chloro-3-fluoro-phényl)-3-(2-méthylsulfanyl-éthyl)-2,4-dioxo-3,4-dihydro-2H-pyrimidin-1-yl]-acétique et 387mg (1,6 mmoles) de 3-pipéridin-4-yl-1,3,4,5-tétrahydro-benzo[d][1,3]diazépin-2-one (préparé comme décrit dans l'exemple 16.4), et après recristallisation dans le n-butanol, 470mg (58%) de 5-(2-chloro-3-fluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione sont obtenus sous la forme d'un solide blanc de point de fusion 207°C.

RMN ¹H (δ, DMSO): 1.50-1.80 (m, 4H), 2.12 (s, 3H), 2.69 (t, 3H, J = 7.19 Hz), 2.90 (m, 2H), 3.16 (m, 1H), 3.38 (m, 2H), 3.95 (d, 1H, J = 13.66 Hz), 4.08 (t, 2H, J = 7.21 Hz), 4.33 (m, 1H), 4.43 (d, 1H, J = 12.91 Hz), 4.79 (s, 2H), 6.79-6.83 (m, 1H), 7.02-7.05 (m, 3H), 7.21-7.23 (m, 1H), 7.44-7.48 (m, 2H), 7.85 (s, 1H), 8.52 (s, 1H).

### Exemple 113: 5-(2,3-difluoro-phényl)-3-(2-méthylsulfanyl-éthyl)-1-{2-oxo-2-[4-(2-oxo-1,2,4,5-tétrahydro-benzo[d][1,3]diazépin-3-yl)-pipéridin-1-yl]-éthyl}-1H-pyrimidine-2,4-dione (schéma réactionnel n°3, composé 113) (example comparatif)

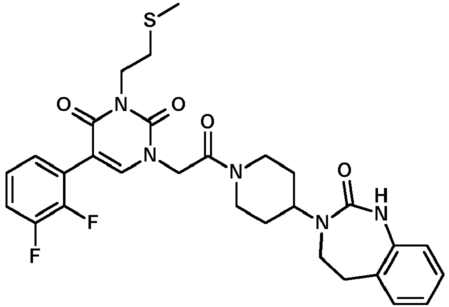

### 113.1: (5-bromo-2,4-dioxo-3,4-dihydro-2H-pyrimidin-1-yl)-acétate de méthyle

23ml (0,2 mmoles) de 1,8-diazabicyclo[5.4.0]undec-7-ène sont additionnés sur une solution contenant 30g (0.2 mmoles) de 5-bromouracile dans 294ml d'acétonitrile. Le milieu passe en solution puis rechute après 5 min. Il est alors refroidi à -5°C avec un bain glace / acétone puis 16ml (0.2 mmoles) de bromoacétate de méthyle sont ajoutés. Le milieu est agité pendant 45 minutes à température ambiante, traité en coulant 150ml d'eau, puis filtré.Le précipité blanc est rincé avec un peu de méthyl tert-butyl éther et séché sous vide à 40°C. 25,5g (63%) de (5-bromo-2,4-dioxo-3,4-dihydro-2H-pyrimidin-1-yl)-acétate de méthyle sont obtenus sous la forme d'un solide blanc.

### 113.2: [5-bromo-3-(2-méthylsulfanyl-éthyl)-2,4-dioxo-3,4-dihydro-2H-pyrimidin-1-yl]-acétate de méthyle

De manière analogue à l'exemple 1.7, à partir de 1,2g (4,6 mmoles) de (5-bromo-2,4-dioxo-3,4-dihydro-2H-pyrimidin-1-yl)-acétate de méthyle et de 1,4ml (13,7 mmoles) de 2-chloroéthyl méthyl sulfide, et après chauffage du mélange à 50 °C pendant toute une nuit, 850mg (55%) de [5-bromo-3-(2-méthylsulfanyl-éthyl)-2,4-dioxo-3,4-dihydro-2H-pyrimidin-1-yl]-acétate de méthyle sont obtenus sous la forme d'une huile incolore qui cristallise.

### 113.3: [5-(2,3-difluoro-phényl)-3-(2-méthylsulfanyl-éthyl)-2,4-dioxo-3,4-dihydro-2H-pyrimidin-1-yl]-acétate de méthyle

De manière analogue à l'exemple 44.1, à partir de 550mg (1,6 mmoles) de [5-bromo-3-(2-méthylsulfanyl-éthyl)-2,4-dioxo-3,4-dihydro-2H-pyrimidin-1-yl]-acétate de méthyle et 1,1g (6,5 mmoles) d'acide 2,3-difluorophénylboronique, 450mg (75%) de [5-(2,3-difluoro-phényl)-3-(2-méthylsulfanyl-éthyl)-2,4-dioxo-3,4-dihydro-2H-pyrimidin-1-yl]-acétate de méthyle sont obtenus sous la forme d'un solide blanc.

### 113.4: acide [5-(2,3-difluoro-phényl)-3-(2-méthylsulfanyl-éthyl)-2,4-dioxo-3,4-dihydro-2H-pyrimidin-1-yl]-acétique

De manière analogue à l'exemple 16.8, à partir de 450mg (1,2 mmoles) de [5-(2,3-difluoro-phényl)-3-(2-méthylsulfanyl-éthyl)-2,4-dioxo-3,4-dihydro-2H-pyrimidin-1-yl]-acétate de méthyle, 430mg (99%) d' acide [5-(2,3-difluoro-phényl)-3-(2-méthylsulfanyl-éthyl)-2,4-dioxo-3,4-dihydro-2H-pyrimidin-1-yl]-acétique sont obtenus sous la forme d'un solide beige.

### 113.5: 5-(2,3-difluoro-phényl)-3-(2-méthylsulfanyl-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione (composé 113)

De manière analogue à l'exemple 13.4, à partir de 200mg (0,6 mmoles) d'acide [5-(2,3-difluoro-phényl)-3-(2-méthylsulfanyl-éthyl)-2,4-dioxo-3,4-dihydro-2H-pyrimidin-1-yl]-acétique et 165mg (0,7 mmoles) de 3-pipéridin-4-yl-1,3,4,5-tétrahydro-benzo[d][1,3]diazépin-2-one, et après cristallisation dans un mélange heptane/acétate d'éthyle 50/50, 230mg (65%) de 5-(2,3-difluoro-phényl)-3-(2-méthylsulfanyl-éthyl)-1-{2-oxo-2-[4-(2-oxo-1,2,4,5-tétrahydro-benzo[d] [1,3]diazépin-3-yl)-pipéridin-1-yl]-éthyl}-1H-pyrimidine-2,4-dione sont obtenus sous la forme d'un solide blanc de point de fusion 220°C.
RMN ¹H (400 MHz, DMSO-d6) δ: 1.50-1.63 (m, 1H), 1.69 (m, 3H), 2.12 (s, 3H), 2.69 (m, 3H), 2.82-2.97 (m, 2H), 3.16 (m, 1H), 3.35-3.48 (m, 2H), 3.95 (m, 1H), 4.03-4.15 (m, 2H), 4.28-4.38 (m, 1H), 4.43 (m, 1H), 4.80 (s, 2H), 6.73-6.88 (m, 1H), 6.96-7.10 (m, 3H), 7.17-7.35 (m, 2H), 7.46 (m, 1H), 7.94 (s, 1H), 8.53 (s, 1H).

### Exemple 114: 3-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle (schéma réactionnel n°3, composé 114) (example comparatif)

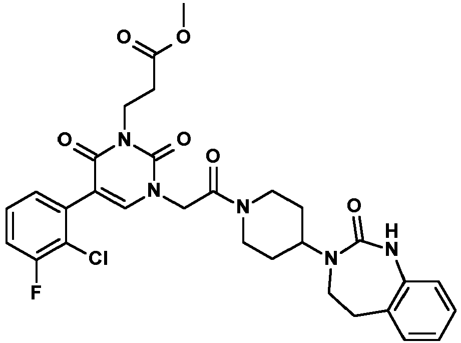

### 114.1: 3-(3-benzhydryl-5-bromo-2,6-dioxo-3,6-dihydro-2H-pyrimidin-1-yl)-propanoate de méthyle

De manière analogue à l'exemple 2.2, à partir de 1g (2,8 mmoles) de 1-benzhydryl-5-bromo-1H-pyrimidine-2,4-dione (préparé comme dans l'exemple 2.1) et 608mg (3,6 mmoles) de 3-bromopropionate de méthyle, 1,1g (85%) de 3-(3-benzhydryl-5-bromo-2,6-dioxo-3,6-dihydro-2H-pyrimidin-1-yl)-propanoate de méthyle sont obtenus sous la forme d'un solide blanc.

### 114.2: 3-[3-benzhydryl-5-(2-chloro-3-fluoro-phényl)-2,6-dioxo-3,6-dihydro-2H-pyrimidin-1-yl]-propanoate de méthyle

De manière analogue à l'exemple 44.1, à partir de 1,1g (2,4 mmoles) de 3-(3-benzhydryl-5-bromo-2,6-dioxo-3,6-dihydro-2H-pyrimidin-1-yl)-propanoate de méthyle et 620mg (3,6 mmoles) d'acide 2-chloro-3-fluorophénylboronique, et après purification par chromatographie sur gel de silice élué avec un mélange heptane / acétate d'éthyle 80/20, 660mg (57%) de 3-[3-benzhydryl-5-(2-chloro-3-fluoro-phényl)-2,6-dioxo-3,6- dihydro-2H-pyrimidin-1-yl]-propanoate de méthyle sont obtenus sous la forme d'un solide jaune.

### 114.3: 3-[5-(2-chloro-3-fluoro-phényl)-2,6-dioxo-3,6-dihydro-2H-pyrimidin-1-yl]-propanoate de méthyle

De manière analogue à l'exemple 2.3, à partir de 500mg (1,0 mmoles) de 3-[3-benzhydryl-5-(2-chloro-3-fluoro-phényl)-2,6-dioxo-3,6-dihydro-2H-pyrimidin-1-yl]-propanoate de méthyle, 310mg (94%) de 3-[5-(2-chloro-3-fluoro-phényl)-2,6-dioxo-3,6-dihydro-2H-pyrimidin-1-yl]-propanoate de méthyle sont obtenus sous la forme d'un solide jaunâtre.

### 114.4: 3-[1-(2-chloro-acétyl)-pipéridin-4-yl]-1,3,4,5-tétrahydro-benzo[d][1,3]diazépin-2-one

De manière analogue à l'exemple 2.4, à partir de 6g (24,5 mmoles) de 3-pipéridin-4-yl-1,3,4,5-tétrahydro-benzo[d][1,3]diazépin-2-one (préparé comme dans l'exemple 16.4) et 5g (53,8 mmoles) d'acide chloroacétique, 6,4g (81%) de 3-[1-(2-chloro-acétyl)-pipéridin-4-yl]-1,3,4,5-tétrahydro-benzo[d] [1,3]diazépin-2-one sont obtenus sous la forme d'un solide blanc.

### 114.5: 3-(5-(2-chloro-3-fluoro-phényl)-2,6-dioxo-3-{2-oxo-2-[4-(2-oxo-1,2,4,5-tétrahydro-benzo[d][1,3]diazépin-3-yl)-pipéridin-1-yl]-éthyl}-3,6-dihydro-2H-pyrimidin-1-yl)-propanoate de méthyle (composé 114)

De manière analogue à l'exemple 111.6, à partir de 150mg (0,5 mmoles) de 3-[5-(2-chloro-3-fluoro-phényl)-2,6-dioxo-3,6-dihydro-2H-pyrimidin-1-yl]-propanoate de méthyle et 163mg (0,5 mmoles) de 3-[1-(2-chloro-acétyl)-pipéridin-4-yl]-1,3,4,5-tétrahydro-benzo[d][1,3]diazépin-2-one, et après cristallisation dans l'acétate d'éthyle puis l'heptane, 230mg (80%) de 3-(5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-propanoate de méthyle sont obtenus sous la forme d'un solide blanc cassé.
RMN ¹H (δ, DMSO): 1.55 (d, J = 13.8 Hz, 1H); 1.69 (d, J = 23.0 Hz, 3H) ; 2.59 (t, J = 7.4 Hz, 2H) ; 2.69 (t, 1H) ; 2.90 (m, 2H); 3.16 (br s, 1H); 3.38 (m, 2H); 3.60 (s, 3H); 3.95 (d, J = 13.5 Hz, 1H); 4.13 (t, J = 7.4 Hz, 2H); 4.33 (br s, 1H); 4.43 (d, J = 12.9 Hz, 1H); 4.78 (s, 2H); 6.81 (dt, J = 7.8, 4.3 Hz, 1H); 7.04 (t, J = 3.8 Hz, 3H) ; 7.21-7.23 (m, 1H); 7.44-7.47 (m, 2H); 7.85 (s, 1H); 8.52 (s, 1H).

Autre composé synthétisé selon un mode opératoire analogue en utilisant les réactifs appropriés commerciaux ou préalablement préparé :

### composé 202: N-[2-(5-(2-chloro-3-fluoro-phényl)-2,6-dioxo-3-{2-oxo-2-[4-(2-oxo-1,2,4,5-tétrahydro-benzo[d] [1,3]diazépin-3-yl)-pipéridin-1-yl]-éthyl}-3,6-dihydro-2H-pyrimidin-1-yl)-éthyl]-acétamide (example comparatif)

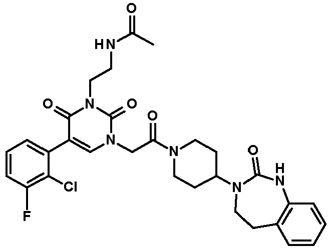

RMN ¹H (δ, DMSO): 1.47-1.83 (m, 4H), 1.76 (s, 3H), 2.62 -2.77 (m, 1H), 2.81-2.97 (m, 2H), 3.07 - 3.20 (m, 1H), 3.26 (q, J = 6.43 Hz, 2H), 3.34 - 3.44 (m, 2H), 3.84 - 4.04 (m, 3H), 4.25 - 4.38 (m, 1H), 4.43 (d, J = 13.11 Hz, 1H), 4.78 (s, 2H), 6.68 - 6.87 (m, 1H), 6.90 - 7.10 (m, 3H), 7.14 - 7.31 (m, 1H), 7.34 - 7.56 (m, 2H), 7.82 (s, 1H), 7.92 (t, J = 5.91 Hz, 1H), 8.52 (s, 1H) .

### Exemple 115: N-[(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-acétamide (schéma réactionnel 3 ou 4, composé 115)

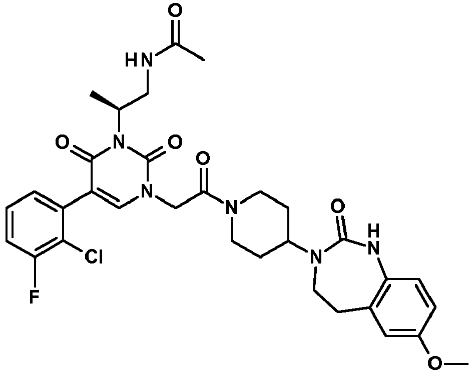

### 115.1: (5-méthoxy-2-nitro-phényl)-acétonitrile

Une solution contenant 30,5g (199 mmoles) de 4-nitroanisole et 40g (239 mmoles) de (4-chloro-phénoxy)-acétonitrile dans 305ml de N,N-diméthylformamide sont rajoutés à -20°C sous azote à une solution contenant 53,6g (478 mmoles) de tert-butylate de potassium dans 610ml de N,N-diméthylformamide. Le milieu réactionnel est laissé remonté jusqu'à 0°C et maintenu à cette température. Après 1 heure, le milieu réactionnel est placé à -50°C et hydrolysé lentement avec 1000ml d'une solution aqueuse glacée d'acide chlorhydrique 6N, puis 1000ml d'acétate d'éthyle et 500ml d'eau sont additionnés. La phase aqueuse est extraite avec de l''acétate d'éthyle. La phase organique est lavée par une solution aqueuse saturée de chlorure d'ammonium puis par de l'eau, et ensuite séchée sur sulfate de sodium anhydre, filtrée et concentrée. Le résidu obtenu est repris dans du toluène et évaporé. L'huile ainsi obtenue est reprise sous agitation dans 250ml d'un mélange acétate d'éthyle / heptane (50/50) pendant une nuit puis le mélange est refroidi dans un bain de glace et filtré. Après rinçage à l'heptane, 26,7g (68%) de (5-méthoxy-2-nitro-phényl)-acétonitrile sont obtenus sous la forme d'un solide orange.

### 115.2: 2-(5-méthoxy-2-nitro-phényl)-éthylamine

200ml (200 mmoles) d'une solution complexée de borane-tétrahydrofurane sont rajoutés à une solution contenant 26g (136 mmoles) de (5-méthoxy-2-nitro-phényl)-acétonitrile dans 130ml de 2-méthyltétrahydrofuranne à 60°C. Après 3 heures, le chauffage est arrêté et 35ml (863 mmoles) de méthanol sont rajoutés lentement ainsi que 200ml d'une solution aqueuse d'hydroxyde de sodium 1N. La phase aqueuse est ensuite extraite avec 250ml de méthyl-tétrahydrofurane. La phase organique est lavée par de l'eau, séchée sur sulfate de sodium anhydre, filtrée et concentrée partiellement. 400ml d'éther tertbutylique et 10ml (175 mmoles) d'acide acétique sont rajoutés. Le mélange est refroidi, laissé sous agitation toute une nuit puis concentré et coévaporé au toluène. 40,6g (100%) d' acétate de 2-(5-méthoxy-2-nitro-phényl)-éthylamine sont obtenus sous la forme d'une huile brune.

### 115.3: 4-[2-(5-méthoxy-2-nitro-phényl)-éthylamino]-pipéridine-1-carboxylate de méthyle

44,5g (210 mmoles) de triacétoxyhydroborate de sodium sont rajoutés à une solution sous azote contenant 40,6g (136 mmoles) d'acide 2-(5-méthoxy-2-nitro-phényl)-éthylamine acétique, 35,9g (180 mmoles) de N-(tert-butoxycarbonyl)-4-pipéridone dans 360ml de 2-méthyltétrahydrofuranne. Après 2 heures, le milieu réactionnel est hydrolysé avec une solution aqueuse d'hydroxyde de sodium 2N. La phase aqueuse est extraite avec du méthyltétrahydrofurane. La phase organique est lavée par de l'eau pour être amenée à pH=6, séchée sur sulfate de sodium, filtrée et évaporée. 69,2g (100%) de 4-[2-(5-méthoxy-2-nitro-phényl)-éthylamino]-pipéridine-1-carboxylate de méthyle sont obtenus sous la forme d'une huile foncée.

### 115.4: 4-[2-(2-amino-5-méthoxy-phényl)-éthylamino]-pipéridine-1-carboxylate de tert-butyle

3,7g (70 mmoles) de chlorure d'ammonium et 31,3g (560 mmoles) de fer sont rajoutés à une solution contenant 69,2g (137 mmoles) de 4-[2-(5-méthoxy-2-nitro-phényl)-éthylamino]-pipéridine-1-carboxylate de méthyle dans 130ml de 2-méthyltétrahydrofuranne,130ml de méthanol et 130ml d'eau. Le milieu réactionnel est chauffé à reflux pendant 4 heures puis laissé revenir à température ambiante. Au bout de 16 heures, le milieu réactionnel est filtré sur célite et le filtrat est concentré jusqu'à évaporation du méthanol. Du méthyltétrahydrofurane est rajouté ainsi qu'une solution à 10 % d'acide éthylènediaminetetraacétique. La phase organique est lavée par de l'eau puis séchée sur sulfate de sodium anhydre, filtrée et concentrée. 53,2g (100%) de 4-[2-(2-amino-5-méthoxy-phényl)-éthylamino]-pipéridine-1-carboxylate de tert-butyle sont obtenus sous la forme d'une huile orange.

### 115.5: 4-(7-méthoxy-2-oxo-1,2,4,5-tétrahydro-benzo[d][1,3]diazépin-3-yl)-pipéridine-1-carboxylate de tert-butyle

27,2g (168 mmoles) de N,N'-carbonyldiimidazole sont rajoutés par portion à une solution contenant 53,2g (137 mmoles) de 4-[2-(2-amino-5-méthoxy-phényl)-éthylamino]-pipéridine-1-carboxylate de tert-butyle à 90% dans du 1000ml de toluène. Après 1 heure, le précipité formé est filtré et le filtrat est concentré partiellement. Le résidu est lavé deux fois par une solution aqueuse de chlorure d'ammonium. La phase organique est séchée sur sulfate de sodium anhydre, filtrée et concentrée. 58g d'une huile orange sont obtenus. Après purification par chromatographie sur gel de silice élué par un mélange d'acétate d'éthyle dans l'heptane, suivant un gradient de polarité (de 20% à 70% d'acétate d'éthyle dans l'heptane), 35g d'un solide jaune sont obtenus et repris dans 200ml d'éther isopropylique au reflux, 100ml d'éther isopropylique, 100ml de tétrahydrofurane, 80ml d'éthanol et 300ml de méthanol. Le mélange est filtré puis concentré. Après purification par chromatographie sur gel de silice élué par un mélange d'acétate d'éthyle dans l'heptane, suivant un gradient de polarité (de 20% à 100% d'acétate d'éthyle dans l'heptane), 21,1g (34%) de 4-(7-méthoxy-2-oxo-1,2,4,5-tétrahydro-benzo[d][1,3]diazépin-3-yl)-pipéridine-1-carboxylate de tert-butyle sont obtenus sous la forme d'un solide beige.

### 115.6: 7-méthoxy-3-pipéridin-4-yl-1,3,4,5-tétrahydro-benzo[d][1,3]diazépin-2-one

35ml (380 mmoles) d'acide trifluoroacétique sont rajoutés à 0°C à une solution contenant 83% de 4-(7-méthoxy-2-oxo-1,2,4,5-tétrahydro-benzo[d][1,3]diazépin-3-yl)-pipéridine-1-carboxylate de tert-butyle_dans 300ml de dichlorométhane. Après 15 heures, le milieu réactionnel est concentré lentement à l'évaporateur rotatif. Le résidu est coévaporé au toluène,repris dans 250ml de dichlorométhane puis le mélange est amené à pH=10 avec une solution aqueuse d'hydroxyde de sodium 1N. La phase aqueuse est extraite par du dichlorométhane, la phase organique est séchée sur sulfate de sodium anhydre, filtrée et concentrée. 12,7g (96%) de 7-méthoxy-3-pipéridin-4-yl-1,3,4,5-tétrahydro-benzo[d][1,3]diazépin-2-one sont obtenus sous la forme d'un solide beige.

### 115.7: [5-bromo-3-((S)-2-tert-butoxycarbonylamino-1-méthyl-éthyl)-2,4-dioxo-3,4-dihydro-2H-pyrimidin-1-yl]-acétate de méthyle

De manière analogue à l'exemple 2.5, à partir de 3,3 g (12,6 mmoles) de (5-bromo-2,4-dioxo-3,4-dihydro-2H-pyrimidin-1-yl)-acétate de méthyle (préparé comme dans l'exemple 13.1) et 2g (11,4 mmoles) de ((R)-2-hydroxy-propyl)-carbamate de tert-butyle, et après purification par chromatographie sur gel de silice élué par un mélange d'acétate d'éthyle dans l'heptane, suivant un gradient de polarité (de 20% à 50% d'acétate d'éthyle dans l'heptane), 4,1g (85%) de [5-bromo-3-((S)-2-tert-butoxycarbonylamino-1-méthyl-éthyl)-2,4-dioxo-3,4-dihydro-2H-pyrimidin-1-yl]-acétate de méthyle sont obtenus sous la forme d'une huile visqueuse.

### 115.8: [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétate de méthyle

De manière analogue à l'exemple 44.1, à partir de 1,9g (11,1 mmoles) d'acide 2-chloro-3-fluorophénylboronique et 3,1g (7,4 mmoles) de [5-bromo-3-((S)-2-tert-butoxycarbonylamino-1-méthyl-éthyl)-2,4-dioxo-3,4-dihydro-2H-pyrimidin-1-yl]-acétate de méthyle, 2,4g (68%) de [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétate de méthyle sont obtenus sous la forme d'une meringue.

### 115.9: acide[3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique

De manière analogue à l'exemple 16.8, à partir de 1g (2,1 mmoles) de [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétate de méthyle, 983mg (101%) d' acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique sont obtenus sous la forme d'un solide beige.

### 115.10: [(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-carbamate de tert-butyle

490mg (2,6 mmoles) de chlorhydrate de 1-éthyl-3-(3-diméthylaminopropyl)carbodiimide et 284mg (2,6 mmoles) de 1-oxy-pyridin-2-ol sont additionnés à une solution contenant 971mg (2,1 mmoles) d'acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique dans 5ml de N,N-diméthylformamide. Le milieu réactionnel est agité pendant 5 minutes puis 645mg (2,3 mmoles) de 7-méthoxy-3-pipéridin-4-yl-1,3,4,5-tétrahydro-benzo[d][1,3]diazépin-2-one et 0,4ml (2,6 mmoles) de triéthylamine sont additionnés. Le milieu réactionnel est agité à température ambiante pendant 12 heures puis dilué par de l'acétate d'éthyle et de l'eau. La phase aqueuse est extraite trois fois par de l'acétate d'éthyle, la phase organique est lavée par une solution saturée de carbonate de sodium puis par une solution aqueuse d'acide chlorhydrique 1N. Après séchage sur sulfate de magnésium, la solution est filtrée et concentrée sous vide. Après purification par chromatographie sur gel de silice élué par un mélange de méthanol dans le dichlorométhane, suivant un gradient de polarité (de 0% à 5% de méthanol dans le dichlorométhane), 1,2g (79%) de [(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle sont obtenus sous la forme d'un solide beige.

### 115.11: 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

0,8ml (7,8 mmoles) d'acide trifluoroacétique sont ajoutés à une solution contenant 800mg (1,1 mmoles) de [(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle dans 7ml de dichlorométhane. Le mélange est agité à température ambiante pendant deux heures puis deux équivalents d'acide trifluoroacétique sont ajoutés . Le mélange est concentré sous vide et repris dans le dichlorométhane et traité avec une solution aqueuse d'hydroxyde de sodium 1N. La phase aqueuse est extraite trois fois par du dichlorométhane, la phase organique est lavée par de l'eau puis par de la saumure. La phase organique est séchée sur sulfate de sodium anhydre, filtrée et concentrée à sec. 646mg (94%) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide blanc cassé.

### 115.12: N-[(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-acétamide (composé 115)

0,1ml (0,8 mmoles) de triéthylamine et 68mg (0,7 mmoles) d'anhydride acétique sont ajoutés à -10°C à une solution contenant 391mg (0,6 mmoles) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione dans 3,2ml de tétrahydrofuranne. Le mélange est hydrolysé après 5 minutes à -10°C par de l'eau et dilué à l'acétate d'éthyle. La phase aqueuse est extraite trois fois par de l'acétate d'éthyle, la phase organique est lavée par de la soude 1N puis par de la saumure. Après séchage sur sulfate de magnésium, la solution est filtrée puis concentrée. Le précipité est repris dans l'acétate d'éthyle et agité pendant 24 heures à température ambiante. 190mg (44%) de N-[ (S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-acétamide sont obtenus sous la forme d'un solide blanc de point de fusion 200°C.
RMN ¹H (δ, DMSO) : 1.34 (d, J = 6.5 Hz, 3H), 1.42-1.75 (m, 4H), 1.77 (s, 3H), 2.60-2.75 (m, 1H), 2.82-2.93 (m, 2H), 3.14 (t, J = 12.6 Hz, 1H), 3.32-3.42 (m, 2H), 3.40-3.61 (m, 2H),3.67 (s, 3H), 3.94 (bt d, J = 13.3 Hz, 1H), 4.18-4.38 (m, 1H), 4.43 (d, J = 12.7 Hz, 1H), 4.58-4.90 (m, 2H), 4.99 (m, 1H), 6.62 (d, J = 2.9 Hz, 1H), 6.66 (dd, J = 2.9, 8.8Hz, 1H), 6.98 (d, J = 8.8 Hz, 1H), 7.13 - 7.31 (m, 1H), 7.35-7.57 (m, 2H), 7.8 (s, 1H), 7.88-8.02 (m, 1H), 8.32 (s, 1H).

Les composés suivants ont été synthétisés selon un mode opératoire analogue en utilisant les réactifs appropriés commerciaux ou préalablement préparés:

### composé 203: N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide

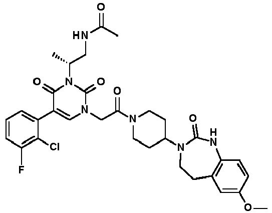

RMN ¹H (δ, DMSO) : 1.34 (dd, J = 6.8, 1.4, 3H), 1.45-1.75 (m, 4H), 1.67-1.87 (m, 3H), 2.62-2.78 (m, 1H), 2.81-2.94 (m, 2H), 3.14 (t, J = 12.5 Hz, 1H), 3.32-3.43 (m, 2H), 3.39-3.62 (m, 2H), 3.67 (s, 3H), 3.87-3.99 (m, 1H), 4.17-4.35 (m, 1H), 4.44 (d, J = 12.6 Hz, 1H), 4.60-4.86 (m, 2H), 4.99 (q, J = 7.2 Hz, 1H), 6.44-6.76 (m, 2H), 6.98 (d, J = 8.8 Hz, 1H), 7.21 (dd, J = 5.7, 3.3 Hz, 1H), 7.39-7.58 (m, 2H), 7.79 (s, 1H), 7.93 (t, J=6.1, 1H), 8.32 (s, 1H).

### composé 204: N-[(R)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-2,2,2-trifluoro-acétamide (example comparatif)

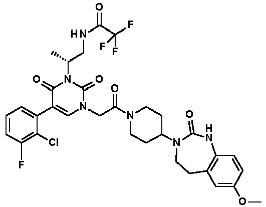

RMN ¹H (δ, DMSO) : 1.39 (dd, J= 7.0 Hz, 2.3, 3H), 1.47-1.83 (m, 4H), 2.63-2.76 (m, 1H), 2.87 (d, J = 5.9 Hz, 2H), 3.04-3.26 (m, 1H), 3.36 (d, J = 4.7 Hz, 2H), 3.56-3.66 (m, 1H), 3.67 (s, 3H), 3.75 (d, J = 8.1 Hz, 1H), 3.96 (d, J = 13.6 Hz, 1H), 4.22-4.37 (m, 1H), 4.44 (d, J = 12.7 Hz, 1H), 4.68 (d, J = 16.5 Hz, 1H), 4.82 (d, J = 16.5 Hz, 1H), 4.98-5.33 (m, 1H), 6.45-6.80 (m, 2H), 6.98 (d, J = 8.8 Hz, 1H), 7.12-7.26 (m, 1H), 7.37-7.58 (m, 2H), 7.80 (s, 1H), 8.33 (s, 1H), 9.58 (d, J = 5.3 Hz, 1H).

### composé 205: N-[(S)-2-(5-(2,3-dichloro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide

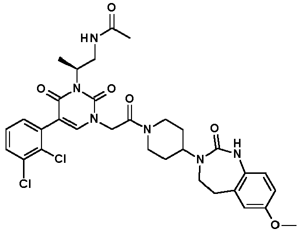

RMN ¹H (δ, DMSO) : 1.34 (d, J = 6.9 Hz, 3H); 1.56-1.71 (m, 4H); 1.77 (s, 3H); 2.68 (t, J = 12.3 Hz, 1H); 2.88 (m, 2H); 3.15 (m, 1H); 3.36 (m, 2H); 3.46-3.58 (m, 2H); 3.67 (s, 3H); 3.95 (d, J = 13.6 Hz, 1H); 4.28 (m, 1H); 4.44 (d, J = 12.8 Hz, 1H); 4.67-4.81 (m, 2H); 4.99 (m, 1H); 6.63-6.68 (m, 2H); 6.98 (d, J = 8.8 Hz, 1H); 7.33 (d, J = 7.7 Hz, 1H); 7.43 (t, J = 7.9 Hz, 1H); 7.68 (dd, J = 8.0, 1.6 Hz, 1H); 7.77 (s, 1H); 7.93 (t, J = 5.9 Hz, 1H); 8.32 (s, 1H).

### composé 206: N-[(S)-2-(5-(2-chloro-3-méthoxy-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide

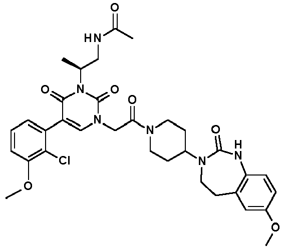

RMN ¹H (δ, DMSO) : 1.33 (d; J = 6.91 Hz; 3 H) ; 1.56 (m; 1 H); 1.68 (m; J = 20.83 Hz; 3 H); 1.77 (s; 3 H); 2.68 (t; J = 12.42 Hz; 1 H); 2.88 (d; J = 6.51 Hz; 2 H); 3.14 (t; J = 12.32 Hz; 1 H); 3.36 (d; J = 6.38 Hz; 2 H); 3.46-3.41 (m; 1 H); 3.59-3.54 (m; 1 H) ; 3.67 (s; 3 H) ; 3.89 (s; 3 H) ; 3.94 (d; J = 13.73 Hz; 1 H); 4.28 (m; 1 H); 4.44 (d; J = 12.77 Hz; 1 H); 4.80-4.66 (m; 2 H); 4.99-4.97 (m; 1 H); 6.69-6.63 (m; 2 H); 6.91 (d; J = 7.64 Hz; 1 H); 6.98 (d; J = 8.79 Hz; 1 H); 7.18 (dd; J = 8.37; 1.40 Hz; 1 H); 7.34 (t; J = 7.96 Hz; 1 H); 7.68 (s; 1 H); 7.92 (t; J= 5.78 Hz; 1 H); 8.32 (s; 1 H).

### Exemple 116: N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide (schéma réactionnel n°4, composé 116) (example comparatif)

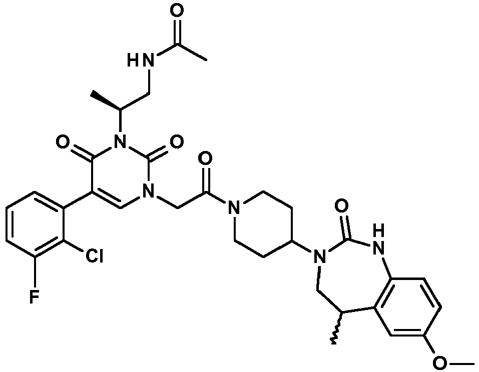

### 116.1: 2-(5-méthoxy-2-nitro-phényl)-propionitrile

Une solution contenant 1g (5,2 mmoles) de (5-méthoxy-2-nitro-phényl)-acétonitrile (préparé comme dans l'exemple 115.1) et 0,6ml (8,9 mmoles) d'iodométhane dans 5ml de N,N-diméthylformamide est ajoutée à 0°C à une solution contenant 208mg (5,2 mmoles) d'hydrure de sodium dans 7ml (91 mmoles) de N,N-diméthylformamide. Après 1 heure, le mélange est dilué par de l'acétate d'éthyle puis par de l'eau. La phase aqueuse est extraite trois fois à l'acétate d'éthyle, la phase organique est lavée à l'eau puis brine. Après séchage sur sulfate de magnésium, la solution est filtrée puis concentrée sous vide. Après purification par chromatographie sur gel de silice, 663mg (62%) de 2-(5-méthoxy-2-nitro-phényl)-propionitrile sont obtenus sous la forme d'une huile claire.

### 116.2: 2-(5-méthoxy-2-nitro-phényl)-propylamine

De manière analogue à l'exmple 115.2, à partir de 663mg (3,2 mmoles) de 2-(5-méthoxy-2-nitro-phényl)-propionitrile, 900mg (104%) de 2-(5-méthoxy-2-nitro-phényl)-propylamine sont obtenus sous la forme d'une huile.

### 116.3: 4-[2-(5-méthoxy-2-nitro-phényl)-propylamino]-pipéridine-1-carboxylate de tert-butyle

De manière analogue à l'exemple 115.3, à partir de 677mg (3,2 mmoles) de 2-(5-méthoxy-2-nitro-phényl)-propylamine, 1,2g (95%) de 4-[2-(5-méthoxy-2-nitro-phényl)-propylamino]-pipéridine-1-carboxylate de tert-butyle sont obtenus sous la forme d'une huile orange.

### 116.4: 4-[2-(2-amino-5-méthoxy-phényl)-propylamino]-pipéridine-1-carboxylate de tert-butyle

De manière analogue à l'exemple 115.4, à partir de 1,2g (3,2 mmoles) de 4-[2-(5-méthoxy-2-nitro-phényl)-propylamino]-pipéridine-1-carboxylate de tert-butyle, 1,2g (103%) de 4-[2-(2-amino-5-méthoxy-phényl)-propylamino]-pipéridine-1-carboxylate de tert-butyle sont obtenus sous la forme d'un solide orange.

### 116.5: 4-(7-méthoxy-5-méthyl-2-oxo-1,2,4,5-tétrahydro-benzo[d][1,3]diazépin-3-yl)-pipéridine-1-carboxylate de tert-butyle

De manière analogue à l'exemple 115.5, à partir de 1,2g (3,2 mmoles) de 4-[2-(2-amino-5-méthoxy-phényl)-propylamino]-pipéridine-1-carboxylate de tert-butyle, 455mg (36%) de 4-(7-méthoxy-5-méthyl-2-oxo-1,2,4,5-tétrahydro-benzo[d][1,3]diazépin-3-yl)-pipéridine-1-carboxylate de tert-butyle sont obtenus sous la forme d'un solide beige.

### 116.6: 7-méthoxy-5-méthyl-3-pipéridin-4-yl-1,3,4,5-tétrahydro-benzo[d][1,3] diazépin-2-one

De manière analogue à l'exemple 115.6, à partir de 455mg (1,2 mmoles) de 4-(7-méthoxy-5-méthyl-2-oxo-1,2,4,5-tétrahydro-benzo[d][1,3]diazépin-3-yl)-pipéridine-1-carboxylate de tert-butyle, 335mg (99%) de 7-méthoxy-5-méthyl-3-pipéridin-4-yl-1,3,4,5-tétrahydro-benzo[d][1,3]diazépin-2-one sont obtenus sou la forme d'un solide beige.

### 116.7: Acide [(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle

De manière analogue à l'exemple 115.10, à partir de 549mg (1,2 mmoles) d'acide [3-((S)-2-tert-butoxycarbonylamino-1-méthyl-éthyl)-5-(2-chloro-3-fluoro-phényl)-2,4-dioxo-3,4-dihydro-2H-pyrimidin-1-yl]-acétique (préparé comme dans l'exemple 115.9) et 332 mg (1,2 mmoles) de 7-méthoxy-5-méthyl-3-pipéridin-4-yl-1,3,4,5-tétrahydro-benzo[d][1,3]diazépin-2-one, 832mg (100%) d'acide [(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle sont obtenus.

### 116.8: 3-((S)-2-amino-1-méthyl-éthyl)-5-(2-chloro-3-fluoro-phényl)-1-{2-[4-(7-méthoxy-5-méthyl-2-oxo-1,2,4,5-tétrahydro-benzo[d][1,3]diazépin-3-yl)-pipéridin-1-yl]-2-oxo-éthyl}-1H-pyrimidine-2,4-dione

De manière analogue à l'exemple 115.11, à partir de 836mg (1,2 mmoles) d'acide [(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-]-carbamate de tert-butyle, et après ajout de 412mg (1,2 mmoles) d'acide(+)-o,o-dibenzoyl-D-tartrique dans le méthanol, chauffage et purification par chromatographie sur gel de silice, 492mg (68%) de 3-((S)-2-amino-1-méthyl-éthyl)-5-(2-chloro-3-fluoro-phényl)-1-{2-[4-(7-méthoxy-5-méthyl-2-oxo-1,2,4,5-tétrahydro-benzo[d][1,3]diazépin-3-yl)-pipéridin-1-yl]-2-oxo-éthyl}-1H-pyrimidine-2,4-dione sont obtenus.

### 116.9: N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide (composé 116)

De manière analogue à l'exemple 115.12, à partir de 100mg (0,2 mmoles) de 3-((S)-2-amino-1-méthyl-éthyl)-5-(2-chloro-3-fluoro-phényl)-1-{2-[4-(7-méthoxy-5-méthyl-2-oxo-1,2,4,5-tétrahydro-benzo[d][1,3]diazépin-3-yl)-pipéridin-1-yl]-2-oxo-éthyl}-1H-pyrimidine-2,4-dione, 7mg (6%) de N-[(S)-2-(5-(2-chloro-3-fluoro-phényl)-3-{2-[4-(7-méthoxy-5-méthyl-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-acétamide sont obtenus sous la forme d'un solide beige.
RMN ¹H (400 MHz, DMSO-d6) δ: 1.18 (m, 3H), 1.34 (dd, J = 7.0, 1.8 Hz, 3H), 1.68 (m, 4H), 1.76 (s, 3H), 2.59-2.73 (m, 1H), 3.04 (m, 1H), 3.13 (m, 1H), 3.30 (m, 2H), 3.51 (m, 2H), 3.68 (s, 3H), 3.95 (m, 1H), 4.25 (m, 1H), 4.44 (m, 1H), 4.57-4.86 (m, 2H), 4.98 (m, 1H), 6.57-6.73 (m, 2H), 6.97 (d, J = 8.6 Hz, 1H), 7.21 (m, 1H), 7.36-7.52 (m, 2H), 7.79 (s, 1H), 7.95 (t, J = 6.1 Hz, 1H), 8.36 (s, 1H).

### Exemple 117: N-[2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-acétamide (schéma réactionnel n°3, composé 117) (example comparatif)

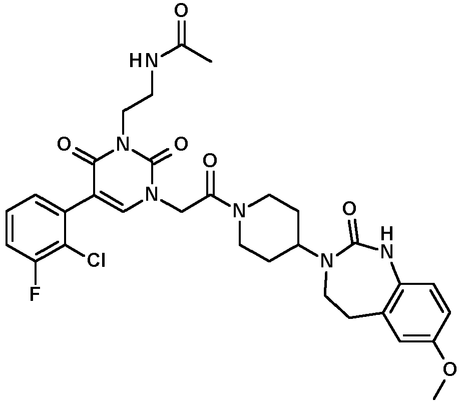

### 117.1: [5-bromo-3-(2-tert-butoxycarbonylamino-éthyl)-2,4-dioxo-3,4-dihydro-2H-pyrimidin-1-yl]-acétate de méthyle

2,4g (17,1 mmoles) de carbonate de potassium et 3,1g (13,7 mmoles) de N-(2-bromoéthyl)carbamate de tert-butyle sont additionnés sur une solution contenant 3g (11,4 mmoles) de (5-bromo-2,4-dioxo-3,4-dihydro-2H-pyrimidin-1-yl)-acétate de méthyle (préparé comme dans l'exemple 13.1) dans 60ml de N,N-diméthylformamide. Le milieu réactionnel est chauffé à 50°C pendant 2 heures puis est hydrolysé et dilué par de l'acétate d'éthyle. Le produit est extrait à l'acétate d'éthyle, la phase organiqus est lavée deux fois par de l'eau et une fois par une solution aqueuse saturée de chlorure de sodium, puis séchée sur sulfate de magnésium, filtrée et concentrée sous vide. Le produit est précipité dans 100ml d'ether diéthylique puis filtré et séché sous vide pendant 2 heures. 3,5g (76%) de [5-bromo-3-(2-tert-butoxycarbonylamino-éthyl)-2,4-dioxo-3,4-dihydro-2H-pyrimidin-1-yl]-acétate de méthyle sont obtenus sous la forme d'un solide blanc.

### 117.2: [3-(2-tert-butoxycarbonylamino-éthyl)-5-(2-chloro-3-fluoro-phényl)-2,4-dioxo-3,4-dihydro-2H-pyrimidin-1-yl]-acétate de méthyle

De manière analogue à l'exemple 115.8, à partir de 429mg (2,5 mmoles) d'acide 2-chloro-3-fluorophénylboronique et 500mg (1,2 mmoles) de [5-bromo-3-(2-tert-butoxycarbonylamino-éthyl)-2,4-dioxo-3,4-dihydro-2H-pyrimidin-1-yl]-acétate de méthyle, et après purification par chromatographie sur gel de silice élué par un mélange d'acétate d'éthyle dans l'heptane, suivant un gradient de polarité (de 40% à 80% d'acétate d'éthyle dans l'heptane), 370mg (66%) de [3-(2-tert-butoxycarbonylamino-éthyl)-5-(2-chloro-3-fluoro-phényl)-2,4-dioxo-3,4-dihydro-2H-pyrimidin-1-yl]-acétate de méthyle sont obtenus sous la forme d'un solide beige.

### 117.3: Acide [3-(2-tert-butoxycarbonylamino-éthyl)-5-(2-chloro-3-fluoro-phényl)-2,4-dioxo-3,4-dihydro-2H-pyrimidin-1-yl]-acétique

De manière analogue à l'exemple 115.9, à partir de 370mg (0,8 mmoles) de [3-(2-tert-butoxycarbonylamino-éthyl)-5-(2-chloro-3-fluoro-phényl)-2,4-dioxo-3,4-dihydro-2H-pyrimidin-1-yl]-acétate de méthyle, 353mg (98%) d'acide [3-(2-tert-butoxycarbonylamino-éthyl)-5-(2-chloro-3-fluoro-phényl)-2,4-dioxo-3,4-dihydro-2H-pyrimidin-1-yl]-acétique sont obtenus sous la forme d'un solide jaune.

### 117.4 [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-carbamate de tert-butyle

De manière analogue à l'exemple 115.10, à partir de 353mg (0,8 mmoles) d'acide [3-(2-tert-butoxycarbonylamino-éthyl)-5-(2-chloro-3-fluoro-phényl)-2,4-dioxo-3,4-dihydro-2H-pyrimidin-1-yl]-acétique et 242mg (0,9 mmoles) de 7-méthoxy-3-pipéridin-4-yl-1,3,4,5-tétrahydro-benzo[d][1,3]diazépin-2-one (préparé comme dans l'exemple 115.6), 270mg (48%) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-carbamate de tert-butyle sont obtenus sous la forme d'une pâte incolore.

### 117.5: 3-(2-amino-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione

De manière analogue à l'exemple 115.11, à partir de 270mg (13,1 mmoles) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-carbamate de tert-butyle, 240mg (104%) de 3-(2-amino-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide beige.

### 117.6: N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide (composé 117)

De manière analogue à l'exemple 115.12, à partir de 240mg (0,4 mmoles) de 3-(2-amino-éthyl)-5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione et 2,5mg (0,02 mmoles) de 4-diméthylaminopyridine, 202mg (76%) de N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-acétamide sont obtenus sous la forme d'un solide blanc de point de fusion 266°C.
RMN ¹H (δ, DMSO): 1.56-1.51 (1 H, m), 1.73-1.65 (3 H, m), 1.76 (3 H, s), 2.68 (1 H, t, J = 12.29 Hz), 2.88 (2 H, m), 3.15 (1 H, t, J = 11.94 Hz), 3.28-3.23 (2 H, m), 3.40-3.30 (2 H, m), 3.67 (3 H, s), 3.95 (3 H, t, J = 6.79 Hz), 4.28 (1 H, m), 4.43 (1 H, d, J = 12.86 Hz), 4.78 (2 H, s), 6.68-6.62 (2 H, m), 6.98 (1 H, d, J = 8.78 Hz), 7.22-7.20 (1 H, m), 7.47-7.44 (2 H, m), 7.82 (1 H, s), 7.93 (1 H, t, J = 5.87 Hz), 8.33 (1 H, s).

Autre composé synthétisé selon un mode opératoire analogue en utilisant les réactifs appropriés ou préalablement préparés: composé 207: N-[2-(5-(2,3-dichloro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-éthyl]-acétamide 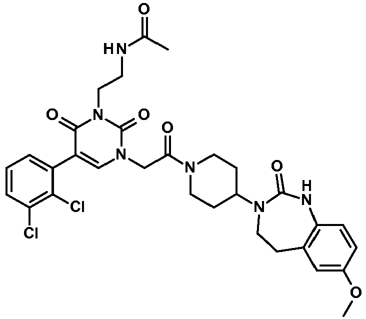 Masse = 657

### Exemple 118: N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide (schéma réactionnel n°3, composé 118) (example comparatif)

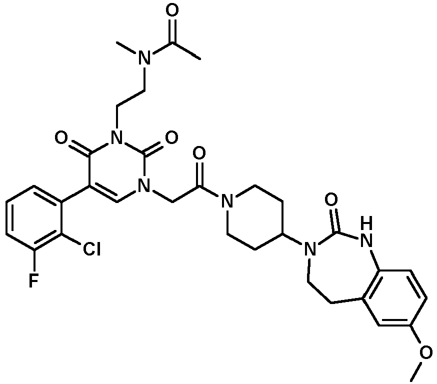

### 118.1: (2-chloro-éthyl)-méthyl-carbamate de tert-butyle

1,2g (5,6 mmoles) de dicarbonate de di-tert-butyle sont additionnés sur une solution contenant 0,5g (5,3 mmoles) de (2-chloro-éthyl)-méthyl-amine dans 10ml de dichlorométhane. Le mélange est agité à température ambainte pendant deux heures, de l'eau est rajoutée puis le produit est extrait avec du dichlorométhane La phase organique est séchée sur sulfate de sodium anhydre puis filtrée et concentrée à sec. 1g (97%) de (2-chloro-éthyl)-méthyl-carbamate de tert-butyle sont obtenus sous la forme d'une huile incolore.

### 118.2: [5-bromo-3-[2-(tert-butoxycarbonyl-méthyl-amino)-éthyl]-2,4-dioxo-3,4-dihydro-2H-pyrimidin-1-yl]-acétate de méthyle

0,6g (4,2 mmoles) de carbonate de potassium et 0,8g (4,2 mmoles) de (2-chloro-éthyl)-méthyl-carbamate de tert-butyle sont additionnés sur une solution contenant 1g (3,8 mmoles) de (5-bromo-2,4-dioxo-3,4-dihydro-2H-pyrimidin-1-yl)-acétate de méthyle (préparé comme dans l'exemple 13.1) dans 13ml de N,N-diméthylformamide. Le mélange est chauffé à 70°C pendant trois heures puis de l'eau est rajoutée et le mélange est extrait deux fois par de l'acétate d'éthyle. La phase organique est lavée à l'eau, séchée sur sulfate de magnésium, filtrée et évaporée. Après purification par chromatographie sur gel de silice, 300mg (19%) de [5-bromo-3-[2-(tert-butoxycarbonyl-méthyl-amino)-éthyl]-2,4-dioxo-3,4-dihydro-2H-pyrimidin-1-yl]-acétate de méthyle sont obtenus sous la forme d'une huile incolore.

### 118.3: [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétate de méthyle

De manière analogue à l'exemple 115.8, à partir de 300mg (0,7 mmoles) de [5-bromo-3-[2-(tert-butoxycarbonyl-méthyl-amino)-éthyl]-2,4-dioxo-3,4-dihydro-2H-pyrimidin-1-yl]-acétate de méthyle et 249mg (1,4 mmoles) d'acide 2-chloro-3-fluorophénylboronique, et après purification par chromatographie sur gel de silice élué par un mélange d'acétate d'éthyle dans l'heptane, suivant un gradient de polarité (de 40% à 70% d'acétate d'éthyle dans l'heptane), 140mg (42%) de [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétate de méthyle sont obtenus sous la forme d'un solide beige.

### 118.4: Acide [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétique

De manière analogue à l'exemple 115.9, à partir de 140mg (0,3 mmoles) de [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétate de méthyle, 160mg (118%) d' acide [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétique ont obtenus sous la forme d'une huile incolore.

### 118.5: [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-méthyl-carbamate de tert-butyle

De manière analogue à l'exemple 115.10, à partir de 160mg (0,4 mmoles) d'acide [3-[2-(tert-butoxycarbonyl-méthyl-amino)-éthyl]-5-(2-chloro-3-fluoro-phényl)-2,4-dioxo-3,4-dihydro-2H-pyrimidin-1-yl]-acétique et 106mg (0,4 mmoles) de 7-méthoxy-3-pipéridin-4-yl-1,3,4,5-tétrahydro-benzo[d] [1,3]diazépin-2-one (préparé comme dans l'exemple 115.6), 120mg (48%) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-méthyl-carbamate de tert-butyle sont obtenus sous la forme d'une huile incolore.

### 118.6: 5-(2-Chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylamino-éthyl)-1H-pyrimidine-2,4-dione

De manière analogue à l'exemple 115.11, à partir de 120mg (0,2 mmoles) de [2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-méthyl-carbamate de tert-butyle, 120mg (116%) de 5-(2-Chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylamino-éthyl)-1H-pyrimidine-2,4-dione sont obtenus sous la forme d'un solide blanc.

### 118.7: N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide (composé 118)

0,03ml (0,2 mmoles) de triéthylamine et 1mg (0,01 mmoles) de 4-diméthylaminopyridine sont additionnés à une solution contenant 120mg (0,2 mmoles) de 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylamino-éthyl)-1H-pyrimidine-2,4-dione dans 6ml de tétrahydrofuranne. Le mélange est refroidi à -10°C, puis 0,01ml (0,2 mmoles) de chlorure d'acétyle sont rajoutés. Après 30 minutes d'agitation à -10°C, le mélange est versé sur une solution aqueuse de carbonate de sodium saturée, puis extrait deux fois à l'acétate d'éthyle. La phase organique est séchée sur sulfate de sodium anhydre puis filtrée et concentrée à sec. Le solide obtenu est purifié par chromatographie sur gel de silice élué par un mélange de méthanol dans le dichlorométhane, suivant un gradient de polarité (de 0% à 5% de méthanol dans le dichlorométhane) . Après reprise dans du dichlorométhane et de l'heptane, précipitation et concentration à sec, 58mg (45%) de N-[2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-éthyl]-N-méthyl-acétamide sont obtenus sous la forme d'un solide blanc.
RMN ¹H (400 MHz, DMSO-d6) δ: 1.46-1.61 (m, 1H), 1.61-1.82 (m, 3H), 1.89 (s, 1.5H), 1.95 (s, 1.5H), 2.67 (m, 1H), 2.87 (m, 2H), 2.82 (s, 1.5H), 2.95 (s, 1.5H), 3.14 (m, 1H), 3.33-3.42 (m, 2H), 3.52 (m, 2H), 3.67 (s, 3H), 3.95 (m, 1H), 4.01-4.18 (m, 2H), 4.28 (m, 1H), 4.42 (d, J = 12.8 Hz, 1H), 4.78 (d, J = 10.3 Hz, 2H), 6.98 (d, J = 8.7 Hz, 1H), 7.19 (m, 1H), 7.45 (m, 2H), 7.84 (d, J = 28 Hz, 1H), 8.32 (s, 1H).

### Exemple 119: 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 119) (example comparatif)

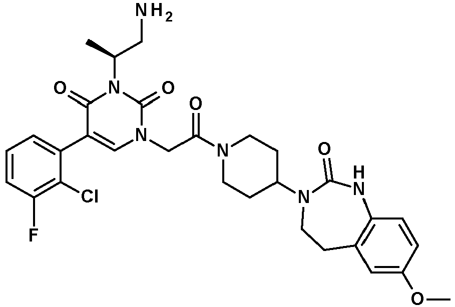

De manière analogue à l'exemple 115.10, à partir de 280mg (0,5 mmoles) de [(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-carbamate de tert-butyle (préparé comme dans l'exemple 115.10), et après purification par chromatographie sur gel de silice, 265 mg (95%) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide blanc cassé de point de fusion 206°C.
RMN ¹H (δ, DMSO): 1.34 (d, J = 6.9 Hz, 3H), 1.38 - 1.58 (m, 3H), 1.67 (m, 3H), 2.68 (m, 1H), 2.80-2.94 (m, 3H), 3.04 (m, 1H), 3.14 (m, 1H), 3.34-3.43 (m, 2H), 3.67 (s, 3H), 3.93 (d, J = 13.5 Hz, 1H), 4.19-4.35 (m, 1H), 4.43 (d, J = 13.0 Hz, 1H), 4.74 (m, 2H), 4.82 (m, 1H), 6.62 (d, J = 3.0 Hz, 1H), 6.67 (dd, J = 8.8, 3.0 Hz, 1H), 6.98 (d, J = 8.8 Hz, 1H), 7.22 (m, 1H), 7.36-7.53 (m, 2H), 7.78 (s, 1H), 8.32 (s, 1H).

### Exemple 120: N-[(S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-propyl]-méthanesulfonamide (schéma réactionnel n°3 ou 4, composé 120) (example comparatif)

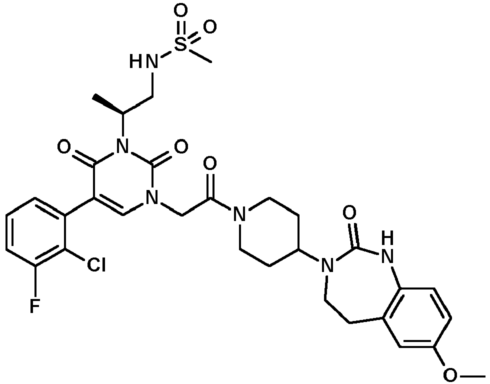

0,3ml (3,7 mmoles) de chlorure de méthanesulfonyle et 0,5ml (3,7 mmoles) de triéthylamine sont ajoutés à 0°C à une solution contenant 1,5g (2,5 mmoles) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (préparé comme dans l'exemple 119) dans 15ml de dichlorométhane. Après 30 minutes, du dichlorométhane est rajouté et le milieu réactionnel est hydrolysé avec une solution aqueuse d'acide chlorhydrique 1N. La phase aqueuse est réextraite au dichlorométhane. La phase organique est séchée sur sulfate de sodium anhydre, filtrée et concentrée. Après purification par chromatographie, reprise dans le pentane et est filtration, 0,8g (45%) de N-[(S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-propyl]-méthanesulfonamide sont obtenus sous la forme d'un solide blanc.
RMN ¹H (δ, DMSO) : 1.36 (dd, J = 2.1, 7.0 Hz, 3H), 1.43 - 1.80 (m, 4H), 2.59 - 2.72 (m, 1H), 2.86 (br s, 3+2H), 3.13 (t, J = 12.4 Hz, 1H), 3.34 - 3.40 (m, 2H), 3.46 - 3.63 (m, 2H), 3.66 (s, 3H), 3.94 (d, J = 13.5 Hz, 1H), 4.18 - 4.35 (m, 1H), 4.42 (d, J = 12.9 Hz, 1H), 4.66 - 4.82 (m, 2H), 4.88 - 5.16 (m, 1H), 6.61 (d, J = 3.0 Hz, 1H), 6.66 (dd, J = 3.0, 8.7 Hz, 1H), 6.97 (d, J = 8.8 Hz, 1H), 7.15 - 7.30 (m, 2H), 7.38 - 7.51 (m, 2H), 7.80 (s, 1H), 8.34 (s, 1H).

### Exemple 121: 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-[(S)-1-méthyl-2-(3-méthyl-oxétan-3-ylamino)-éthyl]-1H-pyrimidine-2,4-dione (schéma réactionnel n°3, composé 121) (example comparatif)

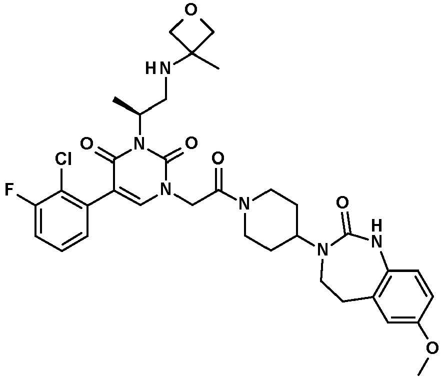

### 121.1: [5-bromo-3-((S)-2-chloro-1-méthyl-éthyl)-2,4-dioxo-3,4-dihydro-2H-pyrimidin-1-yl]-acétate de méthyle

1,3g (5,7 mmoles) d'azodicarboxylate de di-tert-butyle sont additionnés lentement sur une solution préalablement refroidie à 0°C contenant 1g (3,8 mmoles) de (5-bromo-2,4-dioxo-3,4-dihydro-2H-pyrimidin-1-yl)-acétate de méthyle (préparé comme dans l'exemple 13.1), 0,4ml (4,6 mmoles) de (R)-1-chloro-propan-2-ol et 1,5g (5,7 mmoles) de triphénylphosphine dans 20ml de tétrahydrofuranne. Après agitation à température ambiante durant 4 heures, le tétrahydrofurane est éliminé sous vide puis le résidu est repris dans l'acétate d'éthyle. La phase organique est lavée une fois par une solution aqueuse saturée d'hydrogénocarbonate de sodium, une fois par de l'eau puis par une solution aqueuse saturée de chlorure de sodium, séchée sur sulfate de magnésium, filtrée et concentrée sous vide. Après purification par chromatographie sur gel de silice élué par un mélange d'acétate d'éthyle dans l'heptane, suivant un gradient de polarité (de 2% à 50% d'acétate d'éthyle dans l'heptane), 1,2g (93%) de [5-bromo-3-((S)-2-chloro-1-méthyl-éthyl)-2,4-dioxo-3,4-dihydro-2H-pyrimidin-1-yl]-acétate de méthyle sont obtenus sous la forme d'une huile incolore.

### 121.2: [5-bromo-3-((S)-2-hydroxy-1-méthyl-éthyl)-2,4-dioxo-3,4-dihydro-2H-pyrimidin-1-yl]-acétate de méthyle

1ml (7,1 mmoles) de triéthylamine et 3,6ml de méthanol sont additionnés sur une solution contenant 1,2g (3,5 mmoles) de [5-bromo-3-((S)-2-chloro-1-méthyl-éthyl)-2,4-dioxo-3,4-dihydro-2H-pyrimidin-1-yl]-acétate de méthyle dans 12ml de tétrahydrofuranne et 2,7ml (151 mmoles) d'eau. Le mélange réactionnel est chauffé à 70°C pendant 22 heures puis les solvants sont concentrés sous vide. 440mg (39%) de [5-bromo-3-((S)-2-hydroxy-1-méthyl-éthyl)-2,4-dioxo-3,4-dihydro-2H-pyrimidin-1-yl]-acétate de méthyle sont obtenus sous la forme d'une huile claire.

### 121.3: [5-Bromo-3-((S)-1-méthyl-2-oxo-éthyl)-2,4-dioxo-3,4-dihydro-2H-pyrimidin-1-yl]-acétate de méthyle

697mg (1,6 mmoles) de périodinane de Dess-Martin sont additionnés à température ambiante sur une solution contenant 440mg (1,4 mmoles) de [5-bromo-3-((S)-2-hydroxy-1-méthyl-éthyl)-2,4-dioxo-3,4-dihydro-2H-pyrimidin-1-yl]-acétate de méthyle dans 13ml de dichlorométhane. Après 20 heures, de l'eau et du dichlorométhane sont rajoutés. La phase organique est récupérée puis concentrée sous vide. 440mg de [5-Bromo-3-((S)-1-méthyl-2-oxo-éthyl)-2,4-dioxo-3,4-dihydro-2H-pyrimidin-1-yl]-acétate de méthyle sont obtenus.

### 121.4: [5-bromo-3-[(S)-1-méthyl-2-(3-méthyl-oxétan-3-ylamino)-éthyl]-2,4-dioxo-3,4-dihydro-2H-pyrimidin-1-yl]-acétate de méthyle

440mg (1,4 mmoles) de [5-bromo-3-((S)-1-méthyl-2-oxo-éthyl)-2,4-dioxo-3,4-dihydro-2H-pyrimidin-1-yl]-acétate de méthyle, 0,1ml (1,4 mmoles) d'acide acétique et 0,1ml (1,4 mmoles) de 3-méthyloxétan-3-amine sont dilués dans 8,8ml de dichlorométhane. 409mg (1,9 mmoles) de triacétoxyborohydrure de sodium et 87mg (1,4 mmoles) de cyanoborohydrure de sodium sont ensuite rajoutés. L'agitation à température ambiante est maintenue pendant 20 heures. Le mélange est ensuite versé sur une solution aqueuse d'hydroxyde de sodium 1N puis extrait par du dichlorométhane.La phase organique est séchée sur sulfate de sodium anhydre puis filtrée et concentrée à sec. 220mg (41%) de [5-bromo-3-[(S)-1-méthyl-2-(3-méthyl-oxétan-3-ylamino)-éthyl]-2,4-dioxo-3,4-dihydro-2H-pyrimidin-1-yl}-acétate de méthyle sont obtenus sous la forme d'une huile incolore.

### 121.5: [5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétate de méthyle

De manière analogue à l'exemple 115.8, à partir d'une solution contenant 220mg (0,6 mmoles) de [5-bromo-3-[(S)-1-méthyl-2-(3-méthyl-oxétan-3-ylamino)-éthyl]-2,4-dioxo-3,4-dihydro-2H-pyrimidin-1-yl}-acétate de méthyle dans 2,2ml de toluène et 1,4ml de carbonate de potassium et après purification par chromatographie sur gel de silice élué par un mélange de méthanol dans le dichlorométhane, suivant un gradient de polarité (de 0% à 5% de méthanol dans le dichlorométhane), 30mg (12%) de [5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétate de méthyle sont obtenus sous la forme d'une huile jaune.

### 121.6: Acide[5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl}-acétique

De manière analogue à l'exemple 115.9, à partir de 30mg (0,1 mmoles) de [5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétate de méthyle, 29mg (100%) d'acide[5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl}-acétique sont obtenus sous la forme d'une huile marron.

### 121.7: 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-[(S)-1-méthyl-2-(3-méthyl-oxétan-3-ylamino)-éthyl]-1H-pyrimidine-2,4-dione (composé 121)

10mg (0,1 mmoles) de 1-oxy-pyridin-2-ol et 21mg (0,1 mmoles) de 7-méthoxy-3-pipéridin-4-yl-1,3,4,5-tétrahydro-benzo[d][1,3]diazépin-2-one (préparé comme dans l'exemple 115.6) sont additionnés sur une solution contenant 30mg (0,1 mmoles) d'acide[5-(2-chloro-3-fluoro-phényl)-3-[(S)-1-méthyl-2-(3-méthyl-oxétan-3-ylamino)-éthyl]-2,4-dioxo-3,4-dihydro-2H-pyrimidin-1-yl]-acétique dans 0,6ml de N,N-diméthylformamide. 17,6mg (0,1 mmoles) de chlorhydrate de 1-éthyl-3-(3-diméthylaminopropyl) carbodiimide sont rajoutés puis l'agitation est maintenue pendant 20 heures à température ambiante. Le mélange est versé sur de l'eau puis extrait par du dichlorométhane. La phase organique est lavée avec une solution aqueuse d'hydrogénocarbonate de sodium saturée puis rincée à l'eau, séchée sur sulfate de sodium anhydre puis filtrée et concentrée à sec. Après purification par chromatographie sur gel de silice élué par un mélange de méthanol dans le dichlorométhane, suivant un gradient de polarité (de 0% à 10% de méthanol dans le dichlorométhane), 18mg (37%) de 5-(2-chloro-3-fluoro-phényl)-1-[2-[4-(7-méthoxy-2-oxo-1,2,4,5-tétrahydro-benzo[d][1,3]diazépin-3-yl)-pipéridin-1-yl]-2-oxo-éthyl]-3-[(S)-1-méthyl-2-(3-méthyl-oxétan-3-ylamino)-éthyl]-1H-pyrimidine-2,4-dione sont obtenus sous la forme d'un solide blanc.
RMN ¹H (δ, DMSO): 1.33 (s; 3 H); 1.36-1.39 (m; 4 H); 1.53-1.56 (m; 1 H); 1.65-1.75 (m; 3 H); 2.68 (m; 1 H); 2.87-2.92 (m; 3 H); 3.02-3.07 (m; 1 H); 3.12-3.22 (m; 1 H); 3.36 (m ; 2H); 3.67 (s; 3 H); 3.94 (d; J = 13.50 Hz; 1 H); 4.16 (t; J = 5.37 Hz; 2 H); 4.28 (m; 1 H); 4.39 (d; J = 5.31 Hz; 2 H); 4.43 (d; J = 12.35 Hz; 1 H); 4.74 (m; 2 H); 4.89 (m; 1 H); 6.62 (d; J = 2.75 Hz; 1 H); 6.67 (dd; J = 8.79; 2.92 Hz; 1 H); 6.98 (d; J = 8.79 Hz; 1 H); 7.20-7.22 (m; 1 H); 7.43-7.46 (m; 2 H); 7.78 (s; 1H); 8.32 (s; 1 H).

### Exemple 122: 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3 ou 4, composé 122) (example comparatif)

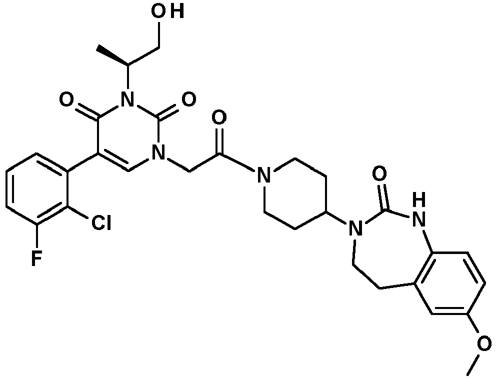

### 122.1: Acide [5-(2-chloro-3-fluoro-phényl)-3-((S)-2-hydroxy-1-méthyl-éthyl)-2,4-dioxo-3,4-dihydro-2H-pyrimidin-1-yl]-acétique

De manière analogue à l'exemple 115.8, à partir de 366mg (2,1 mmoles) d'acide 2-chloro-3-fluorophénylboronique et 450mg (1,4 mmoles) de [5-bromo-3-((S)-2-hydroxy-1-méthyl-éthyl)-2,4-dioxo-3,4-dihydro-2H-pyrimidin-1-yl]-acétate de méthyle (préparé comme dans l'exemple 121.2), 500mg (100%) d' acide [5-(2-chloro-3-fluoro-phényl)-3-((S)-2-hydroxy-1-méthyl-éthyl)-2,4-dioxo-3,4-dihydro-2H-pyrimidin-1-yl]-acétique sont obtenus.

### 122.2: 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (composé 122)

De manière analogue à l'exemple 115.10, à partir de 110mg (0,3 mmoles) d'acide [5-(2-chloro-3-fluoro-phényl)-3-((S)-2-hydroxy-1-méthyl-éthyl)-2,4-dioxo-3,4-dihydro-2H-pyrimidin-1-yl]-acétique et 93mg (0,3 mmoles) de 7-méthoxy-3-pipéridin-4-yl-1,3,4,5-tétrahydro-benzo[d][1,3]diazépin-2-one (préparé comme dans l'exemple 115.6), 150mg (78%) de 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc de point de fusion 138°C.
RMN ¹H (δ, DMSO) : 1.32 (dd, J = 7.1, 2.1 Hz, 3H), 1.55 (m, 1H), 1.61-1.83 (m, 3H), 2.68 (m, 1H), 2.82 - 2.92 (m, 2H), 3.14 (m, 1H), 3.34-3.41 (m, 2H), 3.67 (s, 3H), 3.69 (m, 1H), 3.83 (m, 1H), 3.94 (d, J = 13.7 Hz, 1H), 4.27 (m, 1H), 4.43 (d, J = 13.0 Hz, 1H), 4.74 (m, 2H), 4.79 (t, J = 5.7 Hz, 1H), 4.96 (h, J = 6.9 Hz, 1H), 6.62 (d, J = 3.0 Hz, 1H), 6.67 (dd, J = 8.9, 3.0 Hz, 1H), 6.98 (d, J = 8.8 Hz, 1H), 7.21 (m, 1H), 7.35-7.52 (m, 2H), 7.78 (s, 1H), 8.32 (s, 1H).

### Exemple 123: (S)-2-(5-(2-chloro-3-fluoro-phényl)-3-{2-[4-(7-méthoxy-2-oxo-1,2,4,5-tétrahydro-benzo[d] [1,3]diazépin-3-yl)-pipéridin-1-yl]-2-oxo-éthyl}-2,6-dioxo-3,6-dihydro-2H-pyrimidin-1-yl)-acétate de propyle (schéma réactionnel n°3, composé 123) (example comparatif)

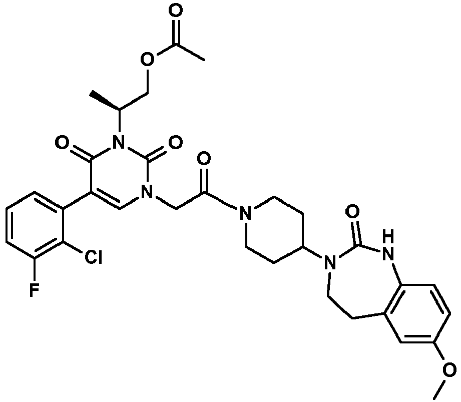

De manière analogue à l'exemple 115.12, à partir de 80mg (0,1 mmoles) de 5-(2-chloro-3-fluoro-phényl)-3-((S)-2-hydroxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (préparé comme dans l'exemple 122.2), 62mg (71%) de (S)-2-(5-(2-chloro-3-fluoro-phényl)-3-{2-[4-(7-méthoxy-2-oxo-1,2,4,5-tétrahydro-benzo[d][1,3]diazépin-3-yl)-pipéridin-1-yl]-2-oxo-éthyl}-2,6-dioxo-3,6-dihydro-2H-pyrimidin-1-yl)-acétate de propyle sont obtenus sous la forme d'un solide blanc de point de fusion 193°C.
RMN ¹H (δ, DMSO) : 1.39 (d, J = 6.9 Hz, 3H), 1.55 (m, 1H), 1.70 (m, 3H), 1.97 (s, 3H), 2.68 (m, 1H), 2.78-2.93 (m, 2H), 3.14 (m, 1H), 3.34-3.43 (m, 2H), 3.67 (s, 3H), 3.93 (d, J = 13.7 Hz, 1H), 4.18 - 4.38 (m, 2H), 4.46 (m, 2H), 4.66-4.85 (m, 2H), 5.18 (q, J = 7.2 Hz, 1H), 6.62 (d, J = 3.0 Hz, 1H), 6.67 (dd, J = 8.7, 2.9 Hz, 1H), 6.98 (d, J = 8.8 Hz, 1H), 7.22 (m, 1H), 7.37-7.55 (m, 2H), 7.82 (s, 1H), 8.32 (s, 1H).

### Exemple 124 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 124) (example comparatif)

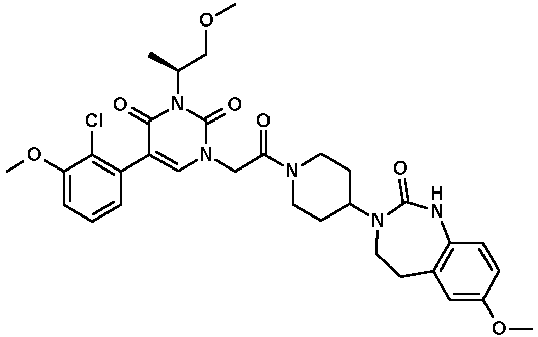

### 124.1 : [5-bromo-3-((S)-2-méthoxy-1-méthyl-éthyl)-2,4-dioxo-3,4-dihydro-2H-pyrimidin-1-yl]-acétate de méthyle

De manière analogue à l'exemple 2.5, à partir de 3g (11,4 mmoles) de (5-bromo-2,4-dioxo-3,4-dihydro-2H-pyrimidin-1-yl)-acétate de méthyle (préparé comme dans l'exemple 13.1) et 1,2ml (12,6 mmoles) de (R)-1-méthoxy-propan-2-ol , et après purification par chromatographie sur gel de silice élué par un mélange d'acétate d'éthyle dans l'heptane, suivant un gradient de polarité (de 30% à 50% d'acétate d'éthyle dans l'heptane), 2,6g (67%) de [5-bromo-3-((S)-2-méthoxy-1-méthyl-éthyl)-2,4-dioxo-3,4-dihydro-2H-pyrimidin-1-yl]-acétate de méthyle sont obtenus sous la forme d'une huile incolore.

### 124.2 : Acide [5-(2-chloro-3-méthoxy-phényl)-3-((S)-2-méthoxy-1-méthyl-éthyl)-2,4-dioxo-3,4-dihydro-2H-pyrimidin-1-yl]-acétique

759mg (7,2 mmoles) de carbonate de sodium et 979mg (5,3 mmoles) d'acide 2-chloro-3-méthoxyphénylboronique sont additionnés sur une solution contenant 800mg (2,4 mmoles) de [5-bromo-3-((S)-2-méthoxy-1-méthyl-éthyl)-2,4-dioxo-3,4-dihydro-2H-pyrimidin-1-yl]-acétate de méthyle dans 80ml de 1,4-dioxanne et 8ml d'eau. Le milieu est dégazé à l'azote pendant 15 minutes, puis 194mg (0,2 mmoles) de complexe de dichlorométhane de 1,1'-bis-(diphénylphosphino)ferrocène-dichloropalladium(II) sont rajoutés et le milieu est chauffé à 100°C pendant 2 heures. Après refroidissement, 3,6ml d'hydroxyde de lithium monohydraté et 16ml d'eau sont rajoutés au millieu réactionnel qui est laissé toute une nuit. De l'eau et de l'acétate d'éthyle sont ajoutés au milieu réactionnel. La phase aqueuse est acidifiée à pH4 avec une solution aqueuse d'acide chlorhydrique 1N. Cette phase est extraite deux fois avec de l'acétate d'éthyle. La phase organique est lavée par de l'eau puis par une solution aqueuse de chlorure de sodium saturée et séchée sur sulfate de sodium anhydre, filtrée et concentrée à sec. 912mg (100%) d'acide[5-(2-chloro-3-méthoxy-phényl)-3-((S)-2-méthoxy-1-méthyl-éthyl)-2,4-dioxo-3,4-dihydro-2H-pyrimidin-1-yl]-acétique sont obtenus sous la forme d'une huile marron.

### 124.3 : 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl} -1H-pyrimidine-2,4-dione (composé 124)

De manière analogue à l'exemple 115.10, à partir de 415mg (1,1 mmoles) d'acide [5-(2-chloro-3-méthoxy-phényl)-3-((S)-2-méthoxy-1-méthyl-éthyl)-2,4-dioxo-3,4-dihydro-2H-pyrimidin-1-yl]-acétique et 328mg (1,2 mmoles) de 7-méthoxy-3-pipéridin-4-yl-1,3,4,5-tétrahydro-benzo[d][1,3]diazépin-2-one (préparé comme dans l'exemple 115.6), 180mg (26%) de 5-(2-chloro-3-méthoxy-phényl)-3-((S)-2-méthoxy-1-méthyl-éthyl)-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide blanc de point de fusion 269°C.
RMN¹H (δ, DMSO): 1.33 (d, J = 6.9 Hz, 3H); 1.55-1.69 (m, 4H); 2.67-2.68 (m, 1H); 2.87 (m, 2H); 3.13 (m, 1H); 3.23 (s, 3H); 3.35-3.37 (m, 2H); 3.52-3.56 (m, 1H); 3.67 (s, 3H); 3.89 (s, 3H), 3.91 (m, 2H); 4.28 (m, 1H); 4.41-4.44 (m, 1H); 4.73 (m, 2H); 5.14 (m, 1H); 6.62 (d, J = 2.9 Hz, 1H); 6.67 (dd, J = 8.8, 2.9 Hz, 1H); 6.91 (dd, J = 7.6, 1.4 Hz, 1H); 6.98 (d, J = 8.8 Hz, 1H); 7.18 (dd, J = 8.4, 1.4 Hz, 1H); 7.35 (m, 1H); 7.69 (s, 1H); 8.33 (s, 1H).

Les composés suivants ont été synthétisés selon un mode opératoire analogue, à partir des réactifs appropriés commerciaux ou préalablement préparés:

### composé 125: 5-(2-chloro-3-fluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

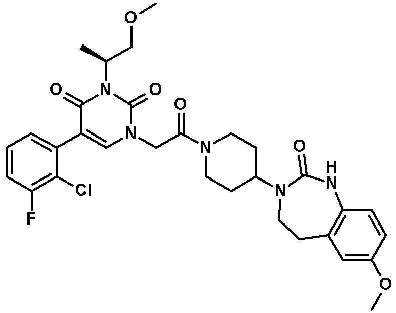

RMN ¹H (δ, DMSO) : 1.34 (d, J = 6.9 Hz, 3H); 1.52-1.80 (m, 4H); 2.68 (t, J = 12.4 Hz, 1H); 2.88 (m, 2H); 3.14 (t, J = 12.4 Hz, 1H); 3.24 (s, 3H); 3.35-3.37 (m, 2H); 3.52-3.56 (m, 1H); 3.67 (s, 3H); 3.92 (m, 2H); 4.28 (m, 1H); 4.43 (d, J = 12.8 Hz, 1H); 4.75 (s, 2H); 5.15 (m, 1H); 6.62-6.68 (m, 2H); 6.98 (d, J = 8.8 Hz, 1H); 7.20-7.22 (m, 1H); 7.43-7.47 (m, 2H); 7.80 (s, 1H); 8.33 (s, 1H).

### Composé 126: 5-(2,3-difluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

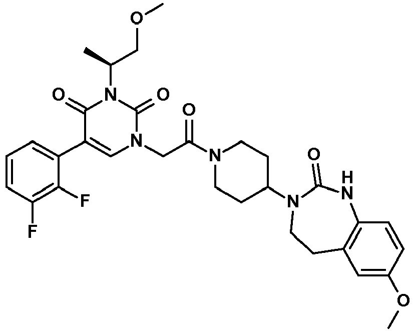

RMN ¹H (δ, DMSO) : 1.34 (d, J= 7.0 Hz, 3H) ; 1.52-1.70 (m, 4H); 2.68 (t, J = 12.5 Hz, 1H); 2.88 (m, 2H); 3.15 (t, J = 12.4 Hz, 1H); 3.23 (s, 3H); 3.36-3.38 (m, 2H); 3.54 (dd, J = 9.9, 5.9 Hz, 1H); 3.67 (s, 3H); 3.90-3.95 (m, 2H); 4.28 (m, 1H); 4.43 (d, J = 12.9 Hz, 1H); 4.77 (s, 2H); 5.16 (m, 1H); 6.63-6.69 (m, 2H) ; 6.98 (d, J = 8.8 Hz, 1H) ; 7.19-7.29 (m, 2H) ; 7.42-7.49 (m, 1H); 7.88 (s, 1H); 8.33 (s, 1H).

### Composé 127: 5-(2,3-dichloro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

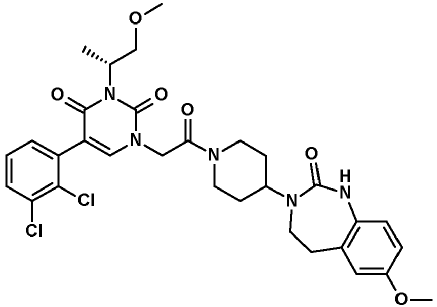

RMN ¹H (δ, DMSO) : 1.33 (d, J = 7.0 Hz, 3H) ; 1.54 (d, J = 14.1 Hz, 1H); 1.67 (d, J = 21.3 Hz, 3H); 2.68 (t, J = 12.4 Hz, 1H); 2.87 (m, 2H); 3.15 (d, J = 13.4 Hz, 1H); 3.23 (s, 3H); 3.36 (d, J = 6.2 Hz, 2H); 3.54 (dd, J = 9.9, 5.9 Hz, 1H); 3.67 (s, 3H); 3.90-3.95 (m, 2H); 4.28 (m, 1H); 4.43 (d, J = 12.8 Hz, 1H); 4.74 (d, J = 4.4 Hz, 2H); 5.14 (m, 1H); 6.62 (d, J = 2.9 Hz, 1H); 6.67 (dd, J = 8.8, 2.9 Hz, 1H); 6.98 (d, J = 8.8 Hz, 1H); 7.33 (dd, J = 7.6, 1.6 Hz, 1H); 7.42 (t, J = 7.9 Hz, 1H); 7.69 (dd, J = 8.0, 1.6 Hz, 1H); 7.79 (s, 1H); 8.34 (s, 1H) .

### Composé 128: 5-(2,3-difluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

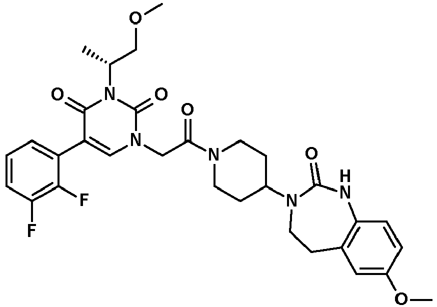

RMN ¹H (δ, DMSO) : 1.34 (d, J = 6.9 Hz, 3H); 1.53-1.56 (m, 1H); 1.65-1.71 (m, 3H); 2.68 (t, J = 12.5 Hz, 1H); 2.88 (d, J = 6.5 Hz, 2H); 3.14 (t, J = 10.2 Hz, 1H); 3.23 (s, 3H); 3.37 (m, 2H); 3.54 (dd, J = 9.9, 5.9 Hz, 1H); 3.67 (s, 3H); 3.94 (d, J = 11.6 Hz, 2H); 4.28 (m, 1H); 4.43 (d, J = 12.8 Hz, 1H); 4.77 (s, 2H); 5.17 (m, 1H); 6.63-6.68 (m, 2H); 6.98 (d, J = 8.8 Hz, 1H); 7.19-7.29 (m, 2H) ; 7.46 (q, J = 8.9 Hz, 1H) ; 7.89 (s, 1H); 8.33 (s, 1H).

### Composé 129: 5-(2,3-diméthoxy-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

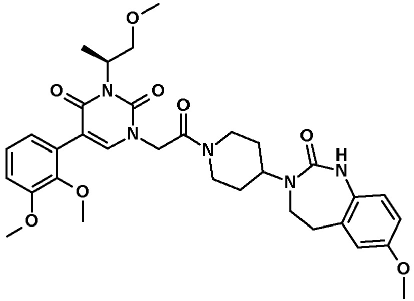

RMN ¹H (δ, DMSO) : 1.34 (d, J = 6.9 Hz, 3H); 1.49-1.58 (m, 1H); 1.62-1.79 (m, 3H); 2.64-2.70 (m, 1H); 2.86-2.88 (m, 2H); 3.13 (t, J = 12.5 Hz, 1H); 3.24 (s, 3H); 3.35-3.37 (m, 2H); 3.55 (dd, J = 9.9, 5.9 Hz, 1H); 3.68 (d, J = 4.4 Hz, 6H); 3.83 (s, 3H); 3.91-3.95 (m, 2H); 4.24-4.32 (m, 1H); 4.43 (d, J = 12.8 Hz, 1H); 4.73 (d, J = 3.6 Hz, 2H); 5.12-5.17 (m, 1H); 6.62 (d, J = 2.9 Hz, 1H); 6.67 (dd, J = 8.8, 2.9 Hz, 1H); 6.79 (t, J = 4.6 Hz, 1H); 6.98 (d, J = 8.8 Hz, 1H); 7.06-7.07 (m, 2H); 7.57 (s, 1H); 8.32 (s, 1H).

### Composé 130: 5-(2-chloro-3-méthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

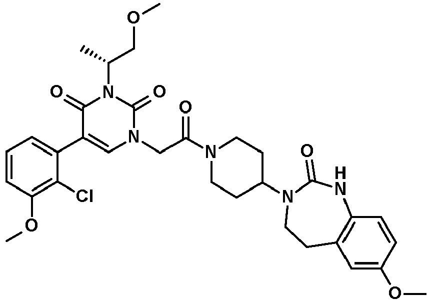

RMN ¹H (δ, DMSO) : 1.33 (d, J = 6.9 Hz, 3H); 1.55 (m, 1H); 1.70 (m, 3H); 2.67 (t, J = 11.9 Hz, 1H); 2.88 (d, J = 6.9 Hz, 2H); 3.13 (m, 1H); 3.23 (s, 3H); 3.36 (d, J = 6.6 Hz, 2H); 3.54 (dd, J = 9.9, 5.9 Hz, 1H) ; 3.67 (s, 3H); 3.89 (s, 3H); 3.93 (m, 2H); 4.28 (m, 1H); 4.43 (d, J = 12.8 Hz, 1H); 4.73 (m, 2H); 5.14 (m, 1H); 6.62-6.68 (m, 2H); 6.91 (dd, J = 7.6, 1.4 Hz, 1H); 6.98 (d, J = 8.8 Hz, 1H); 7.18 (dd, J = 8.4, 1.4 Hz, 1H); 7.35 (t, J = 8.0 Hz, 1H); 7.69 (s, 1H); 8.33 (s, 1H).

### Composé 131: 5-(2-chloro-3-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

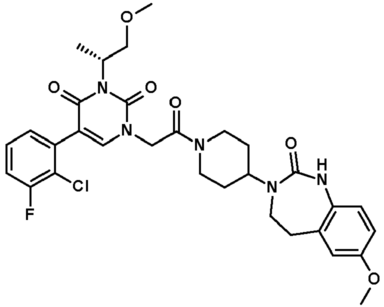

RMN ¹H (δ, DMSO) : 1.34 (d, J = 6.9 Hz, 3H); 1.53-1.56 (m, 1H); 1.65-1.70 (m, 3H); 2.68 (t, J = 12.5 Hz, 1H); 2.88 (m, 2H); 3.14 (m, 1H); 3.24 (s, 3H); 3.36 (d, J = 6.5 Hz, 2H); 3.54 (dd, J = 9.9, 5.9 Hz, 1H) ; 3.67 (s, 3H) ; 3.92 (t, J = 10.3 Hz, 2H); 4.28 (m, 1H); 4.43 (d, J = 12.8 Hz, 1H); 4.75 (s, 2H); 5.15 (m, 1H); 6.62-6.68 (m, 2H); 6.98 (d, J = 8.8 Hz, 1H); 7.20-7.22 (m, 1H); 7.44-7.47 (m, 2H); 7.80 (s, 1H); 8.33 (s, 1H).

### Composé 132: 5-(2,3-dichloro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

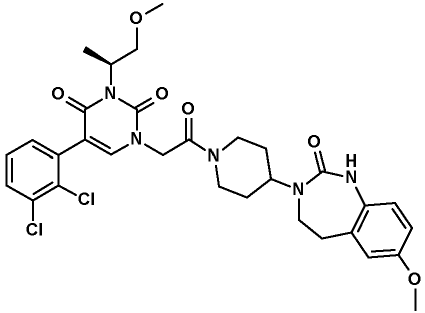

RMN ¹H (δ, DMSO) : 1.32 (d, J= 6.9 Hz, 3H); 1.49-1.58 (m, 1H); 1.63-1.74 (m, 3H); 2.64-2.70 (m, 1H); 2.85-2.87 (m, 2H); 3.10-3.16 (m, 1H) ; 3.22 (s, 3H); 3.34-3.36 (m, 2H); 3.53 (dd, J = 9.9, 5.9 Hz, 1H); 3.66 (s, 3H); 3.89-3.95 (m, 2H); 4.22-4.29 (m, 1H); 4.42 (d, J = 12.8 Hz, 1H); 4.72-4.73 (m, 2H); 5.08-5.15 (m, 1H); 6.61 (d, J = 2.9 Hz, 1H); 6.66 (dd, J = 8.8, 2.9 Hz, 1H); 6.97 (d, J = 8.8 Hz, 1H); 7.32 (dd, J = 7.7, 1.6 Hz, 1H); 7.41 (t, J = 7.9 Hz, 1H); 7.68 (dd, J = 8.0, 1.6 Hz, 1H); 7.77 (s, 1H); 8.32 (s, 1H).

### Composé 133: 5-(2,3-diméthoxy-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

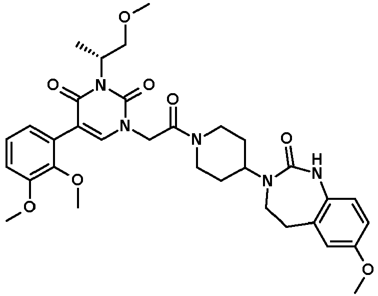

RMN ¹H (δ, DMSO) : 1.34 (d, J = 6.9 Hz, 3H); 1.54 (d, J = 13.8 Hz, 1H) ; 1.67 (br d, J = 19. 0 Hz, 3H) ; 2.68 (m, 1H) ; 2.87 (m, 2H); 3.13 (t, J = 12.5 Hz, 1H); 3.24 (s, 3H); 3.36 (d, J = 6.3 Hz, 2H); 3.55 (dd, J = 9.9, 5.9 Hz, 1H); 3.68 (d, J = 4.4 Hz, 6H); 3.83 (s, 3H); 3.93 (t, J = 9.3 Hz, 2H); 4.28 (m, 1H); 4.43 (d, J = 12.8 Hz, 1H); 4.73 (s, 2H); 5.15 (m, 1H); 6.62-6.68 (m, 2H); 6.79 (t, J = 4.6 Hz, 1H); 6.98 (d, J = 8.8 Hz, 1H); 7.07 (d, J = 4.7 Hz, 2H); 7.57 (s, 1H); 8.33 (s, 1H) .

### Composé 134: 5-(3-bromo-2-fluoro-phényl)-3-((R)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

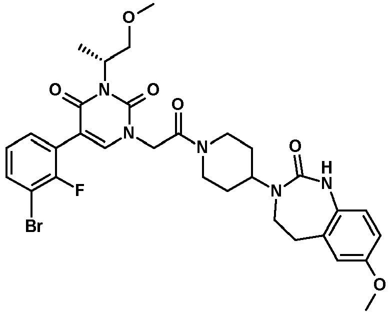

RMN ¹H (δ, DMSO) : 1.21-1.46 (m, 3H), 1.48-1.81 (m, 4H), 2.63-2.74 (m, 1H), 2.83-2.95 (m, 2H), 3.14 (t, J = 11.8 Hz, 1H), 3.23 (s, 3H), 3.34-3.47 (m, 2H), 3.54 (dd, J = 9.9, 5.9 Hz, 1H), 3.67 (s, 3H), 3.85-3.99 (m, 2H), 4.20-4.34 (m, 1H), 4.43 (d, J = 12.9, 1H), 4.76 (s, 2H), 5.07-5.27 (m, 1H), 6.53-6.77 (m, 2H), 6.98 (d, J = 8.8 Hz, 1H), 7.22 (td, J = 7.8, 0.8 Hz, 1H), 7.40 (ddd, J=8.2, 6.7, 1.7 Hz, 1H), 7.72 (ddd, J=8.2, 6.7, 1.7 Hz, 1H), 7.87 (s, 1H), 8.32 (s, 1H).

### Exemple 135 : 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 135) (example comparatif)

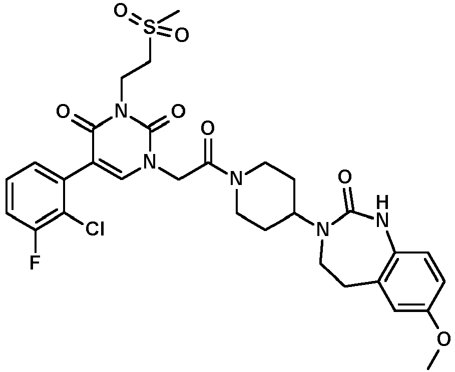

### 135.1 : Acide [5-(2-chloro-3-fluoro-phényl)-3-(2-méthylsulfanyl-éthyl)-2,4-dioxo-3,4-dihydro-2H-pyrimidin-1-yl]-acétique

De manière analogue à l'exemple 115.8, à partir de 300mg (0,9 mmoles) de [5-bromo-3-(2-méthylsulfanyl-éthyl)-2,4-dioxo-3,4-dihydro-2H-pyrimidin-1-yl]-acétate de méthyle (préparé comme dans l'exemple 112.1) et 171mg (1,0 mmoles) d'acide 2-chloro-3-fluorophénylboronique, 320mg (96%) d' acide [5-(2-chloro-3-fluoro-phényl)-3-(2-méthylsulfanyl-éthyl)-2,4-dioxo-3,4-dihydro-2H-pyrimidin-1-yl]-acétique sont obtenus sous la forme d'un solide marron.

### 135.2 : 5-(2-chloro-3-fluoro-phényl)-1-{2-[4-(7-méthoxy-2-oxo-1,2,4,5-tétrahydro-benzo [d][1,3]diazépin-3-yl)-pipéridin-1-yl]-2-oxo-éthyl}-3-(2-méthylsulfanyl-éthyl)-1H-pyrimidine-2,4-dione

De manière analogue à l'exemple 115.10, à partir de 284mg (1,0 mmoles) de 7-méthoxy-3-pipéridin-4-yl-1,3,4,5-tétrahydro-benzo[d] [1,3]diazépin-2-one (préparé comme dans l'exemple 115.6) et 320mg (0,9 mmoles) d' acide [5-(2-chloro-3-fluoro-phényl)-3-(2-méthylsulfanyl-éthyl)-2,4-dioxo-3,4-dihydro-2H-pyrimidin-1-yl]-acétique, et après purification par chromatographie sur gel de silice élué par un mélange dichlorométhane/méthanol 97/3, 325mg (60%) de 5-(2-chloro-3-fluoro-phényl)-1-{2-[4-(7-méthoxy-2-oxo-1,2,4,S-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione sont obtenus sous la forme d'un solide blanc.

### 135.3: 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (composé 135)

Un mélange contenant 71mg (0,06 mmoles) de molybdate d'ammonium tétrahydraté dans 311mg (9,2 mmoles) de péroxyde d'hydrogène est additionné sur une solution contenant 240mg (0,4 mmoles) de 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione dans 4,8ml d'éthanol. Après agitation à température ambiante pendant 46 heures, 5ml de dichlorométhane sont additionnés et le milieu réactionnel est agité à température ambiante pendant 48 heures de plus. Il est ensuite hydrolysé par une solution aqueuse saturée d'hydrogénocarbonate de sodium et le produit est extrait au dichlorométhane. La phase organique est lavée deux fois par une solution aqueuse à 10% de thiosulfate de sodium et une fois par de l'eau, séchée sur sulfate de magnésium, filtrée et concentrée sous vide. Le produit brut est chromatographié sur gel de silice élué par un mélange dichlorométhane /méthanol 97/3, le solide blanc obtenu est repris dans 2ml d'acétate d'éthyle et lentement agité à température ambiante pendant 16 heures.La suspension obtenue est filtrée et rincée une fois avec 2ml d'ether diéthylique, puis séchée sous vide. 150mg (59%) de 5-(2-chloro-3-fluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione sont obtenus sous la forme d'un solide blanc de point de fusion 212°C.
RMN ¹H (δ, DMSO): 1.45-1.61 (m, 1H), 1.70 (m, 3H), 2.68 (m, 1H), 2.80-2.93 (m, 2H), 3.08 (s, 3H), 3.14 (m, 1H), 3.36 (m, 2H), 3.41 (t, J = 7.3 Hz, 2H), 3.67 (s, 3H), 3.95 (d, J = 13.8 Hz, 1H), 4.31 (m, 3H), 4.42 (d, J = 12.9 Hz, 1H), 4.80 (m, 2H), 6.62 (d, J = 3.0 Hz, 1H), 6.67 (dd, J = 8.7, 3.0 Hz, 1H), 6.98 (d, J = 8.8 Hz, 1H), 7.15-7.28 (m, 1H), 7.37-7.54 (m, 2H), 7.88 (s, 1H), 8.35 (s, 1H).

Les composés suivants ont été synthétisés selon un mode opératoire analogue, en utilisant les réactifs appropriés commerciaux ou préalablement préparés:

### composé 136: 5-(2,3-difluoro-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

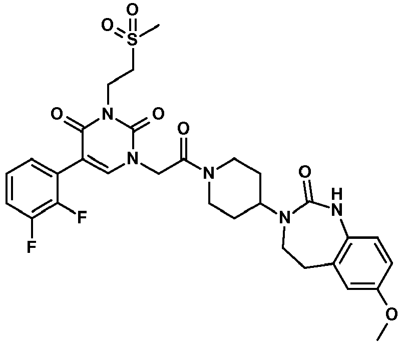

RMN ¹H (δ, DMSO) : 1.51-1.70 (m, 4H); 2.68 (t, J = 12.4 Hz, 1H); 2.88 (m, 2H); 3.09 (s, 3H); 3.15 (t, J = 12.5 Hz, 1H); 3.36-3.44 (m, 4H); 3.67 (s, 3H); 3.96 (d, J = 13.5 Hz, 1H); 4.31 (m, 3H); 4.42 (d, J = 12.7 Hz, 1H); 4.81 (s, 2H); 6.63-6.69 (m, 2H) ; 6.98 (d, J = 8.8 Hz, 1H) ; 7.22-7.31 (m, 2H) ; 7.44-7.51 (m, 1H); 7.97 (s, 1H); 8.35 (s, 1H).

### Composé 137: 5-(2,3-dichlorophényl)-1-[2-[4-(7-méthoxy-2-oxo-4,5-dihydro-1H-1,3-benzodiazépin-3-yl)-1-pipéridyl]-2-oxo-éthyl]-3-(2-méthylsulfonyléthyl)pyrimidine-2,4-dione (example comparatif)

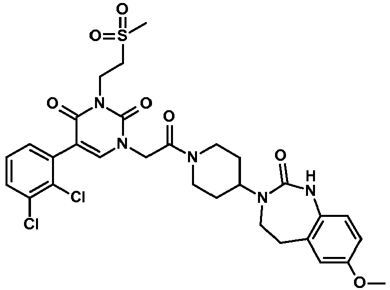

RMN ¹H (δ, DMSO) : 1.52-1.70 (m, 4H); 2.68 (t, J = 12.2 Hz, 1H); 2.88 (m, 2H); 3.08 (s, 3H); 3.15 (m, 1H); 3.36 (m, 2H); 3.41 (t, J = 7.2 Hz, 2H); 3.67 (s, 3H); 3.95 (d, J = 13.4 Hz, 1H); 4.29-4.33 (m, 3H); 4.43 (d, J = 12.7 Hz, 1H); 4.79 (s, 2H); 6.62-6.68 (m, 2H); 6.98 (d, J = 8.8 Hz, 1H); 7.33 (dd, J = 7.7, 1.6 Hz, 1H); 7.44 (t, J = 7.9 Hz, 1H); 7.70 (dd, J = 8.1, 1.6 Hz, 1H); 7.87 (s, 1H); 8.33 (s, 1H).

### Composé 138: 5-(2,3-diméthyl-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

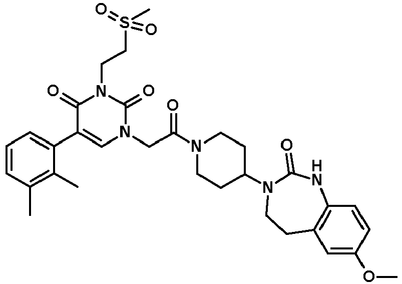

RMN ¹H (δ, DMSO) : 1.56 (m, 1H), 1.70 (m, 3H), 2.07 (s, 3H), 2.28 (s, 3H), 2.71 (m, 1H), 2.87 (m, 2H), 3.08 (s, 3H), 3.14 (m, 1H), 3.34 - 3.38 (m, 2H), 3.41 (t, J = 7.1 Hz, 2H), 3.67 (s, 3H), 3.95 (d, J = 13.7 Hz, 1H), 4.29 (m, 3H), 4.43 (d, J = 13.0 Hz, 1H), 4.77 (m, 2H), 6.62 (d, J = 2.9 Hz, 1H), 6.67 (dd, J = 8.7, 2.9 Hz, 1H), 6.97 (m, 2H), 7.11 (t, J = 7.5 Hz, 1H), 7.19 (d, J = 7.4 Hz, 1H), 7.63 (s, 1H), 8.32 (s, 1H) .

### Composé 139: 5-(2,3-diméthoxy-phényl)-3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

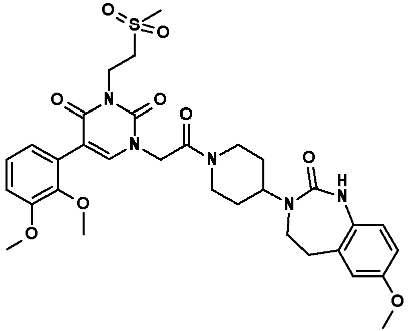

RMN ¹H (δ, DMSO) : 1.50-1.80 (m, 4H); 2.68 (m, 1H); 2.88 (m, 2H); 3.08 (s, 3H); 3.14 (t, J = 13.0 Hz, 1H); 3.36 (m, 2H); 3.41 (t, J = 7.2 Hz, 2H); 3.68 (d, J = 3.5 Hz, 6H); 3.84 (s, 3H); 3.95 (d, J= 13.4 Hz, 1H); 4.29-4.33 (m, 3H); 4.43 (d, J = 12.7 Hz, 1H); 4.78 (s, 2H) ; 6.62 (d, J = 2.9 Hz, 1H) ; 6.67 (dd, J = 8.8, 2.9 Hz, 1H); 6.81 (dd, J = 5.3, 3.9 Hz, 1H); 6.98 (d, J = 8.8 Hz, 1H); 7.08-7.09 (m, 2H); 7.67 (s, 1H); 8.32 (s, 1H).

### Composé 140: 5-(2-chloro-3-méthoxy-phényl)-3-(2-methanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif) (example comparatif)

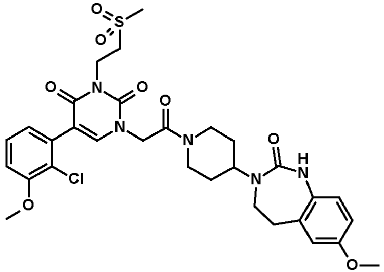

RMN ¹H (δ, DMSO) : 1.55 (1 H, m), 1.68 (3 H, m), 2.68 (1 H, t, J = 12.29 Hz), 2.88 (2 H, m), 3.08 (3 H, s), 3.15 (1 H, t, J = 12Hz), 3.42-3.33 (4 H, m), 3.67 (3 H, s), 3.90 (3 H, s), 3.95 (1 H, d, J = 14.37 Hz), 4.30 (3 H, t, J = 7.38 Hz), 4.43 (1 H, d, J = 12.88 Hz), 4.78 (2 H, s), 6.68-6.62 (2 H, m), 6.93-6.91 (1 H, m), 6.98 (1 H, d, J = 8.78 Hz), 7.20 (1 H, d, J = 8.34 Hz), 7.36 (1 H, t, J = 7.98 Hz), 7.77 (1 H, s), 8.33 (1 H, s).

### Exemple 141 : 3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-5-méthyl-1H-pyrimidine-2,4-dione (schéma réactionnel n°3, composé 141) (example comparatif)

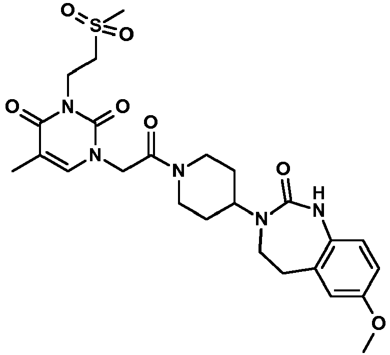

### 141.1: [5-bromo-3-(2-méthanesulfonyl-éthyl)-2,4-dioxo-3,4-dihydro-2H-pyrimidin-1-yl]-acétate de méthyle

De manière analogue à l'exemple 135.3, à partir de 2,4g (7,1 mmoles) de [5-bromo-3-(2-méthylsulfanyl-éthyl)-2,4-dioxo-3,4-dihydro-2H-pyrimidin-1-yl]-acétate de méthyle (préparé comme dans l'exemple 112.1), 2,2g (82%) de [5-bromo-3-(2-méthanesulfonyl-éthyl)-2,4-dioxo-3,4-dihydro-2H-pyrimidin-1-yl]-acétate de méthyle sont obtenus sous la forme d'un solide blanc.

### 141.2: [3-(2-méthanesulfonyl-éthyl)-5-méthyl-2,4-dioxo-3,4-dihydro-2H-pyrimidin-1-yl]-acétate de méthyle

500mg (1,4 mmoles) de [5-bromo-3-(2-méthanesulfonyl-éthyl)-2,4-dioxo-3,4-dihydro-2H-pyrimidin-1-yl]-acétate de méhyle, 562mg (4,1 mmoles) de carbonate de potassium et 0,2ml (1,4 mmoles) de triméthylboroxine sont mis en solution dans 5ml de dioxanne. Le mélange est dégazé avec de l'azote pendant 5 minutes, puis 111mg (0,1 mmoles) de complexe de dichlorométhane de 1,1'-bis-(diphénylphosphino)ferrocène-dichloropalladium(II) sont rajoutés. Le milieu réactionnel est chauffé à 100°C pendant 1 heure. Le mélange est versé sur de l'eau puis extrait deux fois par de l'acétate d'éthyle. La phase organique est séchée sur sulfate de sodium anhydre puis filtrée et concentrée à sec. Après purification par chromatographie sur gel de silice élué par un mélange de méthanol dans le dichlorométhane, suivant un gradient de polarité (de 0% à 10% de méthanol dans le dichlorométhane), 170mg (41%) de [3-(2-méthanesulfonyl-éthyl)-5-méthyl-2,4-dioxo-3,4-dihydro-2H-pyrimidin-1-yl]-acétate de méthyle sont obtenus sous le forme d'huile brune.

### 141.3: Acide [3-(2-méthanesulfonyl-éthyl)-5-méthyl-2,4-dioxo-3,4-dihydro-2H-pyrimidin-1-yl]-acétique

De manière analogue à l'exemple 115.9, à partir de 170mg (0,6 mmoles) de [3-(2-méthanesulfonyl-éthyl)-5-méthyl-2,4-dioxo-3,4-dihydro-2H-pyrimidin-1-yl]-acétate de méthyle, 130mg (80%) d'acide [3-(2-méthanesulfonyl-éthyl)-5-méthyl-2,4-dioxo-3,4-dihydro-2H-pyrimidin-1-yl]-acétique sont obtenus sous la forme d'une huile marron.

### 141.4: 3-(2-méthanesulfonyl-éthyl)-1-{2-[4-(7-méthoxy-2-oxo-1,2, 4,5-tétrahydro-benzo [d][1,3]diazépin-3-yl)-pipéridin-1-yl]-2-oxo-éthyl}-5-méthyl-1H-pyrimidine-2,4-dione (composé 141)

De manière analogue à l'exemple 115.10, à partir de 136mg (0,5 mmoles) de 7-méthoxy-3-pipéridin-4-yl-1,3,4,5-tétrahydro-benzo[d][1,3]diazépin-2-one (préparé comme dans l'exemple 115.6) et 130mg (0,5 mmoles) d'acide [3-(2-méthanesulfonyl-éthyl)-5-méthyl-2,4-dioxo-3,4-dihydro-2H-pyrimidin-1-yl]-acétique, et après purification par chromatographie sur gel de silice élué par un mélange acétonitrile/eau 24/76, 11mg (4%) de 3-(2-méthanesulfonyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-5-méthyl-1H-pyrimidine-2,4-dione sont obtenus sous la forme d'une huile marron.
RMN ¹H (δ, chloroforme): 1.87 (m, 4H), 1.97 (s, 3H), 2.73 (t, J = 12.6 Hz, 1H), 3.00 (m, 2H), 3.06 (s, 3H), 3.27 (t, J = 12.8 Hz, 1H), 3.40 (t, J = 6.9 Hz, 2H), 3.48 (d, J = 8.9 Hz, 2H), 3.78 (s, 3H), 3.93 (d, J = 13.4 Hz, 1H), 4.35 (d, J = 15.8 Hz, 1H), 4.46 (t, J = 7 Hz, 3H), 4.71 (d, J = 13.4 Hz, 1H), 4.81 (d, J = 15.9 Hz, 1H), 6.63 (t, J = 1.5 Hz, 1H), 6.66 - 6.77 (m, 3H), 7.02 (d, J = 1.8 Hz, 1H).

### Exemple 142: 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 142) (example comparatif)

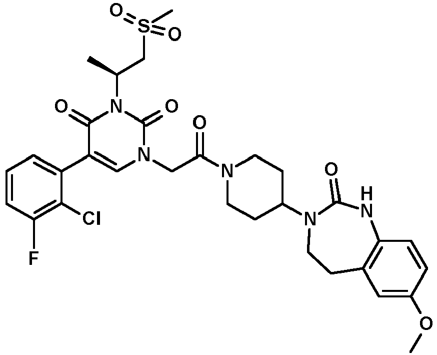

### 142.1: (R)-1-méthylsulfanyl-propan-2-ol

4,8ml (69 mmoles) de (R)-(+)-propylène-1,2 oxyde sont rajoutés goutte à goutte sur une solution contenant 7,2g (103,3 mmoles) de méthanethiolate de sodium dans 32ml d'acétonitrile. Le mélange est agité en tube scellé pendant 4 heures à 80°C puis dilué par de l'eau et par du dichlorométhane. La phase aqueuse est extraite trois fois au dichlorométhane, la phase organique est lavée par l'eau puis à la saumure. Après séchage sur sulfate de magnésium, la solution est filtrée puis concentrée sous vide. 3,5g (48%) de (R)-1-méthylsulfanyl-propan-2-ol sont obtenus sous la forme d'une huile ambrée.

### 142.2: [5-bromo-3-((S)-1-méthyl-2-méthylsulfanyl-éthyl)-2, 4-dioxo-3,4-dihydro-2H-pyrimidin-1-yl]-acétate de méthyle

De manière analogue à l'exemple 121.1, à partir de 3,7g (14,1 mmoles) de (5-bromo-2,4-dioxo-3,4-dihydro-2H-pyrimidin-1-yl)-acétate de méthyle (préparé comme dans l'exemple 13.1) et 1,5g (14,1 mmoles) de (R)-1-méthylsulfanyl-propan-2-ol, 1,6g (32%) de [5-bromo-3-((S)-1-méthyl-2-méthylsulfanyl-éthyl)-2, 4-dioxo-3,4-dihydro-2H-pyrimidin-1-yl]-acétate de méthyle sont obtenus sous la forme d'une huile visqueuse.

### 142.3: [5-(2-chloro-3-fluoro-phényl)-3-((S)-1-méthyl-2-méthylsulfanyl-éthyl)-2,4-dioxo-3,4-dihydro-2H-pyrimidin-1-yl]-acétate de méthyle

De manière analogue à l'exemple 115.8, à partir de 1,6g (4,6 mmoles) de [5-bromo-3-((S)-1-méthyl-2-méthylsulfanyl-éthyl)-2,4-dioxo-3,4-dihydro-2H-pyrimidin-1-yl]-acétate de méthyle et 1,6g (9,1 mmoles) d'acide 2-chloro-3-fluorophénylboronique, 1,7g (93%) de [5-(2-chloro-3-fluoro-phényl)-3-((S)-1-méthyl-2-méthylsulfanyl-éthyl)-2,4-dioxo-3,4-dihydro-2H-pyrimidin-1-yl]-acétate de méthyle sont obtenus sous la forme d'un solide marron.

### 142.4: [5-(2-chloro-3-fluoro-phényl)-3-((S)-2-méthanesulfonyl-1-méthyl-éthyl)-2,4-dioxo-3,4-dihydro-2H-pyrimidin-1-yl]-acétate de méthyle

1,7g (4,2 mmoles) de 5-(2-chloro-3-fluoro-phényl)-3-((S)-1-méthyl-2-méthylsulfanyl-éthyl)-2,4-dioxo-3,4-dihydro-2H-pyrimidin-1-yl]-acétate de méthyle sont additionnés sur un mélange contenant 262mg (0,2 mmoles) de molybdate d'ammonium tétrahydraté dans 4,3ml (42,4 mmoles) de solution aqueuse de péroxyde d'hydrogène à 30% à 0°C. Après 2 heures d'agitation à température ambiante, le mélange est hydrolysé avec 30ml d'eau puis extrait par de l'acétate d'éthyle. La phase organique est lavée par une solution aqueuse de sulfate de sodium, par de l'eau puis brine, séchée sur sulfate de magnésium anhydre et concentrée sous vide. 1,8g (96%) de [5-(2-chloro-3-fluoro-phényl)-3-((S)-2-méthanesulfonyl-1-méthyl-éthyl)-2,4-dioxo-3,4-dihydro-2H-pyrimidin-1-yl]-acétate de méthyle sont obtenus sous la forme d'un solide.

### 142.5: Acide 5-(2-chloro-3-fluoro-phényl)-3-( (S)-2-méthanesulfonyl-1-méthyl-éthyl)-2,4-dioxo-3,4-dihydro-2H-pyrimidin-1-yl]-acétique

1,8g (4,2 mmoles) de [5-(2-chloro-3-fluoro-phényl)-3-((R)-2-méthanesulfonyl-1-méthyl-éthyl)-2,4-dioxo-3,4-dihydro-2H-pyrimidin-1-yl]-acétate de méthyle sont ajoutés à 20ml de tétrahydrofuranne et 2,8ml (5,5 mmoles) de solution aqueuse d'hydroxyde de sodium 1N. Après 2 heures, le mélange est dilué par de l'acétate d'éthyle et par de l'eau, la phase aqueuse est portée à pH 3 avec une solution aqueuse d'acide chlorhydrique 1N. Cette phase est ensuite extraite trois fois par de l'acétate d'éthyle, la phase organique est lavée à l'eau puis brine. Après séchage sur sulfate de magnésium anhydre, la solution est filtrée puis concentrée sous vide. 1,4g (80%) d'acide 5-(2-chloro-3-fluoro-phényl)-3-((S)-2-méthanesulfonyl-1-méthyl-éthyl)-2,4-dioxo-3,4-dihydro-2H-pyrimidin-1-yl]-acétique sont obtenus sous la forme d'une huile.

### 142.6: 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl} -1H-pyrimidine-2,4-dione (composé 142)

De manière analogue à l'exemple 115.10, à partir de 1,4g (3,3 mmoles) d'acide [5-(2-chloro-3-fluoro-phényl)-3-((S)-2-méthanesulfonyl-1-méthyl-éthyl)-2,4-dioxo-3,4-dihydro-2H-pyrimidin-1-yl]-acétique et 1g (3,7 mmoles) de 7-méthoxy-3-pipéridin-4-yl-1,3,4,5-tétrahydro-benzo[d] [1,3]diazépin-2-one (préparé comme dans l'exemple 115.6), 720mg (31%) de 5-(2-chloro-3-fluoro-phényl)-3-((S)-2-méthanesulfonyl-1-méthyl-éthyl)-1-{2-[4-(7-méthoxy-2-oxo-1,2,4,5-tétrahydro-benzo[d] [1,3]diazépin-3-yl)-pipéridin-1-yl]-2-oxo-éthyl} -1H-pyrimidine-2,4-dione sont obtenus sous la forme d'un solide blanc.
RMN ¹H (δ, DMSO): 1.23 (d, J = 6.9 Hz, 3H), 1.44 - 1.83 (m, 4H), 2.67 (dd, J = 3.5, 1.8 Hz, 1H), 2.87 (d, J = 7.6 Hz, 2H), 3.05 (s, 3H), 3.09-3.21 (m, 1H), 3.32 - 3.40 (m, 2H), 3.52 (br s, 1H), 3.66 (s, 3H), 3.93 (d, J = 13.2 Hz, 1H), 4.12-4.35 (m, 3H), 4.42 (d, J = 13.1 Hz, 1H), 4.79 (s, 2H), 6.54 - 6.73 (m, 2H), 6.97 (d, J = 8.8 Hz, 1H), 7.15-7.26 (m, 1H), 7.41-7.51 (m, 2H), 7.89 (s, 1H), 8.31 (s, 1H).

Les composés suivants ont été synthétisés selon un mode opératoire analogue, en utilisant les réactifs appropriés commerciaux ou préalablement préparés:

### composé 143: 5-(2-chloro-3-fluoro-phényl)-3-((R)-2-méthanesulfonyl-1-méthyl-ethyl)-1-{2-[4-(7-méthoxy-2-oxo-1,2, 4,5-tétrahydro-benzo [d][1,3]diazépin-3-yl)-pipéridin-1-yl]-2-oxo-éthyl}-1H-pyrimidine-2,4-dione (example comparatif)

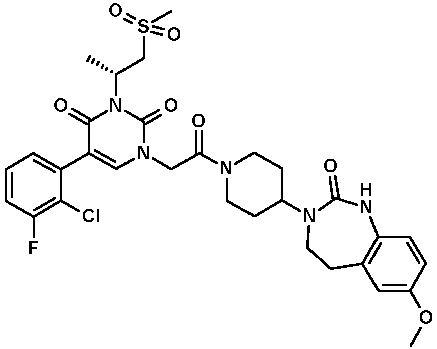

RMN ¹H (δ, DMSO) : 1.24 (d, J = 7.0 Hz, 3 H) ; 1.55 (m, 1 H) ; 1.80-1.60 (m, 3 H); 2.68 (t, J = 12.5 Hz, 1 H) ; 2.88 (m, 2 H); 3.06 (s, 3 H); 3.15 (t, J = 12.5 Hz, 1 H); 3.40-3.32 (m, 2 H); 3.53 (m, 1 H); 3.67 (s, 3 H); 3.94 (d, J = 13.5 Hz, 1 H); 4.28-4.22 (m, 3 H); 4.43 (d, J = 12.8 Hz, 1 H); 4.80 (s, 2 H); 6.68-6.62 (m, 2 H); 6.98 (d, J = 8.8 Hz, 1 H); 7.23 (dd, J = 6.3, 2.8 Hz, 1 H); 7.48-7.45 (m, 2 H); 7.90 (s, 1 H); 8.32 (s, 1 H).

### Composé 144: 5-(2,3-dichloro-phényl)-3-((S)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

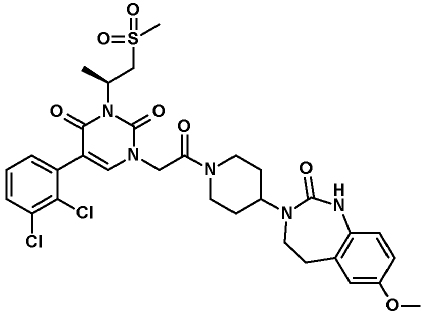

RMN ¹H (δ, DMSO) : 1.24 (d, J = 7.02 Hz, 3H), 1.42 - 1.83 (m, 4H), 2.62 - 2.77 (m, 1H), 2.83 - 2.96 (m, 2H), 3. 06 (s, 3H), 3.10 - 3.23 (m, 1H), 3.33 - 3.39 (m, 2H), 3.52 (dt, J = 7.17, 11.03 Hz, 1H), 3.67 (s, 3H), 3.94 (d, J = 13.53 Hz, 1H), 4.13 - 4.38 (m, 3H), 4.43 (d, J = 12.91 Hz, 1H), 4.79 (s, 2H), 6.62 (d, J = 2.91 Hz, 1H), 6.67 (dd, J = 2.97, 8.78 Hz, 1H), 6. 98 (d, J = 8.78 Hz, 1H), 7.35 (dd, J = 1.59, 7.68 Hz, 1H), 7.44 (t, J = 7.85 Hz, 1H), 7.71 (dd, J = 1.61, 8.03 Hz, 1H), 7.89 (s, 1H), 8.33 (s, 1H).

### Composé 145: 5-(2,3-dichloro-phényl)-3-((R)-2-méthanesulfonyl-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

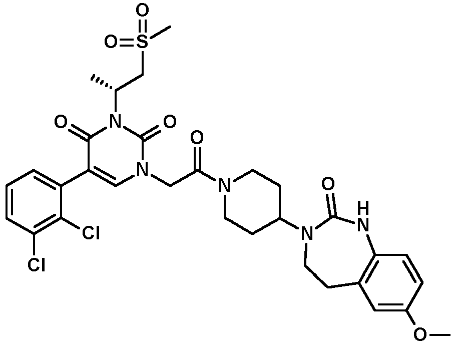

RMN ¹H (δ, DMSO) : 1.23 (d, J = 7.02 Hz, 3H), 1.40 - 1.81 (m, 4H), 2.61 - 2.75 (m, 1H), 2.77 - 2.96 (m, 2H), 3.05 (s, 3H), 3.09 - 3.28 (m, 1H), 3.32 - 3.41 (m, 2H), 3.45 - 3.61 (m, 1H), 3.67 (s, 3H), 3.94 (d, J = 13.4 Hz, 1H), 4.12 - 4.35 (m, 3H), 4.42 (d, J= 12.9 Hz, 1H), 4.79 (br s, 2H), 6.42 - 6.78 (m, 2H), 6.97 (d, J = 8.8 Hz, 1H), 7.34 (dd, J = 1.6, 7.6 Hz, 1H), 7.44 (t, J = 7.9 Hz, 1H), 7.70 (dd, J = 1.57, 8.1 Hz, 1H), 7.88 (s, 1H), 8.32 (s, 1H).

### Exemple 146: 5-(2-chloro-3-fluoro-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (schéma réactionnel n°3, composé 146) (example comparatif)

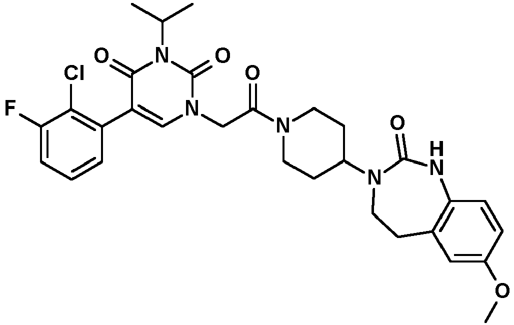

### 146.1: (5-bromo-3-isopropyl-2,4-dioxo-3,4-dihydro-2H-pyrimidin-1-yl)-acétate de méthyle

1,2g (29,7 mmoles) d'hydrure de sodium sont ajoutés par portion à une solution préalablement refroidie à 0°C de 6,5g (24,7 mmoles) de (5-bromo-2,4-dioxo-3,4-dihydro-2H-pyrimidin-1-yl)-acétate de méthyle (préparé comme dans l'exemple 13.1) dans 130ml de N,N-diméthylformamide. Le milieu réactionnel est agité pendant 5 minutes puis 6,3ml (66,7 mmoles) de 2-bromopropane sont additionnés. Le milieu réactionnel est chauffé à 50°C pendant 6 heures puis agité à température ambiante toute une nuit. Le mélange est hydrolysé puis dilué par de l'acétate d'éthyle. Le produit est extrait à l'acétate d'éthyle, la phase organique est lavée une fois par de l'eau et une fois par une solution aqueuse saturée de chlorure de sodium, puis séchée sur sulfate de magnésium, filtrée et concentrée sous vide. Après purification par chromatographie sur gel de silice élué par un mélange heptane/acétate d'éthyle 50/50, 3,5g (46%) de (5-bromo-3-isopropyl-2,4-dioxo-3,4-dihydro-2H-pyrimidin-1-yl)-acétate de méthyle sont obtenus sous la forme d'un solide blanc cassé.

### 146.2: [5-(2-chloro-3-fluoro-phényl)-3-isopropyl-2,4-dioxo-3,4-dihydro-2H-pyrimidin-1-yl]-acétate de méthyle

De manière analogue à l'exemple 115.8, à partir de 3,4g (19,7 mmoles) d'acide 2-chloro-3-fluorophénylboronique et 3,0g (9,8 mmoles) de 5-bromo-3-isopropyl-2,4-dioxo-3,4-dihydro-2H-pyrimidin-1-yl)-acétate de méthyle, 1,4g (34%) de [5-(2-chloro-3-fluoro-phényl)-3-isopropyl-2,4-dioxo-3,4-dihydro-2H-pyrimidin-1-yl]-acétate de méthyle sont obtenus sous la forme d'un solide beige.

### 146.3: Acide [5-(2-chloro-3-fluoro-phényl)-3-isopropyl-2,4-dioxo-3,4-dihydro-2H-pyrimidin-1-yl]-acétique

De manière analogue à l'exemple 142.5, à partir de 500mg (1,4 mmoles) de [5-(2-chloro-3-fluoro-phényl)-3-isopropyl-2,4-dioxo-3,4-dihydro-2H-pyrimidin-1-yl]-acétate de méthyle, 470mg (98%) d'acide [5-(2-chloro-3-fluoro-phényl)-3-isopropyl-2,4-dioxo-3,4-dihydro-2H-pyrimidin-1-yl]-acétique sont obtenus sous la forme d'une huile.

### 146.4: 5-(2-chloro-3-fluoro-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (composé 146)

De manière analogue à l'exemple 115.10, à partir de 427mg (1,6 mmoles) de 7-méthoxy-3-pipéridin-4-yl-1,3,4,5-tétrahydro-benzo[d] [1,3]diazépin-2-one (préparé comme dans l'exemple 115.6) et 480mg (1,4 mmoles) d'acide [5-(2-chloro-3-fluoro-phényl)-3-isopropyl-2,4-dioxo-3,4-dihydro-2H-pyrimidin-1-yl]-acétique, 684mg (81%) de 5-(2-chloro-3-fluoro-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione sont obtenus sous la forme d'un solide blanc de point de fusion 230°C.
RMN ¹H (δ, DMSO) : 1.39 (d, J = 6.9 Hz, 6 H); 1.51-1.69 (m, 4 H); 2.66 (m, 1 H); 2.85 (d, J = 6.7 Hz, 2 H); 3.12 (t, J = 11.9 Hz, 1 H); 3.36-3.33 (m, 2 H); 3.66 (s, 3 H); 3.89-3.93 (m, 1 H); 4.24-4.30 (m, 1 H); 4.41 (d, J = 12.8 Hz, 1 H); 4.72 (d, J = 6.4 Hz, 2 H); 5.05-5.12 (m, 1 H); 6.60 (d, J = 2.9 Hz, 1 H); 6.63-6.66 (m, 1 H); 6.96 (d, J = 8.8 Hz, 1 H); 7.20 (d, J = 2.6 Hz, 1 H); 7.41-7.45 (m, 2 H); 7.76 (s, 1 H); 8.33 (s, 1 H).

Les composés suivants ont été synthétisés selon un mode opératoire analogue, en utilisant les réactifs appropriés commerciaux ou préalablement préparés:

### composé 147: 5-(2,3-diméthoxy-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (example comparatif)

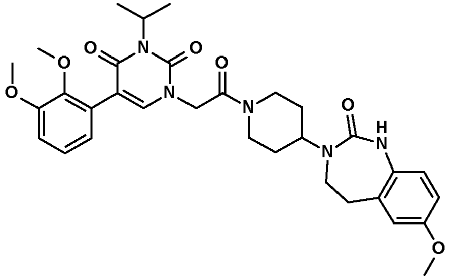

RMN ¹H (δ, DMSO) : 1.39 (6 H, d, J = 6.9 Hz); 1.56-1.47 (1 H, m) ; 1.74-1.62 (3 H, m) ; 2.65 (1 H, br t, J = 12.4 Hz); 2.86-2.84 (2 H, m); 3.11 (1 H, br t, J = 12.6 Hz); 3.35-3.32 (2 H, m); 3.65 (3 H, s); 3.67 (3 H, s); 3.81 (3 H, s); 3.91 (1 H, br d, J = 13.4 Hz); 4.26 (1 H, tt, J = 11.6 ,4.2 Hz); 4.41 (1 H, d, J = 12.9 Hz); 4.70-4.69 (2 H, m) ; 5.13-5.06 (1 H, m) ; 6.60 (1 H, d, J = 2.9 Hz); 6.65 (1 H, dd, J = 8.8, 2.9 Hz); 6.77 (1 H, t, J = 4.6 Hz); 6.96 (1 H, d, J = 8.8 Hz); 7.05 (2 H, d, J = 4.7 Hz); 7.54 (1 H, s); 8.30 (1 H, s).

### Composé 148: 5-(2-chloro-3-méthoxy-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (example comparatif)

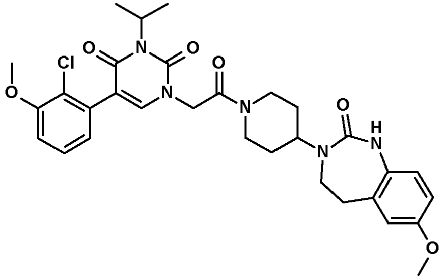

RMN ¹H (δ, DMSO) : 1.38 (6 H, d, J = 6.89 Hz); 1.55-1.68 (4 H, m); 2.69-2.63 (1 H, m); 2.85 (2 H, s); 3.12 (1 H, br t, J = 12.3 Hz); 3.35-3.33 (2 H, m); 3.65 (3 H, s); 3.87 (3 H, s); 3.92 (1 H, m) ; 4.30-4.22 (1 H, m); 4.41 (1 H, d, J = 12.9 Hz); 4.76-4.66 (2 H, m); 5.11-5.04 (1 H, m); 6.66-6.60 (2 H, m); 6.89 (1 H, dd, J = 7.6,1.4 Hz); 6.96 (1 H, d, J = 8.8 Hz); 7.16 (1 H, dd, J = 8.4, 1.4 Hz); 7.32 (1 H, t, J = 8.0 Hz); 7.65 (1 H, s);8.30 (1 H, s).

### Composé 149: 5-(2-fluoro-3-méthoxy-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (example comparatif)

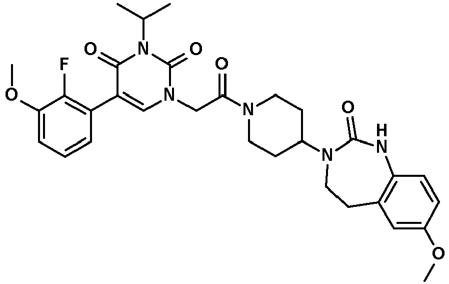

RMN ¹H (δ, DMSO) : 1.38 (6 H, d, J = 6.89 Hz); 1.57-1.48 (1 H, m) ; 1.69-1.63 (3 H, m); 2.65 (1 H, br t, J = 12.5 Hz); 2.87-2.85 (2 H, m); 3.12 (1 H, br t, J = 12.4 Hz); 3.36-3.34 (2 H, m); 3.65 (3 H, s); 3.85 (3 H, s); 3.91 (1 H, br d, J = 13.6 Hz); 4.26 (1 H, tt, J = 11.5, 4.3 Hz); 4.41 (1 H, d, J = 12.9 Hz); 4.72 (2 H, d, J = 3.0 Hz); 5.13-5.06 (1 H, m); 6.61 (1 H, d, J = 2.9 Hz); 6.65 (1 H, dd, J = 8.8, 3.0 Hz); 6.89-6.84 (1 H, m) ; 6.96 (1 H, d, J = 8.8 Hz); 7.18-7.11 (2 H, m); 7.74 (1 H, s); 8.30 (1 H, s).

### Composé 150: 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(2-trifluorométhoxy-phényl)-1H-pyrimidine-2,4-dione (example comparatif)

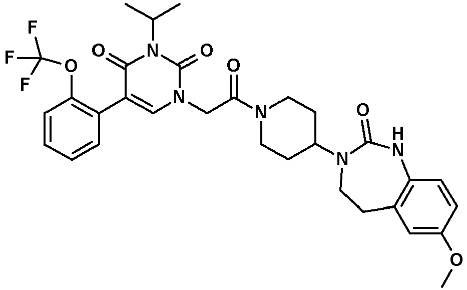

RMN ¹H (δ, DMSO) : 1.38 (d, J = 6.9 Hz, 6H) ; 1.50-1.73 (m, 4H) ; 2.65 (t, J = 12.5 Hz, 1H); 2.86 (br s, 2H); 3.12 (t, J = 12.2 Hz, 1H) ; 3.33-3.35 (m, 2H); 3.65 (s, 3H) ; 3.92 (d, J = 13.6 Hz, 1H); 4.24-4.27 (m, 1H); 4.41 (d, J = 12.9 Hz, 1H); 4.72 (d, J = 2.6 Hz, 2H) ; 5.04-5.11 (m, 1H); 6.60-6.66 (m, 2H) ; 6.96 (d, J = 8.8 Hz, 1H); 7.38-7.48 (m, 4H); 7.73 (s, 1H); 8.31 (s, 1H).

### Composé 151: 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(3-trifluorométhoxy-phényl)-1H-pyrimidine-2,4-dione (example comparatif)

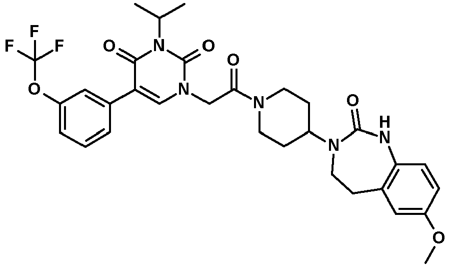

RMN ¹H (δ, DMSO) : 1.40 (d, J = 6.9 Hz, 6H); 1.50-1.68 (m, 4H); 2.63-2.69 (m, 1H); 2.87 (br s, 2H); 3.10-3.17 (m, 1H); 3.35-3.37 (m, 2H); 3.65 (s, 3H); 3.94 (d, J = 13.5 Hz, 1H); 4.23-4.31 (m, 1H); 4.41 (d, J = 12.9 Hz, 1H); 4.76 (br s, 2H); 5.10-5.17 (m, 1H); 6.61-6.67 (m, 2H); 6.96 (d, J = 8.8 Hz, 1H); 7.31 (d, J = 8.0 Hz, 1H); 7.51-7.59 (m, 3H); 7.99 (s, 1H); 8.32 (s, 1H) .

### Composé 152: 3-isopropyl-1-{2-[4-(7-méthoxy-2-oxo-1,2,4,5-tétrahydro-benzo[d][1,3]diazépin-3-yl)-pipéridin-1-yl]-2-oxo-éthyl}-5-(3-méthoxy-2-trifluorométhoxy-phényl)-1H-pyrimidine-2,4-dione (example comparatif)

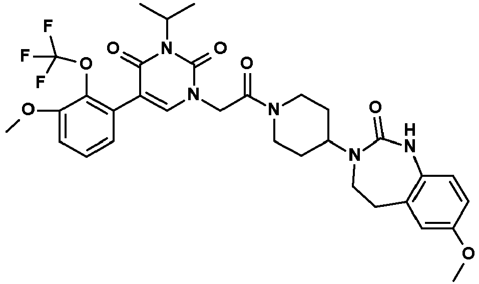

RMN ¹H (δ, DMSO) : 1.37 (d, J = 6.9 Hz, 6 H); 1.49-1.72 (m, 4 H); 2.62-2.68 (m, 1 H); 2.85-2.87 (m, 2 H); 3.06-3.14 (m, 1 H); 3.33-3.35 (m, 2 H); 3.65 (s, 3 H); 3.86 (s, 3 H); 3.92 (d, J = 13.3 Hz, 1 H); 4.24 (d, J = 11.7 Hz, 1 H); 4.41 (d, J = 13.0 Hz, 1 H); 4.71 (d, J = 3.3 Hz, 2 H); 5.04-5.11 (m, 1 H) ; 6.60-6.66 (m, 2 H); 6.89-6.96 (m, 2 H); 7.24 (dd, J = 8.4, 1.5 Hz, 1 H); 7.37 (t, J = 8.0 Hz, 1 H); 7.71 (s, 1 H); 8.31 (s, 1 H).

### Composé 153: 5-(2,3-difluoro-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (example comparatif)

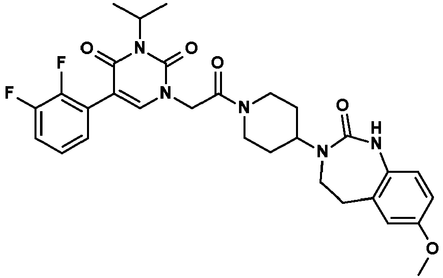

RMN ¹H (δ, DMSO) : 1.39 (d, J = 6.9 Hz, 6 H) ; 1.50-1.73 (m, 4 H); 2.66 (t, J = 12.4 Hz, 1 H); 2.86 (br s, 2 H); 3.13 (t, J = 12.1 Hz, 1 H); 3.34-3.36 (m, 2 H); 3.65 (s, 3 H); 3.92 (d, J = 13.5 Hz, 1 H); 4.21-4.27 (m, 1 H); 4.41 (d, J = 12.9 Hz, 1 H); 4.74 (s, 2 H); 5.06-5.13 (m, 1 H); 6.61-6.67 (m, 2 H); 6.96 (d, J = 8.8 Hz, 1 H); 7.17-7.27 (m, 2 H); 7.40-7.48 (m, 1 H); 7.85 (s, 1 H); 8.31 (s, 1 H).

### Composé 154: 5-(2-chloro-3-éthoxy-phényl)-3-isopropyl-1-{2-[4-(7-méthoxy-2-oxo-1,2,4,5-tétrahydro-benzo[d] [1,3]diazépin-3-yl)-pipéridin-1-yl]-2-oxo-éthyl}-1H-pyrimidine-2,4-dione (example comparatif)

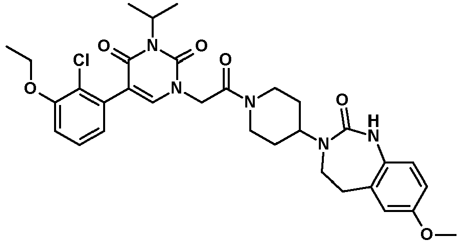

RMN ¹H (δ, DMSO) : 1.36-1.41 (m, 9H); 1.53-1.68 (m, 4H); 2.67-2.68 (m, 1H); 2.87 (m, 2H); 3.14 (m, 1H); 3.37 (m, 2H); 3.67 (s, 3H); 3.93 (d, J = 13.4 Hz, 1H); 4.15 (q, J = 7.0 Hz, 2H); 4.28 (m, 1H); 4.43 (d, J = 12.7 Hz, 1H); 4.71-4.73 (m, 2H); 5.08-5.11 (m, 1H); 6.62-6.68 (m, 2H); 6.89 (dd, J = 7.6, 1.4 Hz, 1H); 6.98 (d, J = 8.8 Hz, 1H); 7.16 (dd, J = 8.4, 1.4 Hz, 1H); 7.31 (t, J = 8.0 Hz, 1H); 7.66 (s, 1H); 8.33 (s, 1H).

### Exemple 155: 5-(2-chloro-3-fluoro-phényl)-1-{2-[4-(7-méthoxy-2-oxo-1,2,4,5-tétrahydro-benzo[d][1,3]diazépin-3-yl)-pipéridin-1-yl]-2-oxo-éthyl}-3-(2-méthylsulfanyl-éthyl)-1H-pyrimidine-2,4-dione (schéma réactionnel n°3, composé 155) (example comparatif)

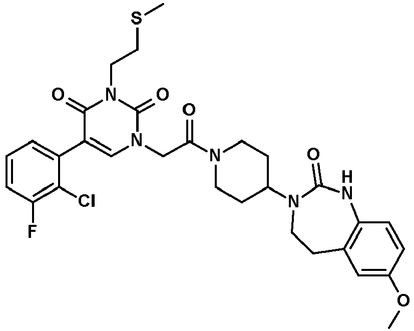

De manière analogue à l'exemple 115.10, à partir de 284mg (1 mmole) de 7-méthoxy-3-pipéridin-4-yl-1,3,4,5-tétrahydro-benzo[d][1,3]diazépin-2-one (préparé comme dans l'exemple 115.6) et 320mg (0,9 mmoles) d'acide [5-(2-chloro-3-fluoro-phényl)-3-(2-méthylsulfanyl-éthyl)-2,4-dioxo-3,4-dihydro-2H-pyrimidin-1-yl]-acétique (préparé comme dans l'exemple 125.1), 325mg (60%) de 5-(2-chloro-3-fluoro-phényl)-1-{2-[4-(7-méthoxy-2-oxo-1,2,4,5-tétrahydro-benzo[d] [1,3]diazépin-3-yl)-pipéridin-1-yl]-2-oxo-éthyl}-3-(2-méthylsulfanyl-éthyl)-1H-pyrimidine-2,4-dione sont obtenus sous la forme d'un solide blanc de point de fusion 230°C.
RMN ¹H (δ, DMSO): 1.50-1.80 (m, 4H); 2.12 (s, 3H); 2.65-2.71 (m, 3H); 2.88 (m, 2H); 3.15 (t, J = 12.1 Hz, 1H); 3.35-3.37 (m, 2H); 3.67 (s, 3H); 3.94 (d, J = 13.6 Hz, 1H); 4.08 (t, J = 7.2 Hz, 2H); 4.22-4.32 (m, 1H); 4.43 (d, J = 12.8 Hz, 1H); 4.79 (s, 2H) ; 6.62 (d, J = 2.9 Hz, 1H) ; 6.67 (dd, J = 8.8, 2.9 Hz, 1H) ; 6.98 (d, J = 8.8 Hz, 1H) ; 7.21-7.23 (m, 1H) ; 7.40-7.50 (m, 2H); 7.85 (s, 1H); 8.33 (s, 1H).

### Exemple 156: 5-(2-chloro-3-fluoro-phényl)-3-(3-méthanesulfonyl-propyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (schéma réactionnel n°3, composé 156) (example comparatif)

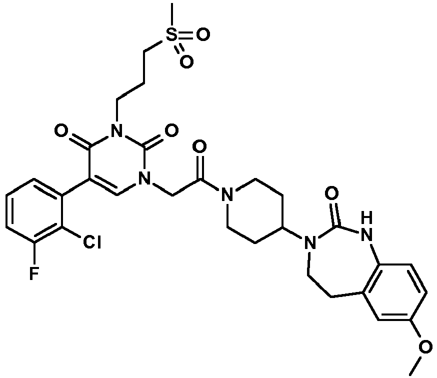

### 156.1: [5-bromo-3-(3-méthylsulfanyl-propyl)-2,4-dioxo-3,4-dihydro-2H-pyrimidin-1-yl]-acétate de méthyle

De manière analogue à l'exemple 121.1, à partir de 3g (11,4 mmoles) de (5-bromo-2,4-dioxo-3,4-dihydro-2H-pyrimidin-1-yl)-acétate de méthyle (préparé comme dans l'exemple 13.1) et 1,4ml (13,7 mmoles) de 3-méthylthiopropanol, 3,5g (87%) de [5-bromo-3-(3-méthylsulfanyl-propyl)-2,4-dioxo-3,4-dihydro-2H-pyrimidin-1-yl]-acétate de méthyle sont obtenus sous la forme d'un solide blanc.

### 156.2: [5-(2-chloro-3-fluoro-phényl)-3-(3-méthylsulfanyl-propyl) -2,4-dioxo-3,4-dihydro-2H-pyrimidin-1-yl]-acétate de méthyle

De manière analogue à l'exemple 115.8, à partir de 695mg (4 mmoles) d'acide 2-chloro-3-fluorophénylboronique et 1g (2,9 mmoles) de [5-bromo-3-(3-méthylsulfanyl-propyl)-2,4-dioxo-3,4-dihydro-2H-pyrimidin-1-yl]-acétate de méthyle, 750mg (66%) de [5-(2-chloro-3-fluoro-phényl)-3-(3-méthylsulfanyl-propyl)-2,4-dioxo-3,4-dihydro-2H-pyrimidin-1-yl]-acétate de méthyle sont obtenus sous la forme d'une huile incolore.

### 156.3: [5-(2-chloro-3-fluoro-phényl)-3-(3-méthanesulfonyl-propyl)-2,4-dioxo-3,4-dihydro-2H-pyrimidin-1-yl]-acétate de méthyle

De manière analogue à l'exemple 125.3, à partir de 750mg (1,9 mmoles) de [5-(2-chloro-3-fluoro-phényl)-3-(3-méthylsulfanyl-propyl)-2,4-dioxo-3,4-dihydro-2H-pyrimidin-1-yl]-acétate de méthyle, 810mg (100%) de [5-(2-chloro-3-fluoro-phényl)-3-(3-méthanesulfonyl-propyl)-2,4-dioxo-3,4-dihydro-2H-pyrimidin-1-yl]- acétate de méthyle sont obtenus sous la forme d'une meringue beige.

### 156.4: Acide [5-(2-chloro-3-fluoro-phényl)-3-(3-méthanesulfonyl-propyl)-2,4-dioxo-3,4-dihydro-2H-pyrimidin-1-yl]-acétique

De manière analogue à l'exemple 115.9, à partir de 810mg (1,9 mmoles) de [5-(2-chloro-3-fluoro-phényl)-3-(3-méthanesulfonyl-propyl)-2,4-dioxo-3,4-dihydro-2H-pyrimidin-1-yl]- acétate de méthyle , 750mg (96%) d'acide [5-(2-chloro-3-fluoro-phényl)-3-(3-méthanesulfonyl-propyl)-2,4-dioxo-3,4-dihydro-2H-pyrimidin-1-yl]-acétique sont obtenus sous la forme d'une meringue beige.

### 156.5: 5-(2-chloro-3-fluoro-phényl)-3-(3-méthanesulfonyl-propyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (composé 156)

De manière analogue à l'exemple 115.10, à partir de 226mg (0,9 mmoles) de 7-méthoxy-3-pipéridin-4-yl-1,3,4,5-tétrahydro-benzo[d][1,3]diazépin-2-one (préparé comme dans l'exemple 115.6) et 343mg (0,8 mmoles) d'acide [5-(2-chloro-3-fluoro-phényl)-3-(3-méthanesulfonyl-propyl)-2,4-dioxo-3,4-dihydro-2H-pyrimidin-1-yl]-acétique, 400mg (72%) de 5-(2-chloro-3-fluoro-phényl)-3-(3-méthanesulfonyl-propyl)-1-{2-[4-(7-méthoxy-2-oxo-1,2,4,5-tétrahydro-benzo [d][1,3]diazépin-3-yl)-piperidin-1-yl]-2-oxo-ethyl}-1H-pyrimidine-2,4-dione sont obtenus sous la forme d'un solide blanc de point de fusion 189°C.
RMN ¹H (δ, DMSO): 1.53-1.70 (m, 4H); 1.96-2.04 (m, 2H); 2.68 (t, J = 12.4 Hz, 1H); 2.88 (m, 2H); 2.98 (s, 3H); 3.15-3.19 (m, 3H); 3.35-3.37 (m, 2H); 3.67 (s, 3H); 3.94 (d, J = 13.7 Hz, 1H); 4.01 (t, J = 7.1 Hz, 2H); 4.28 (m, 1H); 4.43 (d, J = 12.9 Hz, 1H); 4.79 (s, 2H); 6.63 (d, J = 2.9 Hz, 1H); 6.67 (dd, J = 8.8, 3.0 Hz, 1H) ; 6.98 (d, J = 8.8 Hz, 1H) ; 7.23-7.25 (m, 1H); 7.44-7.47 (m, 2H); 7.86 (s, 1H); 8.33 (s, 1H).

### Exemple 157: 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione (schéma réactionnel n°3, composé 157) (example comparatif)

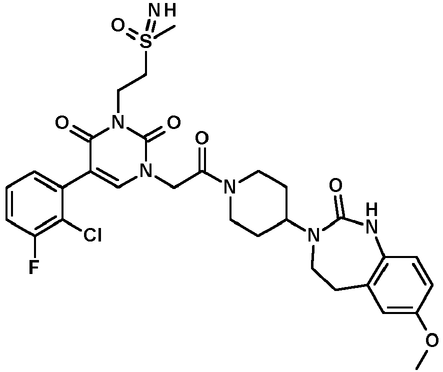

### 157.1: [5-(2-chloro-3-fluoro-phényl)-3-(2-méthylsulfanyl-éthyl)-2,4-dioxo-3,4-dihydro-2H-pyrimidin-1-yl]-acétate de méthyle

De manière analogue à l'exemple 115.8, à partir de 1,9g (11,1 mmoles) d'acide 2-chloro-3-fluorophénylboronique et 2,5g (7,4 mmoles) de [5-bromo-3-(2-méthylsulfanyl-éthyl)-2,4-dioxo-3,4-dihydro-2H-pyrimidin-1-yl]-acétate de méthyle (préparé comme dans l'exemple 112.1), 2,2g (77%) de [5-(2-chloro-3-fluoro-phényl)-3-(2-méthylsulfanyl-éthyl)-2,4-dioxo-3,4-dihydro-2H-pyrimidin-1-yl]- acétate de méthyle sont obtenus sous la forme d'un solide blanc-cassé.

### 157.2: [5-(2-chloro-3-fluoro-phényl)-3-(2-méthanesulfinyl-éthyl)-2,4-dioxo-3,4-dihydro-2H-pyrimidin-1-yl]-acétate de méthyle

0,9g (4,1 mmoles) d'acide 3-chloropéroxybenzoïque sont additionnés à une solution refroidie à 0°C contenant 1,5g (3,9 mmoles) de [5-(2-chloro-3-fluoro-phényl)-3-(2-méthylsulfanyl-éthyl)-2,4-dioxo-3,4-dihydro-2H-pyrimidin-1-yl]-acétate de méthyle dans 30ml de dichlorométhane. Le milieu est ensuite laissé revenir lentement à température ambiante. 20ml d'une solution aqueuse d'hydroxyde de sodium 1N est ajoutée goutte à goutte à 10°C avec 20ml d'eau puis le milieu réactionnel est décanté. La phase aqueuse est extraite deux fois au dichlorométahne. La phase organique est lavée avec une solution aqueuse de thiosulfate de sodium , séchée sur sulfate de magnésium, filtrée et évaporée. L'huile obtenue est précipitée avec du dichlorométhane et de l'heptane. Le solide est filtré, rincé à l'heptane et séché. 1,4g (90%) de [5-(2-chloro-3-fluoro-phényl)-3-(2-méthanesulfinyl-éthyl)-2,4-dioxo-3,4-dihydro-2H-pyrimidin-1-yl]-acétate de méthyle sont obtenus sous la forme d'un solide blanc.

### 157.3: 2-[5-(2-chloro-3-fluoro-phényl)-3-[2-(méthylsulfonimidoyl)éthyl]-2,4-dioxo-pyrimidin-1-yl]acétate de méthyle

1,7g (5,2 mmoles) de diacétate d'iodobenzène sont additionnés sur une solution contenant 1,4g (3,5 mmoles) de [5-(2-chloro-3-fluoro-phényl)-3-(2-méthanesulfinyl-éthyl)-2,4-dioxo-3,4-dihydro-2H-pyrimidin-1-yl]-acétate de méthyle, 786mg (7 mmoles) de 2,2,2-trifluoroacétamide, 560mg (13,9 mmoles) d'oxyde de magnésium et 184mg (0,4 mmoles) de dirhodium tétraacétate dans 42ml de dichlorométhane. Le milieu est agité à température ambiante pendant 4 heures puis filtré et évaporé. Le résidu est repris par 42ml de méthanol puis 2,4g (17,4 mmoles) de carbonate de potassium sont ajoutés. Le mélange est agité à température ambiante pendant 30 minutes puis le méthanol est évaporé et le résidu est repris par de l'acétate d'éthyle puis par de l'eau.La phase aqueuse est extraite deux fois par de l'acétate d'éthyle. La phase organique est lavée à l'eau, séchée sur sulfate de magnésium, filtrée et évaporée. Après purification par chromatographie sur gel de silice élué par de l'acétate d'éthyle puis avec 10% de méthanol, 460mg (32%) de 2-[5-(2-chloro-3-fluoro-phényl)-3-[2-(méthylsulfonimidoyl)éthyl]-2,4-dioxo-pyrimidin-1-yl]acétate de méthyle sont obtenus sous la forme d'un solide jaune pâle.

### 157.4: Acide 2-[5-(2-chloro-3-fluoro-phényl)-3-[2-(méthylsulfonimidoyl)éthyl]-2,4-dioxo-pyrimidin-1-yl]acétique

De manière analogue à l'exemple 115.9, à partir de 180mg (0,4 mmoles) de 2-[5-(2-chloro-3-fluoro-phényl)-3-[2-(méthylsulfonimidoyl)éthyl]-2,4-dioxo-pyrimidin-1-yl]acétate de méthyle, une certaine quantité d'acide 2-[5-(2-chloro-3-fluoro-phényl)-3-[2-(méthylsulfonimidoyl)éthyl]-2,4-dioxo-pyrimidin-1-yl]acétique est obtenue sous la forme d'un solide jaune pâle.

### 157.5: 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione (composé 157)

De manière analogue à l'exemple 115.10, à partir de 113mg (0,4 mmoles) de 7-méthoxy-3-pipéridin-4-yl-1,3,4,5-tétrahydro-benzo[d][1,3]diazépin-2-one (préparé comme dans l'exemple 115.6) et 150mg (0,4 mmoles) d'acide 2-[5-(2-chloro-3-fluoro-phényl)-3-[2-(méthylsulfonimidoyl)éthyl]-2,4-dioxo-pyrimidin-1-yl]acétique, et après purification par chromatographie sur gel de silice élué, 23mg (9 %) de 5-(2-chloro-3-fluoro-phényl)-1-[2-[4-(7-méthoxy-2-oxo-4,5-dihydro-1H-1,3-benzodiazépin-3-yl)-1-pipéridyl]-2-oxo-éthyl]-3-[2-(méthylsulfonimidoyl)éthyl]pyrimidine-2,4-dione sont obtenus sous la forme d'un solide rose pâle de point de fusion 197°C.
RMN ¹H (δ, DMSO): 1.55 (m, 1H), 1.61-1.83 (m, 3H), 2.65 (m, 1H), 2.88 (m, 2H), 2.98 (s, 3H), 3.07-3.22 (m, 1H), 3.67 (s, 3H), 3.84 (s, 1H), 3.95 (d, J = 13.7 Hz, 1H), 4.28 (m, 3H), 4.43 (d, J = 13.0 Hz, 1H), 4.79 (m, 2H), 6.62 (d, J = 2.9 Hz, 1H), 6.67 (dd, J = 8.6, 3.0 Hz, 1H), 6.98 (d, J = 8.9 Hz, 1H), 7.22 (m, 1H), 7.37-7.56 (m, 2H), 7.87 (s, 1H), 8.32 (s, 1H). Les 4 protons manquants sont sous le pic de l'eau et/ou du DMSO.

### Exemple 158: N-[(S)-2-(5-(2-chloro-3-fluoro-phényl)-3-{2-[4-(7-méthoxy-2-oxo-1,2,4,5-tétrahydro-benzo[d] [1,3]diazepin-3-yl)-pipéridin-1-yl]-2-oxo-éthyl}-2,6-dioxo-3,6-dihydro-2H-pyrimidin-1-yl)-propyl]-propionamide (schéma réactionnel n°3, composé 158)

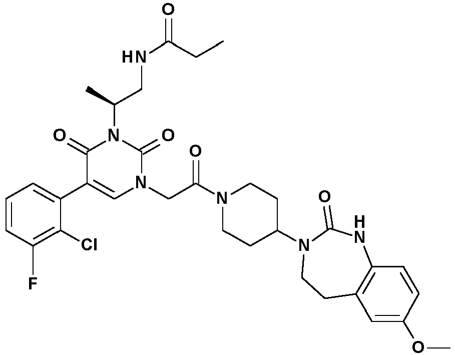

156µL (1,8 mmoles) de chlorure de propanoyle sont ajoutés à une solution de 1g (1,6 mmoles) de 3-((S)-2-amino-1-méthyl-éthyl)-5-(2-chloro-3-fluoro-phényl)-1-{2-[4-(7-méthoxy-2-oxo-1,2,4,5-tétrahydro-benzo[d][1,3]diazepin-3-yl)-piperidin-1-yl]-2-oxo-éthyl}-1H-pyrimidine-2,4-dione préparé comme décrit dans l'exemple 119 et 0,25mL (1,8 mmoles) de triéthylamine dans 20mL de dichlorométhane. Le milieu réactionnel est agité à température ambiante pendant 1h, hydrolysé et extrait avec du dichlorométhane. Les phases organiques sont rassemblées, lavées une fois par de l'eau puis séchées sur sulfate de magnésium, filtrées et concentrées sous vide. Le produit brut obtenu est purifié par chromatographie sur gel de silice élué avec un mélange dichlorométhane/méthanol 96/4. 0,7g (64%) de N-[(S)-2-(5-(2-chloro-3-fluoro-phényl)-3-{2-[4-(7-méthoxy-2-oxo-1,2, 4,5-tétrahydro-benzo [d][1,3]diazepin-3-yl)-pipéridin-1-yl]-2-oxo-éthyl}-2,6-dioxo-3,6-dihydro-2H-pyrimidin-1-yl)-propyl]-propionamide est obtenu sous la forme d'un solide blanc.
RMN ¹H (δ, DMSO): 0.96 (td, J = 7.6, 1.7 Hz, 3H), 1.24-1.41 (d, J = 8.0 Hz, 3H), 1.55 (m, 1H), 1.60-1.84 (m, 3H), 2.03 (qd, J = 9.0, 8.3, 2.6 Hz, 2H), 2.60-2.76 (m, 1H), 2.80-2.96 (m, 2H), 3.15 (m, 1H), 3.36 (m, 2H), 3.52 (m, 2H), 3.67 (s, 3H), 3.95 (d, J = 13.4 Hz, 1H), 4.29 (m, 1H), 4.44 (d, J = 12.8 Hz, 1H), 4.57-4.88 (m, 2H), 5.00 (m, 1H), 6.60 - 6.70 (m, 2H), 6.98 (d, J = 8.8 Hz, 1H), 7.20 (m, 1H), 7.36-7.54 (m, 2H), 7.79 (s, 1H), 7.85 (m, 1H), 8.34 (s, 1H).

### Exemple 159: 5-(2-Chloro-3-fluoro-phenyl)-1-{2-[4-(7-fluoro-2-oxo-1,2,4,5-tetrahydro-benzo[d] [1,3]diazepin-3-yl)-piperidin-1-yl]-2-oxo-ethyl}-3-((S)-2-methoxy-1-methyl-ethyl)-1H-pyrimidine-2,4-dione (schéma réactionnel n°3, composé 159) (example comparatif)

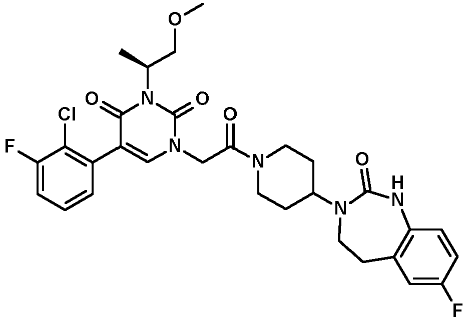

### 159.1 : 7-Fluoro-3-piperidin-4-yl-1,3,4,5-tetrahydro-benzo[d] [1,3]diazepin-2-one

De manière analogue à la séquence décrite dans l'exemple 115 (115.1 à 115.9), à partir de 1g (5,5 mmoles) de 2-(5-fluoro-2-nitrophenyl)acétonitrile, 315mg de 7-Fluoro-3-pipéridin-4-yl-1,3,4,5-tetrahydro-benzo[d][1,3]diazepin-2-one sont obtenus.

### 159.2 : N-[(S)-2-(5-(2-chloro-3-fluoro-phényl)-3-{2-[4-(7-méthoxy-2-oxo-1,2,4,5-tétrahydro-benzo[d] [1,3]diazepin-3-yl)-pipéridin-1-yl]-2-oxo-éthyl}-2,6-dioxo-3,6-dihydro-2H-pyrimidin-1-yl)-propyl]-propionamide

De manière analogue à l'exemple 115.10, à partir de 150mg (0,6 mmole) de 7-Fluoro-3-pipéridin-4-yl-1,3,4,5-tetrahydro-benzo[d][1,3]diazepin-2-one et de 176mg (0,5 mmole) de l'acide [5-(2-Chloro-3-fluoro-phenyl)-3-((S)-2-methoxy-1-methyl-ethyl)-2,4-dioxo-3,4-dihydro-2H-pyrimidin-1-yl]-acétique (préparé comme décrit dans l'exemple 106.3), 60mg (21%) de N-[(S)-2-(5-(2-chloro-3-fluoro-phényl)-3-{2-[4-(7-méthoxy-2-oxo-1,2, 4,5-tétrahydro-benzo [d][1,3]diazepin-3-yl)-pipéridin-1-yl]-2-oxo-éthyl}-2,6-dioxo-3,6-dihydro-2H-pyrimidin-1-yl)-propyl]-propionamide

Les composés suivants ont été synthétisés selon un mode opératoire analogue, en utilisant les réactifs appropriés commerciaux ou préalablement préparés:

### composé 160: 5-(2-Chloro-3-fluoro-phenyl)-1-{2-[4-(7-fluoro-2-oxo-1,2,4,5-tetrahydro-benzo[d] [1,3]diazepin-3-yl)-piperidin-1-yl]-2-oxo-ethyl}-3-(2-methanesulfonyl-ethyl)-1H-pyrimidine-2,4-dione (example comparatif)

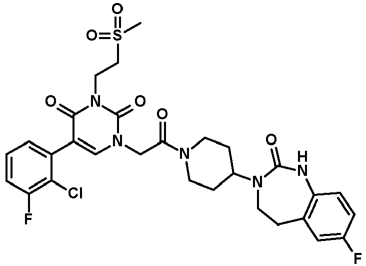

RMN ¹H (δ, DMSO) : 1.56 (m, 1H), 1.68 (m, 3H), 2.69 (m, 1H), 2.84 - 2.94 (m, 2H), 3.08 (s, 3H), 3.15 (m, 1H), 3.35-3.47 (m, 4H), 3.96 (d, J = 13.3 Hz, 1H), 4.31 (m, 3H), 4.43 (d, J = 13.1 Hz, 1H), 4.80 (m, 2H), 6.92 (m, 2H), 7.03 - 7.13 (m, 1H), 7.16-7.29 (m, 1H), 7.37-7.55 (m, 2H), 7.87 (s, 1H), 8.56 (s, 1H).

### Composé 161: N-[(S)-2-(5-(2-Chloro-3-fluoro-phenyl)-3-{2-[4-(7-fluoro-2-oxo-1,2,4,5-tetrahydro-benzo[d] [1,3]diazepin-3-yl)-piperidin-1-yl]-2-oxo-ethyl}-2,6-dioxo-3,6-dihydro-2H-pyrimidin-1-yl)-propyl]-acetamide (example comparatif)

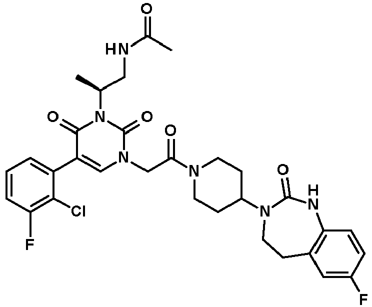

RMN ¹ H (δ, DMSO) : 1.34 (d, J = 6.8 Hz, 3H) 1.56 (m, 1H), 1.68 (m, 3H), 1.77 (s, 3H), 2.68 (m, 1H), 2.84 - 2.94 (m, 2H), 3.15 (m, 1H), 3.38 (m, 2H), 3.41-3.63 (m, 2H), 3.95 (d, J = 13.6 Hz, 1H), 4.29 (m, 1H), 4.44 (d, J = 13.0 Hz, 1H), 4.58-4.87 (m, 2H), 4.91-5.12 (m, 1H), 6.81-6.99 (m, 2H), 7.07 (m, 1H), 7.21 (m, 1H), 7.34-7.51 (m, 2H), 7.78 (s, 1H), 7.93 (t, J = 5.9 Hz, 1H), 8.55 (s, 1H).

### composé 162: N-[(S)-2-(5-(2,3-dichloro-phenyl)-3-{2-[4-(7-fluoro-2-oxo-1,2,4,5-tetrahydro benzo[d][1,3]diazepin-3-yl)-piperidin-1-yl]-2-oxo-ethyl}-2,6-dioxo-3,6-dihydro-2H-pyrimidin-1-yl)-propyl]-acetamide (example comparatif)

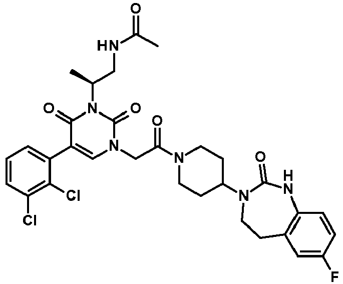

RMN ¹H (δ, DMSO) : 1.34 (d, J = 6.9 Hz, 3H), 1.55 (m, 1H), 1.71 (m, 3H), 1.77 (s, 3H), 2.68 (m, 1H), 2.82-2.97 (m, 2H), 3.14 (m, 1H), 3.37 (m, 2H), 3.51 (m, 2H), 3.95 (d, J = 13.6 Hz, 1H), 4.29 (m, 1H), 4.44 (d, J = 13.0 Hz, 1H), 4.61-4.86 (m, 2H), 5.06 - 4.89 (m, 1H), 6.81-7.00 (m, 2H), 7.07 (m, 1H), 7.33 (m, 1H), 7.42 (t, J = 7.8 Hz, 1H), 7.68 (dd, J = 8.0, 1.6 Hz, 1H), 7.77 (s, 1H), 7.92 (t, J = 5.9 Hz, 1H), 8.55 (s, 1H).

### composé 163: 5-(2-Chloro-3-fluoro-phenyl)-1-{2-[4-(9-fluoro-2-oxo-1,2,4,5-tetrahydro-benzo[d] [1,3]diazepin-3-yl)-piperidin-1-yl]-2-oxo-ethyl}-3-isopropyl-1H-pyrimidine-2,4-dione (example comparatif)

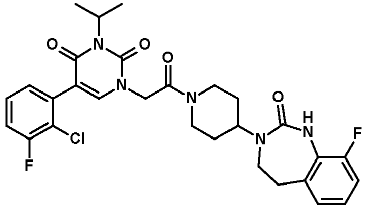

RMN ¹H (δ, DMSO) : 1.41 (d, J = 6.8 Hz, 6H), 1.59 (m, 1H), 1.73 (m, 3H), 2.62-2.77 (m, 1H), 2.87-3.04 (m, 2H), 3.15 (m, 1H), 3.37-3.52 (m, 2H), 3.94 (d, J = 13.8 Hz, 1H), 4.28 (m, 1H), 4.44 (d, J = 13.4 Hz, 1H), 4.74 (m, 2H), 5.11 (hept, J = 6.7 Hz, 1H), 6.87 (m, 1H), 6.94 (d, J = 7.4 Hz, 1H), 7.00-7.12 (m, 1H), 7.15-7.27 (m, 1H), 7.35-7.50 (m, 3H), 7.77 (s, 1H).

### Exemple 164 : 5-(2,3-difluorophenyl)-3-méthyl-1-[2-oxo-2-[4-(2-oxo-3H-imidazo[4,5-b]pyridin-1-yl) -1-piperidyl]ethyl]pyrimidine-2,4-dione (schéma réactionnel n°4, composé 164) (example comparatif)

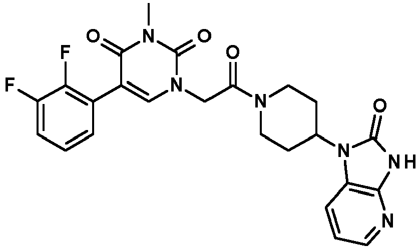

### 164.1: 4-(2-Chloro-pyridin-3-ylamino)-piperidine-1-carboxylic acid ethyl ester

23mL (311 mmoles) d'acide trifluoroacétique sont ajoutés après 5 minutes sur une solution de 20g (156 mmoles) de 3-amino-2-chloropyridine et 26mL (171 mmoles) de 4-oxo-1-pipéridine carboxylate d'éthyle dans 250mL d'isopropylacétate. Le mélange réactionnel est agité 30 minutes à température ambiante puis 40g (19 mmoles) de sodium triacétoxyborohydrure sont ajoutés. Le mélange réactionnel est agité 2heures à température ambiante puis 23mL d'une solution à 10% d'hydroxyde de sodium sont ajoutés jusqu'à pH8-9 et le mélange est chauffé à 50°C. Après refroidissement le milieu réactionnel est décanté puis extrait avec de l'acétate d'éthyle. Les phases organiques sont rassemblées, et séchées sur sulfate de sodium. Les solvants sont évaporés puis le résidu est purifié par chromatographie sur gel de silice élué avec un mélange heptane/acétate d'éthyle 60/40. 43,2g (98%) de 4-(2-chloro-pyridin-3-ylamino)-piperidine-1-carboxylate d'éthyle sont obtenus sous la forme d'une huile incolore.

### 164.2: 4-[1-(2-Chloro-pyridin-3-yl)-ureido]-piperidine-1-carboxylate d'éthyle

43,3g (42,3 mmoles) de 4-(2-chloro-pyridin-3-ylamino)-pipéridine-1-carboxylate d'éthyle en solution dans 200mL d'un mélange tétrahydrofurane/isopropylacétate 1/1 sont ajoutés à une solution de 15,8mL (51 mmoles) de chlorosulfonyl-isocyanate dans 100mL de tetrahydrofuranne. Le mélange réactionnel est agité une heure à température ambiante puis hydrolysé avec 15mL d'eau. Après agitation 18h à température ambiante, la réaction est arrêtée par l'ajout d'une solution à 10% d'hydroxyde de sodium jusqu'à pH 8-9 puis extraite avec de l'acétate d'éthyle. Les phases organiques sont rassemblées, lavées avec une solution saturée de chlorure de sodium et séchées sur sulfate de sodium. Les solvants sont évaporés puis le résidu est repris dans l'éther et essoré. On obtient 32g (74%) de 4-[1-(2-chloro-pyridin-3-yl)-ureido]-pipéridine-1-carboxylate d'éthyle sous la forme d'une poudre blanche.

### 164.3: 4-(2-Oxo-2,3-dihydro-imidazo[4,5-b]pyridin-1-yl)-piperidine-1-carboxylate d'éthyle

13,7mg (0.06 mmoles) de palladiumdiacétate et 52mg (0,12 mmoles) de diphénylphosphinobutane sont ajoutés sur une solution préalablement dégazée à l'azote pendant 1 heure de 1g (3,1 mmoles) de 4-[1-(2-chloro-pyridin-3-yl)-ureido]-pipéridine-1-carboxylate d'éthyle et 1,3g (9,2 mmoles) de carbonate de potassium dans 10mL d'isopropanol. Le mélange réactionnel est agité 3 heures à 83°C, l'isopropanol est concentré, 20mL d'eau sont ajoutés et le milieu est extrait avec de l'acétate d'éthyle. Les phases organiques sont rassemblées, lavées avec une solution saturée de chlorure de sodium et séchées sur sulfate de sodium. Les solvants sont évaporés puis le résidu est repris dans de l'isopropylacétate puis essoré. On obtient 797mg (90%) de 4-(2-oxo-2,3-dihydro-imidazo[4,5-b]pyridin-1-yl)-pipéridine-1-carboxylate d'éthyle sous la forme d'une poudre blanche.

### 164.4 : dichlorure de 1-pipéridin-4-yl-1,3-dihydro-imidazo[4,5-b]pyridin-2-one

1,8mL (34,5 mmoles) d'une solution à 50% d'hydroxyde de sodium et 1,7mL d'eau sont ajoutés sur une solution de 667mg (2,3 mmoles) de 4-(2-oxo-2,3-dihydro-imidazo[4,5-b]pyridin-1-yl)-pipéridine-1-carboxylate d'éthyle dans 1,4mL d'éthanol. Le mélange réactionnel est agité 18h à 80°C. La réaction est arrêtée par l'ajout de 10mL d'eau puis laissée à température ambiante et lavée à l'acétate d'éthyle. La phase aqueuse est concentrée à sec par évaporation azéotropique avec de l'isopropanol. Le résidu brut est repris dans 5mL d'isopropanol puis filtré pour éliminer les sels. Une solution 6N d'acide chlorhydrique dans l'isopropanol est ajoutée, le produit précipite. Après chauffage à 50°C pendant 10 minutes puis retour à température ambiante, le précipité est essoré et rincé à l'isopropanol. 502mg (75%) de dichlorure de 1-pipéridin-4-yl-1,3-dihydro-imidazo[4,5-b]pyridin-2-one sont obtenus sous forme d'une poudre blanche.

### 164.5 : 5-(2,3-difluorophenyl)-3-méthyl-1-[2-oxo-2-[4-(2-oxo-3H-imidazo[4,5-b]pyridin-1-yl)-1-piperidyl]ethyl]pyrimidine-2,4-dione (composé 164)

De manière analogue aux exemples précédemment décrits, à partir de 100mg (0,34 mmole) d'acide 2-[5-(2,3-difluorophenyl)-3-methyl-2,4-dioxo-pyrimidin-1-yl]acétique et de 197mg (0,7 mmoles) de dichlorure de 1-pipéridin-4-yl-1,3-dihydro-imidazo[4,5-b]pyridin-2-one, 100mg (59%) de 5-(2,3-difluorophenyl)-3-méthyl-1-[2-oxo-2-[4-(2-oxo-3H-imidazo[4,5-b]pyridin-1-yl)-1-piperidyl]ethyl]pyrimidine-2,4-dione.
RMN ¹H (δ, DMSO): 1.69-1.91 (m, 2H), 2.09 (m, 1H), 2.34 (m, 1H), 2.70-2.87 (m, 1H), 3.27 (m, 4H), 3.35 (d, 1H), 4.50 (m, 2H), 4.87 (m, 2H), 7.00 (dd, J = 7.8, 5.2 Hz, 1H), 7.14-7.36 (m, 2H), 7.46 (dtd, J = 10.1, 8.1, 1.8 Hz, 1H), 7.59 (dd, J = 8.0, 1.4 Hz, 1H), 7.91 (dd, J = 5.2, 1.3 Hz, 1H), 8.00 (s, 1H), 11.60 (s, 1H).

### Exemple 165 : 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide (schéma réactionnel 4, composé 165) (example comparatif)

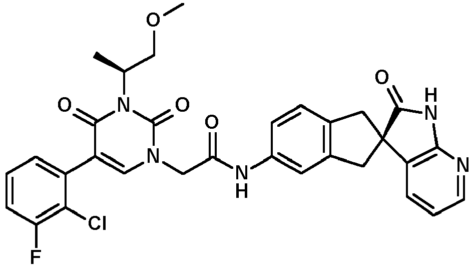

### 165.1: 5-Bromo-2,4-dioxo-3,4-dihydro-2H-pyrimidin-1-yl-acétate de méthyle

21,3g (150 mmoles) de carbonate de potassium et 14,5mL (150 mmoles) de bromoacétate de méthyle sont additionnés sur une solution de 30g (150mmoles) de 5-bromouracile dans 294mL de N,N-diméthylformamide. Le milieu réactionnel est agité à température ambiante pendant 50min puis hydrolysé (250mL d'eau et 50mL de NaOHaq 1N) et extrait par du dichlorométhane (300mL) . Les phases aqueuses sont rassemblées et lavées une fois par 100mL de dichlorométhane. La phase aqueuse contient le produit attendu. Le pH de la phase aqueuse est amené à pH=6-7 par 300mL d'une solution aqueuse d'acide chlorhydrique C=1N et 8mL d'acide chlorhydrique concentré (pour éviter d'avoir un trop grand volume d'eau). Le produit précipite. La suspension est agitée pendant 2h puis filtrée. Le solide obtenu est rincé par de l'eau et de l'éther diéthylique puis séché sous vide à 50°C pendant 24h pour donner 22g (54%) (5-bromo-2,4-dioxo-3,4-dihydro-2H-pyrimidin-1-yl)-acétate de méthyle sous la forme d'un solide blanc.

### 165.2: [5-bromo-3-((S)-2-méthoxy-1-méthyl-éthyl)-2,4-dioxo-3,4-dihydro-2H-pyrimidin-1-yl]-acétate de méthyle

2,7mL (11,4 mmoles) de diéthylazodicarboxylate est additionné goutte à goutte sur une solution refroidie à 0°C de 1g (3,8 mmoles) de 5-bromo-2,4-dioxo-3,4-dihydro-2H-pyrimidin-1-yl)-acétate de méthyle, 0,8mL (8 mmoles) de (R)-1-méthoxy-propan-2-ol et 2g (7,6 mmoles) de triphénylphosphine dans 20mL de tétrahydrofurane. Le milieu réactionnel (suspension blanche) est agité à température ambiante pendant 48 heures. Le tétrahydrofuranne est éliminé sous vide puis le résidu est repris dans l'acétate d'éthyle. La phase organique est lavée une fois par une solution aqueuse saturée d'hydrogénocarbonate de sodium, une fois par de l'eau puis par une solution aqueuse saturée de chlorure de sodium, séchée sur sulfate de magnésium, filtrée et concentrée sous vide. Le produit brut est purifié par chromatographie sur gel de silice élué par un mélange Heptane/Acétate d'éthyle 60/40. 1,2g (78%) de [5-bromo-3-((S)-2-méthoxy-1-méthyl-éthyl)-2,4-dioxo-3,4-dihydro-2H-pyrimidin-1-yl]-acétate de méthyle est obtenu sous la forme d'une huile incolore.

### 165.3: acide 5-(2-chloro-3-fluoro-phenyl)-3-((S)-2-méthoxy-1-méthyl-éthyl)-2,4-dioxo-3,4-dihydro-2H-pyrimidin-1-yl]-acétique

54mg (70µmoles) de 1,1'-bis(diphénylphosphino)ferrocène palladium (II) dichlorure complexé au dichlorométhane sont additionnés à une solution de 400mg (1,3 mmoles) de [5-bromo-3-((S)-2-méthoxy-1-méthyl-éthyl)-2,4-dioxo-3,4-dihydro-2H-pyrimidin-1-yl]-acétate de méthyle, de 417mg (4 mmoles) de carbonate de sodium et 571mg (3,3 mmoles) d'acide 2-chloro-3-fluoro-phényle boronique dans 44mL de 1,4-dioxanne et 4mL d'eau, préalablement dégazée pendant 5min. Le milieu réactionnel est ensuite chauffé à 100°C pendant 2h. 2mL d'une solution aqueuse 1M d'hydroxyde de lithium monohydrate et 2,2mL d'eau sont alors additionnés et le mélange réactionnel est agité pendant 1h. Le milieu réactionnel est hydrolysé puis dilué par de l'acétate d'éthyle. La première phase organique est écartée (impuretés) et la phase aqueuse est amenée à pH acide par une solution aqueuse d'acide chlorhydrique de concentration 1N. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le produit brut est purifié par chromatographie sur gel de silice élué par un mélange dichlorométhane/méthanol 95/5 + 0.1% d'acide acétique. 240mg (49%) d'acide [5-(2-Chloro-3-fluoro-phényl)-3-((S)-2-méthoxy-1-méthyl-éthyl)-2,4-dioxo-3,4-dihydro-2H-pyrimidin-1-yl]-acétique sont obtenus sous la forme d'une huile jaune.

### 165.4: 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide (composé 165)

162mg (0,65 mmoles) de (R)-5-amino-1,3-dihydrospiro[indene-2,3'-pyrrolo[2,3-b)pyridin]-2'(1'H)-one préparé comme décrit dans le brevet WO2011/005731 est additionné sur une solution préalablement agitée pendant 5min, de 240mg (0,65mmoles) d'acide [5-(2-chloro-3-fluoro-phenyl)-3-((S)-2-méthoxy-1-méthyl-éthyl)-2,4-dioxo-3,4-dihydro-2H-pyrimidin-1-yl]-acétique, 114mg (0,8 mmole) de 1-hydroxybenzotriazole et 161mg (0,8 mmole) de chlorhydrate de 1-éthyl-3-(3-diméthylaminopropyl)-carbodiimide dans 5mL de N,N-DIMETHYLFORMAMIDE. Le milieu réactionnel est agité à température ambiante pendant 18h puis dilué par de l'acétate d'éthyle. La phase organique est lavée une fois par une solution aqueuse saturée d'hydrogénocarbonate de sodium, une fois par de l'eau et une fois par une solution aqueuse saturée de chlorure de sodium, séchée sur sulfate de magnésium puis filtrée et concentrée sous vide.Le produit brut est purifié par chromatographie sur gel de silice (HP15) élué par un mélange dichlorométhane/méthanol 96/4. 155mg (39%) de 5-(2-chloro-3-fluoro-phenyl)-3-((S)-2-méthoxy-1-méthyl-éthyl)-2,4-dioxo-3,4-dihydro-2H-pyrimidin-1-yl]-1-[(2R)-2'-oxospiro[1,3-dihydroindene-2,3'-1H-pyrrolo[2,3-b]pyridine]-5-yl]-acetamide sont obtenus sous la forme d'un solide blanc.
RMN ¹H (δ, DMSO): 1.34 (d, J = 6.9 Hz, 3H); 3.04-3.11 (m, 2H); 3.23 (s, 3H); 3.33-3.37 (m, 2H); 3.54 (dd, J = 9.9, 5.9 Hz, 1H); 3.92 (dd, J = 9.9, 8.2 Hz, 1H); 4.65 (s, 2H); 5.13-5.15 (m, 1H); 6.87 (dd, J = 7.3, 5.3 Hz, 1H); 7.18 (dd, J = 7.3, 1.6 Hz, 1H) ; 7.21-7.24 (m, 2H) ; 7.38 (d, J = 8.3 Hz, 1H) ; 7.43-7.46 (m, 2H); 7.62 (s, 1H); 7.93 (s, 1H); 8.07 (dd, J = 5.3, 1.6 Hz, 1H); 10.35 (s, 1H); 11.09 (s, 1H).

Les composés suivants ont été synthétisés selon un mode opératoire analogue à partir des réactifs appropriés commerciaux ou préalablement préparés:

### composé 166: 5-(2-Chloro-3-fluoro-phenyl)-3-isopropyl-pyrimidine-2,4-dione-1-[(2R)-2'-oxospiro[1,3-dihydroindene-2,3'-1H-pyrrolo[2,3-b]pyridine]-5-yl]-acetamide (example comparatif)

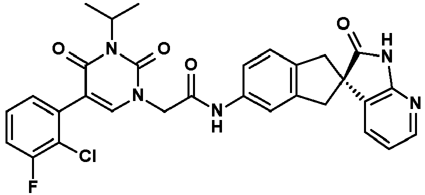

RMN ¹H (δ, DMSO) : 1.41 (d, J = 6.9 Hz, 6H) ; 3.08 (m, 2H); 3.33 (m, 2H) ; 4.65 (m, 2H); 5.10-5.11 (m, 1H); 6.87 (dd, J = 7.3, 5.3 Hz, 1H); 7.18 (m, 1H), 7.23 (m, 2H); 7.38 (m, 1H), 7.45 (m, 2H), 7.63 (m, 1H); 7.91 (s, 1H); 8.07 (dd, J = 5.3, 1.6 Hz, 1H); 10.35 (s, 1H); 11.09 (s, 1H).

### composé 167: 5-(2,3-difluorophenyl)-3-[(1S)-2-methoxy-1-methyl-ethyl]- pyrimidine-2,4-dione-1-[(2R)-2'-oxospiro[1,3-dihydroindene-2,3'-1H-pyrrolo[2,3-b]pyridine]-5-yl]-acetamide (example comparatif)

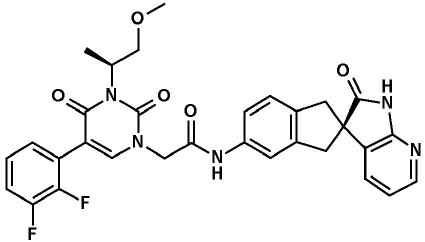

RMN ¹H (δ, DMSO) : 1.34 (d, J = 6.9 Hz, 3H)
, 3.08 (m, 2H), 3.23 (s, 3H), 3.34 (m, 2H), 3.54 (m, 1H), 3.93 (m, 1H), 4.66 (m, 2H), 5.16 (q, J = 7.1 Hz, 1H), 6.87 (dd, J = 7.3, 5.3 Hz, 1H), 7.18 (dd, J = 7.3, 1.7 Hz, 1H), 7.20-7.31 (m, 3H), 7.39 (m, 1H), 7.46 (m, 1H), 7.61 (m, 1H), 8.02 (s, 1H), 8.07 (dd, J = 5.3, 1.7 Hz, 1H), 10.35 (s, 1H), 11.09 (s, 1H).

### composé 168: 2-[5-(2-chloro-3-fluoro-phenyl)-2,4-dioxo-1H-pyrimidin-3-yl]acetic acid-1-[(2R)-2'-oxospiro[1,3 dihydroindene-2,3'-1H-pyrrolo[2,3-b]pyridine]-5-yl]-acetamide (example comparatif)

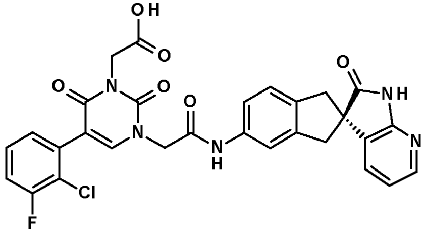

RMN ¹H (δ, DMSO) : 3.08 (dd, J = 16.1, 12.1 Hz, 2H), 3.36 (m, 2H), 4.53 (s, 2H), 4.71 (s, 2H), 6.87 (dd, J = 7.3, 5.3 Hz, 1H), 7.18 (dd, J = 7.4, 1.7 Hz, 1H), 7.24 (m, 2H), 7.38 (dd, J = 8.2, 2.0 Hz, 1H), 7.42-7.53 (m, 2H), 7.61 (m, 1H), 8.03 (s, 1H), 8.07 (dd, J = 5.3, 1.7 Hz, 1H), 10.37 (s, 1H), 11.09 (s, 1H), 13.02 (br-s, 1H).

### composé 169: 5-(2-chloro-3-methoxy-phenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide (example comparatif)

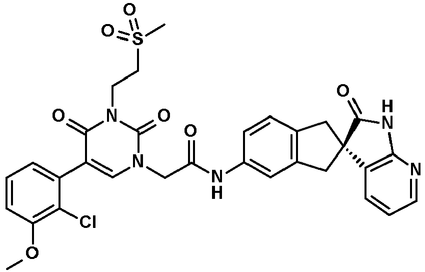

RMN ¹H (δ, DMSO) : 3.08-3.12 (m, 5H); 3.33-3.42 (m, 4H); 3.90 (s, 3H); 4.30 (t, J = 7.2 Hz, 2H); 4.69 (m, 2H); 6.87 (dd, J = 7.3, 5.3 Hz, 1H); 6.93 (dd, J = 7.6, 1.4 Hz, 1H); 7.16-7.24 (m, 3H); 7.34-7.39 (m, 2H); 7.61 (m, 1H); 7.90 (s, 1H); 8.07 (dd, J = 5.3, 1.6 Hz, 1H); 10.35 (s, 1H); 11.08 (s, 1H).

### Composé 170: 5-(2,3-dichlorophenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide (example comparatif)

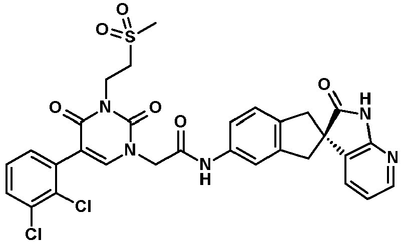

RMN ¹H (δ, DMSO) : 3.03-3.15 (m, 5H), 3.33-3.46 (m, 4H), 4.31 (dd, J = 8.4, 6.1 Hz, 2H), 4.69 (s, 2H), 6.87 (dd, J = 7.3, 5.3 Hz, 1H), 7.18 (dd, J = 7.3, 1.6 Hz, 1H), 7.24 (d, J = 8.1 Hz, 1H), 7.35 (dd, J = 7.7, 1.6 Hz, 1H), 7.38 (dd, J = 8.1, 2.0 Hz, 1H), 7.44 (t, J = 7.9 Hz, 1H), 7.61 (d, J = 2.0 Hz, 1H), 7.70 (dd, J = 8.0, 1.6 Hz, 1H), 8.00 (s, 1H), 8.07 (dd, J = 5.2, 1.6 Hz, 1H), 10.36 (s, 1H), 11.08 (br-s, 1H).

### composé 171: 5-(2,3-dimethoxyphenyl)-3-[(1R)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

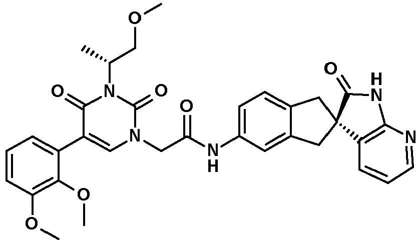

RMN ¹H (δ, DMSO) : 1.34 (d, J = 6.8 Hz, 3H), 3.08 (m, 2H), 3.24 (s, 3H), 3.36 (m, 2H), 3.54 (m, 1H), 3.70 (s, 3H), 3.83 (s, 3H), 3.93 (m, 1H), 4.63 (s, 2H), 5.15 (m, 1H), 6.80 (m, 1H), 6.87 (m, 1H), 7.07 (d, J = 4.5 Hz, 2H), 7.18 (d, J = 7.4 Hz, 1H), 7.23 (d, J = 8.2 Hz, 1H), 7.39 (d, J = 8.1 Hz, 1H), 7.61 (s, 1H), 7.70 (s, 1H), 8.06 (dd, J = 5.3, 1.5 Hz, 1H), 10.32 (s, 1H), 11.08 (s, 1H).

### composé 172: 5-(2-chloro-3-fluoro-phenyl)-3-(2-methylsulfonylethyl)-pyrimidine-2,4-dione-1-[(2R)-2'-oxospiro[1,3-dihydroindene-2,3'-1H-pyrrolo[2,3-b]pyridine]-5-yl]-acetamide (example comparatif)

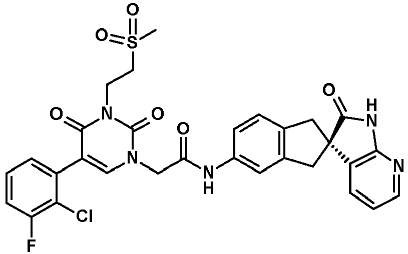

RMN ¹H (δ, DMSO) : 3.09 (m, 5H), 3.28-3.39 (m, 2H), 3.42 (t, J = 7.3 Hz, 2H), 4.31 (t, J = 7.2 Hz, 2H), 4.70 (s, 2H), 6.87 (m, 1H), 7.18 (m, 1H), 7.23 (m, 2H), 7.38 (d, J = 8.1 Hz, 1H), 7.42 - 7.52 (m, 2H), 7.61 (s, 1H), 8.01 (s, 1H), 8.07 (d, J = 5.2 Hz, 1H), 10.36 (s, 1H), 11.08 (s, 1H).

### composé 173: 5-(2-chloro-3-fluoro-phenyl)-3-(3-methylsulfonylpropyl)-pyrimidine-2,4-dione-1-[(2R)-2'-oxospiro[1,3-dihydroindene-2,3'-1H-pyrrolo[2,3-b]pyridine]-5-yl]-acetamide (example comparatif)

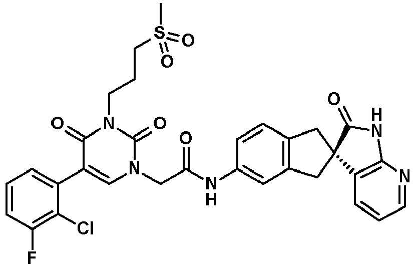

RMN ¹H (δ, DMSO) : 1.96-2.04 (m, 2H) ; 2.98 (s, 3H); 3.09 (m, 2H) ; 3.18 m, 2H); 3.33-3.37 (m, 2H) ; 4.01 (t, J = 7.0 Hz, 2H); 4.69 (s, 2H); 6.87 (dd, J = 7.3, 5.3 Hz, 1H); 7.18 (dd, J = 7.3, 1.6 Hz, 1H); 7.22-7.25 (m, 2H); 7.39 (m, 1H); 7.44-7.47 (m, 2H); 7.61 (m, 1H); 7.99 (s, 1H); 8.07 (dd, J = 5.3, 1.6 Hz, 1H); 10.36 (s, 1H); 11.08 (s, 1H).

### composé 174: 5-(2,3-dichlorophenyl)-3-methyl-pyrimidine-2,4-dione-1-[(2R)-2'-oxospiro[1,3-dihydroindene-2,3'-1H-pyrrolo[2,3-b]pyridine]-5-yl]-acetamide (example comparatif)

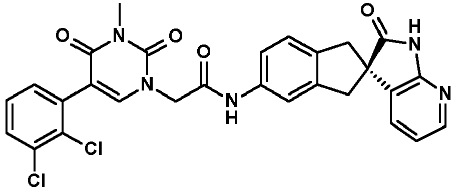

RMN ¹H (δ, DMSO) : 3.09 (dd, J = 16.1, 11.9 Hz, 2H), 3.26 (s, 3H), 3.28-3.40 (m, 2H), 4.68 (s, 2H), 6.87 (dd, J = 7.3, 5.3 Hz, 1H), 7.18 (dd, J = 7.3, 1.6 Hz, 1H), 7.24 (d, J = 8.1 Hz, 1H), 7.34 (dd, J = 7.7, 1.7 Hz, 1H), 7.39 (dd, J = 8.1, 2.0 Hz, 1H), 7.43 (t, J = 7.8 Hz, 1H), 7.61 (d, J = 2.0 Hz, 1H), 7.70 (dd, J = 8.0, 1.6 Hz, 1H), 7.96 (s, 1H), 8.07 (dd, J = 5.2, 1.7 Hz, 1H), 10.35 (s, 1H), 11.09 (s, 1H).

### composé 175: 5-(2-chloro-3-fluoro-phenyl)-3-[(1S)-1-methyl-2-methylsulfonyl-ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

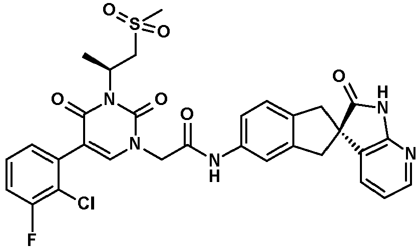

RMN ¹H (δ, DMSO) : 1.24 (d, J = 7.0 Hz, 3H), 3.05 (s, 6H), 3.33-3.40 (m, 1H), 3.54 (m, 1H), 4.16 - 4.36 (m, 2H), 4.70 (s, 2H), 6.87 (m, 1H), 7.19 (dd, J = 7.5, 1.7 Hz, 1H), 7.21 - 7.28 (m, 2H), 7.37 (dd, J = 8.3, 2.0 Hz, 1H), 7.42 - 7.52 (m, 2H), 7.61 (m, 1H), 8.03 (s, 1H), 8.07 (dd, J = 5.1, 1.9 Hz, 1H), 10.35 (s, 1H), 11.08 (s, 1H).

### composé 176: 5-(2-chloro-3-methoxy-phenyl)-3-[2-methoxy-1-(methoxymethyl)ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

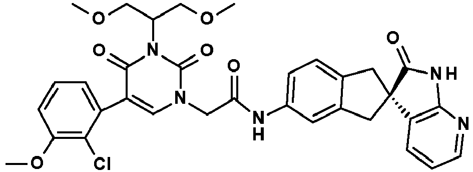

RMN ¹H (δ, DMSO) : 3.04-3.11 (m, 2H) ; 3.23 (s, 6H); 3.33-3.37 (m, 2H); 3.60 (m, 2H); 3.84 (m, 2H); 3.89 (s, 3H); 4.65 (s, 2H); 5.26 (br s, 1H); 6.87 (m, 1H); 6.93 (dd, J = 7.6, 1.4 Hz, 1H); 7.16-7.19 (m, 2H); 7.24 (d, J = 8.2 Hz, 1H); 7.33-7.39 (m, 2H); 7.62 (m, 1H); 7.84 (s, 1H); 8.07 (dd, J = 5.3, 1.6 Hz, 1H); 10.34 (s, 1H); 11.08 (s, 1H).

### composé 177: 5-(2,3-dichlorophenyl)-3-[(1R)-2-methoxy-1-methyl-ethyl]- -pyrimidine-2,4-dione-1-[(2R)-2'-oxospiro[1,3-dihydroindene-2,3'-1H-pyrrolo[2,3-b]pyridine]-5-yl]-acetamide (example comparatif)

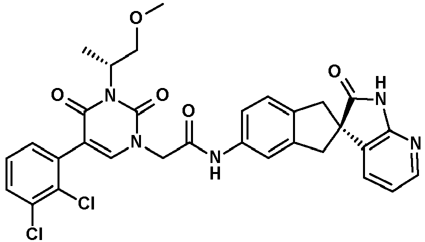

RMN ¹H (δ, DMSO) : 1.33 (d, J = 6.9 Hz, 3H), 3.08 (m, 2H), 3.23 (s, 3H), 3.33-3.40 (m, 2H), 3.54 (m, 1H), 3.92 (m, 1H), 4.65 (s, 2H), 5.14 (m, 1H), 6.87 (m, 1H), 7.18 (dd, J = 7.3, 1.7 Hz, 1H), 7.24 (d, J = 8.2 Hz, 1H), 7.35 (dd, J = 7.7, 1.6 Hz, 1H), 7.38 (dd, J = 8.1, 2.0 Hz, 1H), 7.43 (t, J = 7.9 Hz, 1H), 7.62 (m, 1H), 7.69 (dd, J = 8.0, 1.5 Hz, 1H), 7.92 (s, 1H), 8.07 (dd, J = 5.3, 1.7 Hz, 1H), 10.35 (s, 1H), 11.08 (s, 1H).

### composé 178: 5-(2,3-dichlorophenyl)-3-[(1S)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

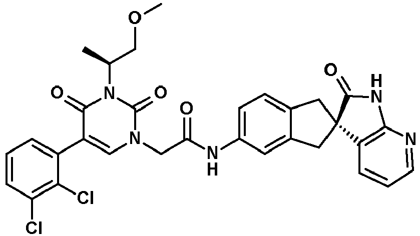

RMN ¹H (δ, DMSO) : 1.33 (d, J = 6.9 Hz, 3H), 3.01 - 3.14 (m, 2H), 3.23 (s, 3H), 3.40 (m, 2 H), 3.54 (m, 1H), 3.92 (m, 1H), 4.64 (s, 2H), 5.14 (m, 1H), 6.87 m, 1H), 7.18 (dd, J = 1.7, 7.3 Hz, 1H), 7.24 (d, J = 8.2 Hz, 1H), 7.30 - 7.47 (m, 3H), 7.62 (m, 1H), 7.69 (dd, J = 1.6, 8 Hz, 1H), 7.92 (s, 1H), 8.07 (dd, J = 1.6, 5.3 Hz, 1H), 10.34 (s, 1H), 11.08 (s, 1H).

### composé 179: 5-(2-chloro-3-fluoro-phenyl)-3-[2-methoxy-1-(methoxymethyl)ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

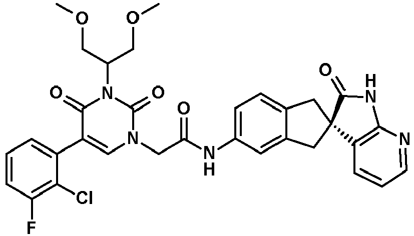

RMN ¹H (δ, DMSO) : 3.02 - 3.14 (m, 2H), 3.24 (s, 6H), 3.33 - 3.39 (m, 2H), 3.60 (m, 2H), 3.85 (m, 2H), 4.66 (s, 2H), 5.27 (br-s, 1H), 6.87 (m, 1H), 7.15 - 7.21 (m, 1H), 7.23 (m, 2H), 7.32 - 7.43 (m, 1H), 7.42 - 7.53 (m, 2H), 7.62 (s, 1H), 7.95 (s, 1H), 8.07 (dd, J = 1.7, 5.4 Hz, 1H), 10.34 (s, 1H), 11.08 (s, 1H).

### composé 180: N-[2-[5-(2-chloro-3-fluoro-phenyl)-2,4-dioxo-pyrimidin-3-yl]ethyl]acetamide-1-[(2R)-2'-oxospiro[1,3-dihydroindene-2,3'-1H-pyrrolo[2,3-b]pyridine]-5-yl]-acetamide (example comparatif)

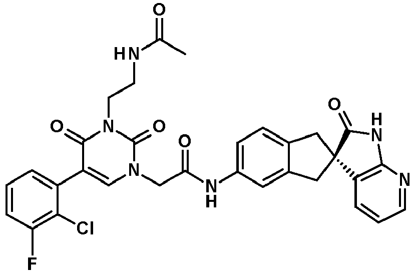

RMN ¹H (δ, DMSO) : 1.75 (s, 3H), 2.98-3.15 (m, 2H), 3.27-3.40 (m, 4H), 3.95 (t, J = 6.3 Hz, 2H), 4.68 (s, 2H), 6.87 (m, 1H), 7.18 (dd, J = 7.2, 1.6 Hz, 1H), 7.20 - 7.28 (m, 2H), 7.39 (dd, J = 8.1, 2.0 Hz, 1H), 7.42-7.51 (m, 2H), 7.61 (m, 1H), 7.93 (t, J = 6.0 Hz, 1H), 7.95 (s, 1H), 8.07 (dd, J = 5.3, 1.7 Hz, 1H), 10.32 (s, 1H), 11.08 (s, 1H).

### composé 181: 5-(2-chloro-3-fluoro-phenyl)-3-[(1R)-2-methoxy-1-methyl-ethyl] -pyrimidine-2,4-dione-1-[(2R)-2'-oxospiro[1,3-dihydroindene-2,3'-1H-pyrrolo[2,3-b]pyridine]-5-yl]-acetamide (example comparatif)

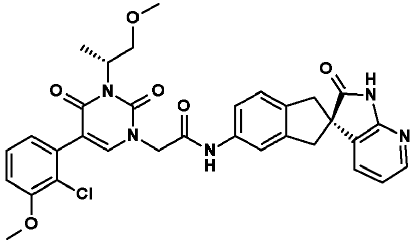

RMN ¹H (δ, DMSO) : 1.33 (d, J = 6.9 Hz, 3H); 3.08 (m, 2H); 3.23 (s, 3H); 3.36 (m, 2H); 3.54 (m, 1H); 3.89-3.91 (m, 4H); 4.64 (s, 2H); 5.13 (m, 1H); 6.87 (m, 1H); 6.93 (d, J = 7.6 Hz, 1H); 7.18 (m, 2H); 7.24 (d, J = 7.7 Hz, 1H); 7.33-7.39 (m, 2H); 7.63 (s, 1H); 7.82 (s, 1H); 8.07 (d, J = 5.2 Hz, 1H); 10.33 (s, 1H); 11.08 (s, 1H).

### composé 182: 5-(2,3-dichlorophenyl)-3-[2-methoxy-1-(methoxymethyl)ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

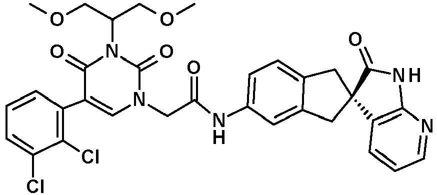

RMN ¹H (δ, DMSO) : 3.04-3.11 (m, 2H) ; 3.24 (s, 6H); 3.33-3.34 (m, 2H); 3.60 (m, 2H); 3.85 (m, 2H); 4.66 (s, 2H); 5.22-5.29 (br-s, 1H); 6.87 (dd, J = 7.3, 5.3 Hz, 1H); 7.18 (dd, J = 7.3, 1.6 Hz, 1H); 7.24 (d, J = 8.2 Hz, 1H); 7.33-7.44 (m, 3H); 7.62 (m, 1H); 7.69 (dd, J = 8.0, 1.6 Hz, 1H); 7.94 (s, 1H); 8.07 (dd, J = 5.3, 1.6 Hz, 1H); 10.35 (s, 1H); 11.08 (s, 1H).

### composé 183: 5-(2-chloro-3-fluoro-phenyl)-3-[(1R)-2-methoxy-1-methyl-ethyl]-pyrimidine-2,4-dione-1-[(2R)-2'-oxospiro[1,3-dihydroindene-2,3'-1H-pyrrolo[2,3-b]pyridine]-5-yl]-acetamide (example comparatif)

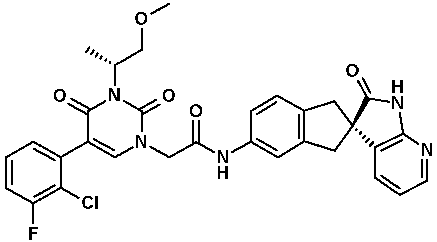

RMN ¹H (δ, DMSO) : 1.34 (d, J = 7.0 Hz, 3H), 3.08 (m, 2H), 3.23 (s, 3H), 3.36 (m, 2H), 3.54 (m, 1H), 3.92 (m, 1H), 4.65 (s, 2H), 5.14 (q, J = 7.2 Hz, 1H), 6.87 (m, 1H), 7.18 (dd, J = 7.2, 1.7 Hz, 1H), 7.21 - 7.28 (m, 2H), 7.38 (dd, J = 8.1, 1.9 Hz, 1H), 7.41-7.50 (m, 2H), 7.63 (m, 1H), 7.93 (s, 1H), 8.07 (dd, J = 5.3, 1.6 Hz, 1H), 10.34 (s, 1H), 11.08 (s, 1H).

### Exemple 184: 5-(2-Chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-piperidin-1-yl]-2-oxo-ethyl}-1H-pyrimidine-2,4-dione (schéma réactionnel n°4, composé 184) (example comparatif)

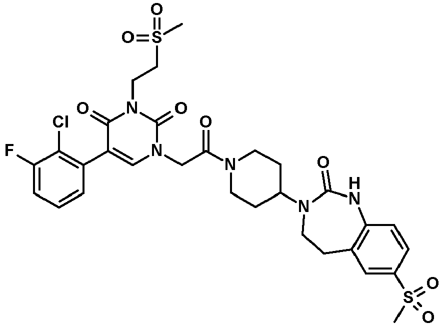

### 184.1 : 2-(5-méthylsulfanyl-2-nitro-phényl)acetonitrile

Une solution de 6g (47mmoles) de (4-chloro-phénoxy)-acétonitrile et 6g (40mmoles) de 1-méthylsulfanyl-4-nitro-benzène dans 60mL de N,N-diméthylformamide est ajouté goutte à goutte à une solution préalablement refroidie à -10°C de 10,6g (94mmoles) de potassium tert-butoxyde dans 120mL de N,N-diméthylformamide. La réaction est agitée à -10°C pendant 45min avant d'être hydrolysée par une solution glaciale d'acide chlorhydrique concentré puis extraite par de l'acétate d'éthyle. La phase organique est lavée à l'eau puis avec une solution saturée de chlorure de sodium, séchée sur sulfate de magnésium, filtrée puis concentrée sous vide. Le brut reactionnel est repris dans une solution 1:1 Heptane:AcOEt et après trituration pendant 1 heure, le mélange est filtré et le solide lavé avec une solution 1:1 Hept:AcOEt pour obtenir 2,9g de 2-(5-méthylsulfanyl-2-nitro-phényl)acétonitrile (58%) sous la forme d'un solide.

### 184.2 : 2-(5-méthylsulfanyl-2-nitro-phényl)éthanamine

28mL d'une solution 1M de boranne complexé au tétrahydrofuranne dans le tétrahydrofuranne est ajoutée goutte à goutte à température ambiante à une solution de 2,9g (14 mmoles) de 2-(5-méthylsulfanyl-2-nitro-phényl)acétonitrile dans 18mL de tétrahydrofuranne.Le mélange est ensuite chauffé à reflux pendant 3h. Le mélange est ensuite refroidi à température ambiante et 6mL de méthanol sont ajoutés lentement. Après 10min d'agitation et de méthanolyse du borane, le mélange est concentré sous vide. Le résidu est repris dans du 2-méthyl-tétrahydrofuranne et la phase organique lavée avec une solution aqueuse d'hydroxyde de sodium 1N. La phase aqueuse est extraite par trois fois au 2-méthyl-tétrahydrofuranne et les phases organiques sont rassemblées et concentrées partiellement. 1mL d'acide acétique est ajouté doucement et le mélange est laissé sous lente agitation pour revenir à température ambiante. Le milieu est donc concentré sous vide 3g (79%) de 2-(5-méthylsulfanyl-2-nitro-phényl)éthanamine est obtenu sous la forme d'une huile.

### 184.3 : 4-[2-(5-méthylsulfanyl-2-nitro-phenyl)-éthylamino]-piperidine-1-carboxylate de tert-butyle

3g (14 mmoles) 2-(5-méthylsulfanyl-2-nitro-phényl)éthanamine et 3,1g (15,4 mmoles) de 1-Boc-4-pipéridone sont ajoutés dans 40mL de 2-méthyltétrahydrofuranne. Le milieu réctionnel est peu soluble mais 4,5g (21 mmoles) de sodium triacétoxyborohydrure sont ajoutés lentement par portions. Le milieu est agité à température ambiante pendant 12h puis hydrolysé avec 15mL d'une solution aqueuse d'hydroxyde de sodium de concentration 2N et extrait avec du 2-méthyl-tétrahydrofuranne. La phase organique est relavée à l'eau jusqu'à pH=6, séchée sur sulfate de magnésium puis concentrée. 6,2g (100%) de 4-[2-(5-méthylsulfanyl-2-nitro-phényl)-éthylamino]-pipéridine-1-carboxylate de tert-butyle sont obtenus sous la forme d'une huile orange.

### 184.4 : 4-[2-(2-amino-5-méthylsulfanyl-phényl)-éthylamino]-pipéridine-1-carboxylate de tert-butyle

3,1g (56 mmoles) de fer et 0,4g (7 mmoles) de chlorure d'ammonium sont ajoutés à une solution de 5,5g (14 mmoles) de 4-[2-(5-méthylsulfanyl-2-nitro-phényl)-éthylamino]-pipéridine-1-carboxylate de tert-butyle dans 14mL de méthanol, 14mL de 2-méthyl-tétrahydrofuranne (14.00 ml) et 14mL d'eau. Le milieu réactionnel non homogène est chauffé au reflux pendant 3h. Une fois la reaction terminée, le milieu est filtré sur célite et le précipité est rincé avec du méthanol .Le filtrat est concentré jusqu'à l'élimination totale du méthanol puis repris dans du 2-méthyl-tétrahydrofuranne et hydrolysé avec 10mL d'une solution d'EDTA à 10%. La phase organique obtenue est lavée avec de l'eau, séchée sur sulfate de magnésium, filtrée et évaporée. 6,1g (100%) de résidu brut de 4-[2-(2-amino-5-méthylsulfanylphényl)-éthylamino]-pipéridine-1-carboxylate de tert-butyle sont obtenus sous la forme d'un solide orange.

### 184.5 : 4-(7-méthylsulfanyl-2-oxo-1,2,4,5-tétrahydro-benzo[d][1,3]diazepin-3-yl)-pipéridine-1-carboxylate de tert-butyle

2,5g (20 mmoles) de N,N'-carbonuldiimidazole sont additionnés par portions sur une solution de 5,1g (14 mmoles) de 4-[2-(2-amino-5-méthylsulfanyl-phényl)-éthylamino]-pipéridine-1-carboxylate de tert-butyle dans 77mL d'acétonitrile. Le milieu réactionnel est agité à température ambiante pendant 18 heures puis dilué par de l'acétate d'éthyle et hydrolysé par une solution aqueuse saturée de chlorure d'ammonium. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le résidu brut est purifié par chromatographie sur gel de silice élué par un mélange heptane / acétate d'éthyle en augmentant la polarité de 7/3 à 5/5. 2,4g (44%) de 4-[2-(2-amino-5-méthylsulfanyl-phényl)-éthylamino]-pipéridine-1-carboxylate de tert-butyle sont obtenus.

### 184.5 : 7-méthylsulfanyl-3-pipéridin-4-yl-1,3,4,5-tetrahydro-benzo[d][1,3]diazepin-2-one

0,8mL (8 mmoles) d'acide trifluoroacétique sont ajoutés à une solution de 1,2g (3 mmoles) de 4-(7-méthylsulfanyl-2-oxo-1,2,4,5-tétrahydro-benzo[d][1,3]diazepin-3-yl)-pipéridine-1-carboxylate de tert-butyle dans 7mL de dichlorométhane. Le mélange est agité 12 heures à température ambiante puis concentré sous vide. Le brut réactionnel est repris dans du toluène et co-evaporé plusieurs fois. 0,8g (90%) de 7-méthylsulfanyl-3-pipéridin-4-yl-1,3,4,5-tetrahydro-benzo[d][1,3]diazepin-2-one sont obtenus et engagés dans l'étape 184.9 sans purification supplémentaire.

### 184.6 : [5-bromo-3-(2-méthylsulfanyl-éthyl)-2,4-dioxo-3,4-dihydro-2H-pyrimidin-1-yl]-acétate de méthyle

11,8g (85,5 mmoles) de carbonate de potassium puis 10,7mL (109 mmoles) de 2-chloroéthylméthyl sulfide sont additionnés sur une solution de 15g (57 mmoles) de 2-(5-bromo-2,4-dioxo-3,4-dihydro-2H-pyrimidin-1-yl)-acétate de méthyle dans 250mL de N,N-diméthylformamide. Le milieu réactionnel est chauffé à 60°C pendant 9h puis hydrolysé et dilué par de l'acétate d'éthyle. Le produit est extrait à l'acétate d'éthyle. Les phases organiques sont rassemblées, lavées deux fois par de l'eau et une fois par une solution aqueuse saturée de chlorure de sodium, séchées sur sulfate de magnésium, filtrées et concentrées sous vide. Le produit brut est purifié par chromatographie sur gel de silice élué par un mélange heptane/acétate d'éthyle 60/40. 13,3g (69%) de [5-bromo-3-(2-méthylsulfanyl-éthyl)-2,4-dioxo-3,4-dihydro-2H-pyrimidin-1-yl]-acétate de méthyle sont obtenus sous la forme d'un solide blanc.

### 184.7 : [5-(2-chloro-3-fluoro-phényl)-3-(2-méthylsulfanyl-éthyl)-2,4-dioxo-3,4-dihydro-2H-pyrimidin-1-yl]-acétate de méthyle

267mg (0,3 mmoles) de dichlorure de 1,1'-bis(diphénylphosphino)ferrocènepalladium (II) complexé au dichlorométhane sont additionnés à une solution de 2,2g (6,5 mmoles) de [5-bromo-3-(2-méthylsulfanyl-éthyl)-2,4-dioxo-3,4-dihydro-2H-pyrimidin-1-yl]-acétate de méthyle, de 2,1g (19,6 mmoles) de carbonate de sodium et de 2,5g (8,5 mmoles) d'acide 2-chloro-3-fluoro phénylboronique. dans 220mL de 1,4-dioxanne et 22mL d'eau, préalablement dégazée sous vide/azote pendant 5min. Le milieu réactionnel est ensuite chauffé à 100°C pendant 1h30 puis hydrolysé et dilué par de l'acétate d'éthyle. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le produit brut est purifié par chromatographie sur gel de silice HP 15 élué par un mélange heptane/acétate d'éthyle 70/30. 1,4g (55%) de [5-(2-chloro-3-fluoro-phényl)-3-(2-méthylsulfanyl-éthyl)-2,4-dioxo-3,4-dihydro-2H-pyrimidin-1-yl]-acétate de méthyle sont obtenus sous la forme d'un solide crème.

### 184.8 : acide [5-(2-chloro-3-fluoro-phenyl)-3-(2-methylsulfanyl-ethyl)-2,4-dioxo-3,4-dihydro-2H-pyrimidin-1-yl]-acétique

5,4mL (5,4 mmoles) d'hydroxyde de lithium monohydrate et 2,8mL d'eau sont additionnés sur une solution de 1,4g (3,6 mmoles) de [5-(2-chloro-3-fluoro-phényl)-3-(2-méthylsulfanyl-éthyl)-2,4-dioxo-3,4-dihydro-2H-pyrimidin-1-yl]-acétate de méthyle dans 28mL de tétrahydrofuranne. Le milieu réactionnel est agité à température ambiante pendant 2h puis hydrolysé et dilué par de l'acétate d'éthyle. Le pH de la phase aqueuse est amené à pH acide par une solution aqueuse d'acide chlorhydrique de concentration 1N. Le produit est extrait à l'acétate d'éthyle, les phases organiques sont rassemblées, lavées une fois par de l'eau et une fois par une solution aqueuse saturée de chlorure de sodium, séchées sur sulfate de magnésium, filtrées puis concentrées sous vide. 1,3g (98%) d'acide [5-(2-chloro-3-fluoro-phényl)-3-(2-méthylsulfanyl-éthyl)-2,4-dioxo-3,4-dihydro-2H-pyrimidin-1-yl]-acétique sont obtenus sous la forme d'un solide crème.

### 184.9 : 5-(2-chloro-3-fluoro-phényl)-3-(2-méthylsulfanyl-éthyl)-1-{2-[4-(7-méthylsulfanyl-2-oxo-1,2,4,5-tetrahydro-benzo[d][1,3]diazepin-3-yl)-pipéridin-1-yl]-2-oxo-éthyl}-1H-pyrimidine-2,4-dione

185mg (1 mmole) de chlorhydrate de 1-éthyl-3-(3-diméthylaminopropyl)carbodiimide et 107mg (1 mmole) de 1-oxy-pyridin-2-ol sont additionnés à 0,3g (0,8 mmoles) d'acide [5-(2-chloro-3-fluoro-phényl)-3-(2-méthylsulfanyl-éthyl)-2,4-dioxo-3,4-dihydro-2H-pyrimidin-1-yl]-acétique dans 2mL de N,N-diméthylformamide. Le milieu réactionnel est agité pendant 5min puis 281mg (1 mmole) de 7-méthylsulfanyl-3-pipéridin-4-yl-1,3,4,5-tetrahydro-benzo[d][1,3]diazepin-2-one (préparé comme décrit en 184.4) et 0,15mL (1 mmole) de triéthylamine sont ajoutés. Le milieu réactionnel est agité à température ambiante pendant 12 heures puis dilué par du dichlorométhane et de l'eau. La phase aqueuse est extraite trois fois au dichlorométhane, les phases organiques sont combinées et lavées avec une solution aqueuse saturée d'hydrogénocarbonate de sodium puis avec une solution aqueuse saturée de chlorure de sodium. Après séchage sur sulfate de magnésium, la solution est filtrée puis concentrée sous vide. Le résidu brut obtenu est purifié par chromatographie sur gel de silice élué avec un mélange heptane / acétate d'éthyle 7/3. 520mg (100%) de 5-(2-chloro-3-fluoro-phényl)-3-(2-méthylsulfanyl-éthyl)-1-{2-[4-(7-méthylsulfanyl-2-oxo-1,2,4,5-tetrahydro-benzo[d][1,3]diazepin-3-yl)-pipéridin-1-yl]-2-oxo-éthyl}-1H-pyrimidine-2,4-dione sont obtenus sous forme d'un solide blanc.

### 184.10 : 5-(2-chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-pipéridin-1-yl]-2-oxo-éthyl}-1H-pyrimidine-2,4-dione (composé 184)

50mg (0,04 mmole) d'ammonium molybdate tétrahydrate et 146uL (24 mmoles) d'une solution aqueuse de peroxyde d'hydrogène 30% sont ajoutés à 517mg (0,8 mmole) de 5-(2-chloro-3-fluoro-phényl)-3-(2-méthylsulfanyl-éthyl)-1-{2-[4-(7-méthylsulfanyl-2-oxo-1,2,4,5-tétrahydro-benzo[d][1,3]diazepin-3-yl)-piperidin-1-yl]-2-oxo-ethyl}-1H-pyrimidine-2,4-dione dans 3mL d'éthanol préalablement refroidie à 0°C. La réaction est agitée à température ambiante pendant 2 heures puis hydrolysée avec de l'eau (30mL) et extraite au dichlorométhane (3x30mL). Les phases organiques sont rassemblées, lavées avec une solution aqueuse saturée de thiosulfate de sodium (30 mL × 4), de l'eau (20 mL × 2), et une solution aqueuse saturée de chlorure de sodium (20 mL × 2), puis séchée sur sulfate de magnésium, filtrées et concentrées sous vide. Après trituration du résidu brut dans l'acétate d'éthyle pendant 12 heures et filtration, 210mg (35%) de 5-(2-chloro-3-fluoro-phényl)-3-(2-méthanesulfonyl-éthyl)-1-{2-[4-(7-méthanesulfonyl-2-oxo-1,2,4,5-tétrahydro-benzo[d][1,3]diazepin-3-yl)-piperidin-1-yl]-2-oxo-éthyl}-1H-pyrimidine-2,4-dione sont obtenus.
RMN ¹H (400 MHz, DMSO): δ (ppm) 1.52-1.75 (m, 4 H); 2.68 (t, J = 11.7 Hz, 1 H); 2.98 (br t, 2 H); 3.06 (s, 3 H); 3.10 (s, 3 H); 3.14-3.18 (br m, 1 H); 3.40 (br m, 4 H); 3.95 (d, J = 13.5 Hz, 1 H); 4.27-4.43 (m, 4 H); 4.79 (s, 2 H); 7.18-7.23 (m, 2 H); 7.42-7.45 (m, 2 H); 7.54-7.58 (m, 2 H); 7.86 (s, 1 H); 9.13 (s, 1 H).

Autre composé synthétisé selon un mode opératoire analogue en utilisant les réactifs appropriés ou préalablement préparés:

### composé 185: 5-(2-Chloro-3-fluoro-phenyl)-3-isopropyl-1-{2-[4-(7-methanesulfonyl-2-oxo-1,2,4,5-tetrahydro-benzo[d][1,3]diazepin-3-yl)-piperidin-1-yl]-2-oxo-ethyl}-1H-pyrimidine-2,4-dione (example comparatif)

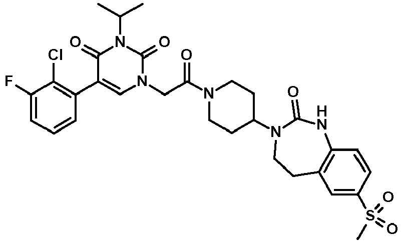

RMN ¹H (δ, DMSO) : 1.41 (d, J = 6.9 Hz, 6H), 1.57 (m, 1H), 1.70 (m, 3H), 2.64-2.77 (m, 1H), 2.93-3.05 (m, 2H), 3.12 (s, 3H), 3.17 (m, 1H), 3.37-3.48 (m, 2H), 3.95 (m, 1H), 4.28-4.50 (m, 2H), 4.64 - 4.84 (m, 2H), 5.10 (h, J = 6.8 Hz, 1H), 7.18-7.27 (m, 2H), 7.40 - 7.49 (m, 2H), 7.54-7.62 (m, 2H), 7.77 (s, 1H), 9.15 (s, 1H).

### Exemple 186: 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d][1,3]diazepin-3-yl)-piperidin-1-yl]-2-oxo-éthyl}-1H-pyrimidine-2,4-dione (schéma réactionnel n°4, composé 186) (example comparatif)

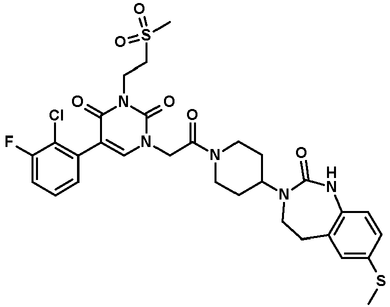

### 186.1 : acide [5-(2-chloro-3-fluoro-phényl)-3-(2-méthanesulfonyl-éthyl)-2,4-dioxo-3,4-dihydro-2H-pyrimidin-1-yl]-acétique

Une solution de 50mg (0,04 mmole) d'ammonium molybdate tétrahydrate et 146uL (24 mmoles) d'une solution aqueuse de peroxyde d'hydrogène 30% sont ajoutés à une solution d'acide [5-(2-chloro-3-fluoro-phenyl)-3-(2-methylsulfanyl-ethyl)-2,4-dioxo-3,4-dihydro-2H-pyrimidin-1-yl]-acétique (préparé comme décrit en 184.8). La réaction est agitée à température ambiante pendant 2 heures, hydrolysée avec de l'eau (30mL) et extraite avec de l'acétate d'éthyle (3x30mL). Les phases organiques sont rassemblées, lavées avec une solution aqueuse saturée de thiosulfate de sodium, de l'eau puis une solution aqueuse saturée de chlorure de sodium. Après séchage sur sulfate de sodium et filtration sous vide, 320mg (98%) d' acide [5-(2-Chloro-3-fluoro-phenyl)-3-(2-methanesulfonyl-ethyl)-2,4-dioxo-3,4-dihydro-2H-pyrimidin-1-yl]-acétique sont obtenus et utilisés dans l'étape suivante sans purification.

### 186.2 : 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d][1,3]diazepin-3-yl)-piperidin-1-yl]-2-oxo-éthyl}-1H-pyrimidine-2,4-dione (composé 186)

182mg (1 mmole) de chlorhydrate de 1-éthyl-3-(3-diméthylaminopropyl)carbodiimide et 105mg (1 mmole) de 1-oxy-pyridin-2-ol sont ajoutés à une solution de 320mg (0,8 mmole) d'acide [5-(2-chloro-3-fluoro-phényl)-3-(2-méthanesulfonyl-éthyl)-2,4-dioxo-3,4-dihydro-2H-pyrimidin-1-yl]-acétique dans 2mL N,N-diméthylformamide. Le milieu réactionnel est agité pendant 5min puis 253mg (0,9 mmole) de 7-méthylsulfanyl-3-piperidin-4-yl-1,3,4,5-tetrahydro-benzo[d][1,3]diazepin-2-one et de 0,15mL (1 mmole) de triéthylamine sont ajoutés. Le milieu réactionnel est agité à température ambiante pendant 12 heures, puis dilué par du dichlorométhane et de l'eau. La phase aqueuse est extraite trois fois au dichlorométhane, les phases organiques sont combinées et lavées avec une solution saturée d'hydrogénocarbonate de sodium, à l'eau puis avec une solution aqueuse saturée de chlorure de sodium. Après séchage sur sulfate de magnésium, la solution est filtrée puis concentrée sous vide. Après trituration du résidu brut dans l'acétate d'éthyle pendant 12 heures et filtration, 210mg (36%) de 5-(2-chloro-3-fluoro-phényl)-3-(2-méthanesulfonyl-éthyl)-1-{2-[4-(7-méthylsulfanyl-2-oxo-1,2,4,5-tetrahydro-benzo[d][1,3]diazepin-3-yl)-piperidin-1-yl]-2-oxo-éthyl}-1H-pyrimidine-2,4-dione sont obtenus.
RMN ¹H (400 MHz, DMSO): δ (ppm) 1.50-1.73 (m, 4 H); 2.38 (s, 3 H); 2.67 (t, J = 12.4 Hz, 1 H); 2.86 (d, J = 6.8 Hz, 2 H) ; 3.06 (s, 3H); 3.13 (t, J = 12.4 Hz, 1 H); 3.33-3.41 (m, 4 H); 3.94 (d, J = 13.6 Hz, 1 H); 4.29 (t, J = 7.4 Hz, 3 H); 4.41 (d, J = 12.9 Hz, 1 H); 4.78 (s, 2 H); 6.99 (d, J = 10.8 Hz, 3 H); 7.19-7.21 (m, 1 H); 7.43-7.46 (m, 2 H); 7.86 (s, 1 H); 8.55 (s, 1 H).

Les composés suivants ont été synthétisés selon un mode opératoire analogue, en utilisant les réactifs appropriés commerciaux ou préalablement préparés:

### composé 187: 5-(2-Chloro-3-fluoro-phenyl)-3-((R)-2-methoxy-1-methyl-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione (example comparatif)

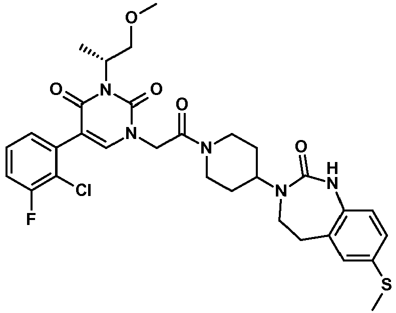

RMN ¹H (δ, DMSO) : 1.33 (d, J = 7.0 Hz, 3H), 1.56 (m, 1H), 1.67 (m, 3H), 2.40 (s, 3H), 2.68 (m, 1H), 2.88 (m, 2H), 3.14 (m, 1H), 3.23 (s, 3H), 3.34-3.42 (m, 2H), 3.54 (m, 1H), 3.83-4.04 (m, 2H), 4.33 (m, 1H), 4.43 (d, J = 12.8 Hz, 1H), 4.75 (m, 2H), 5.15 (m, 1H), 7.01 (m, 3H), 7.21 (m, 1H), 7.45 (m, 2H), 7.79 (s, 1H), 8.57 (s, 1H).

### composé 188: 5-(2-Chloro-3-fluoro-phenyl)-3-(2-methoxy-ethyl)-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione (example comparatif)

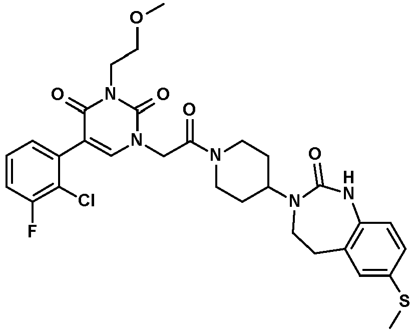

RMN ¹H (δ, DMSO) : 1.45-1.61 (m, 1H), 1.70 (m, 3H), 2.40 (s, 3H), 2.61-2.75 (m, 1H), 2.82-2.93 (m, 2H), 3.09-3.21 (m, 1H), 3.26 (s, 3H), 3.34-3.42 (m, 2H), 3.52 (t, J = 6.1 Hz, 2H), 3.94 (d, J = 13.5 Hz, 1H), 4.07 (m, 2H), 4.32 (m, 1H), 4.43 (d, J = 13.0 Hz, 1H), 4.78 (m, 2H), 6.91-7.09 (m, 3H), 7.17-7.29 (m, 1H), 7.36-7.54 (m, 2H), 7.83 (s, 1H), 8.57 (s, 1H).

### composé 189: 5-(2-Chloro-3-fluoro-phenyl)-3-isopropyl-1-{2-[4-(7-methylsulfanyl-2-oxo-1,2,4,5-tetrahydro-benzo[d][1,3]diazepin-3-yl)-piperidin-1-yl]-2-oxo-ethyl}-1H-pyrimidine-2,4-dione (example comparatif)

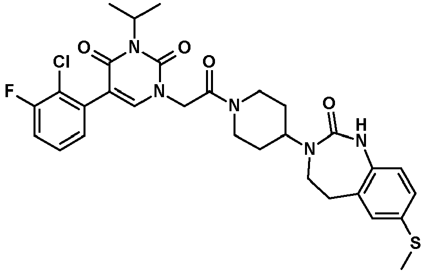

RMN ¹H (δ, DMSO) : 1.41 (d, J = 7.0 Hz, 6H), 1.56 (m, 1H), 1.63-1.86 (m, 3H), 2.40 (s, 3H), 2.68 (m, 1H), 2.78-2.95 (m, 2H), 3.15 (m, 1H), 3.33-3.45 (m, 2H), 3.94 (d, J = 13.6 Hz, 1H), 4.31 (m, 1H), 4.43 (d, J = 12.9 Hz, 1H), 4.74 (m, 2H), 5.11 (p, J = 6.9 Hz, 1H), 7.01 (m, 3H), 7.15-7.27 (m, 1H), 7.35-7.52 (m, 2H), 7.77 (s, 1H), 8.56 (s, 1H).

### Exemple 190 : 3-(1-{2-[5-(2,3-difluoro-phenyl)-3-méthyl-2-oxo-3,4-dihydro-2H-pyrimidin-1-yl]-acetyl}-piperidin-4-yl)-1,3,4,5-tetrahydro-benzo[d][1,3]diazepin-2-one (schéma réactionnel n°3, composé 190) (example comparatif)

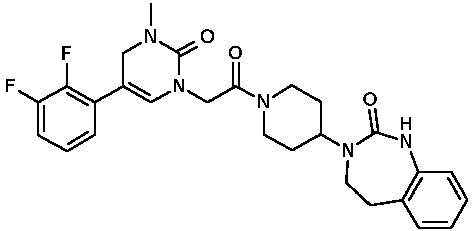

### 190.1 : 5-bromo-1-(4-méthoxy-benzyl)-1H-pyrimidin-2-one

4mL (30 mmoles) de triéthylamine puis 4,3mL de chlorure de 4-méthoxybenzyle sont ajoutés à une solution de 5g (30 mmoles) de 5-bromo-2-hydroxypyrimidine dans 150mL de dichlorométhane. Le milieu réactionnel est agité à température ambiante pendant 48h puis hydrolysé et dilué par du dichlorométhane. Le produit est extrait au dichlorométhane puis à l'acétate d'éthyle. Les phases organiques sont rassemblées et concentrées sous vide. Le produit brut obtenu est repris dans 60mL d'acétate d'éthyle puis filtré. Le solide obtenu est séché à l'azote pendant 2h pour donner 4,9g (46%) de 5-bromo-1-(4-méthoxy-benzyl)-1H-pyrimidin-2-one sous forme d'un solide blanc cassé.

### 190.2 : 5-bromo-1-(4-méthoxy-benzyl)-3,4-dihydro-1H-pyrimidin-2-one

2,5mL (5,5 mmoles) d'une solution d'isopropoxyde de zirconium dans l'heptane de concentration 2M sont additionnés à 4,9g (16,6 mmoles) de 5-bromo-1-(4-méthoxy-benzyl)-1H-pyrimidin-2-one dans 392mL d'isopropanol. Le milieu réactionnel (suspension blanche au début) est chauffé à 90°C pendant 48 heures. Les solvants sont concentrés sous vide. Le résidu est repris dans du dichlorométhane et de l'eau. Le produit est extrait au dichlorométhane. Les phases organiques sont rassemblées, lavées trois fois par de l'eau puis filtrées pour éliminer l'insoluble (sels de zirconium). Le filtrat est concentré sous vide. Le produit brut est purifié par chromatographie sur gel de silice élué par un mélange heptane/acétate d'éthyle 50/50 pour donner 3,45g (70%) de 5-bromo-1-(4-méthoxy-benzyl)-3,4-dihydro-1H-pyrimidin-2-one sous la forme d'un solide blanc.

### 190.3 : 5-bromo-1-(4-méthoxy-benzyl)-3-méthyl-3,4-dihydro-1H-pyrimidin-2-one

242mg (6,1 mmoles) d'hydrure de sodium sont ajoutés par portions à une solution de 1,5g (5,1 mmoles) de 5-bromo-1-(4-méthoxy-benzyl)-3,4-dihydro-1H-pyrimidin-2-one dans 23mL de tétrahydrofuranne. Le mélange réactiionnel est agité à température ambiante pendant 10min avant l'addition de 0,5mL (7,6 mmoles) d'iodométhane. Le mélange réactionnel est agité à température ambiante pendant 1h30 puis hydrolysé et dilué par de l'acétate d'éthyle. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide pour donner 1,6g (100%) de 5-bromo-1-(4-méthoxy-benzyl)-3-méthyl-3,4-dihydro-1H-pyrimidin-2-one sous la forme d'une huile jaune. Le produit est engagé dans l'étape suivante sans purification.

### 190.4 : 5-(2,3-difluoro-phényl)-1-(4-méthoxy-benzyl)-3-méthyl-3,4-dihydro-1H-pyrimidin-2-one

297mg (0,3 mmole) de tetrakis(triphénylphosphine)palladium(0) puis 4g (25,7 mmoles) d'acide 2,3-difluorophénylboronique sont ajoutés à une solution de 1,6g (5,1 mmoles) de 5-bromo-1-(4-méthoxy-benzyl)-3-méthyl-3,4-dihydro-1H-pyrimidin-2-one et 12,9mL (25,7 mmoles) d'une solution aqueuse de carbonate de potassium de concentration 2M dans 48mL de N,N-diméthylformamide et 16mL d'eau, préalablement dégazé. Le milieu réactionnel est chauffé à 90°C pendant 1h30. Après refroidissement, le milieu réactionnel est hydrolysé puis dilué par de l'acétate d'éthyle. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le produit brut est purifié par chromatographie sur gel de silice élué par un mélange heptane/acétate d'éthyle 80/20 pour donner 0,7g (41%) de 5-(2,3-difluoro-phenyl)-1-(4-methoxy-benzyl)-3-methyl-3,4-dihydro-1H-pyrimidin-2-one sous la forme d'une huile jaune.

### 190.5 : 5-(2,3-difluoro-phényl)-3-méthyl-3,4-dihydro-1H-pyrimidin-2-one

640mg (1,9 mmoles) de 5-(2,3-difluoro-phenyl)-1-(4-methoxy-benzyl)-3-methyl-3,4-dihydro-1H-pyrimidin-2-one sont placés en solution dans 5mL d'acide trifluoroacétique. Le milieu réactionnel est agité à température ambiante pendant 30min puis concentré à sec sous vide, dilué par du dichlorométhane, et enfin hydrolysé par une solution aqueuse saturée d'hydrogénocarbonate de sodium. Le produit est extrait au dichlorométhane. Les phases organiques sont rassemblées, lavées une fois par de l'eau puis séchées sur sulfate de magnésium, filtrées et concentrées sous vide.Le produit brut obtenu est purifié par chromatographie sur gel de silice élué par un mélange dichlorométhane/acétate d'éthyle 80/20. 85mg (20%) de 5-(2,3-difluoro-phenyl)-3-méthyl-3,4-dihydro-1H-pyrimidin-2-one sont obtenus.

### 190.6 : [5-(2,3-difluoro-phényl)-3-methyl-2-oxo-3,4-dihydro-2H-pyrimidin-1-yl]-acétate de méthyle

18,2mg (0,45 mmole) d'hydrure de sodium sont ajoutés par portions à 85mg (0,4 mmole) de 5-(2,3-difluoro-phényl)-3-méthyl-3,4-dihydro-1H-pyrimidin-2-one en solution dans 4,5mL de N,N-diméthylformamide. Après 2 min d'agitation à température ambiante, 40uL (0,45 mmole) de méthylchloroacétate sont additionnés. Le milieu réactionnel est agité à température ambiante pendant 40min puis hydrolysé et dilué par de l'acétate d'éthyle. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. 100mg (89%) de [5-(2,3-difluoro-phényl)-3-methyl-2-oxo-3,4-dihydro-2H-pyrimidin-1-yl]-acétate de méthyle sont obtenus sous forme d'un solide blanc.

### 190.7: acide 5-(2,3-difluoro-phényl)-3-méthyl-2-oxo-3,4-dihydro-2H-pyrimidin-1-yl]-acétique

100mg (0,3 mmole) de [5-(2,3-difluoro-phényl)-3-méthyl-2-oxo-3,4-dihydro-2H-pyrimidin-1-yl]-acétate de méthyle sont placés en solution dans 5mL de tétrahydrofuranne et 0,5mL d'eau puis 0,5mL d'une solution aqueuse 1M d'hydroxyde de lithium monohydrate est additionné. Le milieu réactionnel est agité à température ambiante pendant 1h30, hydrolysé puis dilué par de l'acétate d'éthyle. Cette première phase acétate d'éthyle est écartée. La phase aqueuse est acidifiée à pH=5-6 par une solution aqueuse d'acide acétique de contration C=1M. Le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. 80mg (84%) d'acide [5-(2,3-difluoro-phenyl)-3-methyl-2-oxo-3,4-dihydro-2H-pyrimidin-1-yl]-acétique sont obtenus sous forme d'un solide blanc.

### 190.8 : 3-(1-{2-[5-(2,3-difluoro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one (composé 190)

De manière analogue à l'exemple 51.4, à partir de 80mg (0,3 mmoles) d'acide [5-(2,3-difluoro-phenyl)-3-methyl-2-oxo-3,4-dihydro-2H-pyrimidin-1-yl]-acétique et 70mg (0,3 mmoles) de 3-pipéridin-4-yl-1,3,4,5-tétrahydro-benzo[d] [1,3]diazépin-2-one (préparé comme décrit dans l'exemple 16.4), 80mg (55%) de 3-(1-{2-[5-(2,3-difluoro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one sont obtenus.
RMN ¹H (400 MHz, DMSO): δ (ppm) :1.59 - 1.40 (m, 1H), 1.65 (m, 3H), 2.70 - 2.56 (m, 1H), 2.88 (m, 5H), 3.07 (m, 1H), 3. 42 - 3.35 (m, 2H), 3.90 (d, J = 13.6 Hz, 1H), 4.34 - 4.17 (m, 3H), 4.39 (s, 2H), 4.44 (d, 1H), 6.86 - 6.69 (m, 2H), 7.13 - 6.91 (m, 4H), 7.36 - 7.15 (m, 2H), 8.54 (s, 1H).

Les composés suivants ont été synthétisés selon un mode opératoire analogue, en utilisant les réactifs appropriés commerciaux ou préalablement préparés:

### composé 191: 3-(1-{2-[5-(2,3-dichloro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

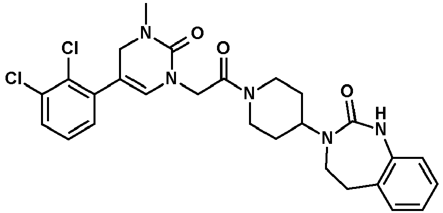

RMN ¹H (δ, DMSO) : 1.45-1.60 (m, 1H), 1.61-1.75 (m, 3H), 2.60 (m, 1H), 2.84 (s, 3H), 2.88 (m, 2H), 3.07 (m, 1H), 3.36 (m, 2H), 3.90 (m, 1H), 4.21 (d, J = 2.5 Hz, 2H), 4.25-4.35 (m, 3H), 4.43 (m, 1H), 6.41 (s, 1H), 6.78-6.82 (m, 1H), 7.01-7.04 (m, 3H), 7.30 (dd, J = 1.7-7.7 Hz, 1H), 7.36 (t, J = 7.8 Hz, 1H), 7.57 (dd, J = 1.7-7.9 Hz, 1H), 8.52 (s, 1H).

### composé 192: 3-(1-{2-[5-(2-chloro-3-fluoro-phenyl)-3-(2-methoxy-ethyl)-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

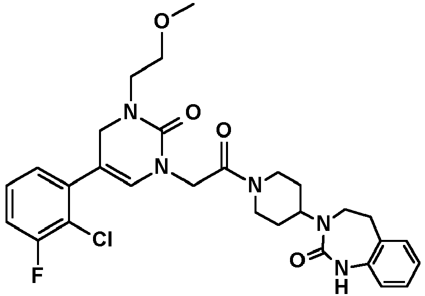

RMN ¹H (δ, DMSO) : 1.53 (m, 1 H); 1.67 (m, 3 H); 2.59-2.64 (m, 1 H); 2.89 (m, 2 H); 3.08 (m, 1 H); 3.27 (s, 3 H); 3.35-3.38 (m, 2 H); 3.46-3.50 (m, 4 H); 3.91 (d, J = 13.5 Hz, 1 H); 4.35 (m, 5 H); 4.44 (d, J = 13.0 Hz, 1 H); 6.46 (s, 1 H); 6.80-6.82 (m, 1 H); 7.04-7.06 (m, 3 H); 7.18 (m, 1 H); 7.35-7.36 (m, 2 H); 8.52 (s, 1 H).

### composé 193: 3-(1-{2-[S-(2-chloro-3-methoxy-phenyl)-3-isopropyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif) (example comparatif)

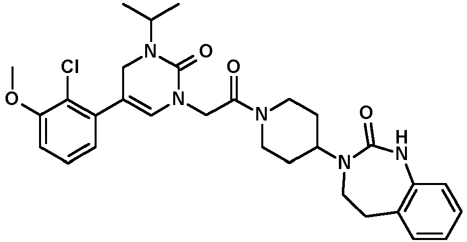

RMN ¹H (δ, DMSO) : 1.21 (d, J = 6.9 Hz, 6H), 1.65-2.00 (m, 4H), 2.67-2.82 (m, 1H), 2.94-3.07 (m, 2H), 3.20 (m, 1H), 3.35 (m, 1H), 3.52 (m, 2H), 3.91 (s, 3H), 4.04 (d, 1H), 4.18 (m, 2H), 4.34 (d, J = 16.6 Hz, 1H), 4.43 (m, 1H), 4.55 (d, J = 16.6 Hz, 1H), 4.60-4.73 (m, 2H), 6.25 (s, 1H), 6.86-6.99 (m, 3H), 7.04 (dd, J = 8.6, 1.3 Hz, 1H), 7.06-7.13 (m, 2H), 7.26 (t, J = 8.0 Hz, 1H).

### composé 194: 3-(1-{2-[5-(2,3-dichloro-phenyl)-2-oxo-3-(2,2,2-trifluoro-ethyl)-3,4-dihydro-2H-pyrimidin-1-yl]-acetyl}-piperidin-4-yl)-1,3,4,5-tetrahydro-benzo[d][1,3]diazepin-2-one (example comparatif)

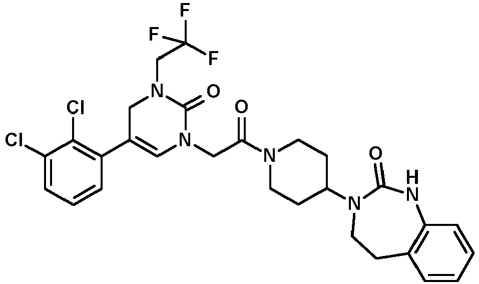

RMN ¹H (δ, DMSO) : 1.53 (m, 1H), 1.65 (m, 3H), 2.62 (m, 1H), 2.79-2.97 (m, 2H), 3.08 (m, 1H), 3.34-3.43 (m, 2H), 3.90 (d, J = 13.4 Hz, 1H), 4.17 (m, 2H), 4.24-4.52 (m, 6H), 6.45 (s, 1H), 6.80 (m, 1H), 7.03 (m, 3H), 7.25-7.51 (m, 2H), 7.56-7.72 (m, 1H), 8.53 (s, 1H).

### composé 195: 1'-[2-[5-(2,3-dichlorophenyl)-3-methyl-2-oxo-4H-pyrimidin-1-yl]acetyl]spiro[1H-pyrido[2,3-d][1,3]oxazine-4,4'-piperidine]-2-one (example comparatif)

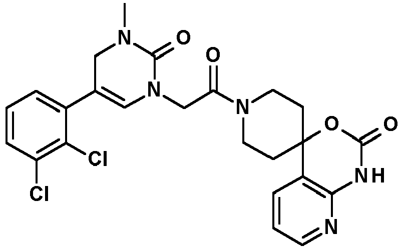

RMN ¹H (δ, DMSO) : 2.00 (m, 4H), 2.85 (s, 3H), 2.93 (m, 1H), 3.39 (m, 1H), 3.85 (d, J = 13.7 Hz, 1H), 4.22 (m, 2H), 4.37 (m, 3H), 6.41 (s, 1H), 7.09 (m, 1H), 7.22-7.45 (m, 2H), 7.58 (dd, J = 7.8, 1.8 Hz, 1H), 7.71 (d, J = 7.6 Hz, 1H), 8.21 (d, J = 4.9 Hz, 1H), 10.86 (s, 1H).

### composé 196: 5-(2,3-Dichloro-phenyl)-3-methyl-1-{2-oxo-2-[4-(2-oxo-4-phenyl-2,3-dihydro-imidazol-1-yl)-piperidin-1-yl]-ethyl}-3,4-dihydro-1H-pyrimidin-2-one(example comparatif)

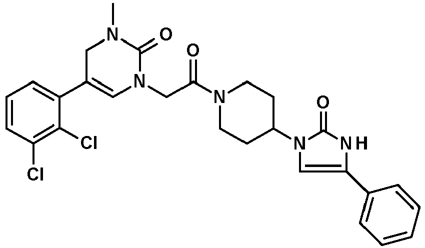

RMN ¹H (δ, DMSO) : 1.54-1.93 (m, 4H), 2.70 (m, 1H), 2.85 (s, 3H), 3.15 (m, 1H), 3.95 (d, J = 13.4 Hz, 1H), 4.11 (m, 1H), 4.22 (m, 2H), 4.37 (s, 2H), 4.48 (d, J = 13.2 Hz, 1H), 6.40 (s, 1H), 7.10-7.22 (m, 2H), 7.35 (m, 4H), 7.50 (d, J = 7.3 Hz, 2H), 7.54-7.61 (m, 1H), 10.71 (br-s, 1H).

### composé 197: 3-(1-{2-[5-(2-Chloro-3-fluoro-phenyl)-3-methyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

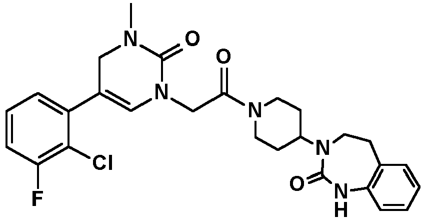

RMN ¹H (δ, DMSO) : 1.52 (m, 1H), 1.65 (m, 3H), 2.61 (m, 1H), 2.85 (s, 3H), 2.88 (m, 2H), 3.08 (m, 1H), 3.37 (m, 2H), 3.90 (d, J = 13.7 Hz, 1H), 4.11 - 4.55 (m, 6H), 6.48 (s, 1H), 6.71-6.88 (m, 1H), 7.03 (m, 3H), 7.19 (dt, J = 7.3, 1.5 Hz, 1H), 7.35 (m, 2H), 8.52 (s, 1H).

### composé 198: 3-(1-{2-[5-(2-Chloro-3-fluoro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

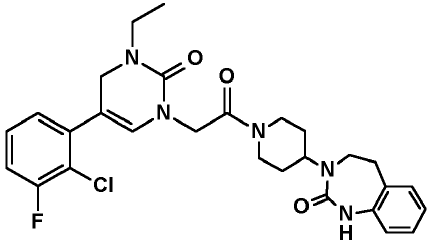

RMN ¹H (δ, DMSO) : 1.08 (t, J = 7.1 Hz, 3H), 1.41-1.59 (m, 1H), 1.66 (m, 3H), 2.60 (m, 2H), 2.80-2.93 (m, 2H), 3.08 (m, 1H), 3.34-3.45 (m, 3H), 3.90 (d, J = 13.5 Hz, 1H), 4.16-4.38 (m, 5H), 4.42 (d, J = 13.5 Hz, 1H), 6.46 (s, 1H), 6.73-6.88 (m, 1H), 7.03 (m, 3H), 7.13 - 7.23 (m, 1H), 7.27 - 7.47 (m, 2H), 8.53 (s, 1H).

### composé 199: 3-(1-{2-[5-(2,3-Dichloro-phenyl)-3-ethyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

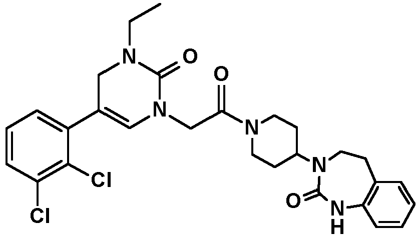

RMN ¹H (δ, DMSO) : 1.08 (t, J = 7.1 Hz, 3H), 1.52 (m, 1H), 1.64 (m, 3H), 2.61 (m, 1H), 2.88 (m, 2H), 3.08 (m, 1H), 3.36 (m, 4H), 3.90 (d, J = 13.4 Hz, 1H), 4.12-4.56 (m, 6H), 6.40 (s, 1H), 6.80 (m, 1H), 7.03 (m, 3H), 7.22-7.45 (m, 2H), 7.58 (dd, J = 7.7, 1.8 Hz, 1H), 8.52 (s, 1H).

### composé 200: 3-(1-{2-[5-(2-Chloro-3-fluoro-phenyl)-3-isopropyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

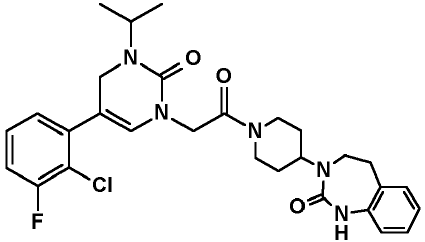

RMN ¹H (δ, DMSO) : 1.11 (d, J = 6.8 Hz, 6H), 1.40-1.57 (m, 1H), 1.67 (m, 3H), 2.53-2.71 (m, 1H), 2.79-2.96 (m, 2H), 2.98-3.16 (m, 1H), 3.34-3.41 (m, 2H), 3.91 (d, J = 13.8 Hz, 1H), 4.09 (m, 2H), 4.21-4.60 (m, 5H), 6.46 (s, 1H), 6.70-6.88 (m, 1H), 6.93-7.10 (m, 3H), 7.15-7.23 (m, 1H), 7.27-7.44 (m, 2H), 8.53 (s, 1H).

### composé 201: 3-(1-{2-[5-(2,3-Dichloro-phenyl)-3-isopropyl-2-oxo-3,4-dihydro-2H-pyrimidin-1-yl]-acetyl}-piperidin-4-yl)-1,3,4,5-tetrahydro-benzo[d][1,3]diazepin-2-one (example comparatif)

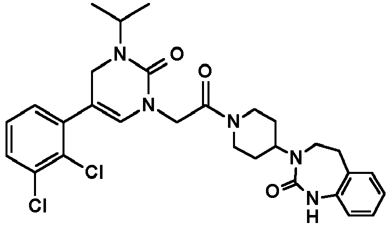

RMN ¹H (δ, DMSO) : 1.17 (d, J = 6.8 Hz, 6H), 1.58 (m, 1H), 1.71 (m, 3H),2.56- 2.73 (m, 1H), 2.94 (m, 2H), 3.14 (m, 1H), 3.41 - 3.51 (m, 2H), 3.97 (d, J = 13.9 Hz, 1H), 4.12 (m, 2H), 4.33 - 4.44 (m, 3H), 4.46-4.62 (m, 2H), 6.46 (s, 1H), 6.86 (m, 1H), 7.09 (m, 3H), 7.33-7.47 (m, 2H), 7.64 (dd, J = 8.0, 1.8 Hz, 1H), 8.58 (s, 1H).

Les composés décrits ci-après sont également préparés selon des modes opératoires analogues à ceux décrits précédemment dans l'ensemble de la partie expérimentale en utilisant les réactifs appropriés commerciaux ou préalablement préparés selon des méthodes classiques en synthèse organique :

### composé 208: 5-(2,3-dichlorophenyl)-3-methyl-1-[2-oxo-2-[4-(2-oxo-5-phenyl-1H-imidazol-3-yl)-1-piperidyl]ethyl]pyrimidine-2,4-dione (example comparatif)

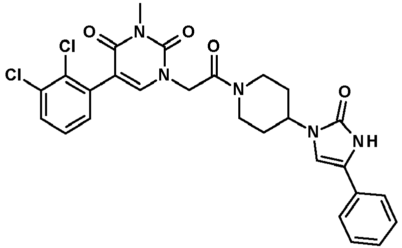

RMN ¹H (δ, DMSO) : 1.54 - 1.94 (m, 4H), 2.69-2.84 (m, 1H), 3.21 (m, 1H), 3.25 (s, 3H), 4.01 (d, J = 13.0 Hz, 1H), 4.09-4.26 (m, 1H), 4.46 (d, J = 12.7 Hz, 1H), 4.66-4.95 (m, 2H), 7.11 - 7.22 (m, 2H), 7.33 (m, 3H), 7.43 (t, J = 7.8 Hz, 1H), 7.47-7.54 (m, 2H), 7.69 (dd, J = 8.0, 1.6 Hz, 1H), 7.84 (s, 1H), 10.74 (m, 1H).

### composé 209: 2-[5-(2,3-dichlorophenyl)-3-methyl-2,4-dioxo-pyrimidin-1-yl]-N-[4-(2-oxo-4,5-dihydro-1H-1,3 benzodiazepin-3-yl)-1-piperidyl]acetamide (example comparatif)

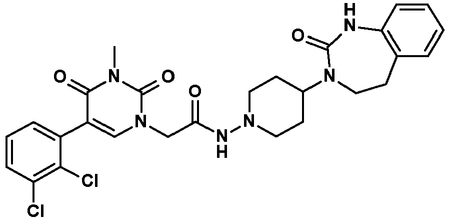

RMN ¹H (δ, DMSO) : 1.48-1.74 (m, 2H), 1.75-1.96 (m, 2H), 2.62 (m, 2H), 2.90 (m, 2H), 2.99 (d, J = 10.6 Hz, 1H), 3.07 (m, 1H), 8.97 (br-s, 1H), 3.24 (m, 3H), 3.40 (m, 2H), 4.03 (m, 1H), 4.76 (s, 2H), 6.68-6.90 (m, 1H), 7.04 (d, J = 4.7 Hz, 3H), 7.32 (m, 1H), 7.43 (t, J = 7.8 Hz, 1H), 7.69 (dd, J = 8.0, 1.6 Hz, 1H), 7.89 (m, 1H), 8.50 (m, 1H).

### composé 210: 5-(2,3-dichlorophenyl)-3-isopropyl-1-[2-oxo-2-[4-(2-oxo-4,5-dihydro-1H-1,3-benzodiazepin-3-yl)-1-piperidyl]ethyl]pyrimidine-2,4-dione (example comparatif)

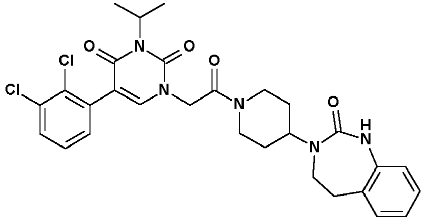

RMN ¹H (δ, DMSO) : 1.40 (d, J = 6.9 Hz, 6H), 1.48-1.60 (m, 1H), 1.64-1.80 (m, 3H), 2.68 (m, 1H), 2.89 (m, 2H), 3.14 (m, 1H), 3.37 (m, 2H), 3.91 (d, J = 12.8 Hz, 1H), 4.25-4.38 (m, 1H), 4.43 (d, J = 12.8 Hz, 1H), 4.73 (m, 2H), 5.10 (m, 1H), 6.78-6.82 (m, 1H), 7.01-7.04 (m, 3H), 7.32 (dd, J = 1.6-7.7 Hz, 1H), 7.41 (t, J = 7.8 Hz, 1H), 7.68 (dd, J = 1.6-8.0 Hz, 1H), 7.76 (s, 1H), 8.54 (s, 1H).

### composé 211: 5-(2,3-dichlorophenyl)-3-methyl-1-[2-oxo-2-(2-oxospiro[1H-pyrido[2,3-d][1,3]oxazine-4,4'-piperidine]-1'-yl)ethyl]pyrimidine-2,4-dione (example comparatif)

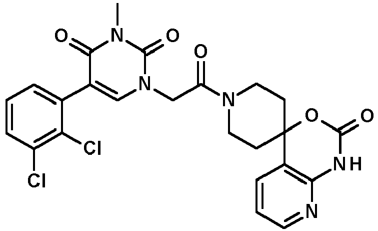

RMN ¹H (δ, DMSO) : 1.90-2.15 (m, 4H), 2.99 (m, 1H), 3.25 (s, 3H), 3.43 (m, 1H), 3.90 (d, J = 12 Hz, 1H), 4.35 (d, J = 12 Hz, 1H), 4.82 (m, 2H), 7.08-7.11 (m, 1H), 7.32 (dd, J = 1.6-7.6 Hz, 1H), 7.43 (t, J = 7.8 Hz, 1H), 7.68-7.71 (m, 2H), 7.81 (s, 1H), 8.21 (dd, J = 1.6-4.9 Hz, 1H), 10.88 (br-s, 1H).

### composé 213: 5-(2,3-dichlorophenyl)-3-methyl-1-[2-oxo-2-[4-(2-oxo-4,5-dihydro-1H-pyrido[2,3-d][1,3]diazepin-3-yl)-1-piperidyl]ethyl]pyrimidine-2,4-dione (example comparatif)

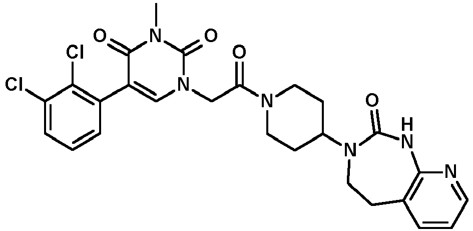

RMN ¹H (δ, DMSO) : 1.56 (m, 1H), 1.71 (m, 3H), 2.69 (m, 1H), 2.84-2.93 (m, 2H), 3.16 (m, 1H), 3.25 (s, 3H), 3.39-3.46 (m, 2H), 3.95 (d, J = 14.2 Hz, 1H), 4.33 (m, 1H), 4.43 (d, J = 12.4 Hz, 1H), 4.78 (s, 2H), 7.06 (m, 1H), 7.32 (dd, J = 7.6, 1.6 Hz, 1H), 7.43 (t, J = 7.9 Hz, 1H), 7.50 (dd, J = 7.6, 1.7 Hz, 1H), 7.68 (m, 1H), 7.82 (s, 1H), 8.18 (dd, J = 5.2, 1.7 Hz, 1H), 8.91 (s, 1H).

### composé 214: 5-(2,3-dimethylphenyl)-3-methyl-1-[2-oxo-2-[4-(2-oxo-5-phenyl-1H-imidazol-3-yl)-1-piperidy]ethyl]pyrimidine-2,4-dione (example comparatif)

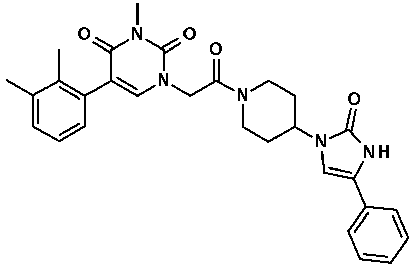

RMN ¹H (δ, DMSO) : 1.54-1.95 (m, 4H), 2.05 (s, 3H), 2.28 (s, 3H), 2.77 (m, 1H), 3.25 (m, 4H), 3.86-4.06 (d, J = 13.1 Hz, 1H), 4.15 (m, 1H), 4.47 (d, J = 13.1 Hz, 1H), 4.79 (m, 2H), 6.96 (d, J = 7.3 Hz, 1H), 7.14 (m, 4H), 7.33 (t, J = 7.6 Hz, 2H), 7.50 (d, J = 7.8 Hz, 2H), 7.59 (s, 1H), 10.72 (s, 1H).

### composé 215: 5-(2,3-dichlorophenyl)-3-methyl-1-[2-oxo-2-[4-(2-oxo-1H-quinolin-3-yl)-1-piperidyl]ethyl]pyrimidine-2,4-dione (example comparatif)

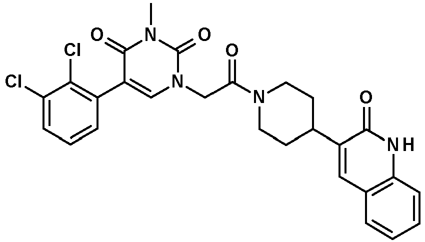

RMN ¹H (δ, DMSO) : 1.36 - 1.66 (m, 2H), 1.91 (m, 2H), 2.66-2.82 (m, 1H), 3.06 (m, 1H), 3.15-3.22 (m, 1H), 3.24 (s, 3H), 4.00 (d, J = 13.1 Hz, 1H), 4.49 (d, J = 13.0 Hz, 1H), 4.79 (s, 2H), 7.10 - 7.19 (m, 1H), 7.28 (d, J = 8.1 Hz, 1H), 7.32 (dd, J = 7.7, 1.6 Hz, 1H), 7.41 (d, J = 7.9 Hz, 1H), 7.43 - 7.48 (m, 1H), 7.63 (dd, J = 8.0, 1.4 Hz, 1H), 7.66 - 7.73 (m, 2H), 7.86 (s, 1H), 11.80 (s, 1H).

### composé 216: 5-(2,3-dimethylphenyl)-3-isopropyl-1-[2-oxo-2-[4-(2-oxo-4,5-dihydro-1H-pyrido[4,3-d] [1,3]diazepin-3-yl)-1-piperidyl]ethyl]pyrimidine-2,4-dione (example comparatif)

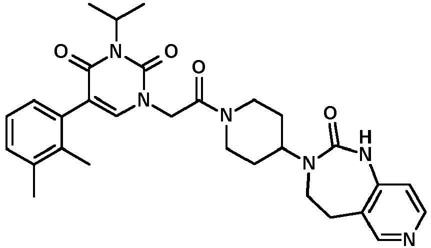

RMN ¹H (δ, DMSO) : 1.40 (d, J = 6.8 Hz, 6H), 1.50-1.62 (m, 1H), 1.63-1.80 (m, 3H), 2.04 (s, 3H), 2.27 (s, 3H), 2.68 (m, 1H), 2.90 (m, 2H), 3.15 (m, 1H), 3.40 (m, 2H), 3.95 (m, 1H), 4.30-4.40 (m, 1H), 4.45 (d, J = 13 Hz, 1H), 4.75 (m, 2H), 5.15 (m, 1H), 6.93-6.98 (m, 2H), 7.10 (t, 1H), 7.18 (d, 1H), 7.54 (s, 1H), 8.09 (d, J = 5.6 Hz, 1H), 8.14 (s, 1H), 9.11 (s, 1H).

### composé 217: 5-(2,3-dimethylphenyl)-3-isopropyl-1-[2-oxo-2-[4-(2-oxo-4,5-dihydro-1H-pyrido[3,2-d] [1,3]diazepin-3-yl)-1-piperidyl]ethyl]pyrimidine-2,4-dione (example comparatif)

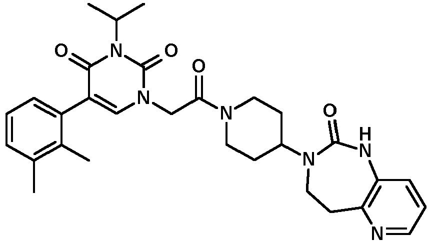

RMN ¹H (δ, DMSO) : 1.40 (d, J = 6.8 Hz, 6H), 1.5-1.62 (m, 1H), 1.65-1.85 (m, 3H), 2.04 (s, 3H), 2.27 (s, 3H), 2.68 (m, 1H), 3.03 (m, 2H), 3.14 (m, 1H), 3.45 (m, 2H), 3.93 (d, J = 12.6 Hz, 1H), 4.25-4.48 (m, 1H), 4.43 (d, J = 13.2 Hz, 1H), 4.72 (m, 2H), 5.11 (m, 1H), 6.96 (d, J = 7 Hz, 1H), 7.08-7.13 (m, 2H), 7.16 (d, J = 7.2 Hz, 1H), 7.42 (dd, J = 1.3-8.2 Hz, 1H), 7.55 (s, 1H), 8.03 (m, 1H), 8.67 (s, 1H).

### composé 218: 5-(2,3-dimethylphenyl)-3-isopropyl-1-[2-oxo-2-[4-(2-oxo-4,5-dihydro-1H-pyrido[2,3-d] [1,3]diazepin-3-yl)-1-piperidyl]ethyl]pyrimidine-2,4-dione (example comparatif)

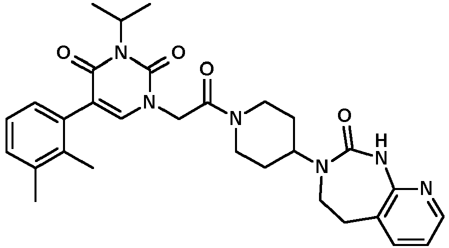

RMN ¹H (δ, DMSO) : 1.40 (d, 6H), 1.5-1.62 (m, 1H), 1.63-1.80 (m, 3H), 2.04 (s, 3H), 2.27 (s, 3H), 2.68 (m, 1H), 2.89 (m, 2H), 3.15 (m, 1H), 3.42 (m, 2H), 3.95 (d, J = 13 Hz, 1H), 4.28-4.38 (m, 1H, 4.45 (d, J = 13 Hz, 1H), 4.72 (m, 2H), 5.11 (m, 1H), 6.89-6.92 (m, 1H), 6.95 (d, J = 7.2 Hz, 1H), 7.09 (t, J = 7.5 Hz, 1H), 7.16 (d, J = 7.2 Hz, 1H), 7.49-7.51 (m, 1H), 7.54 (s, 1H), 8.08 (m, 1H), 8.31 (s, 1H).

### composé 219: 5-(2,3-dichloro-phenyl)-3-methyl-1-{2-oxo-2-[4-(2-oxo-[1,3]diazepan-1-yl)-piperidin-1-yl]-ethyl}-1H-pyrimidine-2,4-dione (example comparatif)

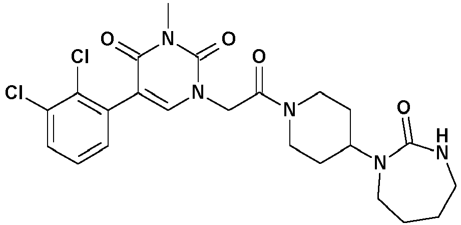

RMN ¹H (δ, DMSO) : 1.33-1.57 (m, 5H), 1.57-1.75 (m, 3H), 2.63 (m, 1H), 2.91 (m, 2H), 3.02 (m, 2H), 3.10 (m, 1H), 3.24 (s, 3H), 3.82-4.06 (m, 2H), 4.39 (d, J = 13.0 Hz, 1H), 4.65-4.87 (m, 2H), 5.93 (t, J = 4.2 Hz, 1H), 7.32 (dd, J = 7.5, 1.5 Hz, 1H), 7.42 (t, J = 7.9 Hz, 1H), 7.69 (dd, J = 8.0, 1.5 Hz, 1H), 7.82 (s, 1H).

### composé 220: 5-(3,4-dichlorophenyl)-3-methyl-1-[2-oxo-2-[4-(2-oxo-4,5-dihydro-1H-1,3-benzodiazepin-3-yl)-1-piperidyl]ethyl]pyrimidine-2,4-dione (example comparatif)

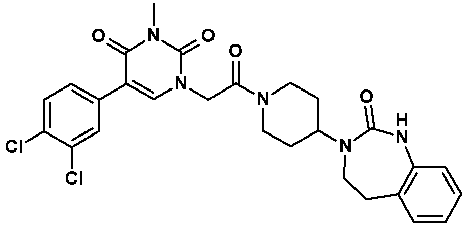

RMN ¹H (δ, DMSO) : 1.39-1.56 (m, 1H), 1.56-1.75 (m, 3H), 2.62 (m, 1H), 2.79-2.88 (m, 2H), 3.03-3.16 (m, 1H), 3.19 (s, 3H), 3.31 (m, 2H), 3.90 (m, 1H), 4.17-4.32 (m, 1H), 4.35 (d, J = 13.6 Hz, 1H), 4.74 (m, 2H), 6.67-6.83 (m, 1H), 6.90-7.06 (m, 3 H), 7.54 (dd, J = 8.5, 2.0 Hz, 1H), 7.62 (d, J = 8.8 Hz, 1H), 7.81 (d, J = 2.0 Hz, 1H), 8.05 (s, 1H), 8.46 (s, 1H).

### composé 221: 1-[2-[4-(7-bromo-2-oxo-4,5-dihydro-1H-1,3-benzodiazepin-3-yl)-1-piperidyl]-2-oxo-ethyl]-5-(2-chloro-3-fluoro-phenyl)-3-isopropyl-pyrimidine-2,4-dione (example comparatif)

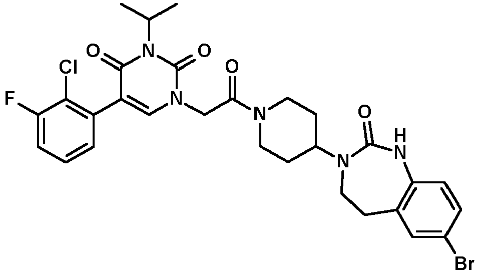

RMN ¹H (δ, DMSO) : 1.41 (d, J = 6.8 Hz, 6H), 1.55 (m, 1H), 1.61-1.82 (m, 3H), 2.65 (m, 1H), 2.84 - 2.94 (m, 2H), 3.15 (m, 1H), 3.34-3.42 (m, 2H), 3.84-4.03 (m, 1H), 4.32 (m, 1H), 4.38-4.51 (m, 1H), 4.64 - 4.84 (m, 2H), 5.11 (hept, J = 7.0 Hz, 1H), 7.01 (d, J = 8.7 Hz, 1H), 7.16-7.30 (m, 3H), 7.35-7.52 (m, 2H), 7.77 (s, 1H), 8.71 (s, 1H).

### composé 222: 5-(2-chloro-3-fluoro-phenyl)-3-isopropyl-1-[2-[4-methyl-4-(2-oxo-4,5-dihydro-1H-1,3-benzodiazepin-3-yl)-1-piperidyl]-2-oxo-ethyl]pyrimidine-2,4-dione (example comparatif)

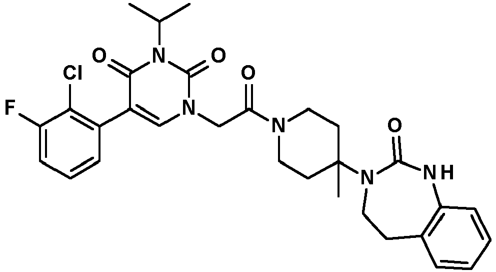

RMN ¹H (δ, DMSO) : 1.47-1.50 (m, 9 H); 1.91-1.93 (m, 1 H); 2.05-2.09 (m, 1 H); 2.22-2.29 (m, 1 H); 2.41-2.46 (m, 1 H); 3.09 (m, 2H); 3.34 (m, 1H), 3.45-3.52 (m, 2H); 3.53-3.60 (m, 2 H); 3.63-3.69 (m, 2 H); 4.68 (d, J = 16.5 Hz, 1H); 4.79 (d, J = 16.4 Hz, 1H); 5.16-5.23 (m, 1 H); 6.99 - 6.88 (m, 2H), 7.12 - 7.06 (m, 1H), 7.14 (dd, J = 7.7, 1.4 Hz, 1H), 7.18 (dt, J = 7.7, 1.3 Hz, 1H), 7.29 - 7.23 (m, 1H), 7.35 (m, 1H); 7.54 (s, 1H).

### composé 223: 5-(2-chloro-3-fluoro-phenyl)-3-isopropyl-1-[2-[(2S)-2-methyl-4-(2-oxo-4,5-dihydro-1H-1,3-benzodiazepin-3-yl)-1-piperidyl]-2-oxo-ethyl]pyrimidine-2,4-dione (example comparatif)

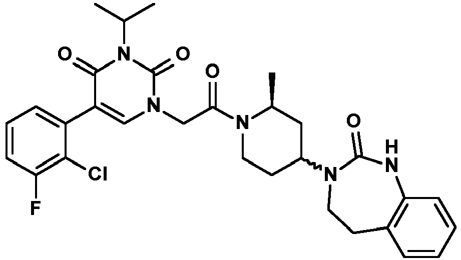

RMN ¹H (δ, DMSO) : 1.19 (d, J = 6.0 Hz, 3H), 1.41 (d, J = 6.7 Hz, 6H), 1.61 (m, 1H), 1.72 - 1.92 (m, 2H), 2.01 (m, 1H), 2.53 (m, 1H), 2.88-2.97 (m, 2H), 3.41-3.50 (m, 2H), 3.77 (br-s, 1H), 4.06 (m, 1H), 4.54-4.86 (m, 2H), 5.10 (hept, J = 6.9 Hz, 1H), 6.82 (m, 1H), 6.95-7.10 (m, 3H), 7.16-7.29 (m, 1H), 7.35-7.52 (m, 2H), 7.84 (s, 1H), 8.52 (s, 1H).

### composé 224: acide 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propanoique (example comparatif)

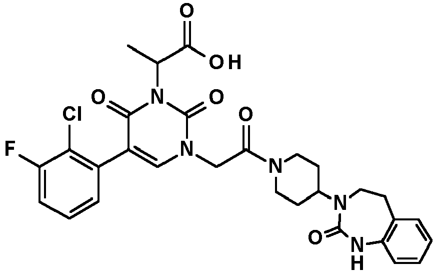

Masse = 598.

### composé 225: 5-(2-chloro-3-fluoro-phenyl)-3-isopropyl-1-[2-[4-(7-methylsulfinyl-2-oxo-4,5-dihydro-1H-1,3-benzodiazepin-3-yl)-1-piperidyl]-2-oxo-ethyl]pyrimidine-2,4-dione (example comparatif)

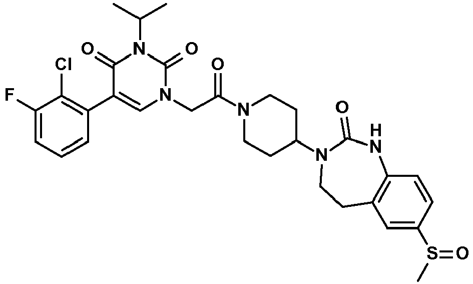

RMN ¹H (δ, DMSO) : 1.41 (d, J = 6.9 Hz, 6H), 1.48-1.63 (m, 1H), 1.63-1.86 (m, 3H), 2.67 (m, 4H), 2.97 (m, 2H), 3.15 (m, 1H), 3.36-3.49 (m, 2H), 3.94 (d, J = 13.5 Hz, 1H), 4.35 (m, 1H), 4.44 (d, J = 13.5 Hz, 1H), 4.75 (m, 2H), 5.11 (hept, J = 6.8 Hz, 1H), 7.18-7.26 (m, 2H), 7.36 (m, 2H), 7.40 - 7.49 (m, 2H), 7.77 (s, 1H), 8.90 (s, 1H).

### composé 226: 2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]acetamide (example comparatif)

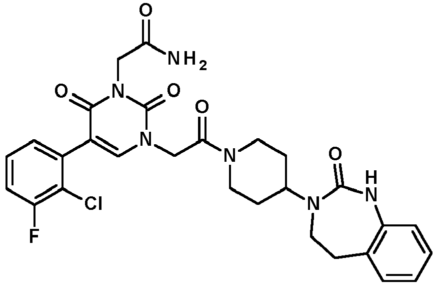

RMN ¹H (δ, DMSO) : 1.55 (m, 1H), 1.71 (m, 3H), 2.69 (m, 1H), 2.83-2.95 (m, 2H), 3.08-3.23 (m, 1H), 3.34 - 3.44 (m, 2H), 3.96 (d, J = 13.7 Hz, 1H), 4.33 (m, 1H), 4.44 (m, 3H), 4.79 (s, 2H), 6.72-6.91 (m, 1H), 6.99-7.07 (m, 3H), 7.11 (br-s, 1H), 7.19 - 7.29 (m, 1H), 7.40-7.52 (m, 2H), 7.56 (br-s, 1H), 7.87 (s, 1H), 8.52 (s, 1H).

### composé 227: 5-(2-chloro-3-fluoro-phenyl)-3-(2-methylsulfinylethyl)-1-[2-oxo-2-[4-(2-oxo-4,5-dihydro-1H-1,3-benzodiazepin-3-yl)-1-piperidyl]ethyl]pyrimidine-2,4-dione (example comparatif)

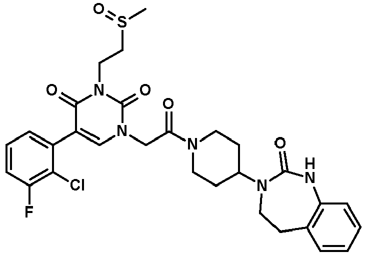

RMN ¹H (δ, DMSO) : 1.47-1.62 (m, 1H), 1.69 (m, 3H), 2.61 (s, 3H), 2.69 (m, 1H), 2.86-2.93 (m, 2H), 2.95 (m, 1H), 3.06 (m, 1H), 3.16 (m, 1H), 3.34-3.47 (m, 2H), 3.95 (d, J = 13.6 Hz, 1H), 4.23 (m, 3H), 4.43 (d, J = 12.7 Hz, 1H), 4.80 (s, 2H), 6.73-6.89 (m, 1H), 6.96-7.10 (m, 3H), 7.16-7.29 (m, 1H), 7.38-7.54 (m, 2H), 7.87 (s, 1H), 8.53 (s, 1H).

### composé 228: 5-(2-chloro-3-fluoro-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione (example comparatif)

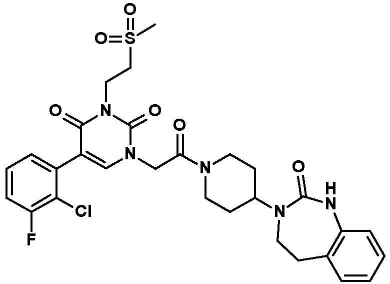

RMN ¹H (δ, DMSO) : 1.55 (m, 1H), 1.71 (m, 3H); 2.69 (m, 1H); 2.90 (m, 2H); 3.09 (s, 3H); 3.16 (m, 1H); 3.36-3.43 (m, 4H); 3.96 (d, J = 13.6 Hz, 1H); 4.29-4.33 (m, 3H); 4.43 (d, J = 12.9 Hz, 1H); 4.80 (s, 2H); 6.79-6.83 (m, 1H); 7.02-7.05 (m, 3H); 7.21-7.23 (m, 1H); 7.45-7.49 (m, 2H); 7.88 (s, 1H); 8.55 (s, 1H).

### composé 229: 5-(2-chloro-3-fluoro-phényl)-3-((S)-2-méthoxy-1-méthyl-éthyl)-1-{2-[4-(7-méthoxy-2-oxo-1,2,4,5-tétrahydro-benzo[d][1,3]diazépin-3-yl)-pipéridin-1-yl]-2-oxo-éthyl}-1H-pyrimidine-2,4-dione (example comparatif)

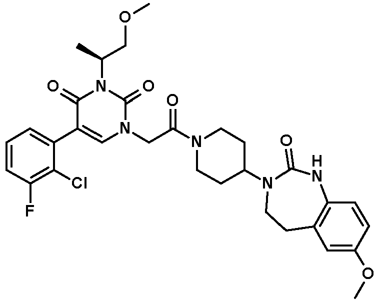

RMN ¹H (δ, DMSO) : 1.33 (dd, J = 7.0, 1.8 Hz, 3H); 1.55 (m, 1H), 1.70 (m, 3H), 2.68 (m, 1H); 2.88 (m, 2H); 3.14 (m, 1H); 3.24 (s, 3H); 3.35-3.37 (m, 2H); 3.52-3.56 (m, 1H); 3.67 (s, 3H); 3.92 (m, 2H); 4.28 (m, 1H); 4.43 (d, J = 12.8 Hz, 1H); 4.75 (m, 2H); 5.15 (m, 1H); 6.62 (d, J = 2.9 Hz, 1H), 6.67 (dd, J = 8.8, 3.0 Hz, 1H), 6.98 (d, J = 8.8 Hz, 1H); 7.20-7.22 (m, 1H); 7.43-7.47 (m, 2H); 7.80 (s, 1H); 8.33 (s, 1H).

### composé 230: 5-(2-chloro-3-fluoro-phenyl)-3-isopropyl-1-[2-[4-(7-methoxy-5,5-dimethyl-2-oxo-1,4-dihydro-1,3-benzodiazepin-3-yl)-1-piperidyl]-2-oxo-ethyl]pyrimidine-2,4-dione (example comparatif)

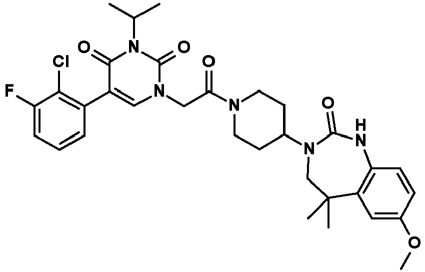

RMN ¹H (δ, DMSO) : 1.27 (d, 6H), 1.41 (d, J = 6.8 Hz, 6H), 1.72 (m, 3H), 1.87 (m, 1H), 2.64 (m, 1H), 3.11 (m, 1H), 3.23 (s, 2H), 3.69 (s, 3H), 3.94 (d, J = 13.4 Hz, 1H), 4.01 (m, 1H), 4.44 (d, J = 12.7 Hz, 1H), 4.74 (m, 2H), 5.11 (p, J = 6.8 Hz, 1H), 6.67 (dd, J = 9.0, 3.0 Hz, 1H), 6.84 (d, J = 3.0 Hz, 1H), 6.96 (d, J = 8.9 Hz, 1H), 7.15-7.28 (m, 1H), 7.37-7.52 (m, 2H), 7.78 (s, 1H), 8.23 (s, 1H).

### composé 231: 5-(2,3-difluorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione (example comparatif)

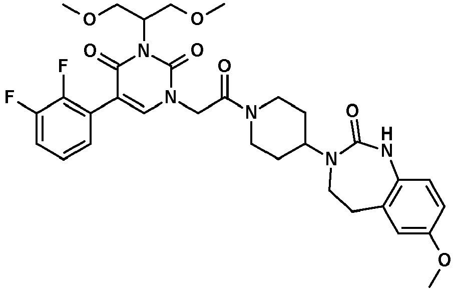

RMN ¹H (δ, DMSO) : 1.44-1.61 (m, 1H), 1.70 (m, 3H), 2.68 (m, 1H), 2.87 (m, 2H), 3.14 (m, 1H), 3.24 (s, 6H), 3.35-3.44 (m, 2H), 3.60 (m, 2H), 3.67 (s, 3H), 3.77-3.90 (m, 2H), 3.94 (d, J = 13.8 Hz, 1H), 4.28 (m, 1H), 4.43 (d, J = 13.2 Hz, 1H), 4.78 (m, 2H), 5.29 (br-s, 1H), 6.63 (d, J = 3.0 Hz, 1H), 6.67 (dd, J = 8.8, 3.0 Hz, 1H), 6.98 (d, J = 8.8 Hz, 1H), 7.12-7.34 (m, 2H), 7.39-7.54 (m, 1H), 7.90 (s, 1H), 8.33 (s, 1H).

### composé 232: 5-(2-chloro-3-fluoro-phenyl)-3-[2-méthoxy-1-(méthoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione (example comparatif)

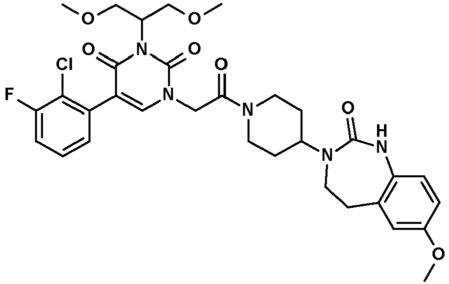

RMN ¹H (δ, DMSO) : 1.55 (m, 1 H); 1.68 (m, 3 H); 2.68 (m, 1 H); 2.88 (m, 2 H); 3.14 (m, 1 H); 3.24 (s, 6H), 3.37 (m, 2 H); 3.60 (m, 2 H); 3.67 (s, 3 H); 3.86 (m, 2 H); 3.94 (d, J = 13.5 Hz, 1 H); 4.28 (m, 1 H); 4.43 (d, J = 12.9 Hz, 1 H); 4.78 (m, 2 H); 5.30 (br-s, 1 H); 6.62 (d, J = 3.0 Hz, 1H); 6.67 (dd, J = 8.8, 3.0 Hz, 1H), 6.98 (d, J = 8.8 Hz, 1 H); 7.21-7.23 (m, 1 H); 7.43-7.46 (m, 2 H); 7.82 (s, 1 H); 8.33 (s, 1 H).

### composé 233: 5-(2-chloro-3-fluoro-phenyl)-3-[(1S)-2-methoxy-1-methyl-ethyl]-1-[2-[4-(7-methylsulfanyl-2-oxo-4,5-dihydro-1H-1,3-benzodiazepin-3-yl)-1-piperidyl]-2-oxo-ethyl]pyrimidine-2,4-dione (example comparatif)

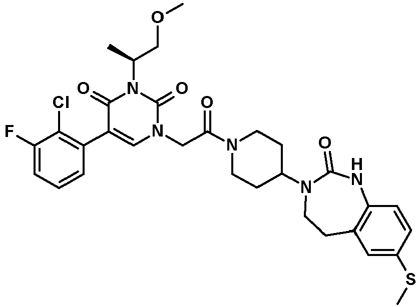

(δ, DMSO) : 1.33 (m, 3H), 1.55 (m, 1H), 1.70 (m, 3H), 2.40 (s, 3H), 2.68 (m, 1H), 2.82-2.93 (m, 2H), 3.14 (m, 1H), 3.23 (s, 3H), 3.34-3.42 (m, 2H), 3.54 (m, 1H), 3.82-3.99 (m, 2H), 4.31 (m, 1H), 4.43 (d, J = 12.7 Hz, 1H), 4.75 (m, 2H), 5.14 (m, 1H), 7.01 (m, 3H), 7.21 (m, 1H), 7.36-7.55 (m, 2H), 7.79 (s, 1H), 8.57 (s, 1H).

### composé 234: 5-(2-chloro-3-fluoro-phenyl)-3-(2-hydroxy-2-methyl-propyl)-1-[2-oxo-2-[4-(2-oxo-4,5-dihydro-1H-1,3-benzodiazepin-3-yl)-1-piperidyl]ethyl]pyrimidine-2,4-dione (example comparatif)

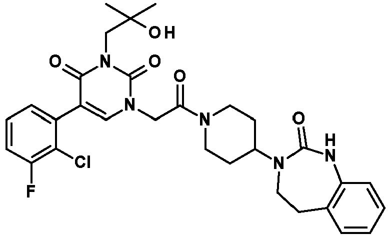

RMN ¹H (δ, DMSO) : 1.10 (s, 6H), 1.55 (m, 1H), 1.68 (m, 3H), 2.69 (m, 1H), 2.83-2.96 (m, 2H), 3.15 (m, 1H), 3.35-3.42 (m, 2H), 3.96 (m, 3H), 4.33 (m, 1H), 4.44 (m, 2H), 4.78 (m, 2H), 6.69 - 6.89 (m, 1H), 6.96-7.10 (m, 3H), 7.15-7.26 (m, 1H), 7.38-7.54 (m, 2H), 7.83 (s, 1H), 8.53 (s, 1H).

### composé 235: 5-(2-chloro-3-fluoro-phenyl)-1-[2-oxo-2-[4-(2-oxo-4,5-dihydro-1H-1,3-benzodiazepin-3-yl)-1-piperidyl]ethyl]-3-(2H-tetrazol-5-ylmethyl)pyrimidine-2,4-dione (example comparatif)

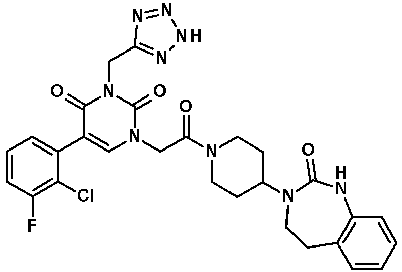

RMN ¹H (δ, DMSO) : 1.55 (m, 1H), 1.68 (m, 3H), 2.70 (m, 1H), 2.89 (m, 2H), 3.18 (m, 2H), 3.37 (m, 2H), 3.95 (d, J = 13.9 Hz, 1H), 4.33 (m, 1H), 4.43 (d, J = 13.1 Hz, 1H), 4.81 (s, 2H), 5.36 (m, 2H), 6.81 (m, 1H), 7.04 (m, 3H), 7.17-7.31 (m, 1H), 7.37-7.56 (m, 2H), 7.94 (s, 1H), 8.53 (s, 1H).

### composé 236: 5-(2,3-difluorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione

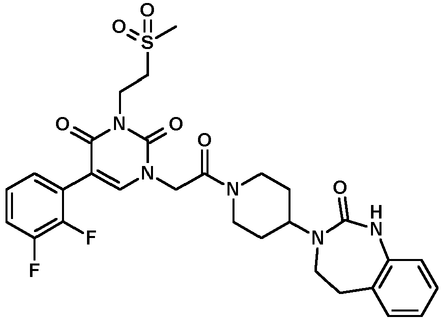

RMN ¹H (δ, DMSO) : 1.55 (m, 1H), 1.71 (m, 3H); 2.70 (m, 1H); 2.90 (m, 2H); 3.08 (s, 3H); 3.16 (m, 1H); 3.36-3.43 (m, 4H); 3.97 (d, J = 13.5 Hz, 1H); 4.32 (m, 3H); 4.43 (d, J = 12.9 Hz, 1H); 4.82 (s, 2H); 6.79-6.83 (m, 1H); 7.02-7.05 (m, 3H); 7.21-7.30 (m, 2H); 7.44-7.51 (m, 1H); 7.97 (s, 1H); 8.54 (s, 1H).

### composé 237: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1S)-1-methylpropyl]pyrimidine-2,4-dione (example comparatif)

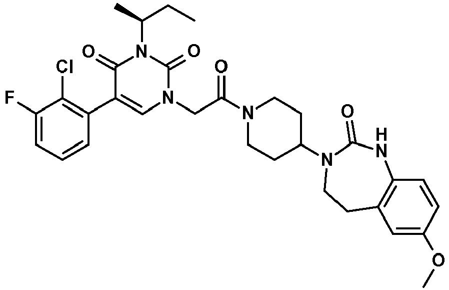

RMN ¹H (δ, DMSO) : 0.80 (t, J = 7.4 Hz, 3H), 1.38 (d, J = 6.8 Hz, 3H), 1.54 (m, 1H), 1.70 (m, 3H), 2.00 - 2.14 (m, 1H), 2.53 (m, 1H), 2.67 (m, 1H), 2.88 (m, 2H), 3.14 (m, 1H), 3.36 (m, 2H), 3.67 (s, 3H), 3.93 (d, J = 13.4 Hz, 1H), 4.28 (m, 1H), 4.43 (d, J = 13.0 Hz, 1H), 4.65-4.84 (m, 2H), 4.89 (m, 1H), 6.62 (d, J = 3.0 Hz, 1H), 6.67 (dd, J = 8.8, 3.0 Hz, 1H), 6.98 (d, J = 8.8 Hz, 1H), 7.14-7.28 (m, 1H), 7.36-7.52 (m, 2H), 7.79 (s, 1H), 8.34 (s, 1H).

### composé 238: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[(1R)-1-methylpropyl]pyrimidine-2,4-dione (example comparatif)

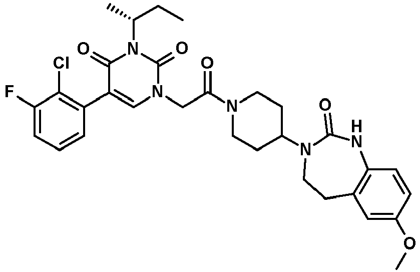

RMN ¹H (δ, DMSO) : 0.80 (t, J = 7.4 Hz, 3H), 1.39 (d, J = 6.8 Hz, 3H), 1.54 (m, 1H), 1.67 (m, 3H), 2.05 (m, 1H), 2.67 (m, 1H), 2.88 (m, 2H), 3.14 (m, 1H), 3.36 (m, 3H), 3.67 (s, 3H), 3.93 (d, J = 13.6 Hz, 1H), 4.28 (m, 1H), 4.43 (d, J = 12.9 Hz, 1H), 4.65-4.83 (m, 2H), 4.88 (m, 1H), 6.62 (d, J = 3.0 Hz, 1H), 6.67 (dd, J = 8.7, 3.0 Hz, 1H), 6.98 (d, J = 8.8 Hz, 1H), 7.21 (m, 1H), 7.37-7.55 (m, 2H), 7.79 (s, 1H), 8.33 (s, 1H).

### composé 239: 5-(2-chloro-3-fluoro-phenyl)-3-[1-(methoxymethyl)propyl]-1-[2-[4-(7-methoxy-2-oxo-4,5-dihydro-1H-1,3-benzodiazepin-3-yl)-1-piperidyl]-2-oxo-ethyl]pyrimidine-2,4-dione (example comparatif)

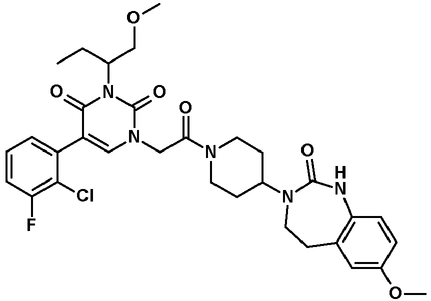

RMN ¹H (δ, DMSO) : 0.82 (t, J = 7.4 Hz, 3H); 1.55 (m, 1H); 1.65-1.75 (m, 3H); 1.97 (m, 1H); 2.53 (m, 2H), 2.67 (m, 1H); 2.88 (m, 2H); 3.14 (m, 1H); 3.23 (s, 3H); 3.36-3.36 (m, 2H); 3.56 (m, 1H); 3.67 (s, 3H); 3.92 (m, 2H); 4.28 (m, 1H); 4.43 (d, J = 12.8 Hz, 1H); 4.76 (br s, 1H); 5.02 (br s, 1H); 6.62 (d, J = 2.9 Hz, 1H); 6.67 (dd, J = 8.8, 3.0 Hz, 1H), 6.98 (d, J = 8.8 Hz, 1H); 7.21-7.23 (m, 1H); 7.43-7.46 (m, 2H); 7.81 (s, 1H); 8.33 (s, 1H).

### composé 240: 5-(2-chloro-3-fluoro-phenyl)-3-(2-methoxy-2-methyl-propyl)-1-[2-[4-(7-methoxy-2-oxo-4,5-dihydro-1H-1,3-benzodiazepin-3-yl)-1-piperidyl]-2-oxo-ethyl]pyrimidine-2,4-dione (example comparatif)

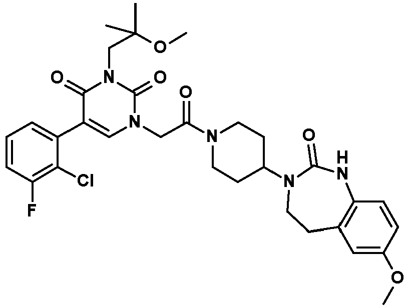

RMN ¹H (δ, DMSO) : 1.12 (s, 6H); 1.55 (m, 1H); 1.68 (m, 3H); 2.68 (m, 1H); 2.88 (m, 2H); 3.16 (m, 4H); 3.36 (m, 2H); 3.67 (s, 3H); 3.94 (d, J = 13.9 Hz, 1H); 4.01 (m, 2H); 4.28 (m, 1H); 4.43 (d, J = 12.8 Hz, 1H); 4.77 (m, 2H); 6.62 (d, J = 2.9 Hz, 1H); 6.67 (dd, J = 8.8, 2.9 Hz, 1H); 6.98 (d, J = 8.8 Hz, 1H); 7.19-7.21 (m, 1H); 7.44-7.47 (m, 2H); 7.82 (s, 1H); 8.33 (s, 1H).

### composé 241: 5-(2-chloro-3-methoxy-phenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione(example comparatif)

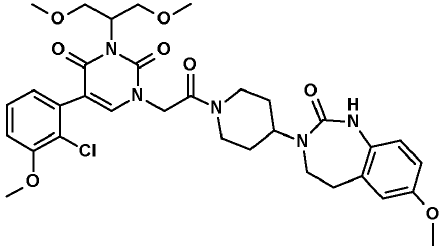

RMN ¹H (δ, DMSO) : 1.44-1.62 (m, 1H), 1.67 (m, 3H), 2.66 (m, 1H), 2.83-2.92 (m, 2H), 3.13 (m, 1H), 3.24 (s, 6H), 3.36 (m, 2H), 3.60 (m, 2H), 3.67 (s, 3H), 3.83 (m, 2H), 3.89 (s, 3H), 3.93 (d, J = 13.4 Hz, 1H), 4.28 (m, 1H), 4.43 (d, J = 12.8 Hz, 1H), 4.74 (m, 2H), 5.26 (br-s, 1H), 6.62 (d, J = 3.0 Hz, 1H), 6.67 (dd, J = 8.9, 3.0 Hz, 1H), 6.91 (dd, J = 7.5, 1.5 Hz, 1H), 6.98 (d, J = 8.7 Hz, 1H), 7.19 (dd, J = 8.5, 1.5 Hz, 1H), 7.31-7.38 (m, 1H), 7.70 (s, 1H), 8.32 (s, 1H).

### composé 242: 5-(2,3-dichlorophenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione(example comparatif)

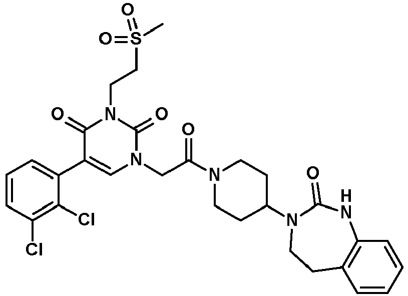

RMN ¹H (δ, DMSO) : 1.55 (m, 1H), 1.72 (m, 3H); 2.69 (m, 1H); 2.90 (m, 2H); 3.08 (s, 3H); 3.20 (m, 1H); 3.43-3.39 (m, 4H); 3.95 (d, J = 11.9 Hz, 1H) 4.31 (m, 3H); 4.44 (d, J = 11.9 Hz, 1H); 4.79 (s, 2H); 6.83-6.79 (m, 1H); 7.04 (m, 3H); 7.33 (dd, J = 7.7, 1.6 Hz, 1H); 7.44 (t, J = 7.9 Hz, 1H); 7.70 (dd, J = 8.1, 1.6 Hz, 1H); 7.87 (s, 1H); 8.53 (s, 1H).

### composé 243: 5-(2-chloro-3-methoxy-phenyl)-3-(2-methylsulfonylethyl)-1-[2-oxo-2-[4-(2-oxo-4,5-dihydro-1H-1,3-benzodiazepin-3-yl)-1-piperidyl]ethyl]pyrimidine-2,4-dione(example comparatif)

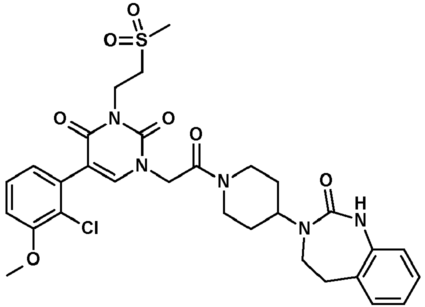

(δ, DMSO) : 1.55 (m, 1H), 1.71 (m, 3H); 2.53 (m, 2H), 2.68 (m, 1H); 2.90 (m, 2H); 3.08 (s, 3H); 3.15 (m, 1H); 3.38 (m, 2H), 3.90 (s, 3H); 3.95 (d, J = 13.6 Hz, 1H); 4.28-4.32 (m, 3H); 4.43 (d, J = 13.0 Hz, 1H); 4.79 (s, 2H); 6.79-6.83 (m, 1H); 6.92 (dd, J = 7.6, 1.4 Hz, 1H); 7.02-7.05 (m, 3H); 7.19-7.21 (m, 1H); 7.36 (t, J = 8.0 Hz, 1H); 7.78 (s, 1H); 8.53 (s, 1H).

### composé 244: 5-(2-chloro-3-fluoro-phenyl)-3-[(1S)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione (example comparatif)

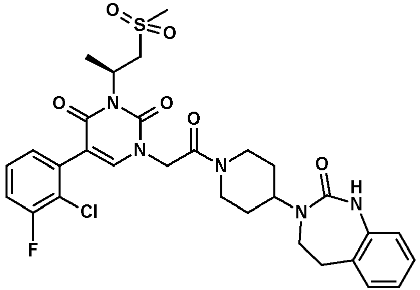

RMN ¹H (δ, DMSO) : 1.24 (d, J = 7.0 Hz, 3H), 1.57 (m, 1H), 1. 69 (m, 3H), 2.70 (s, 1H) 2.84-2.95 (m, 2H), 3.06 (s, 3H), 3.16 (m, 1H), 3.35-3.43 (m, 2H), 3.53 (m, 1H), 3.95 (d, J = 13.6 Hz, 1H), 4.11-4.39 (m, 3H), 4.43 (d, J = 12.4 Hz, 1H), 4.80 (s, 2H), 6.81 (m, 1H), 7.04 (m, 3H), 7.23 (m, 1H), 7.38-7.54 (m, 2H), 7.90 (s, 1H), 8.52 (s, 1H).

### composé 245: 5-(2,3-dichlorophenyl)-3-[2-methoxy-1-(methoxymethyl)ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione (example comparatif)

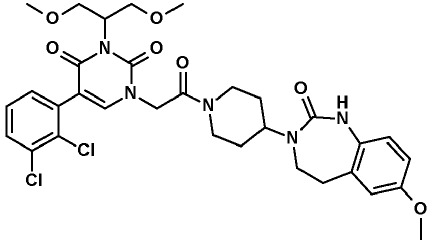

RMN ¹H (δ, DMSO) : 1.50-1.58 (m, 1H); 1.65-1.77 (m, 3H); 2.64-2.70 (m, 1H); 2.85-2.87 (m, 2H); 3.10-3.16 (m, 1H); 3.23 (s, 6H); 3.34-3.36 (m, 2H); 3.59 (m, 2H); 3.66 (s, 3H); 3.80-3.85 (m, 2H); 3.91-3.94 (m, 1H); 4.24-4.30 (m, 1H); 4.40-4.43 (m, 1H); 4.74 (m, 2H); 5.26 (br s, 1H); 6.61 (d, J = 2.9 Hz, 1H); 6.66 (dd, J = 8.8, 2.9 Hz, 1H); 6.97 (d, J = 8.8 Hz, 1H); 7.33 (dd, J = 7.7, 1.6 Hz, 1H); 7.42 (t, J = 7.9 Hz, 1H); 7.68 (dd, J = 8.1, 1.6 Hz, 1H); 7.79 (s, 1H); 8.31 (s, 1H).

### composé 246: acide 3-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]propanoique (example comparatif)

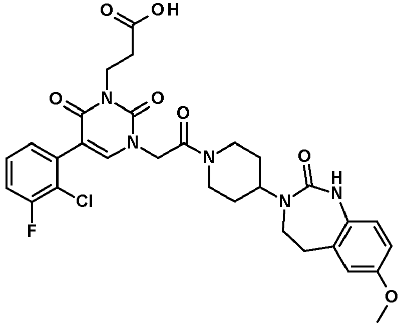

(δ, DMSO) : 1.55 (m, 1H), 1.70 (m, 3H), 2.50 (m, 4H), 2.68 (m, 1H), 2.80-2.95 (m, 2H), 3.15 (m, 1H), 3.67 (s, 3H), 3.94 (d, J = 13.6 Hz, 1H), 4.02-4.17 (m, 2H), 4.28 (m, 1H), 4.43 (d, J = 12.8 Hz, 1H), 4.78 (s, 2H), 6.62 (d, J = 3.0 Hz, 1H), 6.67 (dd, J = 8.8, 3.0 Hz, 1H), 6.98 (d, J = 8.8 Hz, 1H), 7.10 - 7.30 (m, 1H), 7.37-7.54 (m, 2H), 7.84 (s, 1H), 8.32 (s, 1H), 12.45 (br-s, 1H).

### composé 247: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,3,4-oxadiazol-2-yl)ethyl]pyrimidine-2,4-dione(example comparatif)

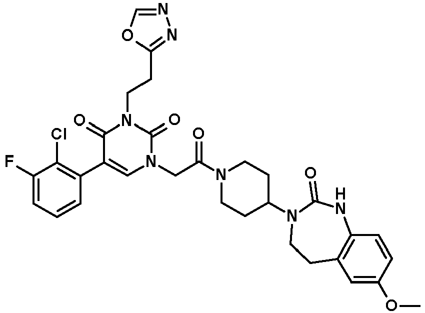

RMN ¹H (δ, DMSO) : 1.54 (m, 1H); 1.68 (m, 3H); 2.68 (m, 1H); 2.88 (m, 2H); 3.19 (m, 3H); 3.36 (m, 2H); 3.67 (s, 3H); 3.94 (d, J = 13.5 Hz, 1H); 4.25 (m, 3H); 4.43 (d, J = 12.8 Hz, 1H); 4.77 (s, 2H); 6.63 (d, J = 2.9 Hz, 1H); 6.67 (dd, J = 8.8, 2.9 Hz, 1H); 6.98 (d, J = 8.8 Hz, 1H); 7.18 (m, 1H); 7.46 (m, 2H); 7.86 (s, 1H); 8.33 (s, 1H); 9.13 (s, 1H).

### composé 248: acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]acetique (example comparatif)

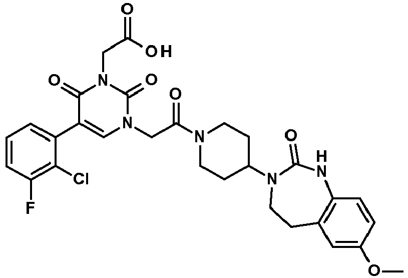

RMN ¹H (δ, DMSO) : 1.49-1.58 (m, 1H); 1.61-1.74 (m, 3H); 2.68 (m, 1H); 2.87-2.89 (m, 2H); 3.12-3.18 (m, 1H); 3.36 (m, 2H), 3.67 (s, 3H); 3.95 (d, J = 13.6 Hz, 1H); 4.25-4.31 (m, 1H); 4.43 (d, J = 12.9 Hz, 1H); 4.52 (m, 2H); 4.81 (s, 2H); 6.62 (d, J = Hz, 1H); 6.67 (dd, J = 8.8, Hz, 1H); 6.98 (d, J = 8.8 Hz, 1H); 7.22-7.24 (m, 1H); 7.44-7.48 (m, 2H); 7.90 (s, 1H); 8.32 (s, 1H); 13.05 (br-s, 1H).

### composé 249: 5-(2-chloro-3-fluoro-phenyl)-3-[(1R)-1-methyl-2-methylsulfonyl-ethyl]-1-[2-oxo-2-[4-(2-oxo-4,5-dihydro-1H-1,3-benzodiazepin-3-yl)-1-piperidyl]ethyl]pyrimidine-2,4-dione (example comparatif)

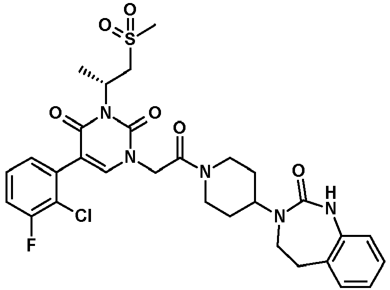

RMN ¹H (δ, DMSO) : 1.24 (d; J = 7.03 Hz; 3 H); 1.53-1.56 (m; 1 H); 1.66-1.72 (m; 3 H); 2.69 (m; 1 H); 2.89-2.91 (m; 2 H); 3.06 (s; 3 H); 3.16 (m; 1 H); 3.38 (m; 2 H); 3.53 (m; 1 H); 3.95 (d; J = 13.62 Hz; 1 H); 4.17-4.24 (m; 1 H); 4.27-4.32 (m; 2 H); 4.43 (d; J = 12.93 Hz; 1 H); 4.81 (m; 2 H); 6.81 (m; 1 H); 7.04 (m; 3 H); 7.22-7.24 (m; 1 H); 7.45-7.49 (m; 2 H); 7.90 (s; 1 H); 8.53 (s; 1 H).

### composé 250: 5-(2-chloro-3-fluoro-phenyl)-3-(2-methylsulfonylethyl)-1-[2-oxo-2-[2-(2-oxo-4,5-dihydro-1H-1,3-benzodiazepin-3-yl)-6-azaspiro[3.3]heptan-6 yl]ethyl]pyrimidine-2,4-dione (example comparatif)

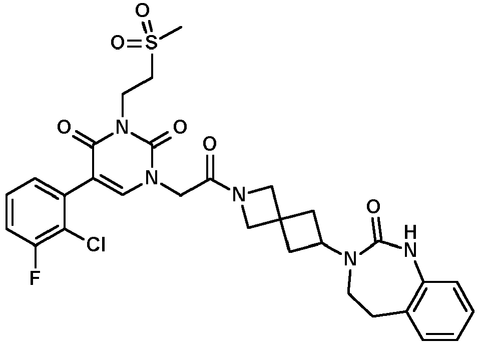

RMN ¹H (δ, DMSO) : 2.32-2.38 (m, 4H); 2.91-2.93 (m, 2H); 3.07 (s, 3H); 3.40-3.43 (m, 4H); 3.88 (s, 1H); 4.01 (s, 1H); 4.18 (s, 1H); 4.29-4.31 (m, 3H); 4.46-4.51 (m, 3H); 6.81-6.85 (m, 1H); 7.04-7.08 (m, 3H); 7.19-7.21 (m, 1H); 7.44-7.47 (m, 2H); 7.88 (m, 1H); 8.53 (m, 1H).

### composé 251: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-[2-(1,2,4-triazol-4-yl)ethyl]pyrimidine-2,4-dione (example comparatif)

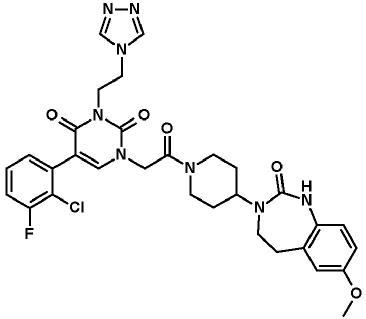

Masse = 651

### composé 252: 5-(2-chloro-3-fluoro-phenyl)-1-[2-[4-(7-fluoro-2-oxo-4,5-dihydro-1H-1,3-benzodiazepin-3-yl)-1-piperidyl]-2-oxo-ethyl]-3-[(1R)-1-methyl-2-methylsulfonyl-ethyl]pyrimidine-2,4-dione (example comparatif)

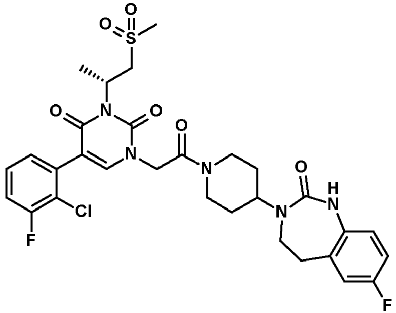

RMN ¹H (δ, DMSO) : 1.14-1.36 (m, 5H), 1.55 (m, 1H), 1.71 (m, 3H), 2.69 (m, 1H), 2.86-2.93 (m, 2H), 3.06 (s, 3H), 3.15 (m, 1H), 3.34-3.41 (m, 2H), 3.53 (m, 1H), 3.94 (d, J = 13.6 Hz, 1H), 4.15-4.33 (m, 3H), 4.43 (d, J = 13.0 Hz, 1H), 4.80 (m, 2H), 6.87-6.96 (m, 2H), 7.02-7.11 (m, 1H), 7.19-7.27 (m, 1H), 7.43-7.51 (m, 2H), 7.89 (s, 1H), 8.55 (s, 1H).

### composé 253: acide 3-[1-[2-[5-(2-chloro-3-fluoro-phenyl)-3-(2-methylsulfonylethyl)-2,4-dioxo-pyrimidin-1-yl]acetyl]-4-piperidyl]-2-oxo-4,5-dihydro-1H-1,3-benzodiazepine-7-carboxylique (example comparatif)

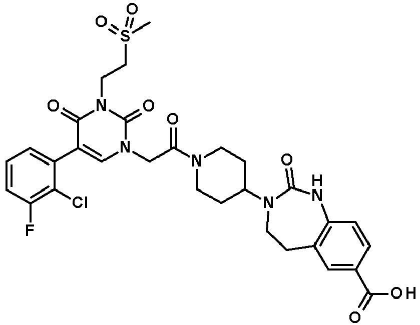

(δ, DMSO) : 1.56 (m, 1H), 1.69 (m, 3H), 2.71 (m, 1H), 2.95 (m, 2H), 3.08 (s, 3H), 3.16 (m, 1H), 3.36-3.48 (m, 4H), 3.96 (d, J = 13.4 Hz, 1H), 4.31 (m, 2H), 4.35-4.51 (m, 2H), 4.80 (m, 2H), 7.11 (d, J = 8.5 Hz, 1H), 7.17 - 7.27 (m, 1H), 7.40-7.54 (m, 2H), 7.61 (dd, J = 8.5, 2.0 Hz, 1H), 7.64 (d, J = 2.0 Hz, 1H), 7.89 (s, 1H), 9.01 (s, 1H), 12.35 (br-s, 1H).

### composé 254: S-[2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethyl] ethanethioate (example comparatif)

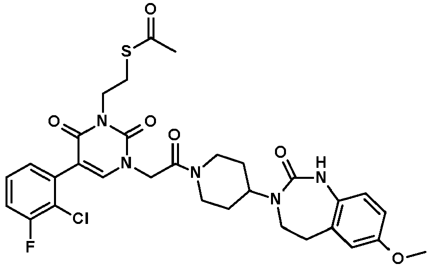

RMN ¹H (δ, DMSO) : 1.56 (m, 1H), 1.68 (m, 3H), 2.33 (m, 4H), 2.81-2.94 (m, 2H), 3.12 (m, 3H), 3.34-3.40 (m, 2H), 3.67 (s, 3H), 3.95 (d, J = 14.0 Hz, 1H), 4.07 (m, 2H), 4.28 (m, 1H), 4.43 (d, J = 12.7 Hz, 1H), 4.78 (s, 2H), 6.63 (d, J = 2.9 Hz, 1H), 6.67 (dd, J = 8.9, 2.9 Hz, 1H), 6.98 (d, J = 8.7 Hz, 1H), 7.17-7.29 (m, 1H), 7.37-7.55 (m, 2H), 7.84 (s, 1H), 8.33 (s, 1H).

### composé 255: N-[(2S)-2-[5-(2,3-dichlorophenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide (example comparatif)

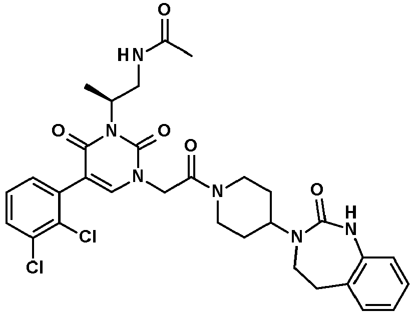

RMN ¹H (δ, DMSO) : 1.34 (3 H, d, J = 6.91 Hz), 1.57 (1 H, m), 1.69 (3 H, m), 1.77 (3 H, s), 2.68-2.72 (1 H, m), 2.90 (2 H, m), 3.15 (1 H, m), 3.38 (2 H, m), 3.44-3.57 (2 H, m), 3.95 (1 H, d, J = 13.54 Hz), 4.33 (1 H, m), 4.44 (1 H, d, J = 12.82 Hz), 4.67-4.81 (2 H, m), 4.99 (1 H, m), 6.81 (1 H, m), 7.04 (3 H, m), 7.33 (1 H, m), 7.43 (1 H, t, J = 7.87 Hz), 7.68 (1 H, dd, J = 8.04, 1.58 Hz), 7.78 (1 H, s), 7.93 (1 H, m), 8.52 (1 H, s).

### composé 256: N-[(2S)-2-[5-(2-chloro-3-fluoro-phenyl)-2,6-dioxo-3-[2-oxo-2-[4-(2-oxo-4,5-dihydro-1H-1,3-benzodiazepin-3-yl)-1-piperidyl]ethyl]pyrimidin-1-yl]propyl]acetamide (example comparatif)

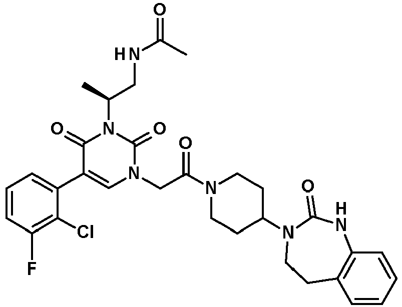

RMN ¹H (δ, DMSO) : 1.35 (d; J = 6.85 Hz; 3 H); 1.57 (m; 1 H); 1.70 (m; 3 H); 1.77 (s; 3 H); 2.69-2.72 (m; 1 H); 2.90 (m; 2 H); 3.16 (m; 1 H); 3.39 (m; 2 H); 3.49-3.57 (m; 2 H); 3.96 (d; J = 13.24 Hz; 1 H); 4.33 (m; 1 H); 4.45 (d; J = 12.80 Hz; 1 H); 4.75 (m; 2 H); 4.99 (m; 1 H); 6.82 (m; 1 H); 7.03-7.05 (m; 3 H); 7.22 (m; 1 H); 7.45 (m; 2 H); 7.79 (s; 1 H); 7.93 (m; 1 H); 8.53 (s; 1 H).

### composé 257: acide 2-[5-(2-chloro-3-fluoro-phenyl)-3-[2-[4-(7-methoxy-2-oxo-4,5-dihydro-1H-1,3-benzodiazepin-3-yl)-1-piperidyl]-2-oxo-ethyl]-2,6-dioxo-pyrimidin-1-yl]ethanesulfonique (example comparatif)

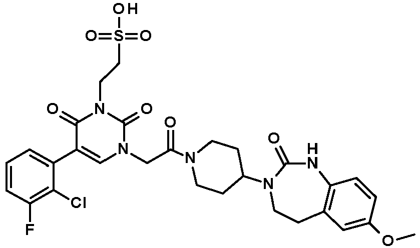

RMN ¹H (δ, DMSO) : 1.56 (m, 1H), 1.62-1.84 (m, 3H), 2.59-2.74 (m, 3H), 2.83-2.95 (m, 2H), 3.06 - 3.16 (m, 1H), 3.18 (s, 2H), 3.28-3.43 (m, 2H), 3.67 (s, 3H), 3.95 (d, J = 13.5 Hz, 1H), 4.06-4.19 (m, 2H), 4.21-4.35 (m, 1H), 4.43 (d, J = 12.8 Hz, 1H), 4.77 (m, 2H), 6.63 (d, J = 3.0 Hz, 1H), 6.66 (dd, J = 8.6, 2.9 Hz, 1H), 6.97 (d, J = 8.8 Hz, 1H), 7.22 (m, 1H), 7.39-7.50 (m, 2H), 7.80 (s, 1H), 8.31 (s, 1H).

### composé 258: 5-(2-chloro-3-fluoro-phenyl)-3-[(1S)-1-(methoxymethyl)-2-methylsulfonyl-ethyl]-1-[2-[4-(7-methoxy-2-oxo-4,5-dihydro-1H-1,3-benzodiazepin-3-yl)-1-piperidyl]-2-oxo-ethyl]pyrimidine-2,4-dione (example comparatif)

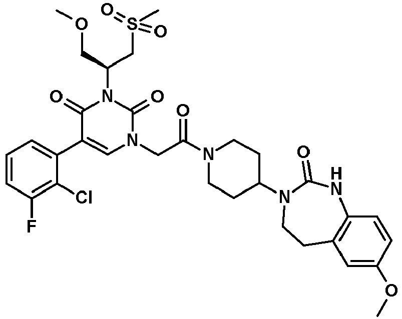

RMN ¹H (δ, DMSO) : 1.54 (m, 1H), 1.71 (m, 3H), 2.65 (m, 2H), 2.87 (m, 2H), 3.07 (s, 3H), 3.15 (m, 1H) , 3.26 (s, 3H), 3.36 (m, 2H), 3.55-3.76 (m, 5H), 3.95 (m, 1H), 4.36 (m, 8.2 Hz, 4H), 4.80 (s, 2H), 6.62 (d, J = 3.0 Hz, 1H), 6.67 (dd, J = 8.6, 3.0 Hz, 1H), 6.98 (d, J = 8.8 Hz, 1H), 7.15 - 7.28 (m, 1H), 7.38-7.53 (m, 2H), 7.89 (s, 1H), 8.32 (s, 1H).

### composé 259: 5-(2-chloro-3-fluoro-phenyl)-1-[2-[4-(7-methoxy-2-oxo-4,5-dihydro-1H-1,3-benzodiazepin-3-yl)-1-piperidyl]-2-oxo-ethyl]-3-(2-methyl-2-methylsulfonyl-propyl)pyrimidine-2,4-dione (example comparatif)

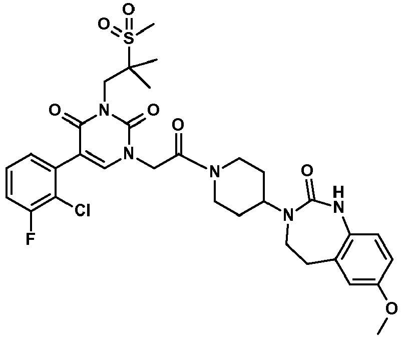

(δ, DMSO) : 1.30 (s, 6 H); 1.52-1.58 (m, 1 H); 1.64-1.77 (m, 3 H); 2.68 (t, J = 12.5 Hz, 1 H); 2.88 (m, 2 H); 3.06 (s, 3 H); 3.15 (t, J = 13.0 Hz, 1 H); 3.36 (m, 2 H); 3.67 (s, 3 H); 3.94 (d, J = 13.5 Hz, 1 H); 4.25-4.35 (m, 3 H); 4.43 (d, J = 13.7 Hz, 1 H); 4.80 (s, 2 H); 6.62 (d, J = 3.0 Hz, 1H), 6.67 (dd, J = 8.9, 3.0 Hz, 1H),
6.98 (d, J = 8.8 Hz, 1 H); 7.22-7.24 (m, 1 H); 7.45-7.48 (m, 2 H); 7.89 (s, 1 H); 8.33 (s, 1 H).

En outre, à titre illustratif, les exemples ci-dessous décrivent de façon non limitative les modes opératoires suivis pour l'obtention de groupements R1 des composés.

### Di-chlorohydrate de 1-pipéridin-4-yl-1,3-dihydro-imidazo[4,5-b]pyridin-2-one : (comparatif)

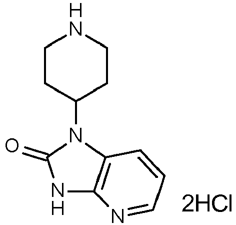

### 4-(2-chloro-pyridin-3-ylamino)-pipéridine-1-carboxyate d'éthyle :

A une solution de 2-chloro-pyridin-3-ylamine et 4-oxo-piperidine-1-carboxylate d'éthyle, dans de l'acétate d'isopropyle, sont rajoutés lentement de l'acide trifluoroacétique. Le milieu réactionnel est agité à température ambiante pendant une heure puis du triacétoxyborohydride de sodium sont additionnés par portions. Après deux heures d'agitation à temperature ambiante, une solution de soude aqueuse à 10% est rajoutée et le mélange est extrait par de l'acétate d'isopropyle. La phase organique est séchée sur sulfate de magnésium, filtrée sous vide puis concentrée à sec. Le 4-(2-chloro-pyridin-3-ylamino)-pipéridine-1-carboxyate d'éthyle est obtenu sous la forme d'une huile orange.

### 4-[1-(2-chloro-pyridin-3-yl)-ureido]-pipéridine-1-carboxylate d'éthyle:

A une solution de chlorosulfonylisocyanate dans du tétrahydrofurane, refroidie à -10°C sous flux d'azote, est rajoutée une solution contenant du 4-(2-chloro-pyridin-3-ylamino)-pipéridine-1-carboxylate d'éthyle, dissous dans du tétrahydrofurane. De l'acétate d'isopropyle est rajouté et le mélange est agité pendant une heure à -10°C. De l'eau est rajoutée, suivi par une solution de soude aqueuse à 10%. La phase organique est récupérée puis concentrée. Un solide beige précipite, il est filtré, lavé avec de l'acétate d'isopropyle puis séché sous vide à 60°C. Le 4-[1-(2-chloro-pyridin-3-yl)-ureido]-pipéridine-1-carboxylate d'éthyle est obtenu sous la forme d'un solide blanc.

### 4-(2-oxo-2,3-dihydro-imidazo[4,5-b]pyridin-1-yl)-pipéridine-1-carboxylate d'éthyle:

A une suspension dégazée sous azote de 4-[1-(2-chloro-pyridin-3-yl)-ureido]-pipéridine-1-carboxylate d'éthyle dans l'isopropanol, sont rajoutés du carbonate de potassium, du trimère d'acétate de palladium et du 1,4-bis(diphénylphosphino)-butane. Le mélange est agité à reflux pendant 21 heures. De l'eau et de l'acétate d'éthyle sont rajoutés. La phase organique est séchée sur sulfate de magnésium, filtrée sous vide puis concentrée à sec. Le brut est purifié par chromatographie flash sur gel de silice élué par un mélange dichlorométhane/méthanol, 95/5. Le 4-(2-oxo-2,3-dihydro-imidazo[4,5-b]pyridin-1-yl)-pipéridine-1-carboxylate d'éthyle est obtenu sous la forme d'un solide jaune pâle. Dichlorhydrate de 1-pipéridin-4-yl-1,3-dihydro-imidazo[4,5-b]pyridin-2-one:
A une solution de 4-(2-oxo-2,3-dihydro-imidazo[4,5-b]pyridin-1-yl)-pipéridine-1-carboxylate d'éthyle dans l'éthanol et l'eau, est rajouté une solution aqueuse de soude. Le milieu réactionnel est agité à reflux pendant 12 heures, puis concentré et repris dans l"isopropanol. Une solution d'acide chlorhydrique aqueuse 5N est rajoutée, le produit précipite, il est filtré, rincé avec de l'isopropanol puis séché sous vide à 60°C. Le dichlorhydrate de 1-pipéridin-4-yl-1,3-dihydro-imidazo[4,5-b]pyridin-2-one est obtenu sous la forme d'un solide beige.

### 1-(4-piperidyl)-3H-imidazo[4,5-b]pyridin-2-one: (example comparatif)

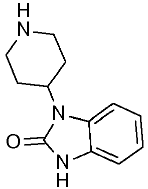

De manière analogue à la synthèse du 1-pipéridin-4-yl-1,3-dihydro-imidazo[4,5-b]pyridin-2-one décrit ci-dessus, à partir de la 2-chloro-phénylamine, le 1-(4-piperidyl)-3H-imidazo[4,5-b]pyridin-2-one est obtenu sous la forme d'un solide blanc.

### 3-pipéridin-4-yl-3,4-dihydro-1H-quinazolin-2-one: (comparatif)

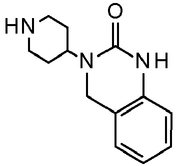

### [2-(1-benzyl-pipéridin-4-ylcarbamoyl)-phényl]-carbamate de tert-butyle:

8,9g (46,3 mmoles) de chorohydrate de 1-éthyl-3-(3-diméthylaminopropyl) carbodiimide sont additionnés à une solution de 10g (42,1 mmoles) d'acide 2-tert-butoxycarbonylamino-benzoïque, 10,3ml (50,6 mmoles) de 1-benzyl-pipéridin-4-ylamine, 6,3g (46,3 mmoles) de 1-hydroxybenzotriazole et 17,6ml (126,3 mmoles) de triéthylamine, dans 120ml de diméthylformamide. Le milieu réactionnel est ensuite agité avant l'addition de. Le milieu réactionnel est chauffé à 70°C pendant 5 heures puis hydrolysé par une solution aqueuse d'hydrogénocarbonate de sodium et dillué par de l'acétate d'éthyle. Le produit est extrait à l'acétate d'éthyle. La phase organique est lavée deux fois par une solution aqueuse saturée d'hydrogénocarbonate de sodium puis par une solution aqueuse saturée de chlorure de sodium et par de l'eau, séchée sur sulfate de magnésium et filtrée. Les solvants sont concentrés sous vide. 17g (100%) de [2-(1-benzyl-pipéridin-4-ylcarbamoyl)-phényl]-carbamate de tert-butyle sont obtenus sous la forme d'un solide beige.

### 2-amino-N-(1-benzyl-pipéridin-4-yl)-benzamide:

30mL (415 mmoles) d' acide trifluoroacétique sont ajoutés goutte à goutte à une solution de 17g (41,5 mmoles) de 2-(1-benzyl-pipéridin-4-ylcarbamoyl)-phényl]-carbamate de tert-butyle dans 170ml de dichlorométhane préalablement refroidie à 0°C. Le milieu réactionnel est agité de 0°C à température ambiante pendant 20 heures. Après concentration sous vide, le résidu est hydrolysé par une solution aqueuse d'hydrogénocarbonate de sodium puis dillué par de l'acétate d'éthyle. Le produit est extrait deux fois par de l'acétate d'éthyle. La phase organique est lavée une fois par de l'eau puis une fois par une solution aqueuse saturée de chlorure de sodium, séchée sur sulfate de magnésium, filtrée et concentrée sous vide. 14 g (100%) de 2-amino-N-(1-benzyl-pipéridin-4-yl)-benzamide sont obtenus sous la forme d'un solide beige.

### (2-amino-benzyl)-(1-benzyl-pipéridin-4-yl)-amine:

12g (38,8 mmoles) de 2-amino-N-(1-benzyl-pipéridin-4-yl)-benzamide dilués dans 72ml de dioxane sont ajoutés très lentement à une solution de 5,2g (135,7 mmoles) d'hydrure de lithium aluminium dans 260ml de dioxane, préalablement chauffé à reflux. Le milieu réactionnel (suspension grise) est ensuite agité et chauffé à reflux pendant 3 heures. Le mélange est refroidi à 0°C puis hydrolysé lentement par 5,2ml de soude aqueuse 15M et 15,5ml d'eau. Le milieu réactionnel est ensuite dillué par 240ml d'ether diéthylique et agité à température ambiante pendant 55 minutes. Après filtration des sels, le filtrat est concentré sous vide pour donner 10,7g (93%) de (2-amino-benzyl)-(1-benzyl-pipéridin-4-yl)-amine sous la forme d'une huile claire.

### 3-(1-benzyl-pipéridin-4-yl)-3,4-dihydro-1H-quinazolin-2-one:

0,9g (5,7 mmoles) de carbonyl diimidazole sont additionnés à une solution de 1,6g (5,2 mmoles) de (2-amino-benzyl)-(1-benzyl-pipéridin-4-yl)-amine dans 25ml de tétrahydrofurane. Le milieu réactionnel est agité à température ambiante pendant 5 heures. Le solvant est éliminé sous vide puis le milieu réactionnel est hydrolysé et dillué par de l'acétate d'éthyle. Après extraction à l'acétate d'éthyle, les phases organiques sont lavées deux fois par de l'eau puis par une solution aqueuse saturée de chlorure de sodium, séchées sur sulfate de magnésium, filtrées et concentrées sous vide. Le solide brut obtenu est trituré dans 15ml d'éther diéthylique puis filtré et séché sous vide pour donner 1,2g (72%) de 3-(1-benzyl-pipéridin-4-yl)-3,4-dihydro-1H-quinazolin-2-one sous la forme d'un solide blanc.

### 3-pipéridin-4-yl-3,4-dihydro-1H-quinazolin-2-one:

120mg de palladium sur charbon (10% massique) sont ajoutés à une solution de 1,2g (3,7 mmoles) de 3-(1-benzyl-pipéridin-4-yl)-3,4-dihydro-1H-quinazolin-2-one dans 30ml de méthanol, préalablement dégazée avec de l'azote. Le milieu est ensuite placé sous une atmosphère de dihydrogène pendant 48 heures puis filtré sur célite. Le filtrat est concentré sous vide pour donner 0,9g (100%) de 3-pipéridin-4-yl-3,4-dihydro-1H-quinazolin-2-one sous la forme d'un solide blanc.

### 3-pipéridin-4-yl-1,3,4,5-tétrahydro-benzo[d] [1,3]diazépin-2-one: (comparatif)

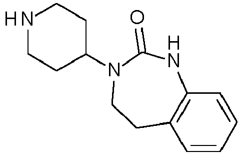

### (1-benzyl-pipéridin-4-yl)-[2-(2-nitro-phényl)-éthyl]-amine:

8ml (40 mmoles) de 1-benzyl-pipéridin-4-ylamine sont additionnés sur 4g (17,4 mmoles) de 1-(2-bromo-éthyl)-2-nitro-benzene et le mélange est chauffé à 100°C pendant 18 heures. Après refroidissement, de l'éther diéthylique est rajouté, le milieu est filtré et le filtrat est concentré. Le résidu brut est purifié par chromatographie sur gel de silice élué par un mélange acétate d'éthyle / heptane, 60/40 puis dichlorométhane / méthanol / ammoniaque, 95/3/2. 4,9g (82%) de (1-benzyl-pipéridin-4-yl)-[2-(2-nitro-phényl)-éthyl]-amine sont obtenus sous la forme d'une huile orangée.

### [2-(2-amino-phényl)-éthyl]-(1-benzyl-pipéridin-4-yl)-amine:

De manière analogue à l'exemple 1.5, à partir de 4,8g (14 mmoles) de (1-benzyl-pipéridin-4-yl)-[2-(2-nitro-phényl)-éthyl]-amine, 140mg d'oxyde de platine (10% molaire) et après purification du résidu par chromatographie sur gel de silice élué par un mélange dichlorométhane / méthanol / ammoniaque 90/8/2, 4,6g (100%) de [2-(2-amino-phényl)-éthyl]-(1-benzyl-pipéridin-4-yl)-amine sont obtenus sous la forme d'une huile brune.

### 3-(1-benzyl-pipéridin-4-yl)-1,3,4,5-tétrahydro-benzo[d] [1,3] diazépin-2-one:

De manière analogue à l'exemple 1.4, à partir de 1g (6,5 mmoles) de [2-(2-amino-phényl)-éthyl]-(1-benzyl-pipéridin-4-yl)-amine, 3,9g (78%) de 3-(1-benzyl-pipéridin-4-yl)-1,3,4,5-tetrahydro-benzo[d][1,3]diazépin-2-one sont obtenus sous la forme d'une poudre blanche.

### 3-pipéridin-4-yl-1,3,4,5-tétrahydro-benzo[d] [1,3]diazépin-2-one:

De manière analogue à l'exemple 1.5, à partir de 3,7g (11 mmoles) de 3-(1-benzyl-pipéridin-4-yl)-1,3,4,5-tetrahydro-benzo[d][1,3]diazépin-2-one, le milieu réactionnel étant placé sous 5bar de dihydrogène, 2,6g (96%) de 3-pipéridin-4-yl-1,3,4,5-tétrahydro-benzo[d][1,3]diazépin-2-one sont obtenus sous la forme d'une poudre blanche.

### 7-bromo-3-(4-pipéridyl)-4,5-dihydro-1H-1,3-benzodiazépin-2-one: (comparatif)

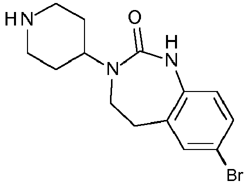

A une solution de 3-pipéridin-4-yl-1,3,4,5-tétrahydro-benzo[d][1,3]diazépin-2-one (préparé comme décrit dans l'exemple 16.4) dans du diméthylformamide, est ajouté du N-bromosuccinimide. Le mélange est agité pendant une heure à température ambiante puis de l'eau et de l'acétate d'éthyle sont rajoutés. Après extraction par de l'acétate d'éthyle, les phases organiques sont combinées, lavées à l'eau puis avec une solution saturée de chlorhydrate de sodium. Après séchage sur sulfate de magnésium, filtration et évaporation, le brut est purifié par phase inverse. Le 7-bromo-3-(4-pipéridyl)-4,5-dihydro-1H-1,3-benzodiazépin-2-one est obtenu sous la forme d'un solide beige.

### 7-méthoxy-3-pipéridin-4-yl-1,3,4,5-tétrahydro-benzo[d][1,3]diazépin-2-one: (selon l'invention)

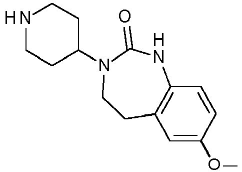

### (5-méthoxy-2-nitro-phényl)-acétonitrile:

Une solution contenant 30,5g (199 mmoles) de 4-nitroanisole et 40g (239 mmoles) de (4-chloro-phénoxy)-acétonitrile dans 305ml de N,N-diméthylformamide sont rajoutés à -20°C sous azote à une solution contenant 53,6g (478 mmoles) de tert-butylate de potassium dans 610ml de N,N-diméthylformamide. Le milieu réactionnel est laissé remonté jusqu'à 0°C et maintenu à cette température. Après 1 heure, le milieu réactionnel est placé à -50°C et hydrolysé lentement avec 1000ml d'une solution aqueuse glacée d'acide chlorhydrique 6N, puis 1000ml d'acétate d'éthyle et 500ml d'eau sont additionnés. La phase aqueuse est extraite avec de l''acétate d'éthyle. La phase organique est lavée par une solution aqueuse saturée de chlorure d'ammonium puis par de l'eau, et ensuite séchée sur sulfate de sodium anhydre, filtrée et concentrée. Le résidu obtenu est repris dans du toluène et évaporé. L'huile ainsi obtenue est reprise sous agitation dans 250ml d'un mélange acétate d'éthyle / heptane (50/50) pendant une nuit puis le mélange est refroidi dans un bain de glace et filtré. Après rinçage à l'heptane, 26,7g (68%) de (5-méthoxy-2-nitro-phényl)-acétonitrile sont obtenus sous la forme d'un solide orange.

### 2-(5-méthoxy-2-nitro-phényl)-éthylamine:

200ml (200 mmoles) d'une solution complexée de borane-tétrahydrofurane sont rajoutés à une solution contenant 26g (136 mmoles) de (5-méthoxy-2-nitro-phényl)-acétonitrile dans 130ml de 2-méthyltétrahydrofuranne à 60°C. Après 3 heures, le chauffage est arrêté et 35ml (863 mmoles) de méthanol sont rajoutés lentement ainsi que 200ml d'une solution aqueuse d'hydroxyde de sodium 1N. La phase aqueuse est ensuite extraite avec 250ml de méthyl-tétrahydrofurane. La phase organique est lavée par de l'eau, séchée sur sulfate de sodium anhydre, filtrée et concentrée partiellement. 400ml d'éther tertbutylique et 10ml (175 mmoles) d'acide acétique sont rajoutés. Le mélange est refroidi, laissé sous agitation toute une nuit puis concentré et coévaporé au toluène. 40,6g (100%) d' acétate de 2-(5-méthoxy-2-nitro-phényl)-éthylamine sont obtenus sous la forme d'une huile brune.

### 4-[2-(5-méthoxy-2-nitro-phényl)-éthylamino]-pipéridine-1-carboxylate de méthyle:

44,5g (210 mmoles) de triacétoxyhydroborate de sodium sont rajoutés à une solution sous azote contenant 40,6g (136 mmoles) d'acide 2-(5-méthoxy-2-nitro-phényl)-éthylamine acétique, 35,9g (180 mmoles) de N-(tert-butoxycarbonyl)-4-pipéridone dans 360ml de 2-méthyltétrahydrofuranne. Après 2 heures, le milieu réactionnel est hydrolysé avec une solution aqueuse d'hydroxyde de sodium 2N. La phase aqueuse est extraite avec du méthyltétrahydrofurane. La phase organique est lavée par de l'eau pour être amenée à pH=6, séchée sur sulfate de sodium, filtrée et évaporée. 69,2g (100%) de 4-[2-(5-méthoxy-2-nitro-phényl)-éthylamino]-pipéridine-1-carboxylate de méthyle sont obtenus sous la forme d'une huile foncée.

### 4-[2-(2-amino-5-méthoxy-phényl)-éthylamino]-pipéridine-1-carboxylate de tert-butyle:

3,7g (70 mmoles) de chlorure d'ammonium et 31,3g (560 mmoles) de fer sont rajoutés à une solution contenant 69,2g (137 mmoles) de 4-[2-(5-méthoxy-2-nitro-phényl)-éthylamino]-pipéridine-1-carboxylate de méthyle dans 130ml de 2-méthyltétrahydrofuranne,130ml de méthanol et 130ml d'eau. Le milieu réactionnel est chauffé à reflux pendant 4 heures puis laissé revenir à température ambiante. Au bout de 16 heures, le milieu réactionnel est filtré sur célite et le filtrat est concentré jusqu'à évaporation du méthanol. Du méthyltétrahydrofurane est rajouté ainsi qu'une solution à 10 % d'acide éthylènediaminetetraacétique. La phase organique est lavée par de l'eau puis séchée sur sulfate de sodium anhydre, filtrée et concentrée. 53,2g (100%) de 4-[2-(2-amino-5-méthoxy-phényl)-éthylamino]-pipéridine-1-carboxylate de tert-butyle sont obtenus sous la forme d'une huile orange.

### 4-(7-méthoxy-2-oxo-1,2,4,5-tétrahydro-benzo[d] [1,3]diazépin-3-yl)-pipéridine-1-carboxylate de tert-butyle:

27,2g (168 mmoles) de N,N'-carbonyldiimidazole sont rajoutés par portion à une solution contenant 53,2g (137 mmoles) de 4-[2-(2-amino-5-méthoxy-phényl)-éthylamino]-pipéridine-1-carboxylate de tert-butyle à 90% dans du 1000ml de toluène. Après 1 heure, le précipité formé est filtré et le filtrat est concentré partiellement. Le résidu est lavé deux fois par une solution aqueuse de chlorure d'ammonium. La phase organique est séchée sur sulfate de sodium anhydre, filtrée et concentrée. 58g d'une huile orange sont obtenus. Après purification par chromatographie sur gel de silice élué par un mélange d'acétate d'éthyle dans l'heptane, suivant un gradient de polarité (de 20% à 70% d'acétate d'éthyle dans l'heptane), 35g d'un solide jaune sont obtenus et repris dans 200ml d'éther isopropylique au reflux, 100ml d'éther isopropylique, 100ml de tétrahydrofurane, 80ml d'éthanol et 300ml de méthanol. Le mélange est filtré puis concentré. Après purification par chromatographie sur gel de silice élué par un mélange d'acétate d'éthyle dans l'heptane, suivant un gradient de polarité (de 20% à 100% d'acétate d'éthyle dans l'heptane), 21,1g (34%) de 4-(7-méthoxy-2-oxo-1,2,4,5-tétrahydro-benzo[d] [1,3]diazépin-3-yl)-pipéridine-1-carboxylate de tert-butyle sont obtenus sous la forme d'un solide beige.

### 7-méthoxy-3-pipéridin-4-yl-1,3,4,5-tétrahydro-benzo[d][1,3]diazépin-2-one:

35ml (380 mmoles) d'acide trifluoroacétique sont rajoutés à 0°C à une solution contenant 83% de 4-(7-méthoxy-2-oxo-1,2,4,5-tétrahydro-benzo[d] [1,3]diazépin-3-yl)-pipéridine-1-carboxylate de tert-butyle_dans 300ml de dichlorométhane. Après 15 heures, le milieu réactionnel est concentré lentement à l'évaporateur rotatif. Le résidu est coévaporé au toluène,repris dans 250ml de dichlorométhane puis le mélange est amené à pH=10 avec une solution aqueuse d'hydroxyde de sodium 1N. La phase aqueuse est extraite par du dichlorométhane, la phase organique est séchée sur sulfate de sodium anhydre, filtrée et concentrée. 12,7g (96%) de 7-méthoxy-3-pipéridin-4-yl-1,3,4,5-tétrahydro-benzo[d][1,3]diazépin-2-one sont obtenus sous la forme d'un solide beige.

### 3-(4-méthyl-pipéridin-4-yl)-1,3,4,5-tétrahydro-benzo[d][1,3]diazépin-2-one: (comparatif)

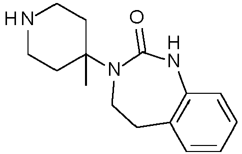

### 4-méthyl-4-[2-(2-nitro-phényl)-acétylamino]-pipéridine-1-carboxylate de tert-butyle:

De manière analogue à l'exemple 1.6, à partir de l'acide 2-nitrophénylacétique et du 4-amino-4-méthyl-pipéridine-1-carboxylate de tert-butyle, le 4-méthyl-4-[2-(2-nitro-phényl)-acétylamino]-pipéridine-1-carboxylate de tert-butyle est obtenu sous la forme d'un solide blanc.

### 4-méthyl-4-[2-(2-nitro-phényl)-éthylamino]-pipéridine-1-carboxylate de tert-butyle:

De manière analogue à l'exemple 115.2, à partir du 4-méthyl-4-[2-(2-nitro-phényl)-acétylamino]-pipéridine-1-carboxylate de tert-butyle et du complexe de borane méthyl sulfide, le 4-méthyl-4-[2-(2-nitro-phényl)-éthylamino]-pipéridine-1-carboxylate de tert-butyle est obtenu sous la forme d'une pâte blanche.

### 4-[2-(2-amino-phényl)-éthylamino]-4-méthyl-pipéridine-1-carboxylate de tert butyle:

De manière analogue à l'exemple 115.4, à partir du 4-méthyl-4-[2-(2-nitro-phényl)-éthylamino]-pipéridine-1-carboxylate de tert-butyle, le 4-[2-(2-amino-phényl)-éthylamino]-4-méthyl-pipéridine-1-carboxylate de tert butyle est obtenu sous la forme d'une huile incolore.

### 4-méthyl-4-(2-oxo-1,2,4,5-tétrahydro-benzo[d] [1,3]diazépin-3-yl)-pipéridine-1-carboxylate de tert butyle:

De manière analogue à l'exemple 1.4, à partir du 4-[2-(2-amino-phényl)-éthylamino]-4-méthyl-pipéridine-1-carboxylate de tert butyle, le 4-méthyl-4-(2-oxo-1,2,4,5-tétrahydro-benzo[d][1,3]diazépin-3-yl)-pipéridine-1-carboxylate de tert butyle est obtenu sous la forme d'un solide blanc.

### 3-(4-méthyl-pipéridin-4-yl)-1,3,4,5-tétrahydro-benzo[d][1,3]diazépin-2-one:

De manière analogue à l'exemple 115.11, à partir du 4-méthyl-4-(2-oxo-1,2,4,5-tétrahydro-benzo[d] [1,3]diazépin-3-yl)-pipéridine-1-carboxylate de tert butyle, le 3-(4-méthyl-pipéridin-4-yl)-1,3,4,5-tétrahydro-benzo[d] [1,3]diazépin-2-one est obtenu sous la forme d'une huile incolore.

### 9-fluoro-3-pipéridin-4-yl-1,3,4,5-tétrahydrobenzo[d] [1,3] diazépin-2-one: (comparatif)

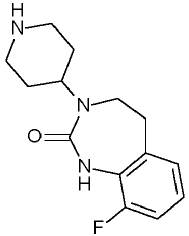

### N-(1-benzyl-pipéridin-4-yl)-2-(3-fluoro-2-nitro-phényl)-acétamide:

De manière analogue à l'exemple 1.6, à partir du 4-amino-1-benzylpipéridine et de l'acide 2-(3-fluoro-2-nitrophényl) acétique, le N-(1-benzyl-pipéridin-4-yl)-2-(3-fluoro-2-nitro-phényl)-acétamide est obtenu sous la forme d'un solide jaune.

### [2-(2-amino-3-fluoro-phényl)-éthyl]-(1-benzyl-pipéridin-4-yl)-amine:

De manière analogue à l'exemple 1.3, à partir du N-(1-benzyl-pipéridin-4-yl)-2-(3-fluoro-2-nitro-phényl)-acétamide, le [2-(2-amino-3-fluoro-phényl)-éthyl]-(1-benzyl-pipéridin-4-yl)-amine est obtenu sous la forme d'une huile brune.

### 3-(1-benzyl-pipéridin-4-yl)-9-fluoro-1,3,4,5-tétrahydro-benzo[d] [1,3]diazépin-2-one:

De manière analogue à l'exemple 1.4, à partir du [2-(2-amino-3-fluoro-phényl)-éthyl]-(1-benzyl-pipéridin-4-yl)-amine, le 3-(1-benzyl-pipéridin-4-yl)-9-fluoro-1,3,4,5-tétrahydro-benzo[d] [1,3]diazépin-2-one est obtenu sous la forme d'une huile jaune.

### 9-fluoro-3-pipéridin-4-yl-1,3,4,5-tétrahydro-benzo[d] [1,3]diazépin-2-one:

De manière analogue à l'exemple 1.5, à partir du 3-(1-benzyl-pipéridin-4-yl)-9-fluoro-1,3,4,5-tétrahydro-benzo[d][1,3]diazépin-2-one, le 9-fluoro-3-pipéridin-4-yl-1,3,4,5-tétrahydro-benzo[d][1,3]diazépin-2-one est obtenu sous la forme d'une huile jaune.

### 7-fluoro-3-pipéridin-4-yl-1,3,4,5-tétrahydro-benzo[d][1,3]diazépin-2-one: (comparatif)

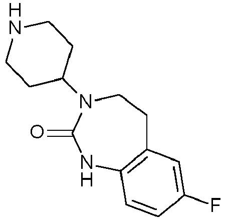

De manière analogue à la synthèse du 9-fluoro-3-pipéridin-4-yl-1,3,4,5-tétrahydro-benzo[d][1,3]diazépin-2-one, à partir du 2-(5-fluoro-2-nitro-phényl)-éthylamine et du 1-Boc-4-pipéridone, le 7-fluoro-3-pipéridin-4-yl-1,3,4,5-tétrahydro-benzo[d] [1,3]diazépin-2-one est obtenu sous la forme d'un solide jaune.

### 7-pipéridin-4-yl-5,6,7,9-tétrahydro-1,7,9-triaza-benzocyclohepten-8-one: (comparatif)

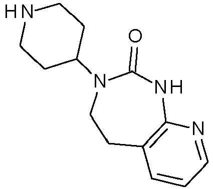

De manière analogue à la synthèse du 9-fluoro-3-pipéridin-4-yl-1,3,4,5-tétrahydro-benzo[d][1,3]diazépin-2-one, à partir de l'acide (2-amino-pyridin-3-yl)-acétique et du 4-amino-1-benzylpipéridine, le 7-pipéridin-4-yl-5,6,7,9-tétrahydro-1,7,9-triaza-benzocyclohepten-8-one est obtenu sous la forme d'un solide blanc.

### 7-pipéridin-4-yl-5,6,7,9-tétrahydro-2,7,9-triaza-benzocyclohepten-8-one: (comparatif)

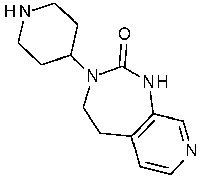

De manière analogue à la synthèse du 7-pipéridin-4-yl-5,6,7,9-tétrahydro-1,7,9-triaza-benzocyclohepten-8-one, à partir de l'acide (3-amino-pyridin-4-yl)-acétique et du 4-amino-1-benzylpipéridine, le 7-pipéridin-4-yl-5,6,7,9-tétrahydro-2,7,9-triaza-benzocyclohepten-8-one est obtenu sous la forme d'un solide blanc.

### 7-pipéridin-4-yl-5,7,8,9-tétrahydro-2,5,7-triaza-benzocyclohepten-6-one: (comparatif)

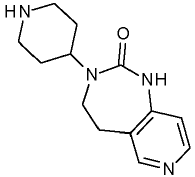

De manière analogue à la synthèse du 7-pipéridin-4-yl-5,6,7,9-tétrahydro-1,7,9-triaza-benzocyclohepten-8-one, à partir de l'acide (4-amino-pyridin-3-yl)-acétique et du 4-amino-1-benzylpipéridine, le 7-pipéridin-4-yl-5,7,8,9-tétrahydro-2,5,7-triaza-benzocyclohepten-6-one est obtenu sous la forme d'un solide blanc.

### 7-pipéridin-4-yl-5,7,8,9-tétrahydro-1,5,7-triaza-benzocyclohepten-6-one:

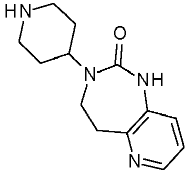

De manière analogue à la synthèse du 7-pipéridin-4-yl-5,6,7,9-tétrahydro-1,7,9-triaza-benzocyclohepten-8-one, à partir de l'acide (5-amino-pyridin-3-yl)-acétique et du 4-amino-1-benzylpipéridine, le 7-pipéridin-4-yl-5,7,8,9-tétrahydro-1,5,7-triaza-benzocyclohepten-6-one est obtenu sous la forme d'un solide blanc.

### 3-pipéridin-4-yl-3,4-dihydro-1H-pyrido[2,3-d]pyrimidin-2-one: (comparatif)

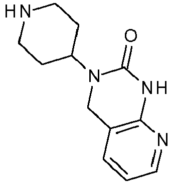

De manière analogue à la synthèse du 3-pipéridin-4-yl-3,4-dihydro-1H-quinazolin-2-one (décrit dans l'exemple 1.5), et à partir de l'acide 2-tert-butoxycarbonylamino-nicotinique, le 3-pipéridin-4-yl-3,4-dihydro-1H-pyrido[2,3-d]pyrimidin-2-one est obtenu sous la forme d'un solide beige.

### 7-méthylsulfanyl-3-pipéridin-4-yl-1,3,4,5-tétrahydro-benzo[d][1,3]diazépin-2-one: (comparatif)

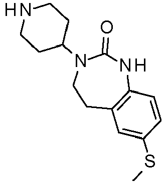

De manière analogue à la synthèse du 7-méthoxy-3-pipéridin-4-yl-1,3,4,5-tétrahydro-benzo[d][1,3]diazépin-2-one (décrit dans l'exemple 115.6), et à partir du 1-Boc-4-pipéridone et du 2-(5-méthylsulfanyl-2-nitro-phényl)-éthylamine, le 7-méthylsulfanyl-3-pipéridin-4-yl-1,3,4,5-tétrahydro-benzo[d] [1,3]diazépin-2-one est obtenu sous la forme d'un solide blanc.

### 7-méthylsulfinyl-3-(4-pipéridyl)-4,5-dihydro-1H-1,3-benzodiazépin-2-one: (comparatif)

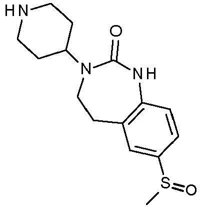

De manière analogue à la synthèse du 7-méthylsulfanyl-3-pipéridin-4-yl-1,3,4,5-tétrahydro-benzo[d][1,3]diazépin-2-one, et après oxydation par l'acide 3-chloroperoxybenzoïque, le 7-méthylsultinyl-3-(4-pipéridyl)-4,5-dihydro-1H-1,3-benzodiazépin-2-one est obtenu sous la forme d'un solide blanc.

### 7-méthylsulfonyl-3-(4-pipéridyl)-4,5-dihydro-1H-1,3-benzodiazépin-2-one: (comparatif)

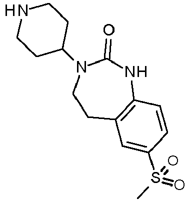

De manière analogue à la synthèse du 7-méthylsulfanyl-3-pipéridin-4-yl-1,3,4,5-tétrahydro-benzo[d][1,3]diazépin-2-one, et après oxydation par le péroxyde d'hydrogène en présence de molybdate d'ammonium tétrahydrate, l'acide 3-chloroperoxybenzoïque, le 7-méthylsulfonyl-3-(4-pipéridyl)-4,S-dihydro-1H-1,3-benzodiazépin-2-one est obtenu sous la forme d'un solide blanc.

### Acide 3-pipéridin-4-yl)-2-oxo-2,3,4,5-tétrahydro-1H-benzo[d][1,3]diazépine-7-carboxylique: (comparatif)

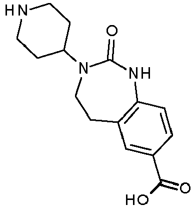

A partir du 7-bromo-3-(4-pipéridyl)-4,5-dihydro-1H-1,3-benzodiazépin-2-one, et après carboxylation avec de l'hydrure de sodium, du n-butyllithium et du dioxyde de carbone, l'acide 3-pipéridin-4-yl)-2-oxo-2,3,4,5-tétrahydro-1H-benzo[d][1,3]diazépine-7-carboxylique est obtenu sous la forme d'un solide blanc.

### 5,5-diméthyl-3-(4-pipéridyl)-1,4-dihydro-1,3-benzodiazépin-2-one:

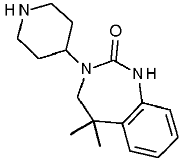

De manière analogue à la synthèse du 7-méthoxy-3-pipéridin-4-yl-1,3,4,5-tétrahydro-benzo[d][1,3]diazépin-2-one (décrit dans l'exemple 115.6), et à partir du 1-Boc-4-pipéridone et du 2-(5-méthoxy-2-nitro-phényl)-2-méthyl-propylamine, le 5,5-diméthyl-3-(4-pipéridyl)-1,4-dihydro-1,3-benzodiazépin-2-one est obtenu sous la forme d'un solide beige.

### 5-méthyl-3-(4-pipéridyl)-4,5-dihydro-1H-1,3-benzodiazépin-2-one: (comparatif)

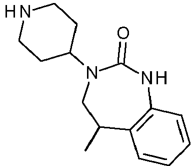

De manière analogue à la synthèse du 7-méthoxy-3-pipéridin-4-yl-1,3,4,5-tétrahydro-benzo[d][1,3]diazépin-2-one (décrit dans l'exemple 115.6), et à partir du 1-Boc-4-pipéridone et du 2-(5-méthoxy-2-nitro-phényl)-propylamine, le 5-méthyl-3-(4-pipéridyl)-4,5-dihydro-1H-1,3-benzodiazépin-2-one est obtenu sous la forme d'un solide blanc.

### 4-méthyl-3-(4-pipéridyl)-4,5-dihydro-1H-1,3-benzodiazépin-2-one: (comparatif)

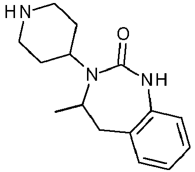

De manière analogue à la synthèse du 7-méthoxy-3-pipéridin-4-yl-1,3,4,5-tétrahydro-benzo[d][1,3]diazépin-2-one (décrit dans l'exemple 115.6), et à partir du 1-Boc-4-pipéridone et du 2-(5-méthoxy-2-nitro-phényl)-1-méthyl-éthylamine, le 4-méthyl-3-(4-pipéridyl)-4,5-dihydro-1H-1,3-benzodiazépin-2-one est obtenu sous la forme d'un solide blanc.

### 3-(6-azaspiro[3.3]heptan-2-yl)-4,5-dihydro-1H-1,3-benzodiazépin-2-one: (comparatif)

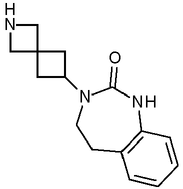

De manière analogue à la synthèse du 7-méthoxy-3-pipéridin-4-yl-1,3,4,5-tétrahydro-benzo[d][1,3]diazépin-2-one (décrit dans l'exemple 115.6), et à partir du 1-(2-bromoéthyl)-2-nitrobenzène et du 6-amino-2-aza-spiro[3.3]heptane-2-carboxylate de tert-butyle, le 3-(6-azaspiro[3.3]heptan-2-yl)-4,5-dihydro-1H-1,3-benzodiazépin-2-one est obtenu sous la forme d'un solide blanc.

### 4-phényl-1-pipéridin-4-yl-1,3-dihydro-imidazol-2-one: (comparatif)

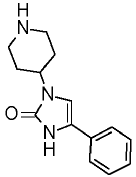

### 4-(2-oxo-4-phényl-2,3-dihydro-imidazol-1-yl)-pipéridine-1-carboxylate de tert butyle:

A une solution de 2-bromoacétophénone dans du dichlorométhane, sont rajoutés du N-Boc-4-amino-pipéridine et de la N,N-diisopropyléthylamine en solution dans du dichlorométhane. Le milieu réactionnel est agité à température ambiante pendant seize heures, puis du cyanate de sodium est rajouté, ainsi que de l'acide acétique glacial. Après extraction par de l'eau et du dichlorométhane, La phase organique est séchée puis filtrée et concentrée à sec. Le 4-(2-oxo-4-phényl-2,3-dihydro-imidazol-1-yl)-pipéridine-1-carboxylate de tert butyle est obtenu sous forme d'un solide crème.

### 4-phényl-1-pipéridin-4-yl-1,3-dihydro-imidazol-2-one:

De manière analogue à l'exemple 1.2, à partir du 4-(2-oxo-4-phényl-2,3-dihydro-imidazol-1-yl)-pipéridine-1-carboxylate de tert butyle, le 4-phényl-1-pipéridin-4-yl-1,3-dihydro-imidazol-2-one est obtenu sous la forme d'un solide orange.

### spiro[1H-pyrido[2,3-d][1,3]oxazine-4,4'-piperidine]-2-one: (comparatif)

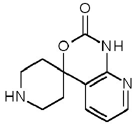

### (6-chloro-pyridin-2-yl)-carbamate de tert-butyle:

2-amino-6-chloropyridine et bis(triméthylsilyl)amide de sodium sont placés en solution dans tétrahydrofuranne. Une solution de di-tert-butyl dicarbonate dans tétrahydrofuranne est rajoutée goutte à goutte. Le milieu réactionnel est agité à température ambiante pendant vingt heures. De l'eau et de l'acétate d'éthyle sont additionnés, le produit est extrait à l'acétate d'éthyle. Les phases organiques sont rassemblées, lavées une fois par de l'eau et une fois par une solution aqueuse saturée de chlorure de sodium, puis séchées sur sulfate de magnésium, filtrées et concentrées sous vide. Le (6-chloro-pyridin-2-yl)-carbamate de tert-butyle est obtenu sous la forme d'un solide marron.

### 7'-chloro-2'-oxo-1',2'-dihydro-1H-spiro[pipéridine-4,4'-pyrido[2,3-d][1,3]oxazine]-1-carboxylate de benzyle: (comparatif)

A une solution à -20°C de N,N,N',N'-tétraméthyléthylènediamine dans tétrahydrofurane est additionné goutte à goutte n-butyllithium. Après trente minutes d'agitation, le milieu est refroidi à -78°C et une solution de (6-chloro-pyridin-2-yl)-carbamate de tert-butyle dans tétrahydrofurane est additionnée. La réaction est maintenue à -50°C pendant deux heures puis une solution de 4-oxo-pipéridine-1-carboxylate de benzyle dans tétrahydrofurane est additionnée goutte à goutte. Le milieu réactionnel est lentement ramené à température ambiante puis chauffé à 40°C pendant dix huit heures. Le milieu réactionnel est hydrolysé par une solution aqueuse saturée d'hydrogénocarbonate de sodium puis dilué par du dichlorométhane. Le produit est extrait au dichlorométhane, les phases organiques sont rassemblées, lavées une fois par de l'eau puis séchées sur sulfate de magnésium, filtrées et concentrées sous vide. Le produit brut est chromatographié sur gel de silice élué par un mélange heptane / acétate d'éthyle 70/30 puis 50/50. 7'-chloro-2'-oxo-1',2'-dihydro-1H-spiro[pipéridine-4,4'-pyrido[2,3-d][1,3]oxazine]-1-carboxylate de benzyle est obtenu sous la forme d'un solide jaune.

### spiro[1H-pyrido[2,3-d][1,3]oxazine-4,4'-piperidine]-2-one: (comparatif)

De manière analogue à l'exemple 98.4, à partir du 7'-chloro-2'-oxo-1',2'-dihydro-1H-spiro[piperidine-4,4'-pyrido[2,3-d][1,3]oxazine]-1-carboxylate de benzyle, le spiro[1H-pyrido[2,3-d][1,3]oxazine-4,4'-piperidine]-2-one est obtenu sous la forme d'un solide jaune.

Un autre objet de l'invention concerne les composés selon l'invention pour leur utilisation comme médicament.

Préférentiellement, les composés selon l'invention sont utilisés dans la prévention et/ou le traitement de maladies inflammatoires avec une composante neurogène.

Le terme « prévention » ou « prévenir » les maladies inflammatoires avec une composante neurogène signifie diminuer et/ou éviter l'apparition des symptômes de ces maladies.

Le terme « traitement » ou « traiter » les maladies inflammatoires avec une composante neurogène signifie diminuer et/ou inhiber le développement de ces maladies et/ou de leurs symptômes.

Par « maladies inflammatoires avec une composante neurogène », on entend préférentiellement les maladies inflammatoires de la peau avec une composante neurogène choisies parmi la rosacée de type I (Erythémateuse) et de type II (Papulopustuleuse), la dermatite atopique, l'eczema chronique des mains, le psoriasis sous ses différentes formes (vulgaire, du scalp, arthritique, pustuleux, de goutte), l'érythème facial, l'érythème pudique, l'urticaire (aigue, prurit sénile, prurigo nodularis) et l'acné, ou encore les céphalées avec une inflammation neurogène telles que les migraines.

Les maladies inflammatoires de la peau avec une composante neurogène sont préférentiellement choisies parmi la rosacée de type I (Erythémateuse), la dermatite atopique, le psoriasis vulgaire et du scalp, et l'uticaire du type prurit sénile et prurigo nodularis, encore plus préférentiellement la rosacée de type I (Erythémateuse).

Les composés selon l'invention peuvent également être utilisés dans la prévention et/ou le traitement de maladies inflammatoires avec une composante vasculaire telles que les hémangiomes infantiles ou le purpura sénile.

L'intérêt pour les nouveaux composés hétérocycliques antagonistes du récepteur CGRP selon l'invention dans le traitement des maladies inflammatoires avec une composante neurogène est basé sur des éléments cliniques soulignant le parallèle entre notamment la rosacée et la migraine :
- la localisation faciale des lésions chez les patients atteints de rosacée est semblable aux zones d'innervation par le nerf trigéminal également impliqué dans la migraine ;
- la prévalence des migraineux chez les patients atteints de rosacée est démontrée par des études épidémiologiques (≥ 27% vs 13% dans la population générale).

Il repose également sur des données expérimentales :
- l'effet vasodilatateur de CGRP sur les vaisseaux cutanés a été démontré en clinique : l'injection de faibles doses de CGRP en sous cutanée induit un érythème persistant de plusieurs heures ;
- l'application topique de capsaïcine induit la libération de neuropeptides, parmi lesquels CGRP, qui se traduit par une vasodilatation cutanée ;
- l'injection par voie intraveineuse de αCGRP chez la souris provoque l'apparition d'un flush majoritairement mesurable au niveau de la face de l'animal. Chez des souris invalidées pour le gène mRamp1, aucune réponse vasodilatatrice à ce flush au CGRP n'est observée. De même chez ces souris, aucun œdème n'apparait dans un modèle d'inflammation neurogène local induit par l'application topique d'un agoniste TRPV1. Ces résultats semblent confirmer un rôle majeur de la libération de CGRP dans l'inflammation neurogène et dans le flush.

A cet effet, de manière générale, les composés selon l'invention présentent une bonne affinité pour le récepteur CGRP et ainsi une bonne activité antagoniste au récepteur CGRP.

Cette activité est plus particulièrement illustrée par le test de dosage cellulaire GPCR GeneBLAzer® réalisé qui fournit une méthode homogène de suivi de la réponse cellulaire à des médicaments candidats.

Dans le modèle GeneBLAzer® GPCR Cell-based Assay fournit par Invitrogen (Lifetechnologies), l'activation du récepteur CALCLR:RAMP1 conduit à l'expression d'un gène rapporteur (la beta-lactamase). L'activité de la beta-lactamase est quantifiée par FRET (fluorescence resonance energy transfer) en présence d'un substrat fluorescent et est directement proportionnelle à l'activation du récepteur.

Les lignées GeneBLAzer® CALCRL:RAMP CRE-bla HEK293F sont des cellules HEK293F qui surexpriment stablement les récepteurs CALCRL et RAMP1 ainsi que le gène rapporteur de la beta-lactamase sous le contrôle de l'élément de réponse à l'AMPc (CRE) .

Le substrat de la beta-lactamase contient deux fluorophores (fluorescéine et coumarine) associés par un noyau beta-lactame. En l'absence de beta-lactamase et lorsqu'il est excité à λ=409nm, le substrat reste intact et la longueur d'onde d'émission de la fluorescence de la coumarine (λ=460nm) excite la fluorescéine qui émet à son tour (λ=530nm). En revanche, lorsque la beta-lactamase est exprimée, le substrat est clivé, séparant les deux fluorophores et interrompant le FRET. L'émission de la coumarine sera alors proportionnelle à l'activation du récepteur.

Les cellules sont ensemencées en microplaques 384 puits à raison de 10 000 cellules par puits et incubées 18 heures à 37°C. Des dilutions sérielles verticales d'agoniste (a-Calcitonin Gene Related Peptide pour RAMP1) sont ajoutées en compétition avec des dilutions sérielles horizontales d'antagonistes à tester. Après 3 heures d'incubation à 37°C, le substrat de la beta-lactamase est ajouté à chaque puits et la microplaque est incubée 2 heures à température ambiante. La fluorescence est alors mesurée à l'aide d'un lecteur de microplaques (λ_{excitation}=409nm, λ_{émission 1}=460nm, λ_{émission 2}=530nm).

La table suivante détaille les différentes conditions appliquées aux différentes lignées utilisées :

| **Lignée** | **Réf. Invitrogen** | **Espèce** | **Récepteur** | **Agoniste** | **Gamme agoniste (nM)** | **Gamme antagoniste (nM)** |
|---|---|---|---|---|---|---|
| **HEK hRAMP1** | K1437 | Humain | CALCRL: RAMP1 | αCGRP (NEOMPS, #SC113) | 0.002-10 | 0.04-10000 |

Les données de fluorescence sont normalisées à l'aide de contrôles négatifs (cellules non stimulées) et de contrôles positifs (agoniste à concentration saturante).
Les constantes de dissociation pour chaque état du récepteur[« Mechanistic analysis of the function of agonists and allosteric modulators: reconciling two-state and operational models » David Roche, British Journal of Pharmacology (2013) 169 1189-1202] (KdR = affinité pour l'état repos et KdA = affinité pour l'état activé) sont calculées et le ratio KdR/KdA représente l'efficacité intrinsèque du ligand testé : si KdR=KdA alors le ligand testé est un antagoniste neutre.
Les différentes EC50 de l'agoniste obtenues à chaque concentration d'antagoniste testé sont linéarisées (régression de Schild [« Quantitation in receptor pharmacology » Terry P.Kenakin, Receptors and Channels (2001) 7 371-385]) afin de déterminer l'affinité apparente (Kdapp) de l'antagoniste. Cette valeur est nommée apparente car elle peut fluctuer en fonction de l'activation constitutive et de l'expression du récepteur et est assimilable au Kb dans le cas d'un antagoniste neutre.

Les résultats d'affinité ainsi obtenus pour chacun des composés testés sont récapitulés dans le tableau I ci-dessous dans lequel :
Kdapp représente l'affinité du composé pour le récepteur CGRP ;
A représente un Kdapp <10 nM;
B représente un Kdapp entre 10 - 100 nM;
C représente un Kdapp entre 101 - 500 nM; et
D représente un Kdapp entre 501 nM - 5000 nM.

**Tableau I :**

| Kdapp **hCGRP (nM)** | **n° Composé** |
|---|---|
| A (<10 nM) | 14; 16-19; 22-24; 28-31; 47; 51-54; 72-73; 83; 89-90; 93; 95-98; 100-101; 103-106; 108; 110; 112-124; 126-128; 130-132; 134-140; 142-146; 148; 152; 155-162; 165-166; 170; 178; 184-189; 192; 199; 202-203; 205-208; 210; 213; 215; 220-221; 225-229; 231-249; 251-259 |
| B (10 - 100 nM) | 1; 4; 8-9; 11-13; 20-21; 25-27; 33; 35; 37-38; 40; 46; 48; 55-57; 59-63; 66-69; 76; 81; 85-88; 91-92; 94; 99; 102; 109; 111; 129; 133; 147; 149-150; 153; 164; 167; 169; 171-177; 179-183; 190; 193-198; 200-201; 204; 211; 214; 250 |
| C (101 - 500 nM) | 2-3; 6; 10; 34; 41-42; 45; 49; 64-65; 70-71; 78; 84; 141; 154; 168; 216-217; 224; 230 |
| D (501 - 5000 nM) | 5; 7; 15; 32; 36; 39; 43-44; 50; 58; 74-75; 77; 79-80; 82; 107; 151; 163; 191; 209; 218-219; 222-223 |

Considérant les résultats répertoriés dans le tableau ci-dessus, les composés présentant un Kdapp classifié A ont une très forte affinité pour le récepteur au CGRP. De plus, ces composés présentent un ratio KdR/KdA = 1 et donc une activité antagoniste du récepteur au CGRP.

Dans la mesure où l'inflammation neurogène joue un rôle important dans la physiopathologie notamment de la rosacée et de la migraine, les antagonistes au récepteur CGRP selon l'invention permettraient ainsi de réduire ou abolir totalement cette inflammation, avec pour résultat un effet persistant à l'arrêt du traitement.

Outre leur potentiel sur le plan de l'activité biologique en tant qu'antagoniste puissant et sélectif de CGRP-R humain, ces composés, avantageusement les dérivés uraciles, présentent une instabilité métabolique au niveau hépatique et devraient ainsi présenter un risque d'hépatotoxicité limité.

Elle est plus particulièrement illustrée par le test de stabilité métabolique en présence de microsomes hépatiques humains ci-dessous.

Des microsomes hépatiques humains (Becton Dickinson) sont incubés à une concentration de protéine de 0,5 mg/mL dans le milieu réactionnel.

Le milieu réactionnel des microsomes est composé de tampon Phosphate pH : 7,4 à 100 mM, de MgCl₂ à 100 mM. (50/50), d'un système de génération d'ATP composé d'un mélange de Nicotinamide Adénine Di-Phosphate (NADP), de Glucose-6-Phosphate (G6P) à 1 mg/mL et de Glucose-6-Phosphate Deshydrogénase (G6PDH) à 4 U/mL. Les composés sont testés à µM (DMSO 0,1%)

Les prélèvements de milieu d'incubation après ajout des microsomes sont réalisés au temps 15 minutes. La réaction métabolique est stoppée par ajout de méthanol (1 volume milieu incubation/3 volumes de méthanol). Le pourcentage du produit parent restant après 15 minutes est mesurée par analyse LC/MS/MS.

Les résultats obtenus sont répertoriés dans le tableau 2 ci-dessous.

**Tableau 2 :**

| n° Composé | % de produit restant à 15min (microsomes hépatiques humains) |
|---|---|
| Olcegepant | 100 |
| Telcagepant | 100 |
| MK-3207 | 100 |
| 25 | 8 |
| 30 | 16 |
| 63 | 45 |
| 110 | 1 |
| 112 | 0 |
| 114 | 41 |
| 115 | 0 |
| 120 | 1 |
| 142 | 65 |
| 143 | 42 |
| 149 | 26 |
| 150 | 0 |
| 158 | 0 |
| 165 | 0 |
| 193 | 18 |
| 206 | 1 |
| 232 | 1 |
| 237 | 3 |
| 240 | 0 |
| 251 | 4 |

D'après les résultats obtenus, les composés de formule (I) présentent une instabilité métabolique au niveau hépatique par rapport aux composés connus de l'art antérieur testés et devraient ainsi présenter un risque d'hépatotoxicité limité.

Les composés selon l'invention peuvent également être formulés dans des compositions pharmaceutiques à usage humain. Lesdites compositions comprennent dans un véhicule pharmaceutiquement acceptable au moins un composé de formule générale (I) selon l'invention.

Par véhicule pharmaceutiquement acceptable, on entend un véhicule adapté pour une utilisation en contact avec des cellules d'humains, compatible avec la peau, les muqueuses et les phanères, sans toxicité, irritation, réponse allergique indue, et proportionné à un rapport avantage/risque raisonnable.

De préférence, le composé de formule générale (I) selon l'invention est présent dans une composition pharmaceutique pour application topique, ce qui réduit considérablement tout effet secondaire au niveau hépatique.

## Revendications

1. Composé hétérocyclique choisi parmi les composés de formule générale (I) : 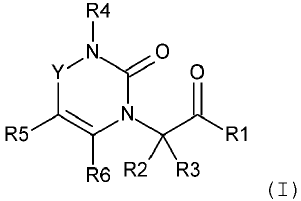 dans laquelle,
- Y est -C (O) ;
- R1 est 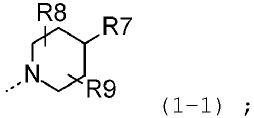
- R2, R3, R6 sont un atome d'hydrogène ;
- R4 est 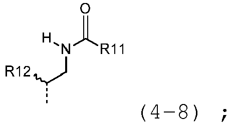
- R5 est 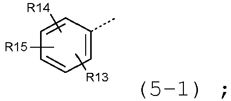
- R7 est 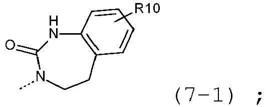
- R8, R9 sont un atome d'hydrogène ;
- R10 est -OR11 ;
- R11, R12 sont identiques ou différents, et choisis parmi -CH₃ ou -CH₂CH₃ ;
- R13, R14, R15 sont identiques ou différents, et choisis parmi un atome d'hydrogène, -OR16, ou un halogène choisi parmi F et Cl ; et
- R16 est un alkyle C1-C3,
ainsi que leurs sels pharmaceutiquement acceptables, et leurs énantiomères.

2. Composé de formule générale (I) selon la revendication 1, dans lequel ledit composé est choisi parmi :
- Composé 158 : N-[(S)-2-(5-(2-chloro-3-fluoro-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-propionamide ;
- Composé 115 : N-[(S)-2-(5-(2-chloro-3-fluoro-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-acétamide ; et
- Composé 206 : N-[(S)-2-(5-(2-chloro-3-méthoxy-phényl)-3-{2-[4-(7-méthoxy-2-oxo-1,2,4,5-tétrahydro-benzo[d][1,3]diazepin-3-yl)-pipéridin-1-yl]-2-oxo-éthyl}-2,6-dioxo-3,6-dihydro-2H-pyrimidin-1-yl)-propyl]-acétamide.

3. Composé de formule générale (I) selon l'une des revendications précédentes, pour son utilisation comme médicament.

4. Composé de formule générale (I) selon l'une des revendications précédentes, pour son utilisation dans la prévention et/ou le traitement de maladies inflammatoires avec une composante neurogène.

5. Composé de formule générale (I) selon la revendication 4, pour son utilisation dans la prévention et/ou le traitement des maladies inflammatoires de la peau avec une composante neurogène choisies parmi la rosacée de type I (Erythémateuse) et de type II (Papulopustuleuse), la dermatite atopique, l'eczema chronique des mains, le psoriasis sous ses différentes formes (vulgaire, du scalp, arthritique, pustuleux, de goutte), l'érythème facial, l'érythème pudique, l'urticaire (aigue, prurit sénile, prurigo nodularis) et l'acné.

6. Composé de formule générale (I) selon la revendication 5, pour son utilisation dans la prévention et/ou le traitement de la rosacée de type I (Erythémateuse), la dermatite atopique, le psoriasis vulgaire et du scalp, et l'uticaire du type prurit sénile et prurigo nodularis.

7. Composé de formule générale (I) selon la revendication 6, pour son utilisation dans la prévention et/ou le traitement de la rosacée de type I (Erythémateuse).

8. Composé de formule générale (I) selon la revendication 4, pour son utilisation dans le traitement de la migraine.

9. Composition pharmaceutique comprenant au moins un composé de formule générale (I) tel que défini dans l'une des revendications 1 ou 2 et un véhicule pharmaceutiquement acceptable, de préférence adapté pour une administration par voie topique.

10. Utilisation cosmétique d'un composé de formule générale (I) selon l'une des revendications 1 ou 2.

## Patentansprüche

1. Heterocyclische Verbindung, ausgewählt aus den Verbindungen der allgemeinen Formel (I): 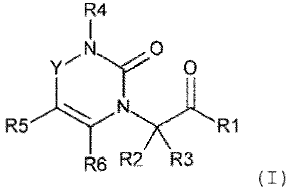 in der
- Y -C(O) ist;
- R1 ist 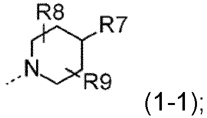
- R2, R3, R6 sind ein Wasserstoffatom;
- R4 ist 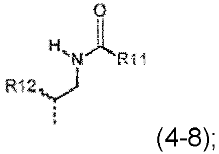
- R5 ist 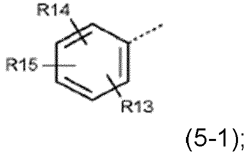
- R7 ist 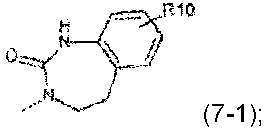
- R8, R9 sind ein Wasserstoffatom;
- R10 ist -OR11;
- R11, R12 sind identisch oder verschieden, und ausgewählt aus -CH₃ oder
- CH₂CH₃;
- R13, R14, R15 sind identisch oder verschieden, und ausgewählt aus einem Wasserstoffatom, -OR16, oder einem Halogen, ausgewählt aus F und Cl; und
- R16 ist ein Alkyl C1-C3,
sowie deren pharmazeutisch akzeptable Salze und deren Enantiomeren.

2. Verbindungen der allgemeinen Formel (I) nach Anspruch 1, wobei besagte Verbindung ausgewählt wird aus:
- Verbindung 158: N-[(S)-2-(5-(2-chloro-3-fluoro-phenyl)-3{2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-propionamid;
- Verbindung 115: N-[(S)-2-(5-(2-chloro-3-fluoro-phenyl)-3-{2-[4-(7-methoxy-2-oxo-1,2,4,5-tetrahydro-benzo[ d][1,3]diazepin-3-yl)-piperidin-1-yl]-2-oxo-ethyl}-2,6-dioxo-3,6-dihydro-2H-pyrimidin-1-yl)-propyl]-acetamid; und
- Verbindung 206: N-[(S)-2-(5-(2-chloro-3-methoxy-phenyl)3-2-[4-(7-methoxy-2-oxo-1,2,4,5-tetrahydro-benzo[d][1,3]diazepin-3-yl)-piperidin-1-yl]-2-oxo-ethyl}-2,6-dioxo-3,6-dihydro-2H-pyrimidin-1-yl)-propyl]-acetamid.

3. Verbindungen der allgemeinen Formel (I) nach einem der vorherigen Ansprüche, zur Verwendung als Medikament.

4. Verbindungen der allgemeinen Formel (I) nach einem der vorherigen Ansprüche, zur Verwendung bei der Vorbeugung und/oder Behandlung von Entzündungskrankheiten mit einer neurogenen Komponente.

5. Verbindungen der allgemeinen Formel (I) nach Anspruch 4, für eine Verwendung in der Vorbeugung und/oder Behandlung von Entzündungskrankheiten der Haut mit neurogener Komponente, ausgewählt aus: Rosacea Typ I (erythematös) und Typ II (papulopustulös), atopische Dermatitis, chronisches Ekzem der Hände, Psoriasis in ihren verschiedenen Formen (vulgaris, Kopfhaut, arthritische, pustulöse, guttata), Gesichtserythem, Röte, Urtikaria (akute, seniler Pruritus, Prurigo nodularis) und Akne.

6. Verbindungen der allgemeinen Formel (I) nach Anspruch 5 zur Verwendung bei der Vorbeugung und/oder Behandlung der Rosacea Typ I (erythematös), atopischer Dermatitis, Psoriasis vulgaris und Kopfhaut, und Urtikaria vom Typ seniler Pruritus und Prurigo nodularis.

7. Verbindungen der allgemeinen Formel (I) nach Anspruch 6 zur Verwendung bei der Vorbeugung und/oder Behandlung der Rosacea Typ I (erythematös).

8. Verbindungen der allgemeinen Formel (I) nach Anspruch 4 zur Verwendung bei der Behandlung von Migräne.

9. Pharmazeutische Zusammensetzung, die mindestens eine Verbindung mit der allgemeinen Formel (I) wie in Anspruch 1 oder 2 und einen pharmazeutisch verträglichen Träger umfasst, vorzugsweise geeignet zur topischen Verabreichung.

10. Kosmetische Verwendung einer Verbindung der allgemeinen Formel (I) nach einem der Ansprüche 1 oder 2.

## Claims

1. Heterocyclic compound selected from the compounds of general formula (I): 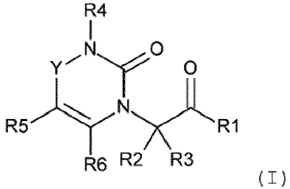 wherein,
- Y is -C(O);
- R1 is 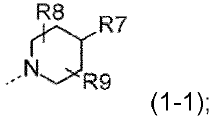
- R2, R3, R6 are a hydrogen atom;
- R4 is 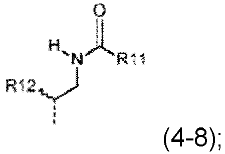
- R5 is 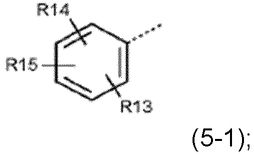
- R7 is 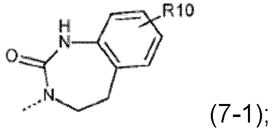
- R8, R9 are a hydrogen atom;
- R10 is -OR11;
- R11, R12 are identical or different, and selected from -CH₃ or -CH₂CH₃;
- R13, R14, R15 are identical or different, and selected from a hydrogen atom, -OR16, or a halogen selected from F and Cl; and
- R16 is a C1-C3 alkyl,
as well as their pharmaceutically acceptable salts, and their enantiomers.

2. Compound of general formula (I) according to claim 1, wherein said compound is selected from:
- Compound 158: N-[(S)-2-(5-(2-chloro-3-fluoro-phenyl)-3-{2-[4-(7-methoxy-2-oxo-1,2,4,5-tetrahydro-benzo[d][1,3]diazepin-3-yl)-piperidin-1-yl]-2-oxoethyl}-2,6-dioxo-3,6-dihydro-2H-pyrimidin-1-yl)-propyl]-propionamide;
- Compound 115: N-[(S)-2-(5-(2-chloro-3-fluoro-phenyl)-3-{2-[4-(7-methoxy-2-oxo-1,2,4,5-tetrahydro-benzo[d][1,3]diazepin-3-yl)-piperidin-1-yl]-2-oxoethyl}-2,6-dioxo-3,6-dihydro-2H-pyrimidin-1-yl)-propyl]-acetamide; and
- Compound 206: N-[(S)-2-(5-(2-chloro-3-methoxy-phenyl)-3-{2-[4-(7-methoxy-2-oxo-1,2,4,5-tetrahydro-benzo[d][1,3]diazepin-3-yl)-piperidin-1-yl]-2-oxoethyl}-2,6-dioxo-3,6-dihydro-2H-pyrimidin-1-yl)-propyl]-acetamide.

3. Compound of general formula (I) according to one of the preceding claims, for use as a medicine.

4. Compound of general formula (I) according to one of the preceding claims, for use in the prevention and/or treatment of inflammatory diseases having a neurogenic component.

5. Compound of general formula (I) according to claim 4, for use in the prevention and/or treatment of inflammatory skin diseases having a neurogenic component selected from type I (Erythematous) and type II (Papulopustular) rosacea, atopic dermatitis, chronic hand eczema, psoriasis in its various forms (vulgar, scalp, arthritic, pustular, guttate), facial erythema, blushing, urticaria (senile pruritus, prurigo nodularis) and acne.

6. Compound of general formula (I) according to claim 5, for use in the prevention and/or treatment of type I (Erythematous) rosacea, atopic dermatitis, vulgar and scalp psoriasis, and urticaria of the senile pruritus and prurigo nodularis types.

7. Compound of general formula (I) according to claim 6, for use in the prevention and/or treatment of type I (Erythematous) rosacea.

8. Compound of general formula (I) according to claim 4, for use in the treatment of migraine.

9. Pharmaceutical composition comprising at least one compound of general formula (I) as defined in one of claims 1 or 2 and a pharmaceutically acceptable carrier, preferably suitable for topical administration.

10. Cosmetic use of a compound of general formula (I) according to one of claims 1 or 2.
